(19) Europäisches Patentamt
European Patent Office
Office européen des brevets

(11) EP 4 285 937 A1

(12) EUROPEAN PATENT APPLICATION
published in accordance with Art. 153(4) EPC

(43) Date of publication:
**06.12.2023 Bulletin 2023/49**

(21) Application number: **22745324.8**

(22) Date of filing: **28.01.2022**

(51) International Patent Classification (IPC):
***A61K 47/68** (2017.01)* ***A61K 47/66** (2017.01)*
***A61K 47/60** (2017.01)* ***A61P 35/00** (2006.01)*

(52) Cooperative Patent Classification (CPC):
**A61K 47/60; A61K 47/66; A61K 47/68; A61P 35/00**

(86) International application number:
**PCT/CN2022/074523**

(87) International publication number:
**WO 2022/161452 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.01.2021 CN 202110116059**
**31.12.2021 CN 202111671761**

(71) Applicant: **Nanjing Chempion Biotechnology Co., Ltd.**
**Nanjing, Jiangsu 210061 (CN)**

(72) Inventors:
- **CHEN, Jian**
**Nanjing, Jiangsu 210061 (CN)**
- **ZHAO, Haibo**
**Nanjing, Jiangsu 210061 (CN)**
- **GU, Rong**
**Nanjing, Jiangsu 210061 (CN)**

(74) Representative: **Cabinet Becker et Associés**
**25, rue Louis le Grand**
**75002 Paris (FR)**

# (54) CONJUGATE AND USE THEREOF

(57) A compound of formula (I), or a tautomer, a stereoisomer or a pharmaceutically acceptable salt thereof. The compound represented by formula (I) or the tautomer, the stereoisomer or the pharmaceutically acceptable salt thereof, as a novel linker compound, can be used for preparing an ADC medicament with a high DAR value. The present invention also relates to an ADC medicament prepared from the linker.

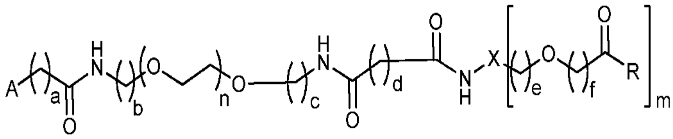

(I)

EP 4 285 937 A1

## Description

### FIELD OF THE INVENTION

The present disclosure relates to the biomedical field, specifically, to conjugates and uses thereof, more specifically, to compounds, conjugates, pharmaceutical compositions and uses thereof for preparing medicaments.

### BACKGROUND OF THE INVENTION

Antibody-Drug Conjugates (ADCs) are a class of biological drugs that link cytotoxic small molecules (cytotoxin) and antibodies by using permanent or unstable chemical linkers. The antibody binds to the specific antigen on the tumor cell membrane and induces endocytosis, allowing the antibody and the cytotoxic small molecule attached to the antibody to enter the cell. Subsequently, after lysosomal degradation, the small molecule drug is released into the cell and induces apoptosis.

The key technical points of ADC drugs include the selection of payload (toxin), linker, antibody, target, and conjugation technology.

The requirements of ADC for linked toxins include: 1. sufficient water solubility and stability in serum, because ADC may circulate in the body for several days; 2. toxins must have a functional group that can be used to conjugate with the linker; 3. toxins must be insensitive to enzymatic degradation reactions by lysosomes; 4. toxins reduce the polymerization effect (lipophilic substances are prone to occur) and change the interaction between ADC and pGp (permeability glycoprotein, drug efflux pump, easy to bind to lipophilic substances), which is the main cause of multitropic drug resistance (MDR) in tumor cells. In addition, for ADCs with cleavable linkers, bystander effects require toxins to kill the target cell and then come out and enter the cell membrane to kill the surrounding cells, requiring the toxins to have a certain lipid-water partition coefficient (LogP) and positive/neutral charge. Currently, the most widely used cytotoxic drugs in clinical practice can be divided into two categories according to their mechanism of action:

DNA damaging agents: calicheamicin (CLM, belonging to enediyne antibacterial drugs) acting on DNA, which causes DNA cleavage and cell death by binding to the minor groove of the DNA double helix.

Tubulin inhibitors: They bind to microtubules to prevent microtubule polymerization, arrest the cell cycle, and then induce tumor cell apoptosis. Tubulin inhibitors mainly include: dolastatin and its derivatives of auristatins (e.g. MMAE, MMAF, and MMAD); maytansine and its derivatives (maytansinoids, such as DM1, DM2, DM3, and DM4); halichondrin B and its derivatives (e.g. eribulin). At present, the vast majority of the ADC projects in clinical research use tubulin inhibitors, and there are products approved for marketing (Adcetris uses MMAE of auristatins, and Kadcyla uses maytansine derivative DM1). Auristatin dominates, accounting for more than 50% of ADC drugs under development. However, these tubulin inhibitors have shown significant ocular toxicity and peripheral neuropathy, which in many cases led to treatment interruption or dose reduction. These results clearly indicate the need for continued investigation of other cytotoxic agents and MTAs as ADC loadings that may provide toxicity and improve the therapeutic index.

Linkers are bridges connecting antibodies and cytotoxic drugs. An ideal conjugate must be stable in vitro or in blood circulation to prevent systemic toxicity due to early release of cytotoxic drugs, and at the same time be able to quickly release effective cytotoxic drugs to kill cancer cells after entering cancer cells. An ideal linker plays a key role in the success or failure of a drug, and the properties of a linker determine the pharmacokinetic properties and therapeutic effect of a drug. An ideal linker is one that does not release cytotoxins until the ADC reaches the target, and releases cytotoxins only after the ADC reaches the cell. The drug release mode of a linker is divided into cleavable and non-cleavable modes. The non-cleavable mode is a form in which a linker is still connected to a cytotoxin after ADC has been digested by lysosome. There are three types of cleavable modes: the first is an acid-sensitive linker, which triggers the hydrolysis of acid-dependent groups in the linker at low pH, such as hydrazone groups; the second is a glutathione-sensitive linker, wherein a linker containing a disulfide bond is reduced and broken by glutathione after reaching the cell because the concentration of glutathione in the cell is higher than that in the plasma; and the last one is a lysosomal protease-sensitive linker, wherein some proteases in lysosomes can recognize and cleave specific peptides in linkers to release drugs.

Traditional cysteine-based site-specific antibody-drug conjugates (ADCs) are limited to one drug per cysteine. However, some applications require a high drug-to-antibody ratio (DAR), for example, when using a payload with a low potency. Higher drug loading can be achieved using classical cysteine conjugation methods, but these methods lead to heterogeneity, as well as suboptimal efficacy and pharmacokinetics.

Due to technical limitations, traditional ADC drugs with a high DAR value often lead to problems of pharmacodynamic instability, increased drug metabolism rate, reduced half-life, and increased systemic toxicity. Therefore, ADC drug technology still needs to be further developed and improved.

## SUMMARY OF THE INVENTION

The present disclosure aims to solve, at least to a certain extent, one of the technical problems in the related art, such as providing ADC drugs with a high DAR value that solve the problems of pharmacokinetic instability, increased drug metabolism rate, reduced half-life, and increased systemic toxicity.

In the first aspect of the present disclosure, the present disclosure relates to a compound of formula (I), or a tautomer, a stereoisomer or a pharmaceutically acceptable salt thereof,

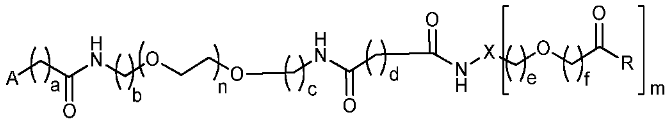
(I)

wherein

a, b, c, d, e, and f are each independently 0, 1, 2, 3, 4, 5, 6, 7, 8, 9 or 10; alternatively 0, 1, 2, 3, 4 or 5; alternatively 0, 1, 2 or 3; alternatively 1 or 2;
n is 1, 2, 3, 4, 5, 6, 7, 8, 9, 10, 11, 12, 13, 14, 15, 16, 17, 18, 19 or 20; alternatively 1, 2, 3, 4, 5, 6, 7, 8, 9 or 10; alternatively 1, 2, 3, 4 or 5; alternatively 1, 2 or 3; alternatively 1;
m is 2 or 3;
Xis C, Nor Si;
A is $-NH_2$, -NH-PG1,

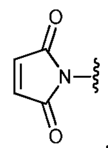
,
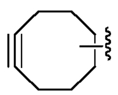
or
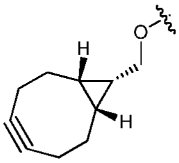
;

R is -OH, -O-PG2,

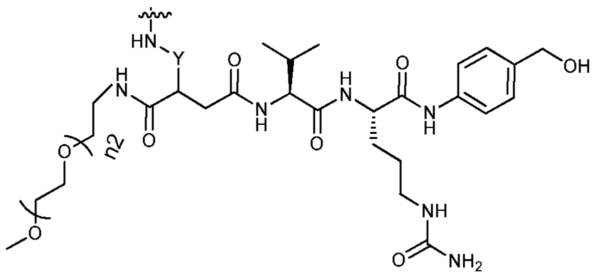
,

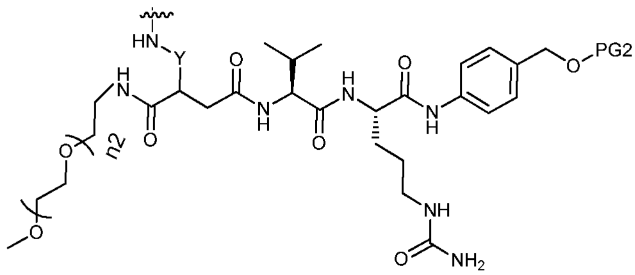
,

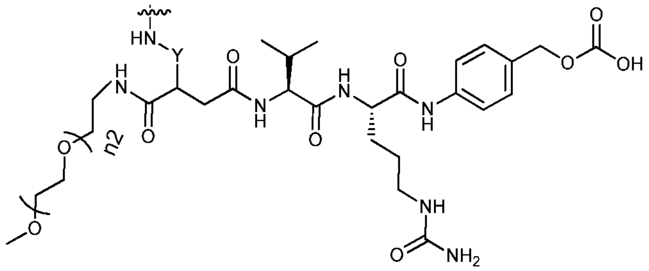

or

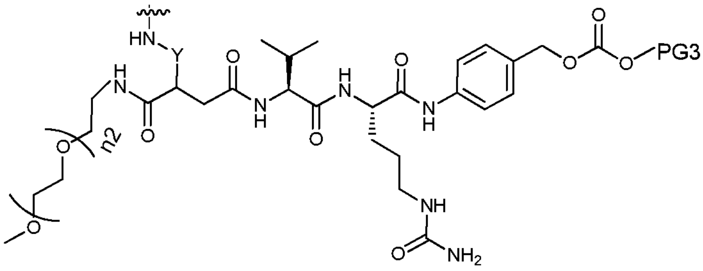

;

wherein PG1, PG2 and PG3 are protecting groups;
n2 is 5, 6, 7, 8, 9, 10, 11, 12, 13, 14, 15, 16, 17, 18, 19 or 20; alternatively 5, 6, 7, 8, 9, 10, 11, 12, 13, 14 or 15; alternatively 7, 8, 9, 10 or 11;
Y is a bond or

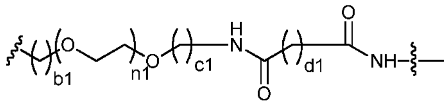

;

wherein n1 is 1, 2, 3, 4, 5, 6, 7, 8, 9, 10, 11, 12, 13, 14, 15, 16, 17, 18, 19 or 20; alternatively 1, 2, 3, 4, 5, 6, 7, 8, 9 or 10; alternatively 1, 2, 3, 4 or 5; alternatively 1, 2 or 3;
b1, c1, and d1 are each independently 0, 1, 2, 3, 4, 5, 6, 7, 8, 9 or 10; alternatively 0, 1, 2, 3, 4 or 5; alternatively 0, 1, 2 or 3; alternatively 1 or 2;
when X is C or Si, m is 3;
when X is N, m is 2.

According to embodiments of the present disclosure, the compound represented by formula (I) or a tautomer, a stereoisomer or a pharmaceutically acceptable salt thereof as a novel linker compound can be used for the manufacture of ADC drugs with high DAR value in a site-specific manner with each cysteine as a conjugation site, thereby reducing the exposure of small molecule drugs in vivo, improving the safety of small molecule drugs, and benefiting drug quality control. Moreover, the prepared ADC drug with high DAR value is more cytotoxic to target cells. According to embodiments of the present disclosure, the compound represented by formula (I) as a linker introduces various types of water-soluble groups, which effectively avoid the phenomenon that ADC is extremely prone to polymerization and precipitation in plasma due to poor water solubility, so that the toxin comes out after killing the target cells and enters the cell membrane to kill peripheral cells, thus showing significant advantages in terms of drug efficacy and side effects. The ADC drug prepared with the compound represented by formula (I) according to embodiments of the present disclosure as a linker solves the problems of pharmacokinetic instability, increased drug metabolism rate, reduced half-life and increased systemic toxicity caused by high DAR value.

According to embodiments of the present disclosure, the above compounds may further comprise at least one of the following additional technical features:

According to embodiments of the present disclosure, provided herein is the structure represented by formula (II), or a tautomer, a stereoisomer or a pharmaceutically acceptable salt thereof,

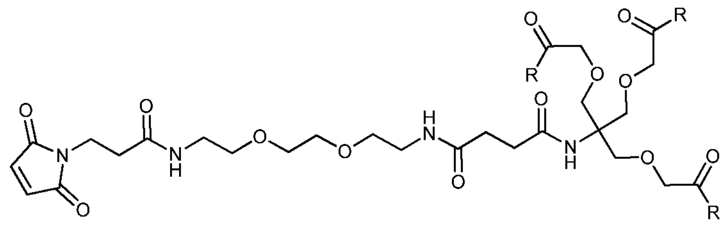 (II).

According to embodiments of the present disclosure, provided herein is the structure represented by formula (III), or a tautomer, a stereoisomer or a pharmaceutically acceptable salt thereof,

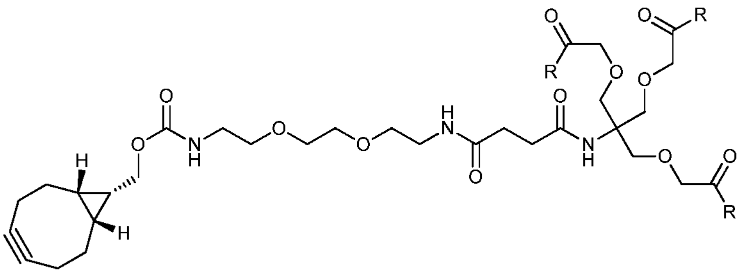

(III).

According to embodiments of the present disclosure, R is -OH, -O-PG2,

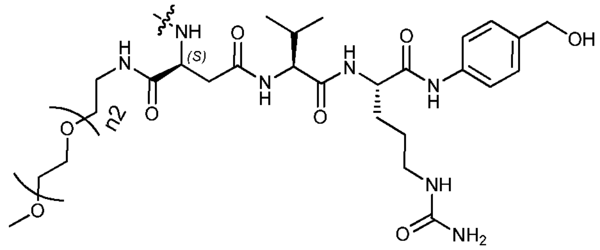

,

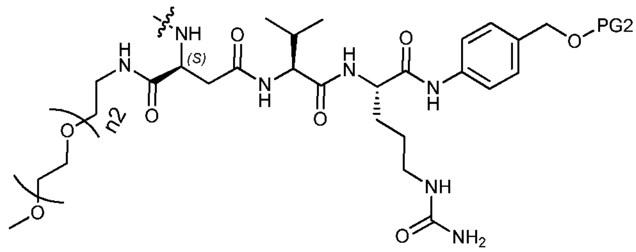

,

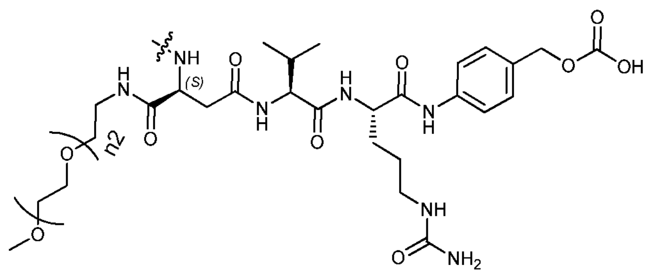

,

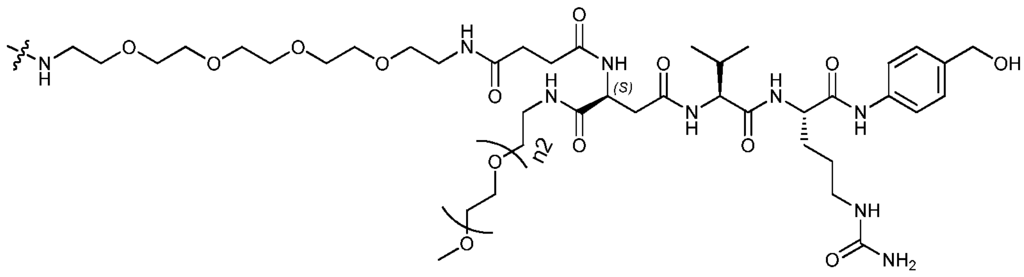

,

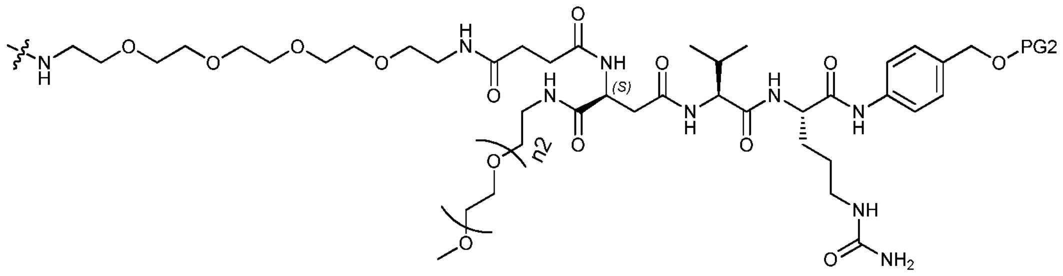

,

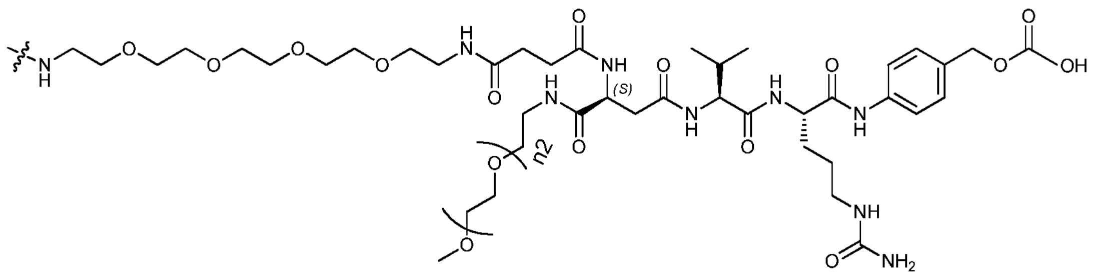

,

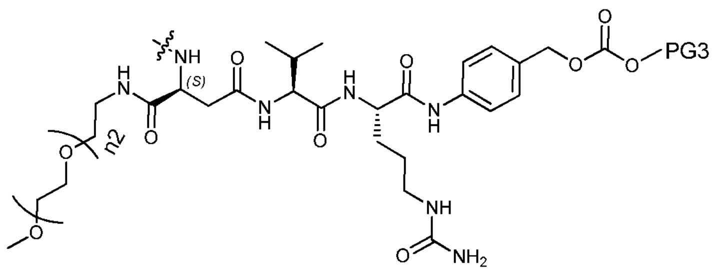

,

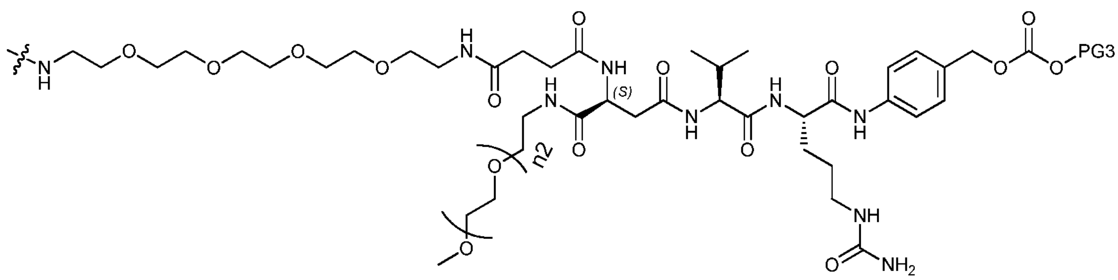

,

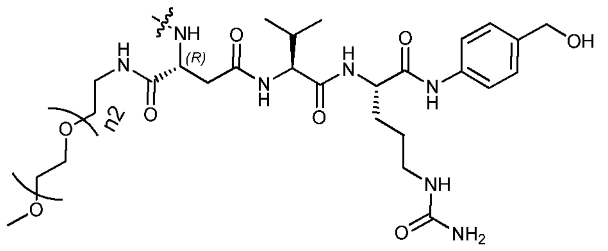

,

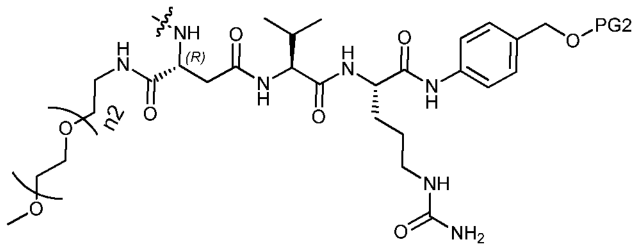

,

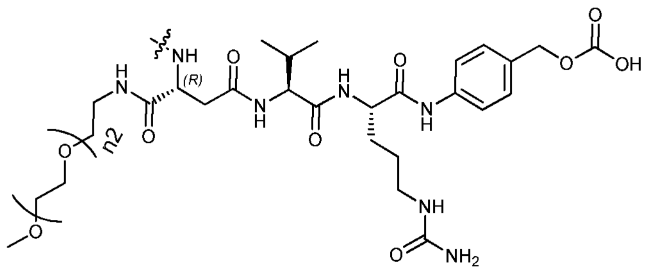

,

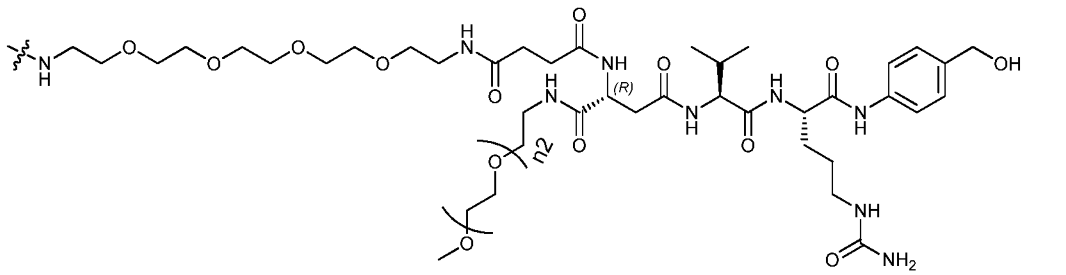

,

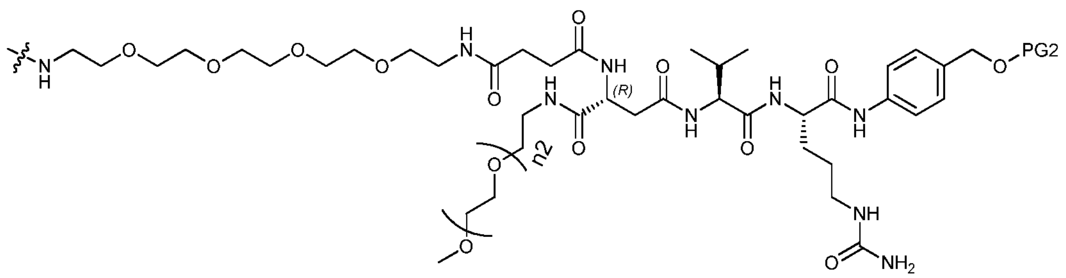

,

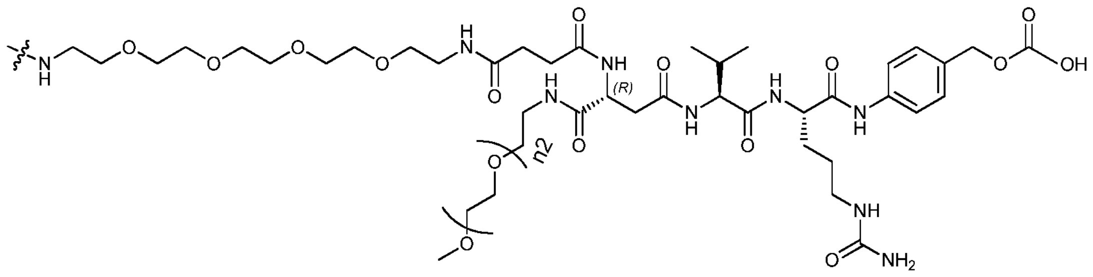

,

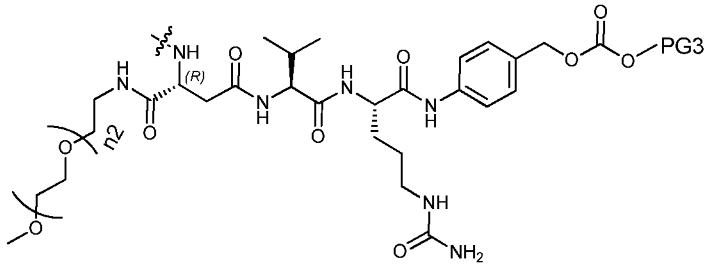

,

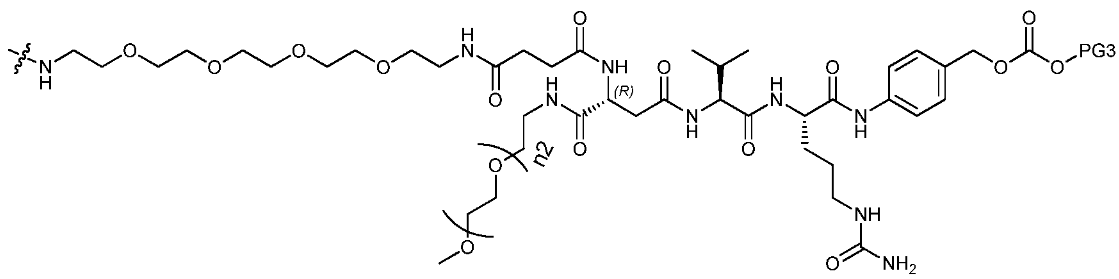

.

According to embodiments of the present disclosure, n2 is 7 or 11.

According to embodiments of the present disclosure, the PG1 is an amino-protecting group; optionally, the amino-protecting group is selected from acetyl, trifluoroacetyl, tert-butoxycarbonyl (BOC, Boc), benzyloxycarbonyl (CBZ, Cbz) and 9-fluorenylmethyleneoxycarbonyl (Fmoc); the PG2 is a hydroxyl-protecting group; optionally, the hydroxyl-protecting group is selected from acetyl and silyl; the PG3 is a carboxyl-protecting group; optionally, the carboxyl-protecting group is selected from $-CH_2CH_2SO_2Ph$, cyanoethyl, 2-(trimethylsilyl)ethyl, 2-(trimethylsilyl)ethoxymethyl, 2-(p-toluenesulfonyl)ethyl, 2-(p-nitrobenzenesulfonyl)ethyl, 2-(diphenylphosphino)ethyl and nitroethyl.

In the second aspect of the present disclosure, provided herein is a conjugate. According to embodiments of the present disclosure, the conjugate comprises the above compound and a drug moiety to which the compound is covalently bound via an R group. According to a specific embodiment of the present disclosure, the drug moiety is a small molecule drug or is called payload.

According to embodiments of the present disclosure, the conjugate of the second aspect further comprises a targeting moiety to which one or more of the conjugates are covalently bound via an A group. In turn, the conjugate in the second aspect can move to the target site in a directed manner under the mediation of the targeting movement of the targeting moiety.

In the third aspect of the present disclosure, provided herein is a conjugate. According to embodiments of the present disclosure, the conjugate comprises a targeting moiety and one or more of the above compounds, wherein the one or more compounds are covalently bound to the targeting moiety via an A group. In turn, the conjugate according to the third aspect can move to the target site in a directed manner under the mediation of the targeting movement of the targeting moiety.

According to embodiments of the present disclosure, the drug is selected from at least one of eribulin, methyl auristatin E, and SN-38, which have the following structures:

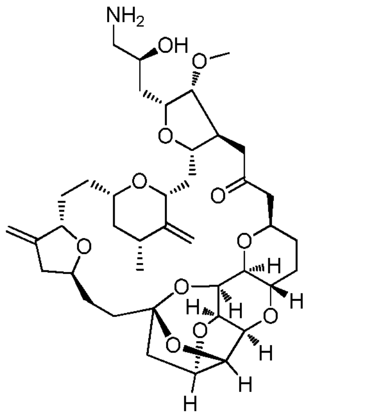

,

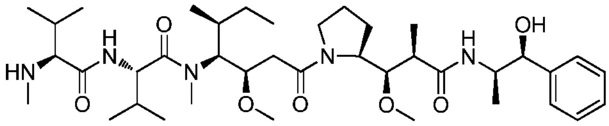

and

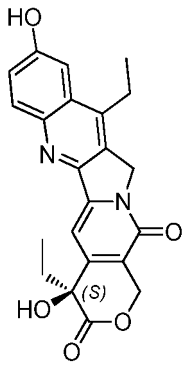

.

According to embodiments of the present disclosure, the conjugate of the second aspect is selected from the following specific compounds or tautomers, stereoisomers or pharmaceutically acceptable salts thereof, wherein the compounds are represented by the structure shown in formula (IV):

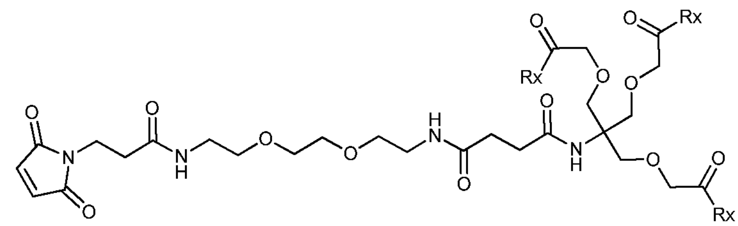

(IV),

wherein Rx is

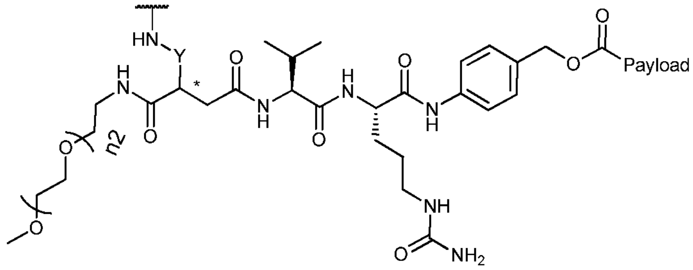

,

and n2, *, and payload are defined in the following table:

| n2 | Chirality of carbon atom at * | Y | Payload |
|---|---|---|---|
| 5 | S | bond | 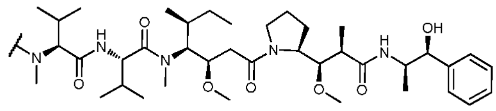 (MMAE) |
| 5 | R | bond | MMAE |
| 5 | S | 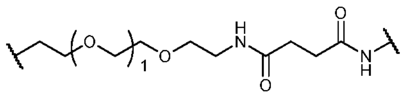 (L1) | MMAE |
| 5 | R | L1 | MMAE |
| 5 | S | 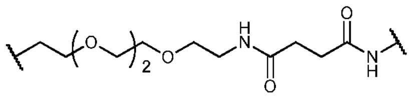 | MMAE |
| | | (L2) | |
| 5 | R | L2 | MMAE |
| 5 | S | 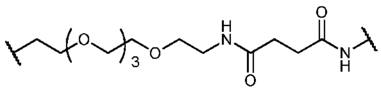 (L3) | MMAE |
| 5 | R | L3 | MMAE |
| 5 | S | 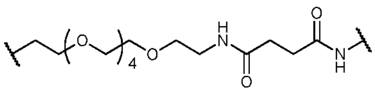 (L4) | MMAE |
| 5 | R | L4 | MMAE |
| 5 | S | 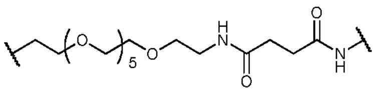 (L5) | MMAE |
| 5 | R | L5 | MMAE |
| 5 | S | 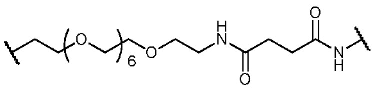 (L6) | MMAE |
| 5 | R | L6 | MMAE |
| 5 | S | 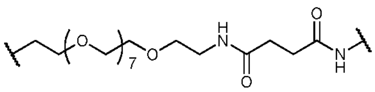 (L7) | MMAE |
| 5 | R | L7 | MMAE |

(continued)

| n2 | Chirality of carbon atom at * | Y | Payload |
|---|---|---|---|
| 5 | S | 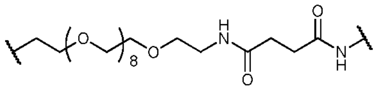 (L8) | MMAE |
| 5 | R | L8 | MMAE |
| 5 | S | 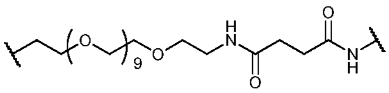 (L9) | MMAE |
| 5 | R | L9 | MMAE |
| 5 | S | 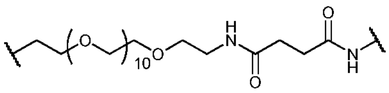 (L10) | MMAE |
| 5 | R | L10 | MMAE |
| 5 | S | 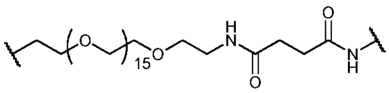 (L15) | MMAE |
| 5 | R | L15 | MMAE |
| 5 | S | 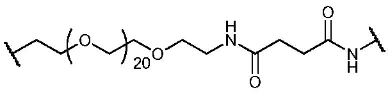 (L20) | MMAE |
| 5 | R | L20 | MMAE |
| 5 | S | bond | 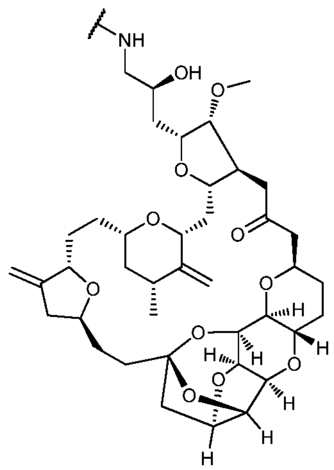 (Eribulin) |
| 5 | R | bond | Eribulin |
| 5 | S | L1 | Eribulin |
| 5 | R | L1 | Eribulin |

(continued)

| n2 | Chirality of carbon atom at * | Y | Payload |
|---|---|---|---|
| 5 | S | L2 | Eribulin |
| 5 | R | L2 | Eribulin |
| 5 | S | L3 | Eribulin |
| 5 | R | L3 | Eribulin |
| 5 | S | L4 | Eribulin |
| 5 | R | L4 | Eribulin |
| 5 | S | L5 | Eribulin |
| 5 | R | L5 | Eribulin |
| 5 | S | L6 | Eribulin |
| 5 | R | L6 | Eribulin |
| 5 | S | L7 | Eribulin |
| 5 | R | L7 | Eribulin |
| 5 | S | L8 | Eribulin |
| 5 | R | L8 | Eribulin |
| 5 | S | L9 | Eribulin |
| 5 | R | L9 | Eribulin |
| 5 | S | L10 | Eribulin |
| 5 | R | L10 | Eribulin |
| 5 | S | L15 | Eribulin |
| 5 | R | L15 | Eribulin |
| 5 | S | L20 | Eribulin |
| 5 | R | L20 | Eribulin |
| 5 | S | bond | 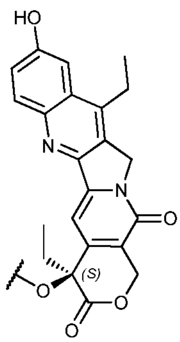 (SN-38) |
| 5 | R | bond | SN-38 |
| 5 | S | L1 | SN-38 |
| 5 | R | L1 | SN-38 |
| 5 | S | L2 | SN-38 |
| 5 | R | L2 | SN-38 |
| 5 | S | L3 | SN-38 |

(continued)

| n2 | Chirality of carbon atom at * | Y | Payload |
|---|---|---|---|
| 5 | R | L3 | SN-38 |
| 5 | S | L4 | SN-38 |
| 5 | R | L4 | SN-38 |
| 5 | S | L5 | SN-38 |
| 5 | R | L5 | SN-38 |
| 5 | S | L6 | SN-38 |
| 5 | R | L6 | SN-38 |
| 5 | S | L7 | SN-38 |
| 5 | R | L7 | SN-38 |
| 5 | S | L8 | SN-38 |
| 5 | R | L8 | SN-38 |
| 5 | S | L9 | SN-38 |
| 5 | R | L9 | SN-38 |
| 5 | S | L10 | SN-38 |
| 5 | R | L10 | SN-38 |
| 5 | S | L15 | SN-38 |
| 5 | R | L15 | SN-38 |
| 5 | S | L20 | SN-38 |
| 5 | R | L20 | SN-38 |
| 6 | S | bond | MMAE |
| 6 | R | bond | MMAE |
| 6 | S | L1 | MMAE |
| 6 | R | L1 | MMAE |
| 6 | S | L2 | MMAE |
| 6 | R | L2 | MMAE |
| 6 | S | L3 | MMAE |
| 6 | R | L3 | MMAE |
| 6 | S | L4 | MMAE |
| 6 | R | L4 | MMAE |
| 6 | S | L5 | MMAE |
| 6 | R | L5 | MMAE |
| 6 | S | L6 | MMAE |
| 6 | R | L6 | MMAE |
| 6 | S | L7 | MMAE |
| 6 | R | L7 | MMAE |
| 6 | S | L8 | MMAE |

(continued)

| n2 | Chirality of carbon atom at * | Y | Payload |
|---|---|---|---|
| 6 | R | L8 | MMAE |
| 6 | S | L9 | MMAE |
| 6 | R | L9 | MMAE |
| 6 | S | L10 | MMAE |
| 6 | R | L10 | MMAE |
| 6 | S | L15 | MMAE |
| 6 | R | L15 | MMAE |
| 6 | S | L20 | MMAE |
| 6 | R | L20 | MMAE |
| 6 | S | bond | Eribulin |
| 6 | R | bond | Eribulin |
| 6 | S | L1 | Eribulin |
| 6 | R | L1 | Eribulin |
| 6 | S | L2 | Eribulin |
| 6 | R | L2 | Eribulin |
| 6 | S | L3 | Eribulin |
| 6 | R | L3 | Eribulin |
| 6 | S | L4 | Eribulin |
| 6 | R | L4 | Eribulin |
| 6 | S | L5 | Eribulin |
| 6 | R | L5 | Eribulin |
| 6 | S | L6 | Eribulin |
| 6 | R | L6 | Eribulin |
| 6 | S | L7 | Eribulin |
| 6 | R | L7 | Eribulin |
| 6 | S | L8 | Eribulin |
| 6 | R | L8 | Eribulin |
| 6 | S | L9 | Eribulin |
| 6 | R | L9 | Eribulin |
| 6 | S | L10 | Eribulin |
| 6 | R | L10 | Eribulin |
| 6 | S | L15 | Eribulin |
| 6 | R | L15 | Eribulin |
| 6 | S | L20 | Eribulin |
| 6 | R | L20 | Eribulin |
| 6 | S | bond | SN-38 |

(continued)

| n2 | Chirality of carbon atom at * | Y | Payload |
|---|---|---|---|
| 6 | R | bond | SN-38 |
| 6 | S | L1 | SN-38 |
| 6 | R | L1 | SN-38 |
| 6 | S | L2 | SN-38 |
| 6 | R | L2 | SN-38 |
| 6 | S | L3 | SN-38 |
| 6 | R | L3 | SN-38 |
| 6 | S | L4 | SN-38 |
| 6 | R | L4 | SN-38 |
| 6 | S | L5 | SN-38 |
| 6 | R | L5 | SN-38 |
| 6 | S | L6 | SN-38 |
| 6 | R | L6 | SN-38 |
| 6 | S | L7 | SN-38 |
| 6 | R | L7 | SN-38 |
| 6 | S | L8 | SN-38 |
| 6 | R | L8 | SN-38 |
| 6 | S | L9 | SN-38 |
| 6 | R | L9 | SN-38 |
| 6 | S | L10 | SN-38 |
| 6 | R | L10 | SN-38 |
| 6 | S | L15 | SN-38 |
| 6 | R | L15 | SN-38 |
| 6 | S | L20 | SN-38 |
| 6 | R | L20 | SN-38 |
| 7 | S | bond | MMAE |
| 7 | R | bond | MMAE |
| 7 | S | L1 | MMAE |
| 7 | R | L1 | MMAE |
| 7 | S | L2 | MMAE |
| 7 | R | L2 | MMAE |
| 7 | S | L3 | MMAE |
| 7 | R | L3 | MMAE |
| 7 | S | L4 | MMAE |
| 7 | R | L4 | MMAE |
| 7 | S | L5 | MMAE |

(continued)

| n2 | Chirality of carbon atom at * | Y | Payload |
|---|---|---|---|
| 7 | R | L5 | MMAE |
| 7 | S | L6 | MMAE |
| 7 | R | L6 | MMAE |
| 7 | S | L7 | MMAE |
| 7 | R | L7 | MMAE |
| 7 | S | L8 | MMAE |
| 7 | R | L8 | MMAE |
| 7 | S | L9 | MMAE |
| 7 | R | L9 | MMAE |
| 7 | S | L10 | MMAE |
| 7 | R | L10 | MMAE |
| 7 | S | L15 | MMAE |
| 7 | R | L15 | MMAE |
| 7 | S | L20 | MMAE |
| 7 | R | L20 | MMAE |
| 7 | S | bond | Eribulin |
| 7 | R | bond | Eribulin |
| 7 | S | L1 | Eribulin |
| 7 | R | L1 | Eribulin |
| 7 | S | L2 | Eribulin |
| 7 | R | L2 | Eribulin |
| 7 | S | L3 | Eribulin |
| 7 | R | L3 | Eribulin |
| 7 | S | L4 | Eribulin |
| 7 | R | L4 | Eribulin |
| 7 | S | L5 | Eribulin |
| 7 | R | L5 | Eribulin |
| 7 | S | L6 | Eribulin |
| 7 | R | L6 | Eribulin |
| 7 | S | L7 | Eribulin |
| 7 | R | L7 | Eribulin |
| 7 | S | L8 | Eribulin |
| 7 | R | L8 | Eribulin |
| 7 | S | L9 | Eribulin |
| 7 | R | L9 | Eribulin |
| 7 | S | L10 | Eribulin |

(continued)

| n2 | Chirality of carbon atom at * | Y | Payload |
|---|---|---|---|
| 7 | R | L10 | Eribulin |
| 7 | S | L15 | Eribulin |
| 7 | R | L15 | Eribulin |
| 7 | S | L20 | Eribulin |
| 7 | R | L20 | Eribulin |
| 7 | S | bond | SN-38 |
| 7 | R | bond | SN-38 |
| 7 | S | L1 | SN-38 |
| 7 | R | L1 | SN-38 |
| 7 | S | L2 | SN-38 |
| 7 | R | L2 | SN-38 |
| 7 | S | L3 | SN-38 |
| 7 | R | L3 | SN-38 |
| 7 | S | L4 | SN-38 |
| 7 | R | L4 | SN-38 |
| 7 | S | L5 | SN-38 |
| 7 | R | L5 | SN-38 |
| 7 | S | L6 | SN-38 |
| 7 | R | L6 | SN-38 |
| 7 | S | L7 | SN-38 |
| 7 | R | L7 | SN-38 |
| 7 | S | L8 | SN-38 |
| 7 | R | L8 | SN-38 |
| 7 | S | L9 | SN-38 |
| 7 | R | L9 | SN-38 |
| 7 | S | L10 | SN-38 |
| 7 | R | L10 | SN-38 |
| 7 | S | L15 | SN-38 |
| 7 | R | L15 | SN-38 |
| 7 | S | L20 | SN-38 |
| 7 | R | L20 | SN-38 |
| 8 | S | bond | MMAE |
| 8 | R | bond | MMAE |
| 8 | S | L1 | MMAE |
| 8 | R | L1 | MMAE |
| 8 | S | L2 | MMAE |

(continued)

| n2 | Chirality of carbon atom at * | Y | Payload |
|---|---|---|---|
| 8 | R | L2 | MMAE |
| 8 | S | L3 | MMAE |
| 8 | R | L3 | MMAE |
| 8 | S | L4 | MMAE |
| 8 | R | L4 | MMAE |
| 8 | S | L5 | MMAE |
| 8 | R | L5 | MMAE |
| 8 | S | L6 | MMAE |
| 8 | R | L6 | MMAE |
| 8 | S | L7 | MMAE |
| 8 | R | L7 | MMAE |
| 8 | S | L8 | MMAE |
| 8 | R | L8 | MMAE |
| 8 | S | L9 | MMAE |
| 8 | R | L9 | MMAE |
| 8 | S | L10 | MMAE |
| 8 | R | L10 | MMAE |
| 8 | S | L15 | MMAE |
| 8 | R | L15 | MMAE |
| 8 | S | L20 | MMAE |
| 8 | R | L20 | MMAE |
| 8 | S | bond | Eribulin |
| 8 | R | bond | Eribulin |
| 8 | S | L1 | Eribulin |
| 8 | R | L1 | Eribulin |
| 8 | S | L2 | Eribulin |
| 8 | R | L2 | Eribulin |
| 8 | S | L3 | Eribulin |
| 8 | R | L3 | Eribulin |
| 8 | S | L4 | Eribulin |
| 8 | R | L4 | Eribulin |
| 8 | S | L5 | Eribulin |
| 8 | R | L5 | Eribulin |
| 8 | S | L6 | Eribulin |
| 8 | R | L6 | Eribulin |
| 8 | S | L7 | Eribulin |

(continued)

| n2 | Chirality of carbon atom at * | Y | Payload |
|---|---|---|---|
| 8 | R | L7 | Eribulin |
| 8 | S | L8 | Eribulin |
| 8 | R | L8 | Eribulin |
| 8 | S | L9 | Eribulin |
| 8 | R | L9 | Eribulin |
| 8 | S | L10 | Eribulin |
| 8 | R | L10 | Eribulin |
| 8 | S | L15 | Eribulin |
| 8 | R | L15 | Eribulin |
| 8 | S | L20 | Eribulin |
| 8 | R | L20 | Eribulin |
| 8 | S | bond | SN-38 |
| 8 | R | bond | SN-38 |
| 8 | S | L1 | SN-38 |
| 8 | R | L1 | SN-38 |
| 8 | S | L2 | SN-38 |
| 8 | R | L2 | SN-38 |
| 8 | S | L3 | SN-38 |
| 8 | R | L3 | SN-38 |
| 8 | S | L4 | SN-38 |
| 8 | R | L4 | SN-38 |
| 8 | S | L5 | SN-38 |
| 8 | R | L5 | SN-38 |
| 8 | S | L6 | SN-38 |
| 8 | R | L6 | SN-38 |
| 8 | S | L7 | SN-38 |
| 8 | R | L7 | SN-38 |
| 8 | S | L8 | SN-38 |
| 8 | R | L8 | SN-38 |
| 8 | S | L9 | SN-38 |
| 8 | R | L9 | SN-38 |
| 8 | S | L10 | SN-38 |
| 8 | R | L10 | SN-38 |
| 8 | S | L15 | SN-38 |
| 8 | R | L15 | SN-38 |
| 8 | S | L20 | SN-38 |

(continued)

| n2 | Chirality of carbon atom at * | Y | Payload |
|---|---|---|---|
| 8 | R | L20 | SN-38 |
| 9 | S | bond | MMAE |
| 9 | R | bond | MMAE |
| 9 | S | L1 | MMAE |
| 9 | R | L1 | MMAE |
| 9 | S | L2 | MMAE |
| 9 | R | L2 | MMAE |
| 9 | S | L3 | MMAE |
| 9 | R | L3 | MMAE |
| 9 | S | L4 | MMAE |
| 9 | R | L4 | MMAE |
| 9 | S | L5 | MMAE |
| 9 | R | L5 | MMAE |
| 9 | S | L6 | MMAE |
| 9 | R | L6 | MMAE |
| 9 | S | L7 | MMAE |
| 9 | R | L7 | MMAE |
| 9 | S | L8 | MMAE |
| 9 | R | L8 | MMAE |
| 9 | S | L9 | MMAE |
| 9 | R | L9 | MMAE |
| 9 | S | L10 | MMAE |
| 9 | R | L10 | MMAE |
| 9 | S | L15 | MMAE |
| 9 | R | L15 | MMAE |
| 9 | S | L20 | MMAE |
| 9 | R | L20 | MMAE |
| 9 | S | bond | Eribulin |
| 9 | R | bond | Eribulin |
| 9 | S | L1 | Eribulin |
| 9 | R | L1 | Eribulin |
| 9 | S | L2 | Eribulin |
| 9 | R | L2 | Eribulin |
| 9 | S | L3 | Eribulin |
| 9 | R | L3 | Eribulin |
| 9 | S | L4 | Eribulin |

(continued)

| n2 | Chirality of carbon atom at * | Y | Payload |
|---|---|---|---|
| 9 | R | L4 | Eribulin |
| 9 | S | L5 | Eribulin |
| 9 | R | L5 | Eribulin |
| 9 | S | L6 | Eribulin |
| 9 | R | L6 | Eribulin |
| 9 | S | L7 | Eribulin |
| 9 | R | L7 | Eribulin |
| 9 | S | L8 | Eribulin |
| 9 | R | L8 | Eribulin |
| 9 | S | L9 | Eribulin |
| 9 | R | L9 | Eribulin |
| 9 | S | L10 | Eribulin |
| 9 | R | L10 | Eribulin |
| 9 | S | L15 | Eribulin |
| 9 | R | L15 | Eribulin |
| 9 | S | L20 | Eribulin |
| 9 | R | L20 | Eribulin |
| 9 | S | bond | SN-38 |
| 9 | R | bond | SN-38 |
| 9 | S | L1 | SN-38 |
| 9 | R | L1 | SN-38 |
| 9 | S | L2 | SN-38 |
| 9 | R | L2 | SN-38 |
| 9 | S | L3 | SN-38 |
| 9 | R | L3 | SN-38 |
| 9 | S | L4 | SN-38 |
| 9 | R | L4 | SN-38 |
| 9 | S | L5 | SN-38 |
| 9 | R | L5 | SN-38 |
| 9 | S | L6 | SN-38 |
| 9 | R | L6 | SN-38 |
| 9 | S | L7 | SN-38 |
| 9 | R | L7 | SN-38 |
| 9 | S | L8 | SN-38 |
| 9 | R | L8 | SN-38 |
| 9 | S | L9 | SN-38 |

(continued)

| n2 | Chirality of carbon atom at * | Y | Payload |
|---|---|---|---|
| 9 | R | L9 | SN-38 |
| 9 | S | L10 | SN-38 |
| 9 | R | L10 | SN-38 |
| 9 | S | L15 | SN-38 |
| 9 | R | L15 | SN-38 |
| 9 | S | L20 | SN-38 |
| 9 | R | L20 | SN-38 |
| 10 | S | bond | MMAE |
| 10 | R | bond | MMAE |
| 10 | S | L1 | MMAE |
| 10 | R | L1 | MMAE |
| 10 | S | L2 | MMAE |
| 10 | R | L2 | MMAE |
| 10 | S | L3 | MMAE |
| 10 | R | L3 | MMAE |
| 10 | S | L4 | MMAE |
| 10 | R | L4 | MMAE |
| 10 | S | L5 | MMAE |
| 10 | R | L5 | MMAE |
| 10 | S | L6 | MMAE |
| 10 | R | L6 | MMAE |
| 10 | S | L7 | MMAE |
| 10 | R | L7 | MMAE |
| 10 | S | L8 | MMAE |
| 10 | R | L8 | MMAE |
| 10 | S | L9 | MMAE |
| 10 | R | L9 | MMAE |
| 10 | S | L10 | MMAE |
| 10 | R | L10 | MMAE |
| 10 | S | L15 | MMAE |
| 10 | R | L15 | MMAE |
| 10 | S | L20 | MMAE |
| 10 | R | L20 | MMAE |
| 10 | S | bond | Eribulin |
| 10 | R | bond | Eribulin |
| 10 | S | L1 | Eribulin |

(continued)

| n2 | Chirality of carbon atom at * | Y | Payload |
|---|---|---|---|
| 10 | R | L1 | Eribulin |
| 10 | S | L2 | Eribulin |
| 10 | R | L2 | Eribulin |
| 10 | S | L3 | Eribulin |
| 10 | R | L3 | Eribulin |
| 10 | S | L4 | Eribulin |
| 10 | R | L4 | Eribulin |
| 10 | S | L5 | Eribulin |
| 10 | R | L5 | Eribulin |
| 10 | S | L6 | Eribulin |
| 10 | R | L6 | Eribulin |
| 10 | S | L7 | Eribulin |
| 10 | R | L7 | Eribulin |
| 10 | S | L8 | Eribulin |
| 10 | R | L8 | Eribulin |
| 10 | S | L9 | Eribulin |
| 10 | R | L9 | Eribulin |
| 10 | S | L10 | Eribulin |
| 10 | R | L10 | Eribulin |
| 10 | S | L15 | Eribulin |
| 10 | R | L15 | Eribulin |
| 10 | S | L20 | Eribulin |
| 10 | R | L20 | Eribulin |
| 10 | S | bond | SN-38 |
| 10 | R | bond | SN-38 |
| 10 | S | L1 | SN-38 |
| 10 | R | L1 | SN-38 |
| 10 | S | L2 | SN-38 |
| 10 | R | L2 | SN-38 |
| 10 | S | L3 | SN-38 |
| 10 | R | L3 | SN-38 |
| 10 | S | L4 | SN-38 |
| 10 | R | L4 | SN-38 |
| 10 | S | L5 | SN-38 |
| 10 | R | L5 | SN-38 |
| 10 | S | L6 | SN-38 |

(continued)

| n2 | Chirality of carbon atom at * | Y | Payload |
|---|---|---|---|
| 10 | R | L6 | SN-38 |
| 10 | S | L7 | SN-38 |
| 10 | R | L7 | SN-38 |
| 10 | S | L8 | SN-38 |
| 10 | R | L8 | SN-38 |
| 10 | S | L9 | SN-38 |
| 10 | R | L9 | SN-38 |
| 10 | S | L10 | SN-38 |
| 10 | R | L10 | SN-38 |
| 10 | S | L15 | SN-38 |
| 10 | R | L15 | SN-38 |
| 10 | S | L20 | SN-38 |
| 10 | R | L20 | SN-38 |
| 11 | S | bond | MMAE |
| 11 | R | bond | MMAE |
| 11 | S | L1 | MMAE |
| 11 | R | L1 | MMAE |
| 11 | S | L2 | MMAE |
| 11 | R | L2 | MMAE |
| 11 | S | L3 | MMAE |
| 11 | R | L3 | MMAE |
| 11 | S | L4 | MMAE |
| 11 | R | L4 | MMAE |
| 11 | S | L5 | MMAE |
| 11 | R | L5 | MMAE |
| 11 | S | L6 | MMAE |
| 11 | R | L6 | MMAE |
| 11 | S | L7 | MMAE |
| 11 | R | L7 | MMAE |
| 11 | S | L8 | MMAE |
| 11 | R | L8 | MMAE |
| 11 | S | L9 | MMAE |
| 11 | R | L9 | MMAE |
| 11 | S | L10 | MMAE |
| 11 | R | L10 | MMAE |
| 11 | S | L15 | MMAE |

(continued)

| n2 | Chirality of carbon atom at * | Y | Payload |
|---|---|---|---|
| 11 | R | L15 | MMAE |
| 11 | S | L20 | MMAE |
| 11 | R | L20 | MMAE |
| 11 | S | bond | Eribulin |
| 11 | R | bond | Eribulin |
| 11 | S | L1 | Eribulin |
| 11 | R | L1 | Eribulin |
| 11 | S | L2 | Eribulin |
| 11 | R | L2 | Eribulin |
| 11 | S | L3 | Eribulin |
| 11 | R | L3 | Eribulin |
| 11 | S | L4 | Eribulin |
| 11 | R | L4 | Eribulin |
| 11 | S | L5 | Eribulin |
| 11 | R | L5 | Eribulin |
| 11 | S | L6 | Eribulin |
| 11 | R | L6 | Eribulin |
| 11 | S | L7 | Eribulin |
| 11 | R | L7 | Eribulin |
| 11 | S | L8 | Eribulin |
| 11 | R | L8 | Eribulin |
| 11 | S | L9 | Eribulin |
| 11 | R | L9 | Eribulin |
| 11 | S | L10 | Eribulin |
| 11 | R | L10 | Eribulin |
| 11 | S | L15 | Eribulin |
| 11 | R | L15 | Eribulin |
| 11 | S | L20 | Eribulin |
| 11 | R | L20 | Eribulin |
| 11 | S | bond | SN-38 |
| 11 | R | bond | SN-38 |
| 11 | S | L1 | SN-38 |
| 11 | R | L1 | SN-38 |
| 11 | S | L2 | SN-38 |
| 11 | R | L2 | SN-38 |
| 11 | S | L3 | SN-38 |

(continued)

| n2 | Chirality of carbon atom at * | Y | Payload |
|---|---|---|---|
| 11 | R | L3 | SN-38 |
| 11 | S | L4 | SN-38 |
| 11 | R | L4 | SN-38 |
| 11 | S | L5 | SN-38 |
| 11 | R | L5 | SN-38 |
| 11 | S | L6 | SN-38 |
| 11 | R | L6 | SN-38 |
| 11 | S | L7 | SN-38 |
| 11 | R | L7 | SN-38 |
| 11 | S | L8 | SN-38 |
| 11 | R | L8 | SN-38 |
| 11 | S | L9 | SN-38 |
| 11 | R | L9 | SN-38 |
| 11 | S | L10 | SN-38 |
| 11 | R | L10 | SN-38 |
| 11 | S | L15 | SN-38 |
| 11 | R | L15 | SN-38 |
| 11 | S | L20 | SN-38 |
| 11 | R | L20 | SN-38 |
| 12 | S | bond | MMAE |
| 12 | R | bond | MMAE |
| 12 | S | L1 | MMAE |
| 12 | R | L1 | MMAE |
| 12 | S | L2 | MMAE |
| 12 | R | L2 | MMAE |
| 12 | S | L3 | MMAE |
| 12 | R | L3 | MMAE |
| 12 | S | L4 | MMAE |
| 12 | R | L4 | MMAE |
| 12 | S | L5 | MMAE |
| 12 | R | L5 | MMAE |
| 12 | S | L6 | MMAE |
| 12 | R | L6 | MMAE |
| 12 | S | L7 | MMAE |
| 12 | R | L7 | MMAE |
| 12 | S | L8 | MMAE |

(continued)

| n2 | Chirality of carbon atom at * | Y | Payload |
|---|---|---|---|
| 12 | R | L8 | MMAE |
| 12 | S | L9 | MMAE |
| 12 | R | L9 | MMAE |
| 12 | S | L10 | MMAE |
| 12 | R | L10 | MMAE |
| 12 | S | L15 | MMAE |
| 12 | R | L15 | MMAE |
| 12 | S | L20 | MMAE |
| 12 | R | L20 | MMAE |
| 12 | S | bond | Eribulin |
| 12 | R | bond | Eribulin |
| 12 | S | L1 | Eribulin |
| 12 | R | L1 | Eribulin |
| 12 | S | L2 | Eribulin |
| 12 | R | L2 | Eribulin |
| 12 | S | L3 | Eribulin |
| 12 | R | L3 | Eribulin |
| 12 | S | L4 | Eribulin |
| 12 | R | L4 | Eribulin |
| 12 | S | L5 | Eribulin |
| 12 | R | L5 | Eribulin |
| 12 | S | L6 | Eribulin |
| 12 | R | L6 | Eribulin |
| 12 | S | L7 | Eribulin |
| 12 | R | L7 | Eribulin |
| 12 | S | L8 | Eribulin |
| 12 | R | L8 | Eribulin |
| 12 | S | L9 | Eribulin |
| 12 | R | L9 | Eribulin |
| 12 | S | L10 | Eribulin |
| 12 | R | L10 | Eribulin |
| 12 | S | L15 | Eribulin |
| 12 | R | L15 | Eribulin |
| 12 | S | L20 | Eribulin |
| 12 | R | L20 | Eribulin |
| 12 | S | bond | SN-38 |

(continued)

| n2 | Chirality of carbon atom at * | Y | Payload |
|---|---|---|---|
| 12 | R | bond | SN-38 |
| 12 | S | L1 | SN-38 |
| 12 | R | L1 | SN-38 |
| 12 | S | L2 | SN-38 |
| 12 | R | L2 | SN-38 |
| 12 | S | L3 | SN-38 |
| 12 | R | L3 | SN-38 |
| 12 | S | L4 | SN-38 |
| 12 | R | L4 | SN-38 |
| 12 | S | L5 | SN-38 |
| 12 | R | L5 | SN-38 |
| 12 | S | L6 | SN-38 |
| 12 | R | L6 | SN-38 |
| 12 | S | L7 | SN-38 |
| 12 | R | L7 | SN-38 |
| 12 | S | L8 | SN-38 |
| 12 | R | L8 | SN-38 |
| 12 | S | L9 | SN-38 |
| 12 | R | L9 | SN-38 |
| 12 | S | L10 | SN-38 |
| 12 | R | L10 | SN-38 |
| 12 | S | L15 | SN-38 |
| 12 | R | L15 | SN-38 |
| 12 | S | L20 | SN-38 |
| 12 | R | L20 | SN-38 |
| 13 | S | bond | MMAE |
| 13 | R | bond | MMAE |
| 13 | S | L1 | MMAE |
| 13 | R | L1 | MMAE |
| 13 | S | L2 | MMAE |
| 13 | R | L2 | MMAE |
| 13 | S | L3 | MMAE |
| 13 | R | L3 | MMAE |
| 13 | S | L4 | MMAE |
| 13 | R | L4 | MMAE |
| 13 | S | L5 | MMAE |

(continued)

| n2 | Chirality of carbon atom at * | Y | Payload |
|---|---|---|---|
| 13 | R | L5 | MMAE |
| 13 | S | L6 | MMAE |
| 13 | R | L6 | MMAE |
| 13 | S | L7 | MMAE |
| 13 | R | L7 | MMAE |
| 13 | S | L8 | MMAE |
| 13 | R | L8 | MMAE |
| 13 | S | L9 | MMAE |
| 13 | R | L9 | MMAE |
| 13 | S | L10 | MMAE |
| 13 | R | L10 | MMAE |
| 13 | S | L15 | MMAE |
| 13 | R | L15 | MMAE |
| 13 | S | L20 | MMAE |
| 13 | R | L20 | MMAE |
| 13 | S | bond | Eribulin |
| 13 | R | bond | Eribulin |
| 13 | S | L1 | Eribulin |
| 13 | R | L1 | Eribulin |
| 13 | S | L2 | Eribulin |
| 13 | R | L2 | Eribulin |
| 13 | S | L3 | Eribulin |
| 13 | R | L3 | Eribulin |
| 13 | S | L4 | Eribulin |
| 13 | R | L4 | Eribulin |
| 13 | S | L5 | Eribulin |
| 13 | R | L5 | Eribulin |
| 13 | S | L6 | Eribulin |
| 13 | R | L6 | Eribulin |
| 13 | S | L7 | Eribulin |
| 13 | R | L7 | Eribulin |
| 13 | S | L8 | Eribulin |
| 13 | R | L8 | Eribulin |
| 13 | S | L9 | Eribulin |
| 13 | R | L9 | Eribulin |
| 13 | S | L10 | Eribulin |

(continued)

| n2 | Chirality of carbon atom at * | Y | Payload |
|---|---|---|---|
| 13 | R | L10 | Eribulin |
| 13 | S | L15 | Eribulin |
| 13 | R | L15 | Eribulin |
| 13 | S | L20 | Eribulin |
| 13 | R | L20 | Eribulin |
| 13 | S | bond | SN-38 |
| 13 | R | bond | SN-38 |
| 13 | S | L1 | SN-38 |
| 13 | R | L1 | SN-38 |
| 13 | S | L2 | SN-38 |
| 13 | R | L2 | SN-38 |
| 13 | S | L3 | SN-38 |
| 13 | R | L3 | SN-38 |
| 13 | S | L4 | SN-38 |
| 13 | R | L4 | SN-38 |
| 13 | S | L5 | SN-38 |
| 13 | R | L5 | SN-38 |
| 13 | S | L6 | SN-38 |
| 13 | R | L6 | SN-38 |
| 13 | S | L7 | SN-38 |
| 13 | R | L7 | SN-38 |
| 13 | S | L8 | SN-38 |
| 13 | R | L8 | SN-38 |
| 13 | S | L9 | SN-38 |
| 13 | R | L9 | SN-38 |
| 13 | S | L10 | SN-38 |
| 13 | R | L10 | SN-38 |
| 13 | S | L15 | SN-38 |
| 13 | R | L15 | SN-38 |
| 13 | S | L20 | SN-38 |
| 13 | R | L20 | SN-38 |
| 14 | S | bond | MMAE |
| 14 | R | bond | MMAE |
| 14 | S | L1 | MMAE |
| 14 | R | L1 | MMAE |
| 14 | S | L2 | MMAE |

(continued)

| n2 | Chirality of carbon atom at * | Y | Payload |
|---|---|---|---|
| 14 | R | L2 | MMAE |
| 14 | S | L3 | MMAE |
| 14 | R | L3 | MMAE |
| 14 | S | L4 | MMAE |
| 14 | R | L4 | MMAE |
| 14 | S | L5 | MMAE |
| 14 | R | L5 | MMAE |
| 14 | S | L6 | MMAE |
| 14 | R | L6 | MMAE |
| 14 | S | L7 | MMAE |
| 14 | R | L7 | MMAE |
| 14 | S | L8 | MMAE |
| 14 | R | L8 | MMAE |
| 14 | S | L9 | MMAE |
| 14 | R | L9 | MMAE |
| 14 | S | L10 | MMAE |
| 14 | R | L10 | MMAE |
| 14 | S | L15 | MMAE |
| 14 | R | L15 | MMAE |
| 14 | S | L20 | MMAE |
| 14 | R | L20 | MMAE |
| 14 | S | bond | Eribulin |
| 14 | R | bond | Eribulin |
| 14 | S | L1 | Eribulin |
| 14 | R | L1 | Eribulin |
| 14 | S | L2 | Eribulin |
| 14 | R | L2 | Eribulin |
| 14 | S | L3 | Eribulin |
| 14 | R | L3 | Eribulin |
| 14 | S | L4 | Eribulin |
| 14 | R | L4 | Eribulin |
| 14 | S | L5 | Eribulin |
| 14 | R | L5 | Eribulin |
| 14 | S | L6 | Eribulin |
| 14 | R | L6 | Eribulin |
| 14 | S | L7 | Eribulin |

(continued)

| n2 | Chirality of carbon atom at * | Y | Payload |
|---|---|---|---|
| 14 | R | L7 | Eribulin |
| 14 | S | L8 | Eribulin |
| 14 | R | L8 | Eribulin |
| 14 | S | L9 | Eribulin |
| 14 | R | L9 | Eribulin |
| 14 | S | L10 | Eribulin |
| 14 | R | L10 | Eribulin |
| 14 | S | L15 | Eribulin |
| 14 | R | L15 | Eribulin |
| 14 | S | L20 | Eribulin |
| 14 | R | L20 | Eribulin |
| 14 | S | bond | SN-38 |
| 14 | R | bond | SN-38 |
| 14 | S | L1 | SN-38 |
| 14 | R | L1 | SN-38 |
| 14 | S | L2 | SN-38 |
| 14 | R | L2 | SN-38 |
| 14 | S | L3 | SN-38 |
| 14 | R | L3 | SN-38 |
| 14 | S | L4 | SN-38 |
| 14 | R | L4 | SN-38 |
| 14 | S | L5 | SN-38 |
| 14 | R | L5 | SN-38 |
| 14 | S | L6 | SN-38 |
| 14 | R | L6 | SN-38 |
| 14 | S | L7 | SN-38 |
| 14 | R | L7 | SN-38 |
| 14 | S | L8 | SN-38 |
| 14 | R | L8 | SN-38 |
| 14 | S | L9 | SN-38 |
| 14 | R | L9 | SN-38 |
| 14 | S | L10 | SN-38 |
| 14 | R | L10 | SN-38 |
| 14 | S | L15 | SN-38 |
| 14 | R | L15 | SN-38 |
| 14 | S | L20 | SN-38 |

(continued)

| n2 | Chirality of carbon atom at * | Y | Payload |
|---|---|---|---|
| 14 | R | L20 | SN-38 |
| 15 | S | bond | MMAE |
| 15 | R | bond | MMAE |
| 15 | S | L1 | MMAE |
| 15 | R | L1 | MMAE |
| 15 | S | L2 | MMAE |
| 15 | R | L2 | MMAE |
| 15 | S | L3 | MMAE |
| 15 | R | L3 | MMAE |
| 15 | S | L4 | MMAE |
| 15 | R | L4 | MMAE |
| 15 | S | L5 | MMAE |
| 15 | R | L5 | MMAE |
| 15 | S | L6 | MMAE |
| 15 | R | L6 | MMAE |
| 15 | S | L7 | MMAE |
| 15 | R | L7 | MMAE |
| 15 | S | L8 | MMAE |
| 15 | R | L8 | MMAE |
| 15 | S | L9 | MMAE |
| 15 | R | L9 | MMAE |
| 15 | S | L10 | MMAE |
| 15 | R | L10 | MMAE |
| 15 | S | L15 | MMAE |
| 15 | R | L15 | MMAE |
| 15 | S | L20 | MMAE |
| 15 | R | L20 | MMAE |
| 15 | S | bond | Eribulin |
| 15 | R | bond | Eribulin |
| 15 | S | L1 | Eribulin |
| 15 | R | L1 | Eribulin |
| 15 | S | L2 | Eribulin |
| 15 | R | L2 | Eribulin |
| 15 | S | L3 | Eribulin |
| 15 | R | L3 | Eribulin |
| 15 | S | L4 | Eribulin |

(continued)

| n2 | Chirality of carbon atom at * | Y | Payload |
|---|---|---|---|
| 15 | R | L4 | Eribulin |
| 15 | S | L5 | Eribulin |
| 15 | R | L5 | Eribulin |
| 15 | S | L6 | Eribulin |
| 15 | R | L6 | Eribulin |
| 15 | S | L7 | Eribulin |
| 15 | R | L7 | Eribulin |
| 15 | S | L8 | Eribulin |
| 15 | R | L8 | Eribulin |
| 15 | S | L9 | Eribulin |
| 15 | R | L9 | Eribulin |
| 15 | S | L10 | Eribulin |
| 15 | R | L10 | Eribulin |
| 15 | S | L15 | Eribulin |
| 15 | R | L15 | Eribulin |
| 15 | S | L20 | Eribulin |
| 15 | R | L20 | Eribulin |
| 15 | S | bond | SN-38 |
| 15 | R | bond | SN-38 |
| 15 | S | L1 | SN-38 |
| 15 | R | L1 | SN-38 |
| 15 | S | L2 | SN-38 |
| 15 | R | L2 | SN-38 |
| 15 | S | L3 | SN-38 |
| 15 | R | L3 | SN-38 |
| 15 | S | L4 | SN-38 |
| 15 | R | L4 | SN-38 |
| 15 | S | L5 | SN-38 |
| 15 | R | L5 | SN-38 |
| 15 | S | L6 | SN-38 |
| 15 | R | L6 | SN-38 |
| 15 | S | L7 | SN-38 |
| 15 | R | L7 | SN-38 |
| 15 | S | L8 | SN-38 |
| 15 | R | L8 | SN-38 |
| 15 | S | L9 | SN-38 |

(continued)

| n2 | Chirality of carbon atom at * | Y | Payload |
|---|---|---|---|
| 15 | R | L9 | SN-38 |
| 15 | S | L10 | SN-38 |
| 15 | R | L10 | SN-38 |
| 15 | S | L15 | SN-38 |
| 15 | R | L15 | SN-38 |
| 15 | S | L20 | SN-38 |
| 15 | R | L20 | SN-38 |
| 16 | S | bond | MMAE |
| 16 | R | bond | MMAE |
| 16 | S | L1 | MMAE |
| 16 | R | L1 | MMAE |
| 16 | S | L2 | MMAE |
| 16 | R | L2 | MMAE |
| 16 | S | L3 | MMAE |
| 16 | R | L3 | MMAE |
| 16 | S | L4 | MMAE |
| 16 | R | L4 | MMAE |
| 16 | S | L5 | MMAE |
| 16 | R | L5 | MMAE |
| 16 | S | L6 | MMAE |
| 16 | R | L6 | MMAE |
| 16 | S | L7 | MMAE |
| 16 | R | L7 | MMAE |
| 16 | S | L8 | MMAE |
| 16 | R | L8 | MMAE |
| 16 | S | L9 | MMAE |
| 16 | R | L9 | MMAE |
| 16 | S | L10 | MMAE |
| 16 | R | L10 | MMAE |
| 16 | S | L15 | MMAE |
| 16 | R | L15 | MMAE |
| 16 | S | L20 | MMAE |
| 16 | R | L20 | MMAE |
| 16 | S | bond | Eribulin |
| 16 | R | bond | Eribulin |
| 16 | S | L1 | Eribulin |

(continued)

| n2 | Chirality of carbon atom at * | Y | Payload |
|---|---|---|---|
| 16 | R | L1 | Eribulin |
| 16 | S | L2 | Eribulin |
| 16 | R | L2 | Eribulin |
| 16 | S | L3 | Eribulin |
| 16 | R | L3 | Eribulin |
| 16 | S | L4 | Eribulin |
| 16 | R | L4 | Eribulin |
| 16 | S | L5 | Eribulin |
| 16 | R | L5 | Eribulin |
| 16 | S | L6 | Eribulin |
| 16 | R | L6 | Eribulin |
| 16 | S | L7 | Eribulin |
| 16 | R | L7 | Eribulin |
| 16 | S | L8 | Eribulin |
| 16 | R | L8 | Eribulin |
| 16 | S | L9 | Eribulin |
| 16 | R | L9 | Eribulin |
| 16 | S | L10 | Eribulin |
| 16 | R | L10 | Eribulin |
| 16 | S | L15 | Eribulin |
| 16 | R | L15 | Eribulin |
| 16 | S | L20 | Eribulin |
| 16 | R | L20 | Eribulin |
| 16 | S | bond | SN-38 |
| 16 | R | bond | SN-38 |
| 16 | S | L1 | SN-38 |
| 16 | R | L1 | SN-38 |
| 16 | S | L2 | SN-38 |
| 16 | R | L2 | SN-38 |
| 16 | S | L3 | SN-38 |
| 16 | R | L3 | SN-38 |
| 16 | S | L4 | SN-38 |
| 16 | R | L4 | SN-38 |
| 16 | S | L5 | SN-38 |
| 16 | R | L5 | SN-38 |
| 16 | S | L6 | SN-38 |

(continued)

| n2 | Chirality of carbon atom at * | Y | Payload |
|---|---|---|---|
| 16 | R | L6 | SN-38 |
| 16 | S | L7 | SN-38 |
| 16 | R | L7 | SN-38 |
| 16 | S | L8 | SN-38 |
| 16 | R | L8 | SN-38 |
| 16 | S | L9 | SN-38 |
| 16 | R | L9 | SN-38 |
| 16 | S | L10 | SN-38 |
| 16 | R | L10 | SN-38 |
| 16 | S | L15 | SN-38 |
| 16 | R | L15 | SN-38 |
| 16 | S | L20 | SN-38 |
| 16 | R | L20 | SN-38 |
| 17 | S | bond | MMAE |
| 17 | R | bond | MMAE |
| 17 | S | L1 | MMAE |
| 17 | R | L1 | MMAE |
| 17 | S | L2 | MMAE |
| 17 | R | L2 | MMAE |
| 17 | S | L3 | MMAE |
| 17 | R | L3 | MMAE |
| 17 | S | L4 | MMAE |
| 17 | R | L4 | MMAE |
| 17 | S | L5 | MMAE |
| 17 | R | L5 | MMAE |
| 17 | S | L6 | MMAE |
| 17 | R | L6 | MMAE |
| 17 | S | L7 | MMAE |
| 17 | R | L7 | MMAE |
| 17 | S | L8 | MMAE |
| 17 | R | L8 | MMAE |
| 17 | S | L9 | MMAE |
| 17 | R | L9 | MMAE |
| 17 | S | L10 | MMAE |
| 17 | R | L10 | MMAE |
| 17 | S | L15 | MMAE |

(continued)

| n2 | Chirality of carbon atom at * | Y | Payload |
|---|---|---|---|
| 17 | R | L15 | MMAE |
| 17 | S | L20 | MMAE |
| 17 | R | L20 | MMAE |
| 17 | S | bond | Eribulin |
| 17 | R | bond | Eribulin |
| 17 | S | L1 | Eribulin |
| 17 | R | L1 | Eribulin |
| 17 | S | L2 | Eribulin |
| 17 | R | L2 | Eribulin |
| 17 | S | L3 | Eribulin |
| 17 | R | L3 | Eribulin |
| 17 | S | L4 | Eribulin |
| 17 | R | L4 | Eribulin |
| 17 | S | L5 | Eribulin |
| 17 | R | L5 | Eribulin |
| 17 | S | L6 | Eribulin |
| 17 | R | L6 | Eribulin |
| 17 | S | L7 | Eribulin |
| 17 | R | L7 | Eribulin |
| 17 | S | L8 | Eribulin |
| 17 | R | L8 | Eribulin |
| 17 | S | L9 | Eribulin |
| 17 | R | L9 | Eribulin |
| 17 | S | L10 | Eribulin |
| 17 | R | L10 | Eribulin |
| 17 | S | L15 | Eribulin |
| 17 | R | L15 | Eribulin |
| 17 | S | L20 | Eribulin |
| 17 | R | L20 | Eribulin |
| 17 | S | bond | SN-38 |
| 17 | R | bond | SN-38 |
| 17 | S | L1 | SN-38 |
| 17 | R | L1 | SN-38 |
| 17 | S | L2 | SN-38 |
| 17 | R | L2 | SN-38 |
| 17 | S | L3 | SN-38 |

(continued)

| n2 | Chirality of carbon atom at * | Y | Payload |
|---|---|---|---|
| 17 | R | L3 | SN-38 |
| 17 | S | L4 | SN-38 |
| 17 | R | L4 | SN-38 |
| 17 | S | L5 | SN-38 |
| 17 | R | L5 | SN-38 |
| 17 | S | L6 | SN-38 |
| 17 | R | L6 | SN-38 |
| 17 | S | L7 | SN-38 |
| 17 | R | L7 | SN-38 |
| 17 | S | L8 | SN-38 |
| 17 | R | L8 | SN-38 |
| 17 | S | L9 | SN-38 |
| 17 | R | L9 | SN-38 |
| 17 | S | L10 | SN-38 |
| 17 | R | L10 | SN-38 |
| 17 | S | L15 | SN-38 |
| 17 | R | L15 | SN-38 |
| 17 | S | L20 | SN-38 |
| 17 | R | L20 | SN-38 |
| 18 | S | bond | MMAE |
| 18 | R | bond | MMAE |
| 18 | S | L1 | MMAE |
| 18 | R | L1 | MMAE |
| 18 | S | L2 | MMAE |
| 18 | R | L2 | MMAE |
| 18 | S | L3 | MMAE |
| 18 | R | L3 | MMAE |
| 18 | S | L4 | MMAE |
| 18 | R | L4 | MMAE |
| 18 | S | L5 | MMAE |
| 18 | R | L5 | MMAE |
| 18 | S | L6 | MMAE |
| 18 | R | L6 | MMAE |
| 18 | S | L7 | MMAE |
| 18 | R | L7 | MMAE |
| 18 | S | L8 | MMAE |

(continued)

| n2 | Chirality of carbon atom at * | Y | Payload |
|---|---|---|---|
| 18 | R | L8 | MMAE |
| 18 | S | L9 | MMAE |
| 18 | R | L9 | MMAE |
| 18 | S | L10 | MMAE |
| 18 | R | L10 | MMAE |
| 18 | S | L15 | MMAE |
| 18 | R | L15 | MMAE |
| 18 | S | L20 | MMAE |
| 18 | R | L20 | MMAE |
| 18 | S | bond | Eribulin |
| 18 | R | bond | Eribulin |
| 18 | S | L1 | Eribulin |
| 18 | R | L1 | Eribulin |
| 18 | S | L2 | Eribulin |
| 18 | R | L2 | Eribulin |
| 18 | S | L3 | Eribulin |
| 18 | R | L3 | Eribulin |
| 18 | S | L4 | Eribulin |
| 18 | R | L4 | Eribulin |
| 18 | S | L5 | Eribulin |
| 18 | R | L5 | Eribulin |
| 18 | S | L6 | Eribulin |
| 18 | R | L6 | Eribulin |
| 18 | S | L7 | Eribulin |
| 18 | R | L7 | Eribulin |
| 18 | S | L8 | Eribulin |
| 18 | R | L8 | Eribulin |
| 18 | S | L9 | Eribulin |
| 18 | R | L9 | Eribulin |
| 18 | S | L10 | Eribulin |
| 18 | R | L10 | Eribulin |
| 18 | S | L15 | Eribulin |
| 18 | R | L15 | Eribulin |
| 18 | S | L20 | Eribulin |
| 18 | R | L20 | Eribulin |
| 18 | S | bond | SN-38 |

(continued)

| n2 | Chirality of carbon atom at * | Y | Payload |
|---|---|---|---|
| 18 | R | bond | SN-38 |
| 18 | S | L1 | SN-38 |
| 18 | R | L1 | SN-38 |
| 18 | S | L2 | SN-38 |
| 18 | R | L2 | SN-38 |
| 18 | S | L3 | SN-38 |
| 18 | R | L3 | SN-38 |
| 18 | S | L4 | SN-38 |
| 18 | R | L4 | SN-38 |
| 18 | S | L5 | SN-38 |
| 18 | R | L5 | SN-38 |
| 18 | S | L6 | SN-38 |
| 18 | R | L6 | SN-38 |
| 18 | S | L7 | SN-38 |
| 18 | R | L7 | SN-38 |
| 18 | S | L8 | SN-38 |
| 18 | R | L8 | SN-38 |
| 18 | S | L9 | SN-38 |
| 18 | R | L9 | SN-38 |
| 18 | S | L10 | SN-38 |
| 18 | R | L10 | SN-38 |
| 18 | S | L15 | SN-38 |
| 18 | R | L15 | SN-38 |
| 18 | S | L20 | SN-38 |
| 18 | R | L20 | SN-38 |
| 19 | S | bond | MMAE |
| 19 | R | bond | MMAE |
| 19 | S | L1 | MMAE |
| 19 | R | L1 | MMAE |
| 19 | S | L2 | MMAE |
| 19 | R | L2 | MMAE |
| 19 | S | L3 | MMAE |
| 19 | R | L3 | MMAE |
| 19 | S | L4 | MMAE |
| 19 | R | L4 | MMAE |
| 19 | S | L5 | MMAE |

(continued)

| n2 | Chirality of carbon atom at * | Y | Payload |
|---|---|---|---|
| 19 | R | L5 | MMAE |
| 19 | S | L6 | MMAE |
| 19 | R | L6 | MMAE |
| 19 | S | L7 | MMAE |
| 19 | R | L7 | MMAE |
| 19 | S | L8 | MMAE |
| 19 | R | L8 | MMAE |
| 19 | S | L9 | MMAE |
| 19 | R | L9 | MMAE |
| 19 | S | L10 | MMAE |
| 19 | R | L10 | MMAE |
| 19 | S | L15 | MMAE |
| 19 | R | L15 | MMAE |
| 19 | S | L20 | MMAE |
| 19 | R | L20 | MMAE |
| 19 | S | bond | Eribulin |
| 19 | R | bond | Eribulin |
| 19 | S | L1 | Eribulin |
| 19 | R | L1 | Eribulin |
| 19 | S | L2 | Eribulin |
| 19 | R | L2 | Eribulin |
| 19 | S | L3 | Eribulin |
| 19 | R | L3 | Eribulin |
| 19 | S | L4 | Eribulin |
| 19 | R | L4 | Eribulin |
| 19 | S | L5 | Eribulin |
| 19 | R | L5 | Eribulin |
| 19 | S | L6 | Eribulin |
| 19 | R | L6 | Eribulin |
| 19 | S | L7 | Eribulin |
| 19 | R | L7 | Eribulin |
| 19 | S | L8 | Eribulin |
| 19 | R | L8 | Eribulin |
| 19 | S | L9 | Eribulin |
| 19 | R | L9 | Eribulin |
| 19 | S | L10 | Eribulin |

(continued)

| n2 | Chirality of carbon atom at * | Y | Payload |
|---|---|---|---|
| 19 | R | L10 | Eribulin |
| 19 | S | L15 | Eribulin |
| 19 | R | L15 | Eribulin |
| 19 | S | L20 | Eribulin |
| 19 | R | L20 | Eribulin |
| 19 | S | bond | SN-38 |
| 19 | R | bond | SN-38 |
| 19 | S | L1 | SN-38 |
| 19 | R | L1 | SN-38 |
| 19 | S | L2 | SN-38 |
| 19 | R | L2 | SN-38 |
| 19 | S | L3 | SN-38 |
| 19 | R | L3 | SN-38 |
| 19 | S | L4 | SN-38 |
| 19 | R | L4 | SN-38 |
| 19 | S | L5 | SN-38 |
| 19 | R | L5 | SN-38 |
| 19 | S | L6 | SN-38 |
| 19 | R | L6 | SN-38 |
| 19 | S | L7 | SN-38 |
| 19 | R | L7 | SN-38 |
| 19 | S | L8 | SN-38 |
| 19 | R | L8 | SN-38 |
| 19 | S | L9 | SN-38 |
| 19 | R | L9 | SN-38 |
| 19 | S | L10 | SN-38 |
| 19 | R | L10 | SN-38 |
| 19 | S | L15 | SN-38 |
| 19 | R | L15 | SN-38 |
| 19 | S | L20 | SN-38 |
| 19 | R | L20 | SN-38 |
| 20 | S | bond | MMAE |
| 20 | R | bond | MMAE |
| 20 | S | L1 | MMAE |
| 20 | R | L1 | MMAE |
| 20 | S | L2 | MMAE |

(continued)

| n2 | Chirality of carbon atom at * | Y | Payload |
|---|---|---|---|
| 20 | R | L2 | MMAE |
| 20 | S | L3 | MMAE |
| 20 | R | L3 | MMAE |
| 20 | S | L4 | MMAE |
| 20 | R | L4 | MMAE |
| 20 | S | L5 | MMAE |
| 20 | R | L5 | MMAE |
| 20 | S | L6 | MMAE |
| 20 | R | L6 | MMAE |
| 20 | S | L7 | MMAE |
| 20 | R | L7 | MMAE |
| 20 | S | L8 | MMAE |
| 20 | R | L8 | MMAE |
| 20 | S | L9 | MMAE |
| 20 | R | L9 | MMAE |
| 20 | S | L10 | MMAE |
| 20 | R | L10 | MMAE |
| 20 | S | L15 | MMAE |
| 20 | R | L15 | MMAE |
| 20 | S | L20 | MMAE |
| 20 | R | L20 | MMAE |
| 20 | S | bond | Eribulin |
| 20 | R | bond | Eribulin |
| 20 | S | L1 | Eribulin |
| 20 | R | L1 | Eribulin |
| 20 | S | L2 | Eribulin |
| 20 | R | L2 | Eribulin |
| 20 | S | L3 | Eribulin |
| 20 | R | L3 | Eribulin |
| 20 | S | L4 | Eribulin |
| 20 | R | L4 | Eribulin |
| 20 | S | L5 | Eribulin |
| 20 | R | L5 | Eribulin |
| 20 | S | L6 | Eribulin |
| 20 | R | L6 | Eribulin |
| 20 | S | L7 | Eribulin |

(continued)

| n2 | Chirality of carbon atom at * | Y | Payload |
|---|---|---|---|
| 20 | R | L7 | Eribulin |
| 20 | S | L8 | Eribulin |
| 20 | R | L8 | Eribulin |
| 20 | S | L9 | Eribulin |
| 20 | R | L9 | Eribulin |
| 20 | S | L10 | Eribulin |
| 20 | R | L10 | Eribulin |
| 20 | S | L15 | Eribulin |
| 20 | R | L15 | Eribulin |
| 20 | S | L20 | Eribulin |
| 20 | R | L20 | Eribulin |
| 20 | S | bond | SN-38 |
| 20 | R | bond | SN-38 |
| 20 | S | L1 | SN-38 |
| 20 | R | L1 | SN-38 |
| 20 | S | L2 | SN-38 |
| 20 | R | L2 | SN-38 |
| 20 | S | L3 | SN-38 |
| 20 | R | L3 | SN-38 |
| 20 | S | L4 | SN-38 |
| 20 | R | L4 | SN-38 |
| 20 | S | L5 | SN-38 |
| 20 | R | L5 | SN-38 |
| 20 | S | L6 | SN-38 |
| 20 | R | L6 | SN-38 |
| 20 | S | L7 | SN-38 |
| 20 | R | L7 | SN-38 |
| 20 | S | L8 | SN-38 |
| 20 | R | L8 | SN-38 |
| 20 | S | L9 | SN-38 |
| 20 | R | L9 | SN-38 |
| 20 | S | L10 | SN-38 |
| 20 | R | L10 | SN-38 |
| 20 | S | L15 | SN-38 |
| 20 | R | L15 | SN-38 |
| 20 | S | L20 | SN-38 |

(continued)

| n2 | Chirality of carbon atom at * | Y | Payload |
|---|---|---|---|
| 20 | R | L20 | SN-38; |

Still alternatively, the following compounds: wherein Rx is:

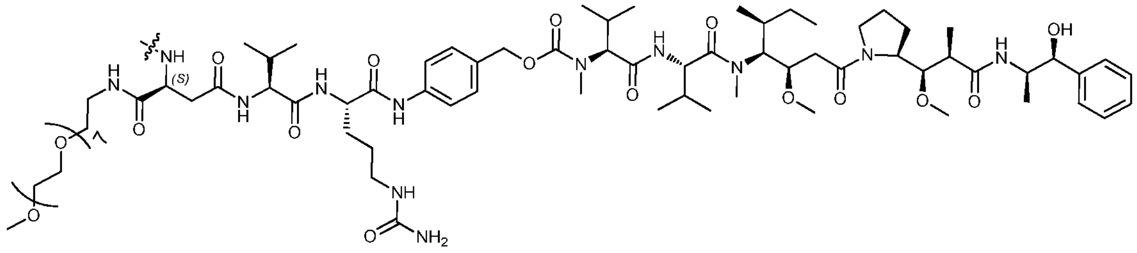

,

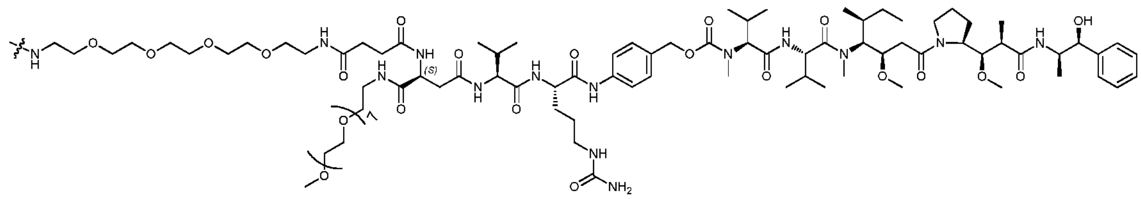

,

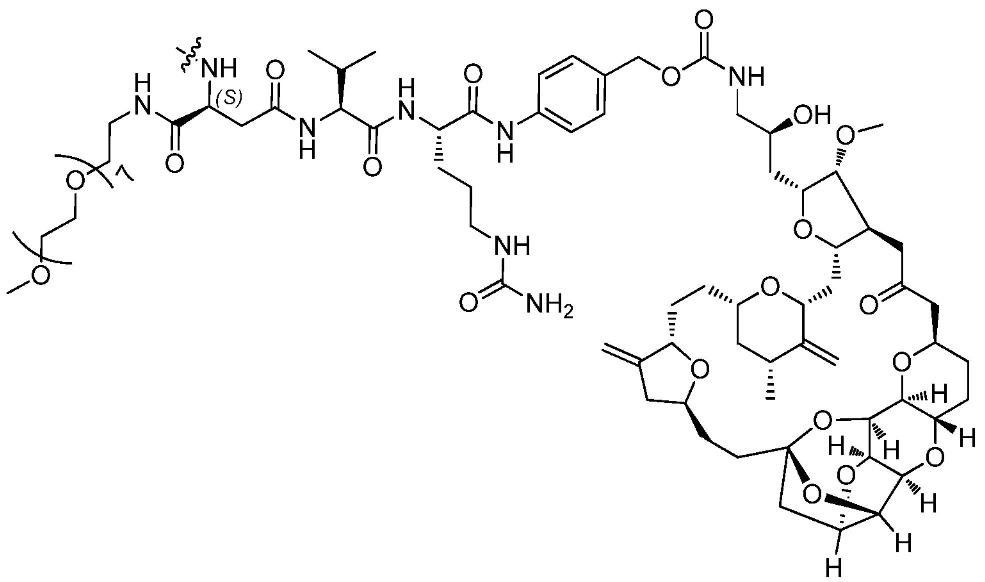

,

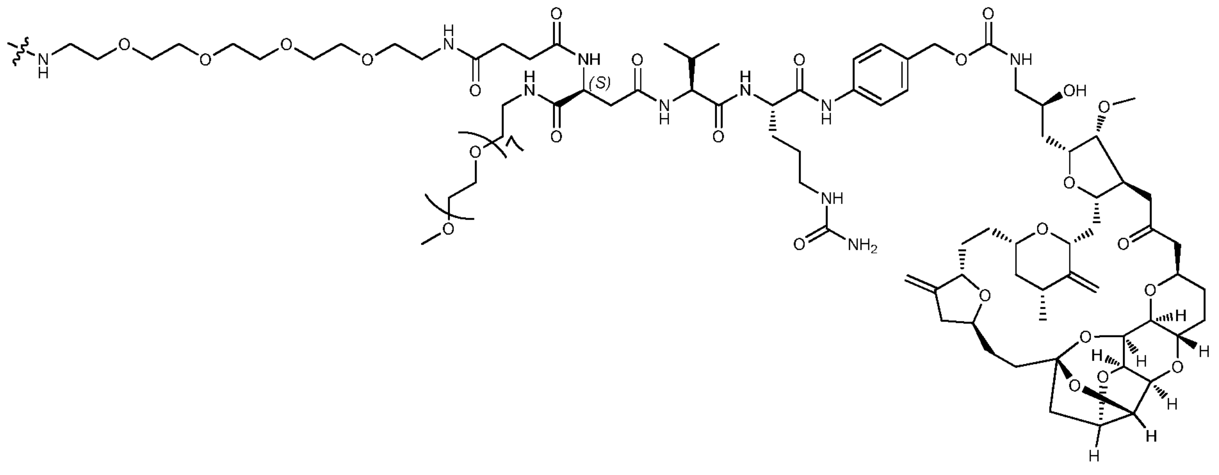

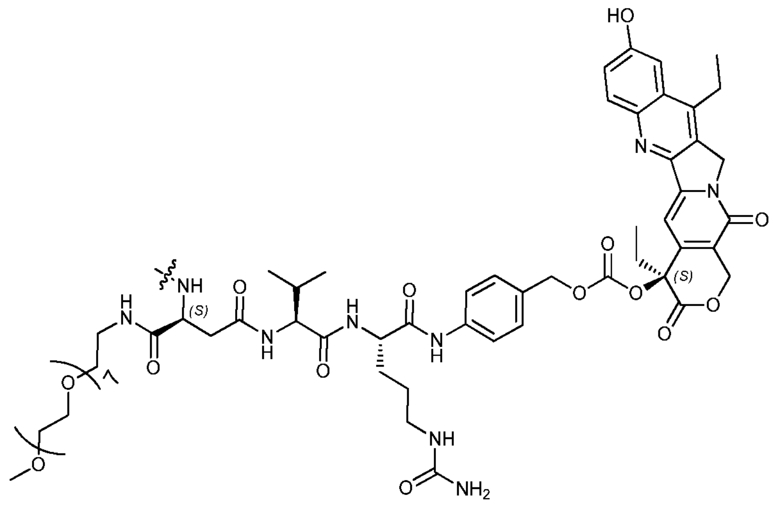

, ,

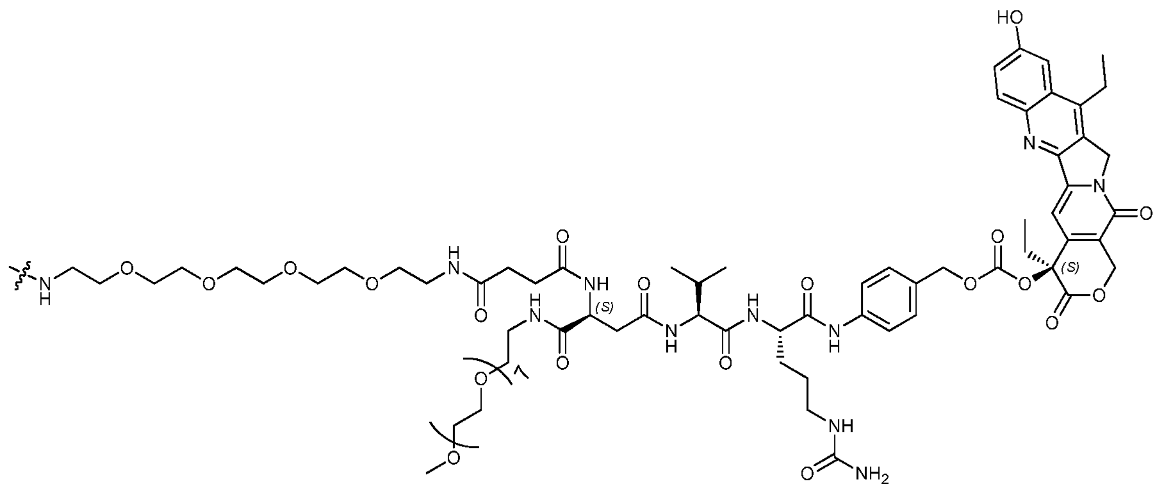

,

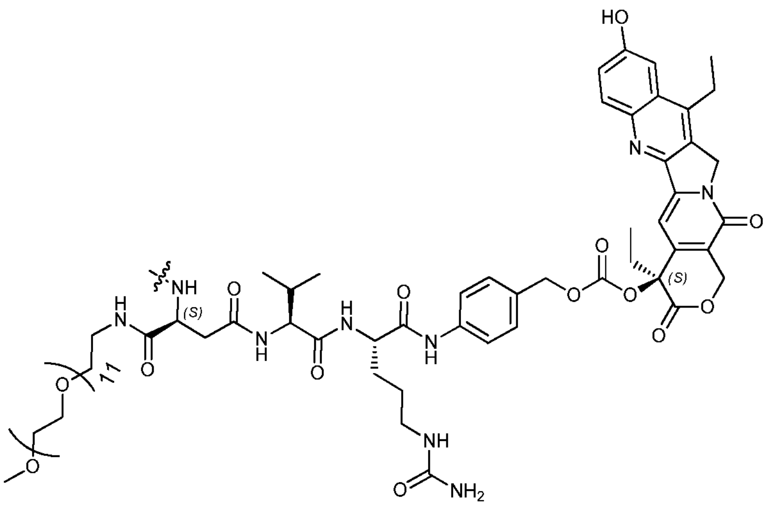

,

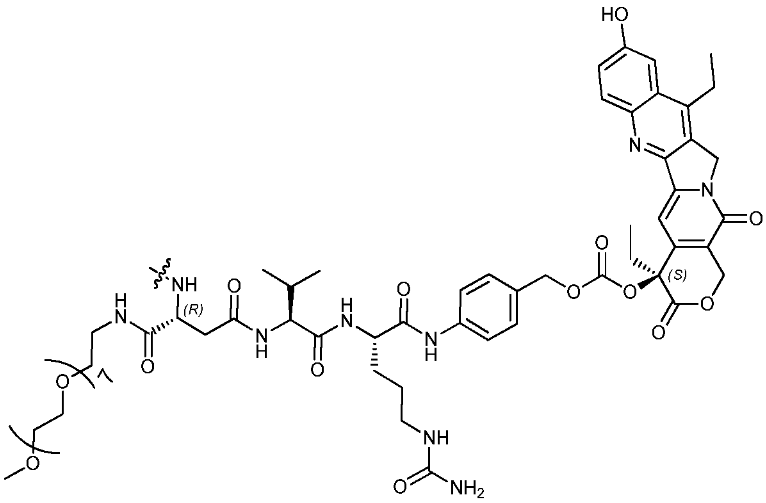

,

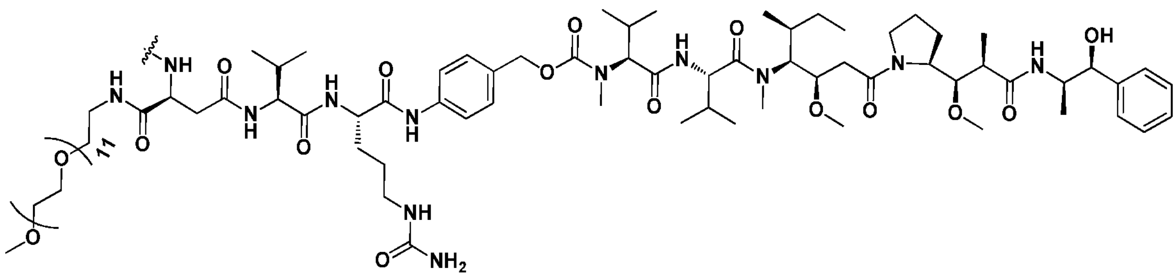

or

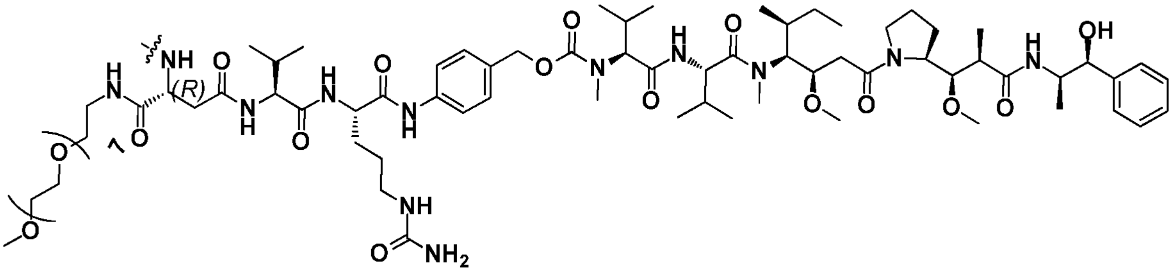

.

According to embodiments of the present disclosure, the conjugate is selected from the following specific compounds, or tautomers, stereoisomers or pharmaceutically acceptable salts thereof, wherein the compounds are represented by the structure shown in formula (V):

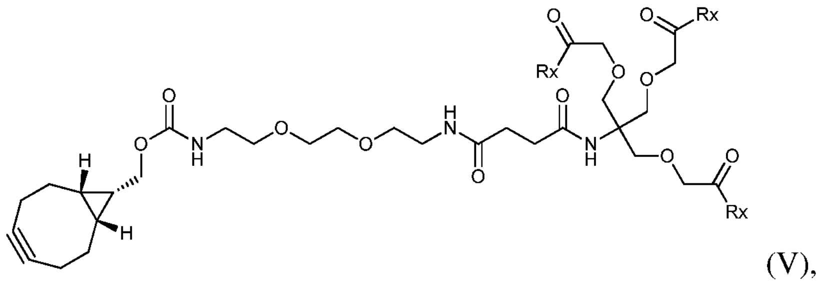

(V),

wherein Rx is

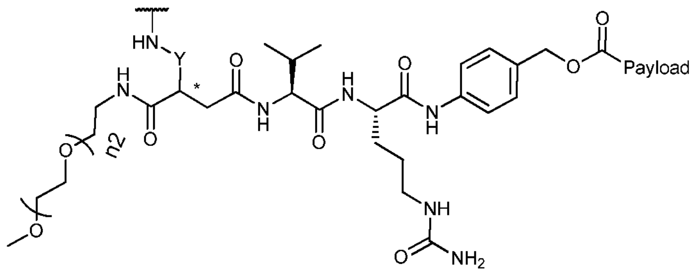

,

and n2, *, and payload are defined in the following table:

| n2 | Chirality of carbon atom at * | Y | Payload |
|---|---|---|---|
| 5 | S | bond | MMAE |
| 5 | R | bond | MMAE |
| 5 | S | L1 | MMAE |
| 5 | R | L1 | MMAE |
| 5 | S | L2 | MMAE |
| 5 | R | L2 | MMAE |
| 5 | S | L3 | MMAE |
| 5 | R | L3 | MMAE |
| 5 | S | L4 | MMAE |
| 5 | R | L4 | MMAE |
| 5 | S | L5 | MMAE |
| 5 | R | L5 | MMAE |
| 5 | S | L6 | MMAE |
| 5 | R | L6 | MMAE |
| 5 | S | L7 | MMAE |
| 5 | R | L7 | MMAE |
| 5 | S | L8 | MMAE |
| 5 | R | L8 | MMAE |
| 5 | S | L9 | MMAE |

(continued)

| n2 | Chirality of carbon atom at * | Y | Payload |
|---|---|---|---|
| 5 | R | L9 | MMAE |
| 5 | S | L10 | MMAE |
| 5 | R | L10 | MMAE |
| 5 | S | L15 | MMAE |
| 5 | R | L15 | MMAE |
| 5 | S | L20 | MMAE |
| 5 | R | L20 | MMAE |
| 5 | S | bond | Eribulin |
| 5 | R | bond | Eribulin |
| 5 | S | L1 | Eribulin |
| 5 | R | L1 | Eribulin |
| 5 | S | L2 | Eribulin |
| 5 | R | L2 | Eribulin |
| 5 | S | L3 | Eribulin |
| 5 | R | L3 | Eribulin |
| 5 | S | L4 | Eribulin |
| 5 | R | L4 | Eribulin |
| 5 | S | L5 | Eribulin |
| 5 | R | L5 | Eribulin |
| 5 | S | L6 | Eribulin |
| 5 | R | L6 | Eribulin |
| 5 | S | L7 | Eribulin |
| 5 | R | L7 | Eribulin |
| 5 | S | L8 | Eribulin |
| 5 | R | L8 | Eribulin |
| 5 | S | L9 | Eribulin |
| 5 | R | L9 | Eribulin |
| 5 | S | L10 | Eribulin |
| 5 | R | L10 | Eribulin |
| 5 | S | L15 | Eribulin |
| 5 | R | L15 | Eribulin |
| 5 | S | L20 | Eribulin |
| 5 | R | L20 | Eribulin |
| 5 | S | bond | SN-38 |
| 5 | R | bond | SN-38 |
| 5 | S | L1 | SN-38 |
| 5 | R | L1 | SN-38 |
| 5 | S | L2 | SN-38 |

(continued)

| n2 | Chirality of carbon atom at * | Y | Payload |
|---|---|---|---|
| 5 | R | L2 | SN-38 |
| 5 | S | L3 | SN-38 |
| 5 | R | L3 | SN-38 |
| 5 | S | L4 | SN-38 |
| 5 | R | L4 | SN-38 |
| 5 | S | L5 | SN-38 |
| 5 | R | L5 | SN-38 |
| 5 | S | L6 | SN-38 |
| 5 | R | L6 | SN-38 |
| 5 | S | L7 | SN-38 |
| 5 | R | L7 | SN-38 |
| 5 | S | L8 | SN-38 |
| 5 | R | L8 | SN-38 |
| 5 | S | L9 | SN-38 |
| 5 | R | L9 | SN-38 |
| 5 | S | L10 | SN-38 |
| 5 | R | L10 | SN-38 |
| 5 | S | L15 | SN-38 |
| 5 | R | L15 | SN-38 |
| 5 | S | L20 | SN-38 |
| 5 | R | L20 | SN-38 |
| 6 | S | bond | MMAE |
| 6 | R | bond | MMAE |
| 6 | S | L1 | MMAE |
| 6 | R | L1 | MMAE |
| 6 | S | L2 | MMAE |
| 6 | R | L2 | MMAE |
| 6 | S | L3 | MMAE |
| 6 | R | L3 | MMAE |
| 6 | S | L4 | MMAE |
| 6 | R | L4 | MMAE |
| 6 | S | L5 | MMAE |
| 6 | R | L5 | MMAE |
| 6 | S | L6 | MMAE |
| 6 | R | L6 | MMAE |
| 6 | S | L7 | MMAE |
| 6 | R | L7 | MMAE |
| 6 | S | L8 | MMAE |

(continued)

| n2 | Chirality of carbon atom at * | Y | Payload |
|---|---|---|---|
| 6 | R | L8 | MMAE |
| 6 | S | L9 | MMAE |
| 6 | R | L9 | MMAE |
| 6 | S | L10 | MMAE |
| 6 | R | L10 | MMAE |
| 6 | S | L15 | MMAE |
| 6 | R | L15 | MMAE |
| 6 | S | L20 | MMAE |
| 6 | R | L20 | MMAE |
| 6 | S | bond | Eribulin |
| 6 | R | bond | Eribulin |
| 6 | S | L1 | Eribulin |
| 6 | R | L1 | Eribulin |
| 6 | S | L2 | Eribulin |
| 6 | R | L2 | Eribulin |
| 6 | S | L3 | Eribulin |
| 6 | R | L3 | Eribulin |
| 6 | S | L4 | Eribulin |
| 6 | R | L4 | Eribulin |
| 6 | S | L5 | Eribulin |
| 6 | R | L5 | Eribulin |
| 6 | S | L6 | Eribulin |
| 6 | R | L6 | Eribulin |
| 6 | S | L7 | Eribulin |
| 6 | R | L7 | Eribulin |
| 6 | S | L8 | Eribulin |
| 6 | R | L8 | Eribulin |
| 6 | S | L9 | Eribulin |
| 6 | R | L9 | Eribulin |
| 6 | S | L10 | Eribulin |
| 6 | R | L10 | Eribulin |
| 6 | S | L15 | Eribulin |
| 6 | R | L15 | Eribulin |
| 6 | S | L20 | Eribulin |
| 6 | R | L20 | Eribulin |
| 6 | S | bond | SN-38 |
| 6 | R | bond | SN-38 |
| 6 | S | L1 | SN-38 |

(continued)

| n2 | Chirality of carbon atom at * | Y | Payload |
|---|---|---|---|
| 6 | R | L1 | SN-38 |
| 6 | S | L2 | SN-38 |
| 6 | R | L2 | SN-38 |
| 6 | S | L3 | SN-38 |
| 6 | R | L3 | SN-38 |
| 6 | S | L4 | SN-38 |
| 6 | R | L4 | SN-38 |
| 6 | S | L5 | SN-38 |
| 6 | R | L5 | SN-38 |
| 6 | S | L6 | SN-38 |
| 6 | R | L6 | SN-38 |
| 6 | S | L7 | SN-38 |
| 6 | R | L7 | SN-38 |
| 6 | S | L8 | SN-38 |
| 6 | R | L8 | SN-38 |
| 6 | S | L9 | SN-38 |
| 6 | R | L9 | SN-38 |
| 6 | S | L10 | SN-38 |
| 6 | R | L10 | SN-38 |
| 6 | S | L15 | SN-38 |
| 6 | R | L15 | SN-38 |
| 6 | S | L20 | SN-38 |
| 6 | R | L20 | SN-38 |
| 7 | S | bond | MMAE |
| 7 | R | bond | MMAE |
| 7 | S | L1 | MMAE |
| 7 | R | L1 | MMAE |
| 7 | S | L2 | MMAE |
| 7 | R | L2 | MMAE |
| 7 | S | L3 | MMAE |
| 7 | R | L3 | MMAE |
| 7 | S | L4 | MMAE |
| 7 | R | L4 | MMAE |
| 7 | S | L5 | MMAE |
| 7 | R | L5 | MMAE |
| 7 | S | L6 | MMAE |
| 7 | R | L6 | MMAE |
| 7 | S | L7 | MMAE |

(continued)

| n2 | Chirality of carbon atom at * | Y | Payload |
|---|---|---|---|
| 7 | R | L7 | MMAE |
| 7 | S | L8 | MMAE |
| 7 | R | L8 | MMAE |
| 7 | S | L9 | MMAE |
| 7 | R | L9 | MMAE |
| 7 | S | L10 | MMAE |
| 7 | R | L10 | MMAE |
| 7 | S | L15 | MMAE |
| 7 | R | L15 | MMAE |
| 7 | S | L20 | MMAE |
| 7 | R | L20 | MMAE |
| 7 | S | bond | Eribulin |
| 7 | R | bond | Eribulin |
| 7 | S | L1 | Eribulin |
| 7 | R | L1 | Eribulin |
| 7 | S | L2 | Eribulin |
| 7 | R | L2 | Eribulin |
| 7 | S | L3 | Eribulin |
| 7 | R | L3 | Eribulin |
| 7 | S | L4 | Eribulin |
| 7 | R | L4 | Eribulin |
| 7 | S | L5 | Eribulin |
| 7 | R | L5 | Eribulin |
| 7 | S | L6 | Eribulin |
| 7 | R | L6 | Eribulin |
| 7 | S | L7 | Eribulin |
| 7 | R | L7 | Eribulin |
| 7 | S | L8 | Eribulin |
| 7 | R | L8 | Eribulin |
| 7 | S | L9 | Eribulin |
| 7 | R | L9 | Eribulin |
| 7 | S | L10 | Eribulin |
| 7 | R | L10 | Eribulin |
| 7 | S | L15 | Eribulin |
| 7 | R | L15 | Eribulin |
| 7 | S | L20 | Eribulin |
| 7 | R | L20 | Eribulin |
| 7 | S | bond | SN-38 |

(continued)

| n2 | Chirality of carbon atom at * | Y | Payload |
|---|---|---|---|
| 7 | R | bond | SN-38 |
| 7 | S | L1 | SN-38 |
| 7 | R | L1 | SN-38 |
| 7 | S | L2 | SN-38 |
| 7 | R | L2 | SN-38 |
| 7 | S | L3 | SN-38 |
| 7 | R | L3 | SN-38 |
| 7 | S | L4 | SN-38 |
| 7 | R | L4 | SN-38 |
| 7 | S | L5 | SN-38 |
| 7 | R | L5 | SN-38 |
| 7 | S | L6 | SN-38 |
| 7 | R | L6 | SN-38 |
| 7 | S | L7 | SN-38 |
| 7 | R | L7 | SN-38 |
| 7 | S | L8 | SN-38 |
| 7 | R | L8 | SN-38 |
| 7 | S | L9 | SN-38 |
| 7 | R | L9 | SN-38 |
| 7 | S | L10 | SN-38 |
| 7 | R | L10 | SN-38 |
| 7 | S | L15 | SN-38 |
| 7 | R | L15 | SN-38 |
| 7 | S | L20 | SN-38 |
| 7 | R | L20 | SN-38 |
| 8 | S | bond | MMAE |
| 8 | R | bond | MMAE |
| 8 | S | L1 | MMAE |
| 8 | R | L1 | MMAE |
| 8 | S | L2 | MMAE |
| 8 | R | L2 | MMAE |
| 8 | S | L3 | MMAE |
| 8 | R | L3 | MMAE |
| 8 | S | L4 | MMAE |
| 8 | R | L4 | MMAE |
| 8 | S | L5 | MMAE |
| 8 | R | L5 | MMAE |
| 8 | S | L6 | MMAE |

(continued)

| n2 | Chirality of carbon atom at * | Y | Payload |
|---|---|---|---|
| 8 | R | L6 | MMAE |
| 8 | S | L7 | MMAE |
| 8 | R | L7 | MMAE |
| 8 | S | L8 | MMAE |
| 8 | R | L8 | MMAE |
| 8 | S | L9 | MMAE |
| 8 | R | L9 | MMAE |
| 8 | S | L10 | MMAE |
| 8 | R | L10 | MMAE |
| 8 | S | L15 | MMAE |
| 8 | R | L15 | MMAE |
| 8 | S | L20 | MMAE |
| 8 | R | L20 | MMAE |
| 8 | S | bond | Eribulin |
| 8 | R | bond | Eribulin |
| 8 | S | L1 | Eribulin |
| 8 | R | L1 | Eribulin |
| 8 | S | L2 | Eribulin |
| 8 | R | L2 | Eribulin |
| 8 | S | L3 | Eribulin |
| 8 | R | L3 | Eribulin |
| 8 | S | L4 | Eribulin |
| 8 | R | L4 | Eribulin |
| 8 | S | L5 | Eribulin |
| 8 | R | L5 | Eribulin |
| 8 | S | L6 | Eribulin |
| 8 | R | L6 | Eribulin |
| 8 | S | L7 | Eribulin |
| 8 | R | L7 | Eribulin |
| 8 | S | L8 | Eribulin |
| 8 | R | L8 | Eribulin |
| 8 | S | L9 | Eribulin |
| 8 | R | L9 | Eribulin |
| 8 | S | L10 | Eribulin |
| 8 | R | L10 | Eribulin |
| 8 | S | L15 | Eribulin |
| 8 | R | L15 | Eribulin |
| 8 | S | L20 | Eribulin |

(continued)

| n2 | Chirality of carbon atom at * | Y | Payload |
|---|---|---|---|
| 8 | R | L20 | Eribulin |
| 8 | S | bond | SN-38 |
| 8 | R | bond | SN-38 |
| 8 | S | L1 | SN-38 |
| 8 | R | L1 | SN-38 |
| 8 | S | L2 | SN-38 |
| 8 | R | L2 | SN-38 |
| 8 | S | L3 | SN-38 |
| 8 | R | L3 | SN-38 |
| 8 | S | L4 | SN-38 |
| 8 | R | L4 | SN-38 |
| 8 | S | L5 | SN-38 |
| 8 | R | L5 | SN-38 |
| 8 | S | L6 | SN-38 |
| 8 | R | L6 | SN-38 |
| 8 | S | L7 | SN-38 |
| 8 | R | L7 | SN-38 |
| 8 | S | L8 | SN-38 |
| 8 | R | L8 | SN-38 |
| 8 | S | L9 | SN-38 |
| 8 | R | L9 | SN-38 |
| 8 | S | L10 | SN-38 |
| 8 | R | L10 | SN-38 |
| 8 | S | L15 | SN-38 |
| 8 | R | L15 | SN-38 |
| 8 | S | L20 | SN-38 |
| 8 | R | L20 | SN-38 |
| 9 | S | bond | MMAE |
| 9 | R | bond | MMAE |
| 9 | S | L1 | MMAE |
| 9 | R | L1 | MMAE |
| 9 | S | L2 | MMAE |
| 9 | R | L2 | MMAE |
| 9 | S | L3 | MMAE |
| 9 | R | L3 | MMAE |
| 9 | S | L4 | MMAE |
| 9 | R | L4 | MMAE |
| 9 | S | L5 | MMAE |

(continued)

| n2 | Chirality of carbon atom at * | Y | Payload |
|---|---|---|---|
| 9 | R | L5 | MMAE |
| 9 | S | L6 | MMAE |
| 9 | R | L6 | MMAE |
| 9 | S | L7 | MMAE |
| 9 | R | L7 | MMAE |
| 9 | S | L8 | MMAE |
| 9 | R | L8 | MMAE |
| 9 | S | L9 | MMAE |
| 9 | R | L9 | MMAE |
| 9 | S | L10 | MMAE |
| 9 | R | L10 | MMAE |
| 9 | S | L15 | MMAE |
| 9 | R | L15 | MMAE |
| 9 | S | L20 | MMAE |
| 9 | R | L20 | MMAE |
| 9 | S | bond | Eribulin |
| 9 | R | bond | Eribulin |
| 9 | S | L1 | Eribulin |
| 9 | R | L1 | Eribulin |
| 9 | S | L2 | Eribulin |
| 9 | R | L2 | Eribulin |
| 9 | S | L3 | Eribulin |
| 9 | R | L3 | Eribulin |
| 9 | S | L4 | Eribulin |
| 9 | R | L4 | Eribulin |
| 9 | S | L5 | Eribulin |
| 9 | R | L5 | Eribulin |
| 9 | S | L6 | Eribulin |
| 9 | R | L6 | Eribulin |
| 9 | S | L7 | Eribulin |
| 9 | R | L7 | Eribulin |
| 9 | S | L8 | Eribulin |
| 9 | R | L8 | Eribulin |
| 9 | S | L9 | Eribulin |
| 9 | R | L9 | Eribulin |
| 9 | S | L10 | Eribulin |
| 9 | R | L10 | Eribulin |
| 9 | S | L15 | Eribulin |

(continued)

| n2 | Chirality of carbon atom at * | Y | Payload |
|---|---|---|---|
| 9 | R | L15 | Eribulin |
| 9 | S | L20 | Eribulin |
| 9 | R | L20 | Eribulin |
| 9 | S | bond | SN-38 |
| 9 | R | bond | SN-38 |
| 9 | S | L1 | SN-38 |
| 9 | R | L1 | SN-38 |
| 9 | S | L2 | SN-38 |
| 9 | R | L2 | SN-38 |
| 9 | S | L3 | SN-38 |
| 9 | R | L3 | SN-38 |
| 9 | S | L4 | SN-38 |
| 9 | R | L4 | SN-38 |
| 9 | S | L5 | SN-38 |
| 9 | R | L5 | SN-38 |
| 9 | S | L6 | SN-38 |
| 9 | R | L6 | SN-38 |
| 9 | S | L7 | SN-38 |
| 9 | R | L7 | SN-38 |
| 9 | S | L8 | SN-38 |
| 9 | R | L8 | SN-38 |
| 9 | S | L9 | SN-38 |
| 9 | R | L9 | SN-38 |
| 9 | S | L10 | SN-38 |
| 9 | R | L10 | SN-38 |
| 9 | S | L15 | SN-38 |
| 9 | R | L15 | SN-38 |
| 9 | S | L20 | SN-38 |
| 9 | R | L20 | SN-38 |
| 10 | S | bond | MMAE |
| 10 | R | bond | MMAE |
| 10 | S | L1 | MMAE |
| 10 | R | L1 | MMAE |
| 10 | S | L2 | MMAE |
| 10 | R | L2 | MMAE |
| 10 | S | L3 | MMAE |
| 10 | R | L3 | MMAE |
| 10 | S | L4 | MMAE |

(continued)

| n2 | Chirality of carbon atom at * | Y | Payload |
|---|---|---|---|
| 10 | R | L4 | MMAE |
| 10 | S | L5 | MMAE |
| 10 | R | L5 | MMAE |
| 10 | S | L6 | MMAE |
| 10 | R | L6 | MMAE |
| 10 | S | L7 | MMAE |
| 10 | R | L7 | MMAE |
| 10 | S | L8 | MMAE |
| 10 | R | L8 | MMAE |
| 10 | S | L9 | MMAE |
| 10 | R | L9 | MMAE |
| 10 | S | L10 | MMAE |
| 10 | R | L10 | MMAE |
| 10 | S | L15 | MMAE |
| 10 | R | L15 | MMAE |
| 10 | S | L20 | MMAE |
| 10 | R | L20 | MMAE |
| 10 | S | bond | Eribulin |
| 10 | R | bond | Eribulin |
| 10 | S | L1 | Eribulin |
| 10 | R | L1 | Eribulin |
| 10 | S | L2 | Eribulin |
| 10 | R | L2 | Eribulin |
| 10 | S | L3 | Eribulin |
| 10 | R | L3 | Eribulin |
| 10 | S | L4 | Eribulin |
| 10 | R | L4 | Eribulin |
| 10 | S | L5 | Eribulin |
| 10 | R | L5 | Eribulin |
| 10 | S | L6 | Eribulin |
| 10 | R | L6 | Eribulin |
| 10 | S | L7 | Eribulin |
| 10 | R | L7 | Eribulin |
| 10 | S | L8 | Eribulin |
| 10 | R | L8 | Eribulin |
| 10 | S | L9 | Eribulin |
| 10 | R | L9 | Eribulin |
| 10 | S | L10 | Eribulin |

(continued)

| n2 | Chirality of carbon atom at * | Y | Payload |
|---|---|---|---|
| 10 | R | L10 | Eribulin |
| 10 | S | L15 | Eribulin |
| 10 | R | L15 | Eribulin |
| 10 | S | L20 | Eribulin |
| 10 | R | L20 | Eribulin |
| 10 | S | bond | SN-38 |
| 10 | R | bond | SN-38 |
| 10 | S | L1 | SN-38 |
| 10 | R | L1 | SN-38 |
| 10 | S | L2 | SN-38 |
| 10 | R | L2 | SN-38 |
| 10 | S | L3 | SN-38 |
| 10 | R | L3 | SN-38 |
| 10 | S | L4 | SN-38 |
| 10 | R | L4 | SN-38 |
| 10 | S | L5 | SN-38 |
| 10 | R | L5 | SN-38 |
| 10 | S | L6 | SN-38 |
| 10 | R | L6 | SN-38 |
| 10 | S | L7 | SN-38 |
| 10 | R | L7 | SN-38 |
| 10 | S | L8 | SN-38 |
| 10 | R | L8 | SN-38 |
| 10 | S | L9 | SN-38 |
| 10 | R | L9 | SN-38 |
| 10 | S | L10 | SN-38 |
| 10 | R | L10 | SN-38 |
| 10 | S | L15 | SN-38 |
| 10 | R | L15 | SN-38 |
| 10 | S | L20 | SN-38 |
| 10 | R | L20 | SN-38 |
| 11 | S | bond | MMAE |
| 11 | R | bond | MMAE |
| 11 | S | L1 | MMAE |
| 11 | R | L1 | MMAE |
| 11 | S | L2 | MMAE |
| 11 | R | L2 | MMAE |
| 11 | S | L3 | MMAE |

(continued)

| n2 | Chirality of carbon atom at * | Y | Payload |
|---|---|---|---|
| 11 | R | L3 | MMAE |
| 11 | S | L4 | MMAE |
| 11 | R | L4 | MMAE |
| 11 | S | L5 | MMAE |
| 11 | R | L5 | MMAE |
| 11 | S | L6 | MMAE |
| 11 | R | L6 | MMAE |
| 11 | S | L7 | MMAE |
| 11 | R | L7 | MMAE |
| 11 | S | L8 | MMAE |
| 11 | R | L8 | MMAE |
| 11 | S | L9 | MMAE |
| 11 | R | L9 | MMAE |
| 11 | S | L10 | MMAE |
| 11 | R | L10 | MMAE |
| 11 | S | L15 | MMAE |
| 11 | R | L15 | MMAE |
| 11 | S | L20 | MMAE |
| 11 | R | L20 | MMAE |
| 11 | S | bond | Eribulin |
| 11 | R | bond | Eribulin |
| 11 | S | L1 | Eribulin |
| 11 | R | L1 | Eribulin |
| 11 | S | L2 | Eribulin |
| 11 | R | L2 | Eribulin |
| 11 | S | L3 | Eribulin |
| 11 | R | L3 | Eribulin |
| 11 | S | L4 | Eribulin |
| 11 | R | L4 | Eribulin |
| 11 | S | L5 | Eribulin |
| 11 | R | L5 | Eribulin |
| 11 | S | L6 | Eribulin |
| 11 | R | L6 | Eribulin |
| 11 | S | L7 | Eribulin |
| 11 | R | L7 | Eribulin |
| 11 | S | L8 | Eribulin |
| 11 | R | L8 | Eribulin |
| 11 | S | L9 | Eribulin |

(continued)

| n2 | Chirality of carbon atom at * | Y | Payload |
|---|---|---|---|
| 11 | R | L9 | Eribulin |
| 11 | S | L10 | Eribulin |
| 11 | R | L10 | Eribulin |
| 11 | S | L15 | Eribulin |
| 11 | R | L15 | Eribulin |
| 11 | S | L20 | Eribulin |
| 11 | R | L20 | Eribulin |
| 11 | S | bond | SN-38 |
| 11 | R | bond | SN-38 |
| 11 | S | L1 | SN-38 |
| 11 | R | L1 | SN-38 |
| 11 | S | L2 | SN-38 |
| 11 | R | L2 | SN-38 |
| 11 | S | L3 | SN-38 |
| 11 | R | L3 | SN-38 |
| 11 | S | L4 | SN-38 |
| 11 | R | L4 | SN-38 |
| 11 | S | L5 | SN-38 |
| 11 | R | L5 | SN-38 |
| 11 | S | L6 | SN-38 |
| 11 | R | L6 | SN-38 |
| 11 | S | L7 | SN-38 |
| 11 | R | L7 | SN-38 |
| 11 | S | L8 | SN-38 |
| 11 | R | L8 | SN-38 |
| 11 | S | L9 | SN-38 |
| 11 | R | L9 | SN-38 |
| 11 | S | L10 | SN-38 |
| 11 | R | L10 | SN-38 |
| 11 | S | L15 | SN-38 |
| 11 | R | L15 | SN-38 |
| 11 | S | L20 | SN-38 |
| 11 | R | L20 | SN-38 |
| 12 | S | bond | MMAE |
| 12 | R | bond | MMAE |
| 12 | S | L1 | MMAE |
| 12 | R | L1 | MMAE |
| 12 | S | L2 | MMAE |

(continued)

| n2 | Chirality of carbon atom at * | Y | Payload |
|---|---|---|---|
| 12 | R | L2 | MMAE |
| 12 | S | L3 | MMAE |
| 12 | R | L3 | MMAE |
| 12 | S | L4 | MMAE |
| 12 | R | L4 | MMAE |
| 12 | S | L5 | MMAE |
| 12 | R | L5 | MMAE |
| 12 | S | L6 | MMAE |
| 12 | R | L6 | MMAE |
| 12 | S | L7 | MMAE |
| 12 | R | L7 | MMAE |
| 12 | S | L8 | MMAE |
| 12 | R | L8 | MMAE |
| 12 | S | L9 | MMAE |
| 12 | R | L9 | MMAE |
| 12 | S | L10 | MMAE |
| 12 | R | L10 | MMAE |
| 12 | S | L15 | MMAE |
| 12 | R | L15 | MMAE |
| 12 | S | L20 | MMAE |
| 12 | R | L20 | MMAE |
| 12 | S | bond | Eribulin |
| 12 | R | bond | Eribulin |
| 12 | S | L1 | Eribulin |
| 12 | R | L1 | Eribulin |
| 12 | S | L2 | Eribulin |
| 12 | R | L2 | Eribulin |
| 12 | S | L3 | Eribulin |
| 12 | R | L3 | Eribulin |
| 12 | S | L4 | Eribulin |
| 12 | R | L4 | Eribulin |
| 12 | S | L5 | Eribulin |
| 12 | R | L5 | Eribulin |
| 12 | S | L6 | Eribulin |
| 12 | R | L6 | Eribulin |
| 12 | S | L7 | Eribulin |
| 12 | R | L7 | Eribulin |
| 12 | S | L8 | Eribulin |

(continued)

| n2 | Chirality of carbon atom at * | Y | Payload |
|---|---|---|---|
| 12 | R | L8 | Eribulin |
| 12 | S | L9 | Eribulin |
| 12 | R | L9 | Eribulin |
| 12 | S | L10 | Eribulin |
| 12 | R | L10 | Eribulin |
| 12 | S | L15 | Eribulin |
| 12 | R | L15 | Eribulin |
| 12 | S | L20 | Eribulin |
| 12 | R | L20 | Eribulin |
| 12 | S | bond | SN-38 |
| 12 | R | bond | SN-38 |
| 12 | S | L1 | SN-38 |
| 12 | R | L1 | SN-38 |
| 12 | S | L2 | SN-38 |
| 12 | R | L2 | SN-38 |
| 12 | S | L3 | SN-38 |
| 12 | R | L3 | SN-38 |
| 12 | S | L4 | SN-38 |
| 12 | R | L4 | SN-38 |
| 12 | S | L5 | SN-38 |
| 12 | R | L5 | SN-38 |
| 12 | S | L6 | SN-38 |
| 12 | R | L6 | SN-38 |
| 12 | S | L7 | SN-38 |
| 12 | R | L7 | SN-38 |
| 12 | S | L8 | SN-38 |
| 12 | R | L8 | SN-38 |
| 12 | S | L9 | SN-38 |
| 12 | R | L9 | SN-38 |
| 12 | S | L10 | SN-38 |
| 12 | R | L10 | SN-38 |
| 12 | S | L15 | SN-38 |
| 12 | R | L15 | SN-38 |
| 12 | S | L20 | SN-38 |
| 12 | R | L20 | SN-38 |
| 13 | S | bond | MMAE |
| 13 | R | bond | MMAE |
| 13 | S | L1 | MMAE |

(continued)

| n2 | Chirality of carbon atom at * | Y | Payload |
|---|---|---|---|
| 13 | R | L1 | MMAE |
| 13 | S | L2 | MMAE |
| 13 | R | L2 | MMAE |
| 13 | S | L3 | MMAE |
| 13 | R | L3 | MMAE |
| 13 | S | L4 | MMAE |
| 13 | R | L4 | MMAE |
| 13 | S | L5 | MMAE |
| 13 | R | L5 | MMAE |
| 13 | S | L6 | MMAE |
| 13 | R | L6 | MMAE |
| 13 | S | L7 | MMAE |
| 13 | R | L7 | MMAE |
| 13 | S | L8 | MMAE |
| 13 | R | L8 | MMAE |
| 13 | S | L9 | MMAE |
| 13 | R | L9 | MMAE |
| 13 | S | L10 | MMAE |
| 13 | R | L10 | MMAE |
| 13 | S | L15 | MMAE |
| 13 | R | L15 | MMAE |
| 13 | S | L20 | MMAE |
| 13 | R | L20 | MMAE |
| 13 | S | bond | Eribulin |
| 13 | R | bond | Eribulin |
| 13 | S | L1 | Eribulin |
| 13 | R | L1 | Eribulin |
| 13 | S | L2 | Eribulin |
| 13 | R | L2 | Eribulin |
| 13 | S | L3 | Eribulin |
| 13 | R | L3 | Eribulin |
| 13 | S | L4 | Eribulin |
| 13 | R | L4 | Eribulin |
| 13 | S | L5 | Eribulin |
| 13 | R | L5 | Eribulin |
| 13 | S | L6 | Eribulin |
| 13 | R | L6 | Eribulin |
| 13 | S | L7 | Eribulin |

(continued)

| n2 | Chirality of carbon atom at * | Y | Payload |
|---|---|---|---|
| 13 | R | L7 | Eribulin |
| 13 | S | L8 | Eribulin |
| 13 | R | L8 | Eribulin |
| 13 | S | L9 | Eribulin |
| 13 | R | L9 | Eribulin |
| 13 | S | L10 | Eribulin |
| 13 | R | L10 | Eribulin |
| 13 | S | L15 | Eribulin |
| 13 | R | L15 | Eribulin |
| 13 | S | L20 | Eribulin |
| 13 | R | L20 | Eribulin |
| 13 | S | bond | SN-38 |
| 13 | R | bond | SN-38 |
| 13 | S | L1 | SN-38 |
| 13 | R | L1 | SN-38 |
| 13 | S | L2 | SN-38 |
| 13 | R | L2 | SN-38 |
| 13 | S | L3 | SN-38 |
| 13 | R | L3 | SN-38 |
| 13 | S | L4 | SN-38 |
| 13 | R | L4 | SN-38 |
| 13 | S | L5 | SN-38 |
| 13 | R | L5 | SN-38 |
| 13 | S | L6 | SN-38 |
| 13 | R | L6 | SN-38 |
| 13 | S | L7 | SN-38 |
| 13 | R | L7 | SN-38 |
| 13 | S | L8 | SN-38 |
| 13 | R | L8 | SN-38 |
| 13 | S | L9 | SN-38 |
| 13 | R | L9 | SN-38 |
| 13 | S | L10 | SN-38 |
| 13 | R | L10 | SN-38 |
| 13 | S | L15 | SN-38 |
| 13 | R | L15 | SN-38 |
| 13 | S | L20 | SN-38 |
| 13 | R | L20 | SN-38 |
| 14 | S | bond | MMAE |

(continued)

| n2 | Chirality of carbon atom at * | Y | Payload |
|---|---|---|---|
| 14 | R | bond | MMAE |
| 14 | S | L1 | MMAE |
| 14 | R | L1 | MMAE |
| 14 | S | L2 | MMAE |
| 14 | R | L2 | MMAE |
| 14 | S | L3 | MMAE |
| 14 | R | L3 | MMAE |
| 14 | S | L4 | MMAE |
| 14 | R | L4 | MMAE |
| 14 | S | L5 | MMAE |
| 14 | R | L5 | MMAE |
| 14 | S | L6 | MMAE |
| 14 | R | L6 | MMAE |
| 14 | S | L7 | MMAE |
| 14 | R | L7 | MMAE |
| 14 | S | L8 | MMAE |
| 14 | R | L8 | MMAE |
| 14 | S | L9 | MMAE |
| 14 | R | L9 | MMAE |
| 14 | S | L10 | MMAE |
| 14 | R | L10 | MMAE |
| 14 | S | L15 | MMAE |
| 14 | R | L15 | MMAE |
| 14 | S | L20 | MMAE |
| 14 | R | L20 | MMAE |
| 14 | S | bond | Eribulin |
| 14 | R | bond | Eribulin |
| 14 | S | L1 | Eribulin |
| 14 | R | L1 | Eribulin |
| 14 | S | L2 | Eribulin |
| 14 | R | L2 | Eribulin |
| 14 | S | L3 | Eribulin |
| 14 | R | L3 | Eribulin |
| 14 | S | L4 | Eribulin |
| 14 | R | L4 | Eribulin |
| 14 | S | L5 | Eribulin |
| 14 | R | L5 | Eribulin |
| 14 | S | L6 | Eribulin |

(continued)

| n2 | Chirality of carbon atom at * | Y | Payload |
|---|---|---|---|
| 14 | R | L6 | Eribulin |
| 14 | S | L7 | Eribulin |
| 14 | R | L7 | Eribulin |
| 14 | S | L8 | Eribulin |
| 14 | R | L8 | Eribulin |
| 14 | S | L9 | Eribulin |
| 14 | R | L9 | Eribulin |
| 14 | S | L10 | Eribulin |
| 14 | R | L10 | Eribulin |
| 14 | S | L15 | Eribulin |
| 14 | R | L15 | Eribulin |
| 14 | S | L20 | Eribulin |
| 14 | R | L20 | Eribulin |
| 14 | S | bond | SN-38 |
| 14 | R | bond | SN-38 |
| 14 | S | L1 | SN-38 |
| 14 | R | L1 | SN-38 |
| 14 | S | L2 | SN-38 |
| 14 | R | L2 | SN-38 |
| 14 | S | L3 | SN-38 |
| 14 | R | L3 | SN-38 |
| 14 | S | L4 | SN-38 |
| 14 | R | L4 | SN-38 |
| 14 | S | L5 | SN-38 |
| 14 | R | L5 | SN-38 |
| 14 | S | L6 | SN-38 |
| 14 | R | L6 | SN-38 |
| 14 | S | L7 | SN-38 |
| 14 | R | L7 | SN-38 |
| 14 | S | L8 | SN-38 |
| 14 | R | L8 | SN-38 |
| 14 | S | L9 | SN-38 |
| 14 | R | L9 | SN-38 |
| 14 | S | L10 | SN-38 |
| 14 | R | L10 | SN-38 |
| 14 | S | L15 | SN-38 |
| 14 | R | L15 | SN-38 |
| 14 | S | L20 | SN-38 |

(continued)

| n2 | Chirality of carbon atom at * | Y | Payload |
|---|---|---|---|
| 14 | R | L20 | SN-38 |
| 15 | S | bond | MMAE |
| 15 | R | bond | MMAE |
| 15 | S | L1 | MMAE |
| 15 | R | L1 | MMAE |
| 15 | S | L2 | MMAE |
| 15 | R | L2 | MMAE |
| 15 | S | L3 | MMAE |
| 15 | R | L3 | MMAE |
| 15 | S | L4 | MMAE |
| 15 | R | L4 | MMAE |
| 15 | S | L5 | MMAE |
| 15 | R | L5 | MMAE |
| 15 | S | L6 | MMAE |
| 15 | R | L6 | MMAE |
| 15 | S | L7 | MMAE |
| 15 | R | L7 | MMAE |
| 15 | S | L8 | MMAE |
| 15 | R | L8 | MMAE |
| 15 | S | L9 | MMAE |
| 15 | R | L9 | MMAE |
| 15 | S | L10 | MMAE |
| 15 | R | L10 | MMAE |
| 15 | S | L15 | MMAE |
| 15 | R | L15 | MMAE |
| 15 | S | L20 | MMAE |
| 15 | R | L20 | MMAE |
| 15 | S | bond | Eribulin |
| 15 | R | bond | Eribulin |
| 15 | S | L1 | Eribulin |
| 15 | R | L1 | Eribulin |
| 15 | S | L2 | Eribulin |
| 15 | R | L2 | Eribulin |
| 15 | S | L3 | Eribulin |
| 15 | R | L3 | Eribulin |
| 15 | S | L4 | Eribulin |
| 15 | R | L4 | Eribulin |
| 15 | S | L5 | Eribulin |

(continued)

| n2 | Chirality of carbon atom at * | Y | Payload |
|---|---|---|---|
| 15 | R | L5 | Eribulin |
| 15 | S | L6 | Eribulin |
| 15 | R | L6 | Eribulin |
| 15 | S | L7 | Eribulin |
| 15 | R | L7 | Eribulin |
| 15 | S | L8 | Eribulin |
| 15 | R | L8 | Eribulin |
| 15 | S | L9 | Eribulin |
| 15 | R | L9 | Eribulin |
| 15 | S | L10 | Eribulin |
| 15 | R | L10 | Eribulin |
| 15 | S | L15 | Eribulin |
| 15 | R | L15 | Eribulin |
| 15 | S | L20 | Eribulin |
| 15 | R | L20 | Eribulin |
| 15 | S | bond | SN-38 |
| 15 | R | bond | SN-38 |
| 15 | S | L1 | SN-38 |
| 15 | R | L1 | SN-38 |
| 15 | S | L2 | SN-38 |
| 15 | R | L2 | SN-38 |
| 15 | S | L3 | SN-38 |
| 15 | R | L3 | SN-38 |
| 15 | S | L4 | SN-38 |
| 15 | R | L4 | SN-38 |
| 15 | S | L5 | SN-38 |
| 15 | R | L5 | SN-38 |
| 15 | S | L6 | SN-38 |
| 15 | R | L6 | SN-38 |
| 15 | S | L7 | SN-38 |
| 15 | R | L7 | SN-38 |
| 15 | S | L8 | SN-38 |
| 15 | R | L8 | SN-38 |
| 15 | S | L9 | SN-38 |
| 15 | R | L9 | SN-38 |
| 15 | S | L10 | SN-38 |
| 15 | R | L10 | SN-38 |
| 15 | S | L15 | SN-38 |

(continued)

| n2 | Chirality of carbon atom at * | Y | Payload |
|---|---|---|---|
| 15 | R | L15 | SN-38 |
| 15 | S | L20 | SN-38 |
| 15 | R | L20 | SN-38 |
| 16 | S | bond | MMAE |
| 16 | R | bond | MMAE |
| 16 | S | L1 | MMAE |
| 16 | R | L1 | MMAE |
| 16 | S | L2 | MMAE |
| 16 | R | L2 | MMAE |
| 16 | S | L3 | MMAE |
| 16 | R | L3 | MMAE |
| 16 | S | L4 | MMAE |
| 16 | R | L4 | MMAE |
| 16 | S | L5 | MMAE |
| 16 | R | L5 | MMAE |
| 16 | S | L6 | MMAE |
| 16 | R | L6 | MMAE |
| 16 | S | L7 | MMAE |
| 16 | R | L7 | MMAE |
| 16 | S | L8 | MMAE |
| 16 | R | L8 | MMAE |
| 16 | S | L9 | MMAE |
| 16 | R | L9 | MMAE |
| 16 | S | L10 | MMAE |
| 16 | R | L10 | MMAE |
| 16 | S | L15 | MMAE |
| 16 | R | L15 | MMAE |
| 16 | S | L20 | MMAE |
| 16 | R | L20 | MMAE |
| 16 | S | bond | Eribulin |
| 16 | R | bond | Eribulin |
| 16 | S | L1 | Eribulin |
| 16 | R | L1 | Eribulin |
| 16 | S | L2 | Eribulin |
| 16 | R | L2 | Eribulin |
| 16 | S | L3 | Eribulin |
| 16 | R | L3 | Eribulin |
| 16 | S | L4 | Eribulin |

(continued)

| n2 | Chirality of carbon atom at * | Y | Payload |
|---|---|---|---|
| 16 | R | L4 | Eribulin |
| 16 | S | L5 | Eribulin |
| 16 | R | L5 | Eribulin |
| 16 | S | L6 | Eribulin |
| 16 | R | L6 | Eribulin |
| 16 | S | L7 | Eribulin |
| 16 | R | L7 | Eribulin |
| 16 | S | L8 | Eribulin |
| 16 | R | L8 | Eribulin |
| 16 | S | L9 | Eribulin |
| 16 | R | L9 | Eribulin |
| 16 | S | L10 | Eribulin |
| 16 | R | L10 | Eribulin |
| 16 | S | L15 | Eribulin |
| 16 | R | L15 | Eribulin |
| 16 | S | L20 | Eribulin |
| 16 | R | L20 | Eribulin |
| 16 | S | bond | SN-38 |
| 16 | R | bond | SN-38 |
| 16 | S | L1 | SN-38 |
| 16 | R | L1 | SN-38 |
| 16 | S | L2 | SN-38 |
| 16 | R | L2 | SN-38 |
| 16 | S | L3 | SN-38 |
| 16 | R | L3 | SN-38 |
| 16 | S | L4 | SN-38 |
| 16 | R | L4 | SN-38 |
| 16 | S | L5 | SN-38 |
| 16 | R | L5 | SN-38 |
| 16 | S | L6 | SN-38 |
| 16 | R | L6 | SN-38 |
| 16 | S | L7 | SN-38 |
| 16 | R | L7 | SN-38 |
| 16 | S | L8 | SN-38 |
| 16 | R | L8 | SN-38 |
| 16 | S | L9 | SN-38 |
| 16 | R | L9 | SN-38 |
| 16 | S | L10 | SN-38 |

(continued)

| n2 | Chirality of carbon atom at * | Y | Payload |
|---|---|---|---|
| 16 | R | L10 | SN-38 |
| 16 | S | L15 | SN-38 |
| 16 | R | L15 | SN-38 |
| 16 | S | L20 | SN-38 |
| 16 | R | L20 | SN-38 |
| 17 | S | bond | MMAE |
| 17 | R | bond | MMAE |
| 17 | S | L1 | MMAE |
| 17 | R | L1 | MMAE |
| 17 | S | L2 | MMAE |
| 17 | R | L2 | MMAE |
| 17 | S | L3 | MMAE |
| 17 | R | L3 | MMAE |
| 17 | S | L4 | MMAE |
| 17 | R | L4 | MMAE |
| 17 | S | L5 | MMAE |
| 17 | R | L5 | MMAE |
| 17 | S | L6 | MMAE |
| 17 | R | L6 | MMAE |
| 17 | S | L7 | MMAE |
| 17 | R | L7 | MMAE |
| 17 | S | L8 | MMAE |
| 17 | R | L8 | MMAE |
| 17 | S | L9 | MMAE |
| 17 | R | L9 | MMAE |
| 17 | S | L10 | MMAE |
| 17 | R | L10 | MMAE |
| 17 | S | L15 | MMAE |
| 17 | R | L15 | MMAE |
| 17 | S | L20 | MMAE |
| 17 | R | L20 | MMAE |
| 17 | S | bond | Eribulin |
| 17 | R | bond | Eribulin |
| 17 | S | L1 | Eribulin |
| 17 | R | L1 | Eribulin |
| 17 | S | L2 | Eribulin |
| 17 | R | L2 | Eribulin |
| 17 | S | L3 | Eribulin |

(continued)

| n2 | Chirality of carbon atom at * | Y | Payload |
|---|---|---|---|
| 17 | R | L3 | Eribulin |
| 17 | S | L4 | Eribulin |
| 17 | R | L4 | Eribulin |
| 17 | S | L5 | Eribulin |
| 17 | R | L5 | Eribulin |
| 17 | S | L6 | Eribulin |
| 17 | R | L6 | Eribulin |
| 17 | S | L7 | Eribulin |
| 17 | R | L7 | Eribulin |
| 17 | S | L8 | Eribulin |
| 17 | R | L8 | Eribulin |
| 17 | S | L9 | Eribulin |
| 17 | R | L9 | Eribulin |
| 17 | S | L10 | Eribulin |
| 17 | R | L10 | Eribulin |
| 17 | S | L15 | Eribulin |
| 17 | R | L15 | Eribulin |
| 17 | S | L20 | Eribulin |
| 17 | R | L20 | Eribulin |
| 17 | S | bond | SN-38 |
| 17 | R | bond | SN-38 |
| 17 | S | L1 | SN-38 |
| 17 | R | L1 | SN-38 |
| 17 | S | L2 | SN-38 |
| 17 | R | L2 | SN-38 |
| 17 | S | L3 | SN-38 |
| 17 | R | L3 | SN-38 |
| 17 | S | L4 | SN-38 |
| 17 | R | L4 | SN-38 |
| 17 | S | L5 | SN-38 |
| 17 | R | L5 | SN-38 |
| 17 | S | L6 | SN-38 |
| 17 | R | L6 | SN-38 |
| 17 | S | L7 | SN-38 |
| 17 | R | L7 | SN-38 |
| 17 | S | L8 | SN-38 |
| 17 | R | L8 | SN-38 |
| 17 | S | L9 | SN-38 |

(continued)

| n2 | Chirality of carbon atom at * | Y | Payload |
|---|---|---|---|
| 17 | R | L9 | SN-38 |
| 17 | S | L10 | SN-38 |
| 17 | R | L10 | SN-38 |
| 17 | S | L15 | SN-38 |
| 17 | R | L15 | SN-38 |
| 17 | S | L20 | SN-38 |
| 17 | R | L20 | SN-38 |
| 18 | S | bond | MMAE |
| 18 | R | bond | MMAE |
| 18 | S | L1 | MMAE |
| 18 | R | L1 | MMAE |
| 18 | S | L2 | MMAE |
| 18 | R | L2 | MMAE |
| 18 | S | L3 | MMAE |
| 18 | R | L3 | MMAE |
| 18 | S | L4 | MMAE |
| 18 | R | L4 | MMAE |
| 18 | S | L5 | MMAE |
| 18 | R | L5 | MMAE |
| 18 | S | L6 | MMAE |
| 18 | R | L6 | MMAE |
| 18 | S | L7 | MMAE |
| 18 | R | L7 | MMAE |
| 18 | S | L8 | MMAE |
| 18 | R | L8 | MMAE |
| 18 | S | L9 | MMAE |
| 18 | R | L9 | MMAE |
| 18 | S | L10 | MMAE |
| 18 | R | L10 | MMAE |
| 18 | S | L15 | MMAE |
| 18 | R | L15 | MMAE |
| 18 | S | L20 | MMAE |
| 18 | R | L20 | MMAE |
| 18 | S | bond | Eribulin |
| 18 | R | bond | Eribulin |
| 18 | S | L1 | Eribulin |
| 18 | R | L1 | Eribulin |
| 18 | S | L2 | Eribulin |

(continued)

| n2 | Chirality of carbon atom at * | Y | Payload |
|---|---|---|---|
| 18 | R | L2 | Eribulin |
| 18 | S | L3 | Eribulin |
| 18 | R | L3 | Eribulin |
| 18 | S | L4 | Eribulin |
| 18 | R | L4 | Eribulin |
| 18 | S | L5 | Eribulin |
| 18 | R | L5 | Eribulin |
| 18 | S | L6 | Eribulin |
| 18 | R | L6 | Eribulin |
| 18 | S | L7 | Eribulin |
| 18 | R | L7 | Eribulin |
| 18 | S | L8 | Eribulin |
| 18 | R | L8 | Eribulin |
| 18 | S | L9 | Eribulin |
| 18 | R | L9 | Eribulin |
| 18 | S | L10 | Eribulin |
| 18 | R | L10 | Eribulin |
| 18 | S | L15 | Eribulin |
| 18 | R | L15 | Eribulin |
| 18 | S | L20 | Eribulin |
| 18 | R | L20 | Eribulin |
| 18 | S | bond | SN-38 |
| 18 | R | bond | SN-38 |
| 18 | S | L1 | SN-38 |
| 18 | R | L1 | SN-38 |
| 18 | S | L2 | SN-38 |
| 18 | R | L2 | SN-38 |
| 18 | S | L3 | SN-38 |
| 18 | R | L3 | SN-38 |
| 18 | S | L4 | SN-38 |
| 18 | R | L4 | SN-38 |
| 18 | S | L5 | SN-38 |
| 18 | R | L5 | SN-38 |
| 18 | S | L6 | SN-38 |
| 18 | R | L6 | SN-38 |
| 18 | S | L7 | SN-38 |
| 18 | R | L7 | SN-38 |
| 18 | S | L8 | SN-38 |

(continued)

| n2 | Chirality of carbon atom at * | Y | Payload |
|---|---|---|---|
| 18 | R | L8 | SN-38 |
| 18 | S | L9 | SN-38 |
| 18 | R | L9 | SN-38 |
| 18 | S | L10 | SN-38 |
| 18 | R | L10 | SN-38 |
| 18 | S | L15 | SN-38 |
| 18 | R | L15 | SN-38 |
| 18 | S | L20 | SN-38 |
| 18 | R | L20 | SN-38 |
| 19 | S | bond | MMAE |
| 19 | R | bond | MMAE |
| 19 | S | L1 | MMAE |
| 19 | R | L1 | MMAE |
| 19 | S | L2 | MMAE |
| 19 | R | L2 | MMAE |
| 19 | S | L3 | MMAE |
| 19 | R | L3 | MMAE |
| 19 | S | L4 | MMAE |
| 19 | R | L4 | MMAE |
| 19 | S | L5 | MMAE |
| 19 | R | L5 | MMAE |
| 19 | S | L6 | MMAE |
| 19 | R | L6 | MMAE |
| 19 | S | L7 | MMAE |
| 19 | R | L7 | MMAE |
| 19 | S | L8 | MMAE |
| 19 | R | L8 | MMAE |
| 19 | S | L9 | MMAE |
| 19 | R | L9 | MMAE |
| 19 | S | L10 | MMAE |
| 19 | R | L10 | MMAE |
| 19 | S | L15 | MMAE |
| 19 | R | L15 | MMAE |
| 19 | S | L20 | MMAE |
| 19 | R | L20 | MMAE |
| 19 | S | bond | Eribulin |
| 19 | R | bond | Eribulin |
| 19 | S | L1 | Eribulin |

(continued)

| n2 | Chirality of carbon atom at * | Y | Payload |
|---|---|---|---|
| 19 | R | L1 | Eribulin |
| 19 | S | L2 | Eribulin |
| 19 | R | L2 | Eribulin |
| 19 | S | L3 | Eribulin |
| 19 | R | L3 | Eribulin |
| 19 | S | L4 | Eribulin |
| 19 | R | L4 | Eribulin |
| 19 | S | L5 | Eribulin |
| 19 | R | L5 | Eribulin |
| 19 | S | L6 | Eribulin |
| 19 | R | L6 | Eribulin |
| 19 | S | L7 | Eribulin |
| 19 | R | L7 | Eribulin |
| 19 | S | L8 | Eribulin |
| 19 | R | L8 | Eribulin |
| 19 | S | L9 | Eribulin |
| 19 | R | L9 | Eribulin |
| 19 | S | L10 | Eribulin |
| 19 | R | L10 | Eribulin |
| 19 | S | L15 | Eribulin |
| 19 | R | L15 | Eribulin |
| 19 | S | L20 | Eribulin |
| 19 | R | L20 | Eribulin |
| 19 | S | bond | SN-38 |
| 19 | R | bond | SN-38 |
| 19 | S | L1 | SN-38 |
| 19 | R | L1 | SN-38 |
| 19 | S | L2 | SN-38 |
| 19 | R | L2 | SN-38 |
| 19 | S | L3 | SN-38 |
| 19 | R | L3 | SN-38 |
| 19 | S | L4 | SN-38 |
| 19 | R | L4 | SN-38 |
| 19 | S | L5 | SN-38 |
| 19 | R | L5 | SN-38 |
| 19 | S | L6 | SN-38 |
| 19 | R | L6 | SN-38 |
| 19 | S | L7 | SN-38 |

(continued)

| n2 | Chirality of carbon atom at * | Y | Payload |
|---|---|---|---|
| 19 | R | L7 | SN-38 |
| 19 | S | L8 | SN-38 |
| 19 | R | L8 | SN-38 |
| 19 | S | L9 | SN-38 |
| 19 | R | L9 | SN-38 |
| 19 | S | L10 | SN-38 |
| 19 | R | L10 | SN-38 |
| 19 | S | L15 | SN-38 |
| 19 | R | L15 | SN-38 |
| 19 | S | L20 | SN-38 |
| 19 | R | L20 | SN-38 |
| 20 | S | bond | MMAE |
| 20 | R | bond | MMAE |
| 20 | S | L1 | MMAE |
| 20 | R | L1 | MMAE |
| 20 | S | L2 | MMAE |
| 20 | R | L2 | MMAE |
| 20 | S | L3 | MMAE |
| 20 | R | L3 | MMAE |
| 20 | S | L4 | MMAE |
| 20 | R | L4 | MMAE |
| 20 | S | L5 | MMAE |
| 20 | R | L5 | MMAE |
| 20 | S | L6 | MMAE |
| 20 | R | L6 | MMAE |
| 20 | S | L7 | MMAE |
| 20 | R | L7 | MMAE |
| 20 | S | L8 | MMAE |
| 20 | R | L8 | MMAE |
| 20 | S | L9 | MMAE |
| 20 | R | L9 | MMAE |
| 20 | S | L10 | MMAE |
| 20 | R | L10 | MMAE |
| 20 | S | L15 | MMAE |
| 20 | R | L15 | MMAE |
| 20 | S | L20 | MMAE |
| 20 | R | L20 | MMAE |
| 20 | S | bond | Eribulin |

(continued)

| n2 | Chirality of carbon atom at * | Y | Payload |
|---|---|---|---|
| 20 | R | bond | Eribulin |
| 20 | S | L1 | Eribulin |
| 20 | R | L1 | Eribulin |
| 20 | S | L2 | Eribulin |
| 20 | R | L2 | Eribulin |
| 20 | S | L3 | Eribulin |
| 20 | R | L3 | Eribulin |
| 20 | S | L4 | Eribulin |
| 20 | R | L4 | Eribulin |
| 20 | S | L5 | Eribulin |
| 20 | R | L5 | Eribulin |
| 20 | S | L6 | Eribulin |
| 20 | R | L6 | Eribulin |
| 20 | S | L7 | Eribulin |
| 20 | R | L7 | Eribulin |
| 20 | S | L8 | Eribulin |
| 20 | R | L8 | Eribulin |
| 20 | S | L9 | Eribulin |
| 20 | R | L9 | Eribulin |
| 20 | S | L10 | Eribulin |
| 20 | R | L10 | Eribulin |
| 20 | S | L15 | Eribulin |
| 20 | R | L15 | Eribulin |
| 20 | S | L20 | Eribulin |
| 20 | R | L20 | Eribulin |
| 20 | S | bond | SN-38 |
| 20 | R | bond | SN-38 |
| 20 | S | L1 | SN-38 |
| 20 | R | L1 | SN-38 |
| 20 | S | L2 | SN-38 |
| 20 | R | L2 | SN-38 |
| 20 | S | L3 | SN-38 |
| 20 | R | L3 | SN-38 |
| 20 | S | L4 | SN-38 |
| 20 | R | L4 | SN-38 |
| 20 | S | L5 | SN-38 |
| 20 | R | L5 | SN-38 |
| 20 | S | L6 | SN-38 |

(continued)

| n2 | Chirality of carbon atom at * | Y | Payload |
|---|---|---|---|
| 20 | R | L6 | SN-38 |
| 20 | S | L7 | SN-38 |
| 20 | R | L7 | SN-38 |
| 20 | S | L8 | SN-38 |
| 20 | R | L8 | SN-38 |
| 20 | S | L9 | SN-38 |
| 20 | R | L9 | SN-38 |
| 20 | S | L10 | SN-38 |
| 20 | R | L10 | SN-38 |
| 20 | S | L15 | SN-38 |
| 20 | R | L15 | SN-38 |
| 20 | S | L20 | SN-38 |
| 20 | R | L20 | SN-38. |

The following compounds are still alternative: wherein Rx is

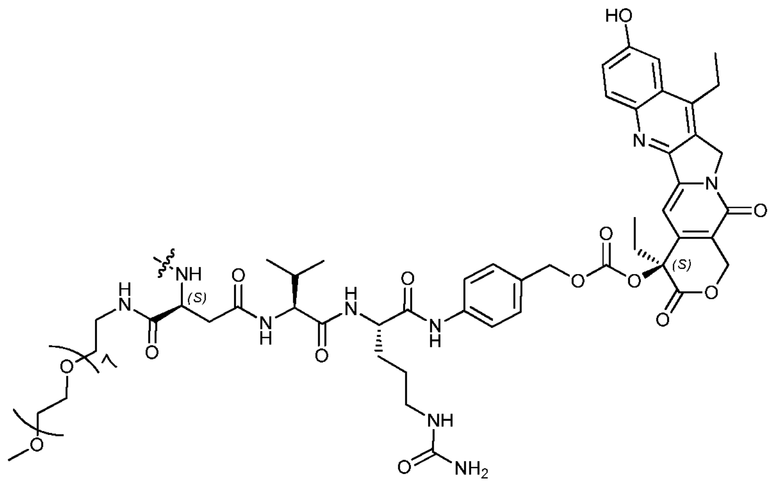

,

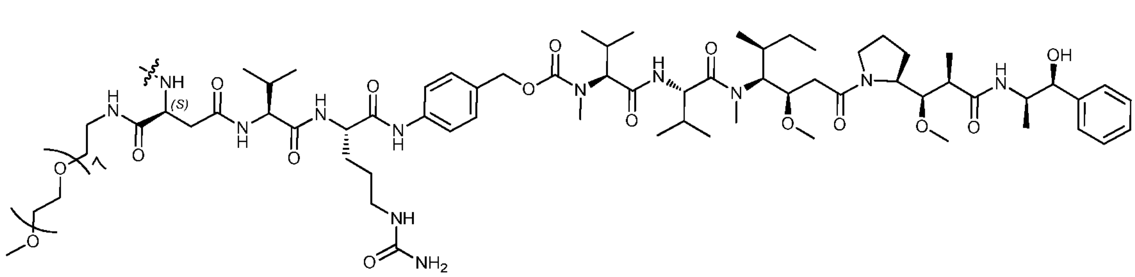

or

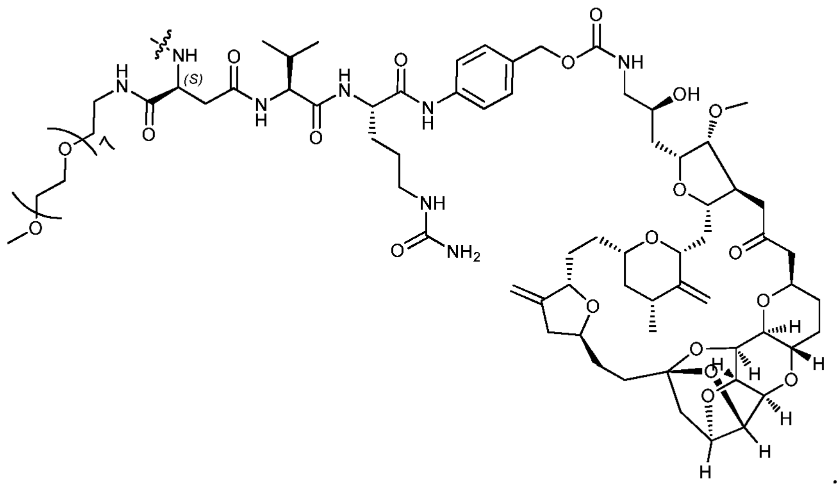

According to embodiments of the present disclosure, the targeting moiety is a protein-based recognition molecule (PBRM).

According to embodiments of the present disclosure, the recognition molecule is an internalizing antibody or an internalizing antigen-binding fragment thereof targeting tumor cells. In turn, the conjugate of the second aspect is covalently bound to an internalizing antibody or an internalizing antigen-binding fragment thereof targeting tumor cells via an A group to form an antibody-drug conjugate (ADC).

According to embodiments of the present disclosure, the antibody or antigen-binding fragment binds to anti-human epidermal growth factor receptor (HER2) antibody, EGFR, GPNMB, CD56, TACSTD2 (TROP2), CEACAM5, folate receptor-a, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin outer domain B, endothelin receptor ETB, tenascin c, collagen IV, VEGFR2, periostin, CD30, CD79b, CD19, CD22, CD138, CD37, CD33, CD74, etc.

In the fourth aspect of the present disclosure, provided herein is a pharmaceutical composition. According to embodiments of the present disclosure, the pharmaceutical composition comprises the above conjugate and a pharmaceutically acceptable carrier, wherein the conjugate comprises a targeting moiety, the compound of the first aspect, and a drug moiety, wherein the targeting moiety is covalently linked to one or more of the compounds, and the compound is covalently linked to the drug moiety via an R group.

In the fifth aspect of the present disclosure, provided herein is use of the above conjugate or the above pharmaceutical composition in the manufacture of a medicament for treating a patient having or at risk of having a cancer expressing a target antigen, wherein the conjugate comprises a targeting moiety, the compound of the first aspect, and a drug moiety, wherein the targeting moiety is covalently linked to one or more of the compounds, and the compound is covalently linked to the drug moiety via an R group.

According to embodiments of the present disclosure, the target antigen is human epidermal growth factor receptor 2.

According to embodiments of the present disclosure, the cancer expresses a high level of human epidermal growth factor receptor 2.

According to embodiments of the present disclosure, the cancer is breast cancer, gastric cancer, bladder cancer or urothelial cell carcinoma.

In the sixth aspect of the present disclosure, provided herein is treating a patient having or at risk of having a cancer expressing a target antigen. According to embodiments of the present disclosure, the method comprises administering to the patient a therapeutically effective amount of the above conjugate which is an ADC comprising a targeting moiety, the compound of the first aspect and a drug moiety.

In the seventh aspect of the present disclosure, provided herein is a method for reducing or inhibiting the growth of a tumor expressing a target antigen, comprising administering a therapeutically effective amount of the above conjugate which is an ADC comprising a targeting moiety, the compound of the first aspect and a drug moiety.

In the eighth aspect of the present disclosure, provided herein is use of the above conjugate in treating a cancer expressing a target antigen, wherein the conjugate is an ADC comprising a targeting moiety, the compound of the first aspect and a drug moiety.

In the ninth aspect of the present disclosure, provided herein is use of the above conjugate in the manufacture of a medicament for treating a cancer expressing a target antigen, wherein the conjugate is an ADC comprising a targeting moiety, the compound of the first aspect and a drug moiety.

In the tenth aspect of the present disclosure, provided herein is a method for producing the above conjugate,

which includes reacting an antibody or an antigen-binding fragment with the above linker compound bound to a small molecule drug under conditions that allow the conjugation.

In the eleventh aspect of the present disclosure, provided herein is a method of determining whether a patient will respond to the treatment with the above conjugate, comprising providing a biological sample from the patient and contacting the biological sample with the above conjugate which is an ADC comprising a targeting moiety, the compound of the first aspect and a drug moiety.

According to embodiments of the present disclosure, the biological sample is a tumor biopsy derived from a patient having or at risk of having a cancer expressing human epidermal growth factor receptor 2, wherein the cancer is breast cancer, gastric cancer, bladder cancer, or urothelial cell carcinoma.

## DETAILED DESCRIPTION OF THE INVENTION

The following embodiments are provided to further illustrate the present disclosure. It should be understood that these embodiments are only for illustrating the present disclosure, but are not intended to limit the scope of the present disclosure.

### TERMINOLOGY USED HEREIN

Unless defined otherwise, all technical and scientific terms used herein have the same meanings as commonly understood by those skilled in the art.

In the description of the present specification, descriptions with reference to terms "embodiment", "embodiments", "example", "specific embodiment", or "examples" etc. mean that a specific feature, structure, material, or characteristic described in conjunction with the embodiment or example is included in at least one embodiment or example of the present disclosure. In this specification, exemplary descriptions of the above terms do not necessarily refer to the same embodiment or example. Moreover, the described specific feature, structure, material or characteristic can be combined in any one or more embodiments or examples in a suitable manner. In addition, those skilled in the art can combine and incorporate different embodiments or examples and features of different embodiments or examples described in this specification without contradicting each other.

As used herein, the articles "a", "an" and "the" are intended to include "at least one" or "one or more" unless otherwise stated or clearly contradicted by context. Therefore, these articles used herein refer to articles of one or more than one (i.e., at least one) object. For example, "a component" refers to one or more components, that is, more than one component may be considered to be adopted or used in an implementation of the described embodiment.

As used herein, the term "subject" refers to an animal. Generally, the animal is a mammal. The subject, for example, also refers to primates (e.g. humans, males or females), bovines, sheep, goats, horses, canines, cats, rabbits, rats, mice, fish, birds, and the like. In some embodiments, the subject is a primate. In other embodiments, the subject is a human being.

As used herein, the term "patient" refers to human beings (including adults and children) or other animals. In some embodiments, "patient" refers to a human being.

The term "include" or "comprise" is an open-ended expression, i.e., including the content specified in the present disclosure but not excluding the content in other aspects.

"Stereoisomer" refers to a compound that has the same chemical structure but differs in the spatial arrangement of atoms or moieties. Stereoisomers include enantiomers, diastereomers, conformational isomers (rotamers), geometric isomers (*cis*/*trans* isomers), atropisomers, etc.

"Chirality" means that a molecule has the property that the molecule and its mirror image are non-superimposable; and "achirality" means that a molecule has the property that the molecule and its mirror image are superimposable.

"Enantiomer" refers to two isomers of a compound that are each a mirror image of the other one but cannot overlap with each other.

"Diastereoisomer" refers to stereoisomers that have two or more chiral centers and whose molecules are not mirror images of each other. Diastereomers have different physical properties such as melting point, boiling point, spectral property, and reactivity. A mixture of diastereomers can be separated by high-resolution analytical operations, for example, electrophoresis and chromatography such as HPLC.

The definitions and rules of stereochemistry used in the present disclosure generally follow S. P. Parker, Ed., "McGraw-Hill Dictionary of Chemical Terms (1984)", McGraw-Hill Book Company, New York; and Eliel, E. and Wilen, S., "Stereochemistry of Organic Compounds", John Wiley & Sons, Inc., New York, 1994.

Many organic compounds exist in optically active forms, i.e., they have the ability to rotate the plane of plane polarized light. When describing optically active compounds, the prefixes D and L, or *R* and *S* are used to denote the absolute configurations of the molecule with respect to one or more chiral centers. The prefixes *d* and *1*, or (+) and (-) are symbols used to designate the rotation of plane polarized light due to a compound, wherein (-) or *1* indicates that

the compound is levorotatory, and (+) or *d* indicates that the compound is dextrorotatory. One specific stereoisomer is an enantiomer, and a mixture of such isomers is called an enantiomeric mixture. A mixture of enantiomers in 50:50 is called a racemic mixture or a racemate, which may occur when a chemical reaction or process is not stereoselective or stereospecific.

Any asymmetric atom (e.g., carbon, etc.) of the compound of the present disclosure can be present in a racemic or enantiomer-enriched form, for example, present in (*R*)-, (*S*)-, or (*R*, *S*)-configuration. In some embodiments, in terms of (*R*)- or (*S*)-configuration, each asymmetric atom has an enantiomeric excess of at least 50%, an enantiomeric excess of at least 60%, an enantiomeric excess of at least 70%, an enantiomeric excess of at least 80%, an enantiomeric excess of at least 90%, an enantiomeric excess of at least 95%, or an enantiomeric excess of at least 99%.

In accordance with the selection of starting materials and methods, the compounds of the present disclosure may be present as one of the possible isomers or a mixture thereof, such as a racemate and a mixture of diastereomers, depending on the number of asymmetric carbon atoms. Optically active (*R*)- or (*S*)-isomers can be prepared using chiral synthons or chiral reagents, or resolved using conventional techniques. If a compound contains a double bond, the substituents may be in an E or Z configuration; and if a compound contains disubstituted cycloalkyl, the substituents of the cycloalkyl may have a *cis*- or trans-configuration.

Any obtained mixture of stereoisomers can be separated into pure or substantially pure geometric isomers, enantiomers, or diastereomers according to the differences in physical and chemical properties of components, for example, by chromatography and/or fractional crystallization process.

A racemate of any obtained end-product or intermediate can be resolved into optical enantiomers by methods known to those skilled in the art, for example, by separating salts of the obtained diastereomers. Racemic products can also be separated by chiral chromatography, such as high-performance liquid chromatography (HPLC) using chiral adsorbents. Particularly, enantiomers can be prepared by asymmetric synthesis, for example, referring to Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers that have different energies and can be interconverted by crossing a low energy barrier. If tautomerism is possible (e.g., in solution), a chemical equilibrium of tautomers can be reached. For example, protontautomer (also known as prototropic tautomer) includes interconversion through proton migration, such as ketone-enol isomerization and imine-enamine isomerization. Valence tautomer includes interconversion through recombination of some bonding electrons. A specific example of ketone-enol tautomerization is interconversion of pentane-2,4-dione and 4-hydroxy-3-penten-2-one tautomeric isomers. Another example of tautomerism is phenol-ketone tautomerization. A specific example of phenol-ketone tautomerization is interconversion of 4-hydroxypyridine and pyridin-4(1*H*)-one tautomeric isomers. Unless otherwise indicated, all tautomeric forms of the compound of the present disclosure shall fall within the scope of the present disclosure.

As described in the present disclosure, the compound of the present disclosure may be optionally substituted with one or more substituents, for example, compounds represented by the above general formulas, or specific example compounds in the examples, and a class of compounds contained in the present disclosure.

The term "protecting group" or "PG" refers a substituent which, when reacted with other functional groups, is usually used to block or protect specific functionality. For example, "amino-protecting group" refers to a substituent that is connected to an amino group to block or protect the functionality of the amino group in a compound. Suitable amino-protecting groups include acetyl, trifluoroacetyl, tert-butoxycarbonyl (BOC, Boc), benzyloxycarbonyl (CBZ, Cbz), and 9-fluorenylmethyleneoxycarbonyl (Fmoc). Similarly, "hydroxyl-protecting group" refers to a substituent of a hydroxyl group, which is used to block or protect the functionality of the hydroxyl group. Suitable hydroxyl-protecting groups include acetyl and silyl groups. "Carboxy-protecting group" refers to a substituent of a carboxyl group, which is used to block or protect the functionality of the carboxyl group. The carboxyl-protecting groups generally include $-CH_2CH_2SO_2Ph$, cyanoethyl, 2-(trimethylsilyl)ethyl, 2-(trimethylsilyl)ethoxymethyl, 2-(p-toluenesulfonyl)ethyl, 2-(p-nitrobenzenesulfonyl)ethyl, 2-(diphenylphosphino)ethyl, nitroethyl, etc. For a general description of protecting groups, please refer to: T W. Greene, Protective Groups in Organic Synthesis, John Wiley&Sons, New York, 1991; and P. J. Kocienski, Protecting Groups, Thieme, Stuttgart, 2005.

As used herein, "pharmaceutically acceptable salt" refers to an organic or inorganic salt of a compound of the present disclosure. Pharmaceutically acceptable salts are well known in the art, as described in the literature: S. M. Berge *et al.* describe pharmaceutically acceptable salts in detail in J. Pharmaceutical Sciences, 1977, 66: 1-19. Salts formed by pharmaceutically acceptable non-toxic acids include, but are not limited to: inorganic acid salts formed through the reaction with amino groups, such as hydrochloride, hydrobromide, phosphate, sulfate, and perchlorate; organic acid salts such as acetate, oxalate, maleate, tartrate, citrate, succinate, and malonate; or salts obtained by other methods such as ion exchange described in books and literatures. Other pharmaceutically acceptable salts include adipate,

alginate, ascorbate, aspartate, benzenesulfonate, benzoate, bisulfate, borate, butyrate, camphorate, camphorsulfonate, cyclopentyl propionate, digluconate, lauryl sulfate, ethanesulfonate, formate, fumarate, gluceptate, glycerophosphate, gluconate, hemisulfate, heptanoate, caproate, hydroiodide, 2-hydroxy-ethanesulfonate, lactobionate, lactate, laurate, lauryl sulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate, etc. Salts obtained from appropriate bases include salts of alkali metals, alkaline earth metals, ammonium, and $N^+(C_1\text{-}C_4\ \text{alkyl})_4$. The present disclosure also intends to contemplate any quaternary ammonium salts formed by compounds containing a group containing N. Water-soluble or oil-soluble or dispersed products can be obtained by quaternization. Salts of alkali metals or alkaline earth metals include salts of sodium, lithium, potassium, calcium, magnesium, etc. Pharmaceutically acceptable salts further include suitable non-toxic ammonium, and amine cations formed by quaternary ammonium salts and counterions such as halides, hydroxides, carboxylates, sulfates, phosphates, nitrates, $C_{1\text{-}8}$ sulfonates and aromatic sulfonates.

The term "conjugate" refers to a substance formed by covalently binding two or more compounds, such as a conjugate formed by covalently binding a compound of formula (I) of this application to a small molecule drug, or a conjugate formed by covalently binding the compound of formula (I) to an antibody or an antigen-binding fragment, or a conjugate formed by covalently binding the compound of formula (I) to an antibody or an antigen-binding fragment and a small molecule drug where the conjugate is an antibody-drug conjugate (ADC).

The terms "antibody-drug conjugate", "antibody conjugate", "conjugate", "immunoconjugate", and "ADC" are used interchangeably, and refer to a compound that is linked to an antibody, or a derivative thereof (e.g., an anti-HER2 antibody).

The term "antibody" is used in the broadest sense to refer to an immunoglobulin molecule that recognizes and specifically binds to a target, such as a protein, polypeptide, carbohydrate, polynucleotide, lipid, or a combination thereof through antigen recognition sites within the variable region of the immunoglobulin molecule. The heavy chain of an antibody is composed of a heavy chain variable domain (VH) and a heavy chain constant region (CH). The light chain is composed of a light chain variable domain (VL) and a light chain constant domain (CL). For the purposes of this application, mature heavy chain and light chain variable domains each comprise three complementarity determining regions (CDR1, CDR2 and CDR3) within four framework regions (FR1, FR2, FR3 and FR4) arranged from N-terminus to C-terminus: FR1, CDR1, FR2, CDR2, FR3, CDR3 and FR4. An "antibody" can be naturally occurring or man-made, such as monoclonal antibodies produced by conventional hybridoma technology. The term "antibody" includes full-length monoclonal antibodies and full-length polyclonal antibodies, as well as antibody fragments such as Fab, Fab', F(ab')2, Fv, and single chain antibodies. An antibody can be any one of the five major classes of immunoglobulins: IgA, IgD, IgE, IgG, and IgM, or a subclass thereof (e.g., isotypes IgG1, IgG2, IgG3, and IgG4). The term further encompasses human antibodies, chimeric antibodies, humanized antibodies and any modified immunoglobulin molecule containing an antigen recognition site, so long as it demonstrates the desired biological activity.

As used herein, the term "antigen-binding fragment" or "antigen-binding portion" of an antibody refers to one or more fragments of an antibody that retain the ability to specifically bind to an antigen (e.g., HER2). Antigen-binding fragments preferably also retain the ability to internalize into an antigen-expressing cell. In some embodiments, antigen-binding fragments also retain immune effector activity. It has been shown that fragments of a full-length antibody can perform the antigen-binding function of a full-length antibody. Examples of binding fragments encompassed within the term "antigen-binding fragment" or "antigen-binding portion" of an antibody include (i) a Fab fragment, a monovalent fragment consisting of the VL, VH, CL, and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody; (v) a dAb fragment, which comprises a single variable domain, e.g., a VH domain (see, e.g., Ward et al. (1989) Nature 341:544-6; and Winter et al., WO 90/05144); and (vi) an isolated complementarity determining region (CDR). Furthermore, although the two domains of the Fv fragment, VL and VH, are coded by separate genes, they can be joined, using recombinant methods, by a synthetic linker that enables them to be made as a single protein chain in which the VL and VH regions pair to form monovalent molecules (known as single chain Fv(scFv)). See, e.g., Bird et al. (1988) Science 242:423-6; and Huston et al. (1988) Proc. Natl. Acad. Sci. USA 85:5879-83. Such single chain antibodies are also intended to be encompassed within the term "antigen-binding fragment" or "antigen-binding portion" of an antibody, and are known in the art as an exemplary type of binding fragment that can internalize into cells upon binding. See, e.g., Zhu et al. (2010) 9:2131-41; He et al. (2010) J. Nucl. Med. 51:427-32; and Fitting et al. (2015) MAbs 7:390-402. In certain embodiments, scFv molecules may be incorporated into a fusion protein. Other forms of single chain antibodies, such as bifunctional antibodies are also encompassed. Bifunctional antibodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., Holliger et al. (1993) Proc. Natl. Acad. Sci. USA 90:6444-8; and Poljak et al. (1994) Structure 2:1121-3). Antigen-binding fragments are obtained using conventional techniques known to those

of skill in the art, and the binding fragments are screened for utility (e.g., binding affinity, internalization) in the same manner as are intact antibodies. Antigen-binding fragments may be prepared by cleavage of the intact protein, e.g., by protease or chemical cleavage.

As used herein in reference to an antibody or antigen-binding fragment, "internalizing" means that an antibody or antigen-binding fragment is capable of being taken through the cell's lipid bilayer membrane to an internal compartment (i.e., "internalized") upon binding to the cell, preferably into a degradative compartment in the cell. For example, an internalizing anti-HER2 antibody is one that is capable of being taken into the cell after binding to HER2 on the cell membrane.

The term "human epidermal growth factor receptor 2", "her2", or "her2/neu" refers to any native form of human her2. The term encompasses full- length her2 (e.g., NCBI Reference Sequence: NP_004439.2; SEQ ID NO: 21), as well as any form of human her2 that results from cellular processing. The term also encompasses naturally occurring variants of her2, including but not limited to splice variants, allelic variants, and isoforms. Her2 can be isolated from human, or may be produced recombinantly or by synthetic methods.

The term "anti-her2 antibody" or "antibody that specifically binds her2" refers to any form of antibody or fragment thereof that specifically binds her2, and encompasses monoclonal antibodies (including full length monoclonal antibodies), polyclonal antibodies, and biologically functional antibody fragments so long as they specifically bind her2. U.S. Pat. No. 5,821,337 (incorporated herein by reference) provides exemplary her2-binding sequences, including exemplary anti-her2 antibody sequences. Alternatively, the anti-her2 antibody used in the ADCs disclosed herein is an internalizing antibody or internalizing antibody fragment. Trastuzumab is an exemplary internalizing anti-human her2 antibody.

The term "kon" or "ka" refers to the on-rate constant for association of an antibody to an antigen to form an antibody/antigen complex. The rate can be determined using standard assays, such as a Biacore or ELISA assay.

The term "koff" or "kd" refers to the off-rate constant for dissociation of an antibody from an antibody/antigen complex. The rate can be determined using standard assays, such as a Biacore or ELISA assay.

The term "$K_D$" refers to the equilibrium dissociation constant of a particular antibody-antigen interaction. $K_D$ is calculated by ka/kd. The rate can be determined using standard assays, such as a Biacore or ELISA assay. The antibody or antigen-binding fragment in the conjugates of the present application can bind to the target antigen at an equilibrium dissociation constant ($K_D \leq 1$ mM, $\leq 100$ nM or $\leq 10$ nM, or any amount therebetween). In certain embodiments, the $K_D$ is 1 pM to 500 pM. In some embodiments, the $K_D$ is between 500 pM and 1 $\mu$M.

The term "antibody:drug ratio" or "drug-to-antibody ratio" or "DAR" refers to the number of attached drug moieties per antibody moiety, i.e., drug loading.

The term "therapeutic agent", "drug" or "drug moiety" refers to an agent capable of modulating a biological process and/or having a biological activity.

The term "cytotoxic agent" refers to a substance that causes cell death primarily by interfering with a cell's expression activity and/or functioning. Examples of cytotoxic agents include, but are not limited to, anti-mitotic agents, such as eribulin, auristatins (e.g., monomethyl auristatin E (MMAE)).

The term "cancer" refers to the physiological condition in mammals in which a population of cells is characterized by unregulated cell growth.

A "pharmaceutical composition" refers to a preparation which is in such form as to permit administration and subsequently provide the intended biological activity of the active ingredient (s) and/or to achieve a therapeutic effect, and which contains no additional components which are unacceptably toxic to a subject to which the formulation would be administered. The pharmaceutical composition may be sterile.

A "pharmaceutical excipient" comprises a material such as an adjuvant, a carrier, pH-adjusting and buffering agents, tonicity adjusting agents, wetting agents, preservative, and the like.

"Pharmaceutically acceptable" means, within the scope of sound medical judgment, suitable for contact with human and lower animal tissues without undue toxicity, irritation, allergic reaction, etc., and commensurate with a reasonable benefit/risk ratio.

An "effective amount" of an ADC as disclosed herein is an amount sufficient to perform a specifically stated purpose, for example to produce a therapeutic effect after administration, such as a reduction in tumor growth rate or tumor volume, a reduction in a symptom of cancer, or some other indicia of treatment efficacy. An effective amount can be determined in a routine manner in relation to the stated purpose. The term "therapeutically effective amount" refers to an amount of an ADC effective to treat a disease or condition in a subject. In the case of cancer, a therapeutically effective amount of ADC can reduce the number of cancer cells, reduce tumor size, inhibit (e.g., slow or stop) tumor metastasis, inhibit (e.g., slow or stop) tumor growth, and/or relieve one or more symptoms. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

An "effective amount" of an ADC as disclosed herein is an amount sufficient to perform a specifically stated purpose, for example to produce a therapeutic effect after administration, such as a reduction in tumor growth rate or

tumor volume, a reduction in a symptom of cancer, or some other indicia of treatment efficacy. An effective amount can be determined in a routine manner in relation to the stated purpose. The term "therapeutically effective amount" refers to an amount of an ADC effective to treat a disease or condition in a subject. In the case of cancer, a therapeutically effective amount of ADC can reduce the number of cancer cells, reduce tumor size, inhibit (e.g., slow or stop) tumor metastasis, inhibit (e.g., slow or stop) tumor growth, and/or relieve one or more symptoms. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Now some embodiments of the present disclosure are described in more detail, examples of which are illustrated by the accompanying structural formulas and chemical formulas. The present disclosure is intended to cover all alternatives, modifications and equivalent technical solutions falling within the scope of the present disclosure as defined by the claims. Those skilled in the art should recognize that many methods and materials similar or equivalent to those described herein can be used to implement the present disclosure. The present disclosure is not limited to the methods and materials described herein. In the event that one or more of the incorporated literature, patents, and similar materials differs from or contradicts this application (including but not limited to defined terms, term applications, described technologies, etc.), this application shall prevail.

It should be further noted that some features of the present disclosure, for the sake of clarity, have been described in multiple independent embodiments, but may alternatively be provided in combination in a single embodiment. On the contrary, the various features of the present disclosure are described in a single embodiment for the sake of brevity, but they can alternatively be provided individually or in any suitable sub-combination.

Unless otherwise specified, all scientific and technological terms used in the present disclosure have the same meanings as commonly understood by those skilled in the art to which the present disclosure belongs. All patents and publications involved in the present disclosure are incorporated into the present disclosure in their entireties by reference.

Unless otherwise stated, the following definitions used herein shall apply. For the purposes of the present disclosure, the chemical elements correspond to the Periodic Table of the Elements, CAS Edition, and Handbook of Chemistry and Physics, 75th Edition, 1994. In addition, general principles of organic chemistry can be found in the descriptions of "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference.

**Preparation Example**

**Example 1 Synthesis of MS-1**

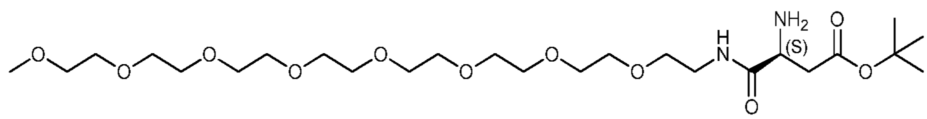

MS-1

**Step 1: Synthesis of Cbz-Ms-1**

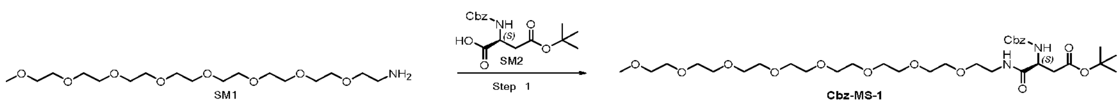


SM2 (8 g, 23.5 mmol) and 150 mL of DMF were added to a 250 mL three-necked flask, and the mixture was cooled to 0°C under argon gas in an ice salt bath. SM1 (9 g, 23.5 mmol) and HBTU (13.3 g, 35.2 mmol) were added, and then DIPEA (4.5 g, 35.2 mmol) was added dropwise. After the addition was completed, the mixture was reacted at room temperature (20°C) for 1h. The reaction was monitored by TLC (DCM:MeOH=10:1), and SM2 disappeared.

500 mL of water was added to the reaction solution, and the mixture was extracted 3 times with 200 mL of EA. The organic phase was dried over anhydrous sodium sulfate and concentrated. The crude product was purified via a silica gel column eluted with MeOH/DCM (2%-3%) to give 11 g (15.9 mmol, 68.0%) of product as a colorless oil.

$^1H$ NMR (300 MHz, DMSO-$d_6$) δ 7.95-7.91 (m, 1H), 7.53 (d, J=8.7Hz, 1H), 737-7.34 (m, 5H), 5.10-4.98 (m, 2H), 4.38-4.30 (m, *1*H), 3.51 - 3.44 (m, 26H), 3.43 - 3.38 (m, 4H), 3.24 - 3.18 (m, 5H), 2.67 - 2.60 (m, 1H), 2.48 - 2.43 (m, 1H), 1.40 (s, 9H).

**Step 2: Synthesis of Ms-1**

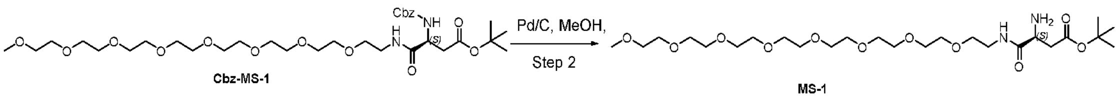


Cbz-MS-1 MS-1

To a solution of Cbz-Ms-1 (1.0 g, 1.5 mmol, 1.0 eq.) in methanol was added palladium on carbon (0.2 g), and the atmosphere in the reaction system was replaced 5 times with hydrogen. The mixture was hydrogenated with stirring at 25 °C for 4 hours. The reaction solution was filtered and concentrated to dryness by rotary-evaporation under reduced pressure to give 650 mg of Ms-1 (81%) as a colorless oil. This product can be used directly in the next step without further purification.

MS m/z [M+H]+ (ESI): 555.45.

**Example 2 Synthesis of CS-2**

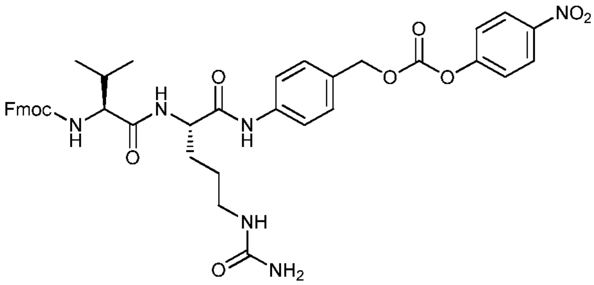

CS-2

**Step 1: Synthesis of INA**

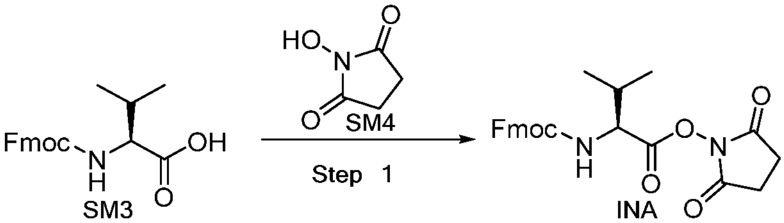


SM3 INA

300 mL of DCM was added to a three-necked flask, then SM3 (30 g, 0.0885 mol), SM4 (13.2 g, 0.115 mol), and EDCI (22.1 g, 0.115 mol) were added, and the mixture was stirred at room temperature for 6 h. The reaction was monitored by TLC with DCM/MeOH = 10:1, and the raw material was completely converted. The reaction solution was diluted to 600 mL with dichloromethane, then washed twice with 200 mL of 0.25 M dilute hydrochloric acid and once with saturated brine, dried over anhydrous sodium sulfate, and filtered. The filtrate was rotary-evaporated to dryness to give 35 g (0.08 mol, 90.39%) of white foamy solid, which was used directly in the next step without purification.

**Step 2: Synthesis of INB**

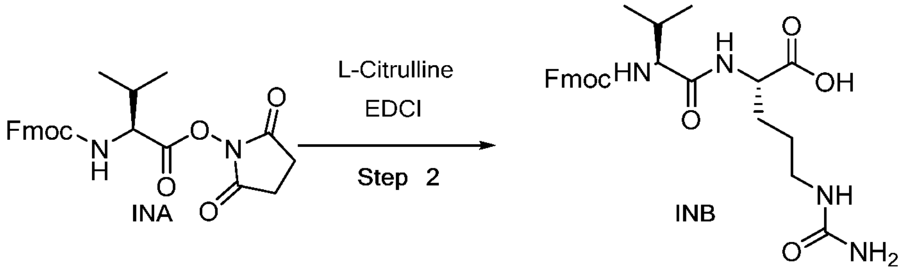


INA

INB

INA (35 g, 0.08 mmol) was dissolved in 400 mL of DMF, and L-citrulline (30.9 g, 0.177 mol) and sodium bicarbonate (18.5 g, 0.221 mol) were added. 200 mL of water was then added, and the mixture was reacted at 25°C for 16 hours. The reaction was monitored by TLC with DCM/MeOH=10/1, and there was no raw material remaining as the reaction was completed. DMF was removed by rotary-evaporation, and 500 mL of water and 50 g of citric acid were added. The mixture was slurried for 1 hour, and filtered. The solid was rotary-evaporated to dryness. The solid was slurried with 500 mL of dichloromethane for 1 hour, and filtered with suction. The solid was sucked with an oil pump to dryness to give 34.5 g (0.07 mmol, 87.5%) of the target product as a white solid.

$^1$H NMR (300 MHz, DMSO-d$_6$) δ 12.40 (s, 1H), 8.15 (d, *J* = 7.3 Hz, 1H), 7.89 (d, *J* = 7.5 Hz, 2H), 7.80 - 7.69 (m, 2H), 7.46 - 7.29 (m, 6H), 6.00 - 5.88 (m, 1H), 5.37 (s, 2H), 4.30 - 4.14 (m, 4H), 3.95 - 3.89 (m, 1H), 2.98 - 2.89 (m, 2H), 2.05 - 1.87 (m, 1H), 1.83 - 1.50 (m, 2H), 1.45 - 1.31 (m, 2H), 0.87 (dd, *J* = 9.3, 6.7 Hz, 6H).

**Step 3: Synthesis of INC**

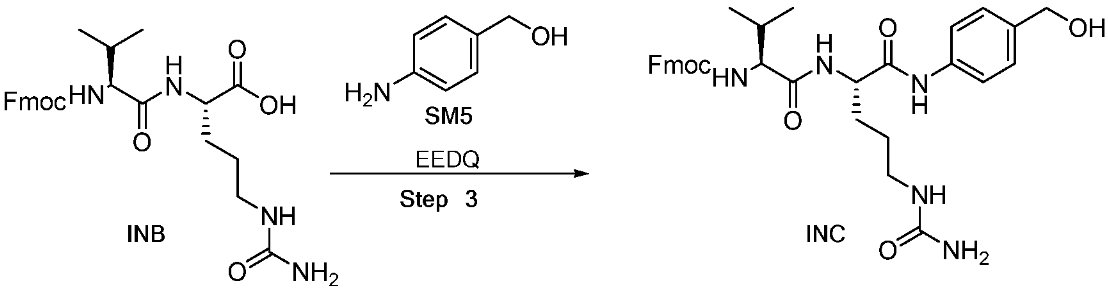


INB

INC

To a 3L single-necked flask were added INB (26.9 g, 54.23 mmol) and a mixture of DCM/MeOH (2L, 2:1). The mixture was stirred to be dissolved until clear. SM5 (8 g, 65.08 mmol) and 2-ethoxy-1-ethoxycarbonyl-1,2-dihydroquinoline (EEDQ) (24.1g, 97.6mmol) were added, and the mixture was reacted at room temperature (20°C) in the dark for 16 h. The mixture was then transferred to an oil bath at 30 °C and stirred for 5 h. The reaction was monitored by TLC with DCM/MeOH=5:1, and the raw material was completely converted. The reaction solution was rotary evaporated to remove most of the reaction solution. The residue was transferred to a 1L single-necked flask and rotary-evaporated to dryness. The residue was slurried with 500 mL of methyl tert-butyl ether for 1 h, then filtered, and slurried with 300 mL of tetrahydrofuran for 16 h. The mixture was filtered to give 20 g of white solid (33.2 mmol, 61.3%).

$^1$H NMR (300 MHz, DMSO-d$_6$) δ 9.97 (s, 1H), 8.10 (d, *J* = 7.1 Hz, 1H), 7.89 (d, *J* = 7.3 Hz, 2H), 7.78 - 7.65 (m, 2H), 7.54 (d, *J* = 8.1 Hz, 2H), 7.42 (t, *J* = 7.6 Hz, 3H), 7.32 (t, *J* = 7.3 Hz, 2H), 7.23 (d, *J* = 8.0 Hz, 2H), 6.04 - 5.88 (m, 1H), 5.47 - 5.30 (m, 2H), 5.15 - 5.00 (m, 1H), 4.50 - 4.17 (m, 6H), 3.93 (t, *J* = 7.5 Hz, 1H), 3.10 - 2.88 (m, 2H), 2.05 - 1.95 (m, 1H), 1.76 - 1.57 (m, 2H), 1.50 - 1.34 (m, 2H), 0.96 - 0.81 (m, 6H).

**Step 4: Synthesis of CS-2**

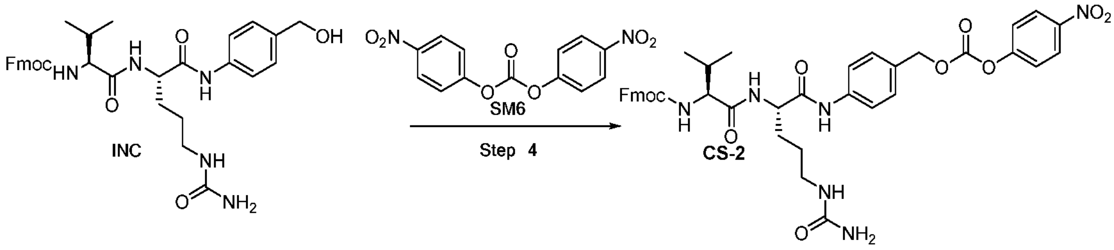


INC (20 g, 33 mmol) and 1L of DMF were added to a single-necked flask, and the mixture was stirred until completely dissolved. SM6 (63 g, 209 mmol) was added. The mixture was cooled down to 0-5°C in an ice bath, and DIPEA (27g, 209mmol) was added dropwise. The mixture was then reacted under an ice bath for 3h.

The reaction was monitored by TLC with dichloromethane:methanol = 10:1, and INC was completely converted. The reaction was stopped. The mixture was sucked with an oil pump to dryness at 55°C. The solid was crushed, slurried with 100 mL of methyl tert-butyl ether for 1 hour, and then filtered. The filter cake was slurried with 300mL of 1:1 DCM/PE for 1h and filtered. The filter cake was slurried again with 300mL of 1:1 DCM/PE and filtered to give 15.5g (20.2 mmol, 61.3%) of a khaki solid.

1H NMR (300 MHz, DMSO-$d_6$) δ 10.14 (s, 1H), 8.37 - 8.26 (m, 2H), 8.14 (d, J = 7.4 Hz, 1H), 7.89 (d, J = 7.3 Hz, 2H), 7.74 (t, J = 6.6 Hz, 2H), 7.65 (d, J = 8.4 Hz, 2H), 7.60 - 7.52 (m, 2H), 7.47 - 7.37 (m, 5H), 7.32 (t, J = 7.1 Hz, 2H), 6.02 - 5.90 (m, 1H), 5.41 (s, 2H), 5.25 (s, 2H), 4.49 - 4.37 (m, 1H), 4.34 - 4.18 (m, 3H), 3.97 - 3.88 (m, 1H), 3.09 - 2.91 (m, 2H), 2.07 - 1.92 (m, 1H), 1.76 - 1.54 (m, 2H), 1.52 - 1.30 (m, 2H), 0.93 - 0.79 (m, 6H).

**Example 3 Synthesis of FS-1**

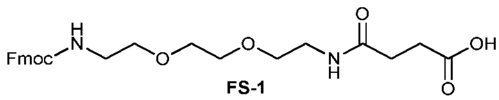

FS-1

**Step 1: Synthesis of IND**

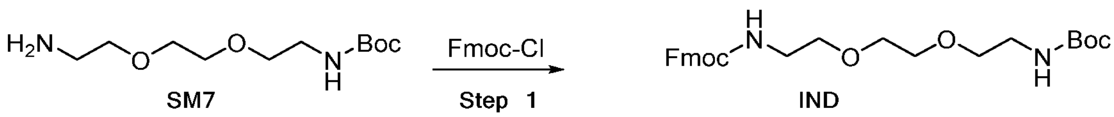


To a single-necked flask, SM7 (9 g, 36.24 mmol), 1,4-dioxane (45 mL) and aqueous $Na_2CO_3$ (10%, 90 mL) were added. A solution of Fmoc-Cl (10.3 g, 39.87 mmol) in 1,4-dioxane (45 mL) was added dropwise under an ice bath, and then the mixture was reacted under an ice bath. The reaction was monitored by TLC (DCM:MeOH=10:1), and the raw material was completely converted. 300 mL of EA was added to the reaction solution. The mixture was washed 2 times with 100 mL of water and 2 times with 100 mL of saturated brine, and concentrated. The crude product was purified via a silica gel column eluted with EA/PE (30%-50%) to give 16.5g (35.09 mmol, 96.8%) of product as a colorless oil.

$^1$H NMR (300 MHz, DMSO-$d_6$) δ 8.00 - 7.76 (m, 3H), 7.72 - 7.65 (m, 2H), 7.44 - 7.30 (m, 5H), 4.47 - 4.10 (m, 3H), 3.52 - 3.42 (m, 4H), 3.42 - 3.32 (m, 4H), 3.23 - 3.02 (m, 4H), 1.37 (s, 9H).

**Step 2: Synthesis of INE**

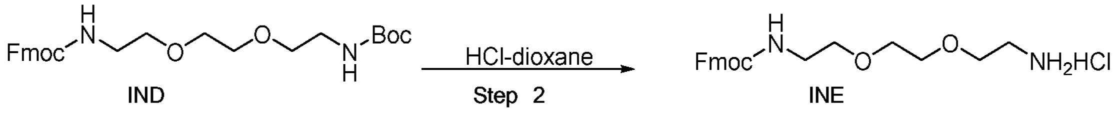

IND (16.5g, 35.09mmol) was added into a 250 mL single-necked flask, and HCl-dioxane solution (4 N, 50 mL) was added with stirring. The mixture was reacted at room temperature for 1h. The reaction was monitored by TLC with PE:EA=1:1, and the raw material was completely converted. The solvent was removed by rotary-evaporation to give 14 g (34.4 mmol, 98.05%) of product as a colorless oil.

$^1H$ NMR (300 MHz, DMSO-$d_6$) δ 8.05 (s, 3H), 7.90 (d, *J* = 7.4 Hz, 2H), 7.73 - 7.61 (m, 2H), 7.38 (dt, *J* = 13.8, 7.3 Hz, 5H), 4.44 - 4.14 (m, 3H), 3.56 - 3.51 (m, 4H), 3.49 - 3.26 (m, 4H), 3.15 (dd, *J* = 11.7, 5.8 Hz, 2H), 2.94 (dd, *J* = 10.1, 5.0 Hz, 2H).

**Step 3: Synthesis of FS-1**

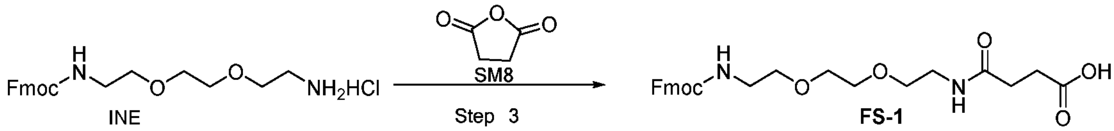

INE
FS-1

DCM (250mL), INE (14g, 34.4mmol) and succinic anhydride (11.4g, 114.2mmol) were added into a 500mL single-necked flask, and the mixture was cooled in an ice bath. Triethylamine (3.86g, 38.1mmol) was added dropwise, and then the mixture was reacted at room temperature for 3 hours. The reaction was monitored by TLC, DCM/MeOH=10/1, and the raw material was completely converted. The reaction solution was diluted with 800 mL of dichloromethane, washed twice with water (200 mL) and once with saturated brine (200 mL), dried over anhydrous sodium sulfate, and filtered. The filtrate was concentrated. The crude product was purified via a silica gel column eluted with DCM/MeOH=20:1 to give 15.5 g (32.9mmol, 95.9%) of light yellow oil. $^1H$ NMR (300 MHz, DMSO-$d_6$) δ 12.08 (s, 1H), 7.98 - 7.80 (m, 3H), 7.73 - 7.60 (m, 2H), 7.37 (dt, *J* = 13.9, 7.3 Hz, 5H), 4.46 - 4.14 (m, 3H), 3.55 - 3.43 (m, 4H), 3.42 - 3.34 (m, 4H), 3.25 - 3.06 (m, 4H), 2.44 - 2.39 (m, 2H), 2.37 - 2.26 (m, 2H).

**Example 4 Synthesis of Bn-LS-1**

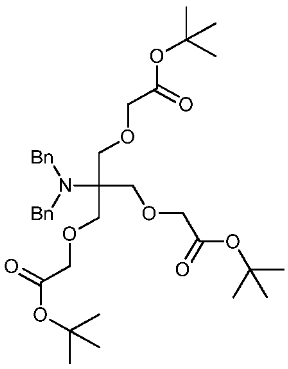

Bn-LS-1

**Step 1: Synthesis of INF**

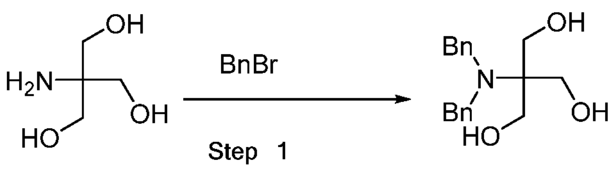


SM9 INF

DMF (450mL) was added to a 1L single-necked flask, followed by SM9 (50g, 0.413mol), benzyl bromide (155g, 0.908mol), and $K_2CO_3$ powder (143g, 1.038mol). The mixture was refluxed at 155°C in an oil bath for 20 hours. The reaction was monitored by LCMS. The solvent was removed by rotary-evaporation, and then 1L of dichloromethane was added. The mixture was washed twice with water (300mL) and twice with saturated brine (200mL), dried over anhydrous sodium sulfate, and filtered. The filtrate was rotary-evaporated to dryness. 40 mL of ethyl acetate was added to the crude product. The mixture was heated to reflux until completely dissolved, then cooled and crystallized overnight. The solid was filtered, and dried in vacuum to give 55 g (0.182 mmol, 44.07%) of off-white crystals.

$^1H$ NMR (300 MHz, DMSO-$d_6$) δ 7.31 - 7.00 (m, 10H), 4.29 (t, *J* = 5.1 Hz, 3H), 3.97 (s, 4H), 3.52 (d, J = 5.1 Hz, 6H).

**Step 2: Synthesis of Bn-LS-1**

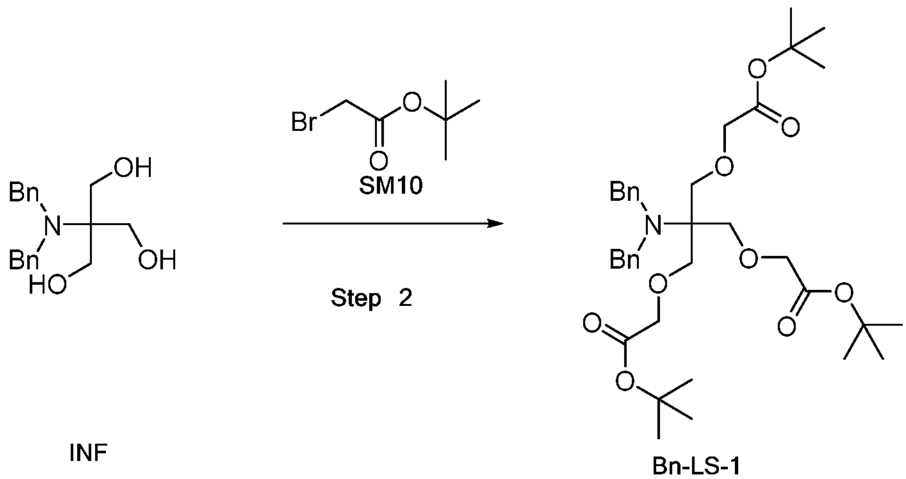


INF

Bn-LS-1

To a 500 mL three-necked flask were added 80 mL of water, and sodium hydroxide (80g, 1.99mol). DCM (160mL), tetrabutylammonium bromide (3.2g, 10mmol), and INF (20g, 66.36mmol) were then added. The mixture was stirred mechanically. The mixture was cooled down to below 10°C in an ice bath, and tert-butyl bromoacetate (52 g, 265.44 mmol) was added dropwise. After the addition was completed, the mixture was allowed to warm to 20°C and stirred at room temperature overnight (16 h). The reaction was monitored by TLC (PE:EA=1:1), and there was no raw material remaining. To the reaction system was added 1L of DCM, and the mixture was washed twice with water and twice with saturated aqueous sodium bicarbonate solution, and concentrated. The crude product was purified via a silica gel column eluted with PE:EA=20:1 to give 17g of product Bn-LS-1 (26.40mmol, 39.78%) .

$^1H$ NMR (300 MHz, DMSO-$d_6$) δ 7.29 - 7.19 (m, 4H), 7.17 - 7.00 (m, 6H), 3.98 (s, 4H), 3.88 (s, 6H), 3.63 (s, 6H), 1.41 (s, 27H).

**Example 5 Synthesis of HG-PL1**

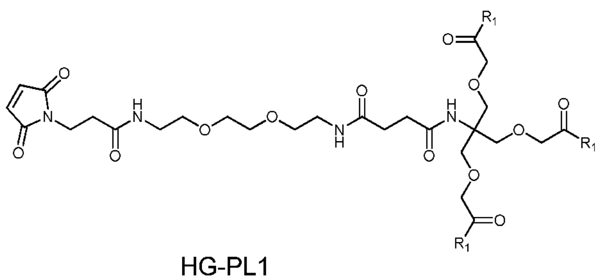

HG-PL1

R1=

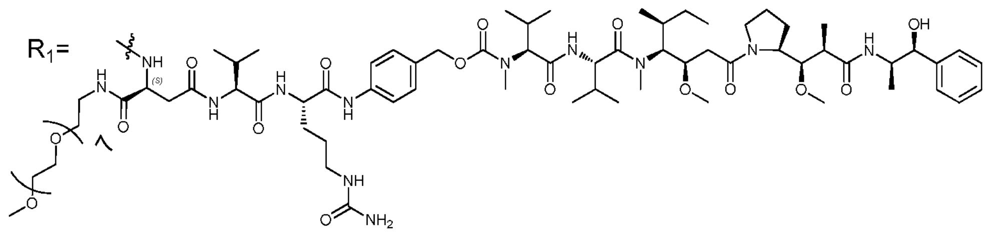

Step 1: Synthesis of PH-HG-001-7

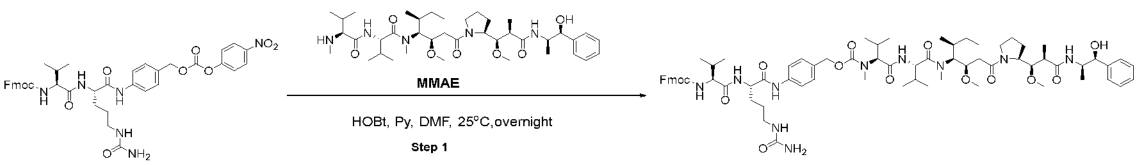


CS-2

PH-HG-001-7

To a solution of CS-2 (527.0 mg, 0.7 mmol, 1.0 eq.) in DMF (10.0 mL) were added MMAE (740.2 mg, 1.05 mmol, 1.5 eq.), HOBt (18.6 mg, 0.14 mmol, 0.2 eq.) and pyridine (16.3 mg, 0.21 mmol, 0.3 eq.) in batches. The reaction solution was stirred at 25 °C under nitrogen overnight. 100 mL of water was added to the reaction system, and the mixture was extracted with dichloromethane (3×100mL). The combined organic phase was washed with saturated brine (3×100 mL), dried over anhydrous sodium sulfate, and filtered. The filtrate was concentrated under reduced pressure. The resulting crude product was purified by preparative HPLC (X Bridge Prep OBD C18 column; mobile phase, water (10 mmol ammonium bicarbonate) and acetonitrile (from 42.0% acetonitrile to 72.0% acetonitrile, 10 min); detector. UV 254 nm) to give 550 mg (59%) of PH-HG-001-7, as a white solid.

MS m/z $[M+H]^+$ (ESI): 1345.45.

**Step 2: Synthesis of PH-HG-001-8**

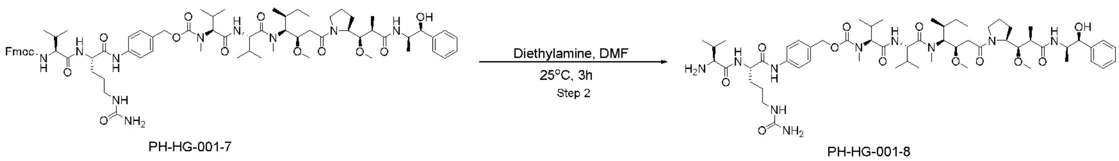


PH-HG-001-7

PH-HG-001-8

To PH-HG-001-7 (600.0 mg, 0.4 mmol, 1.0 eq.) in DMF (8.0 mL) was added diethylamine (4 mL) under nitrogen. The reaction solution was stirred at 25°C for 3 hours and then concentrated by rotary-evaporation. The crude product was purified by Flash-Prep-HPLC (C8 column, mobile phase: water (10 mmol ammonium bicarbonate) and acetonitrile (10% to 80%), detector UV 210 nm) to give 340 mg (68%) of PH- HG-001-8, as a white solid.

MS m/z $[M+H]^+$ (ESI): 1123.75. $^1H$ NMR (300 MHz, DMSO-$d_6$) δ: 0.78-0.92 (m, 24H), 0.99-1.06 (m, 7H), 1.21-1.27 (m, 1H), 1.31-1.37 (m, 3H), 1.49-1.86 (m, 7H), 1.90-2.21 (m, 4H), 2.37-2.45 (m, 1H), 2.81-3.01 (m, 6H), 3.10-3.22 (m, 9H), 3.51-3.81 (m, 3H), 3.90-4.08 (m, 2H), 4.14-4.28 (m, 2H), 4.34-4.80 (m, 4H), 4.84-5.17 (m, 2H), 5.39-5.58 (m, 2H), 6.01-6.35 (m, 1H), 7.10-7.37 (m, 7H), 7.55-7.92 (m, 3H), 7.99-8.41 (m, 1H), 10.12-10.52 (m, 1H).

**Step 3: Synthesis of PH-HG-001-1**

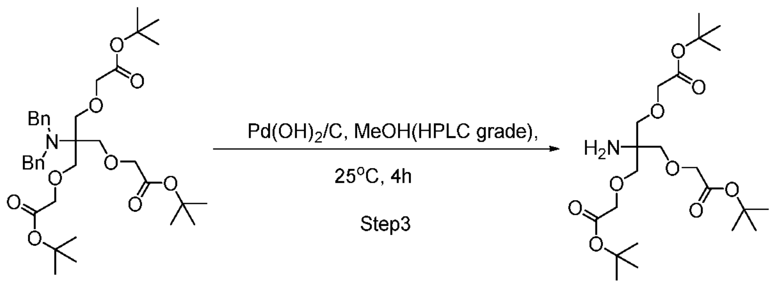


Bn-LS-1

PH-HG-001-1

To a solution of Bn-LS-1 (1.0 g, 1.6 mmol, 1.0 eq.) in methanol (20 mL, chromatography grade) was added palladium hydroxide on carbon (0.2 g). The atmosphere in the reaction system was replaced 5 times with hydrogen, and the mixture was hydrogenated with stirring at 25°C for 4 hours. The reaction solution was filtered. The filtrate was concentrated to dryness by rotary-evaporation under reduced pressure to give 650 mg of PH-HG-001-1 (90%) as a colorless oil. This product was used directly in the next step without further purification.

MS m/z $[M+H]^+$ (ESI): 464.30.

**Step 4: Synthesis of PH-HG-001-2**

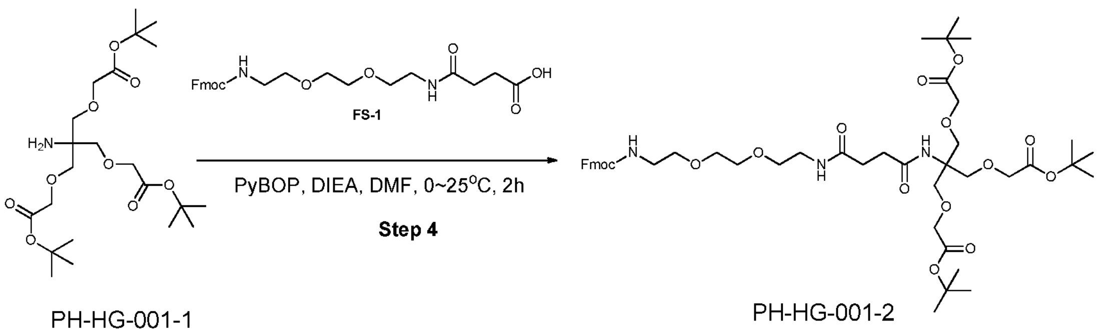


PH-HG-001-1

PH-HG-001-2

To a solution of PH-HG-001-1 (600.0 mg, 1.3 mmol, 1.0 eq.) in DMF (10.0 mL) were added FS-1 (650.3 mg, 1.4 mmol, 1.1 eq.), N,N-diisopropylethylamine (494.4 mg, 3.8 mmol, 3.0 eq.) and 1H-benzotriazole-1-yl-oxytripyrrolidinophosphonium hexafluorophosphate (PyBOP) (995.4 mg, 1.9 mmol, 1.5 eq.) under nitrogen at 0°C. The reaction solution was stirred at 25°C for 2 hours, and then quenched with 100 mL of water. The mixture was extracted with ethyl acetate (3×100 mL). The combined organic phase was washed with saturated brine (3×100 mL), dried over anhydrous sodium sulfate, and filtered. The filtrate was concentrated by rotary-evaporation. The crude product was purified via a silica gel column eluted with dichloromethane:methanol=30:1 to give 900 mg (77%) of PH-HG-001-2 as a colorless oil.

MS m/z $[M+H]^+$ (ESI): 916.50.

**Step 5: Synthesis of PH-HG-001-3**

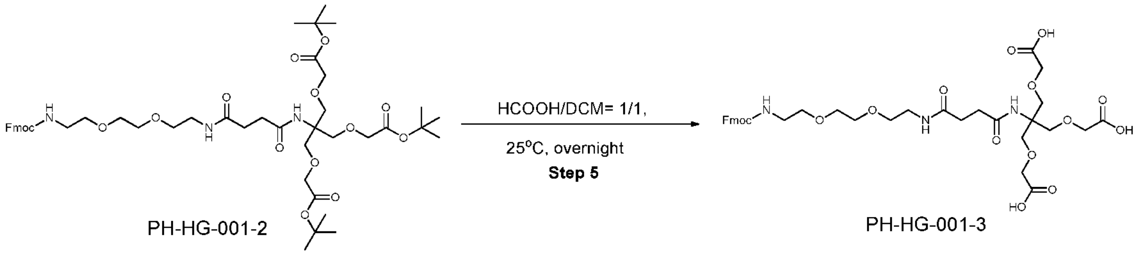


PH-HG-001-2

PH-HG-001-3

To a solution of PH-HG-001-2 (1.1 g, 1.2 mmol, 1.0 eq.) in dichloromethane (5.0 mL) was added formic acid (5.0 mL) under nitrogen. The reaction solution was stirred overnight at 25°C and concentrated by rotary-evaporation. The crude product was purified by Flash-Prep-HPLC (conditions: C18 silica gel column; mobile phase water and acetonitrile (10% to 80% acetonitrile, 20 min), detector UV 210 nm) to give 720 mg (80%) of PH-HG-001-3, as a colorless oil.

MS m/z $[M+H]^+$ (ESI): 748.15. $^1H$ NMR (400 MHz, Methanol-$d_4$) $\delta$: 2.42-2.58 (m, 4H), 3.36-3.40 (m, 4H), 3.47-3.55 (m, 4H), 3.57-3.64 (m, 4H), 3.89 (s, 6H), 4.09 (s, 6H), 4.20-4.24 (m, 1H), 4.37-4.39 (m, 2H), 7.30-7.32 (m, 2H), 7.38-7.42 (m, 2H), 7.66 (d, *J* = 7.2 Hz, 2H), 7.81 (d, *J* = 7.2 Hz, 2H).

**Step 6: Synthesis of PH-HG-001-14**

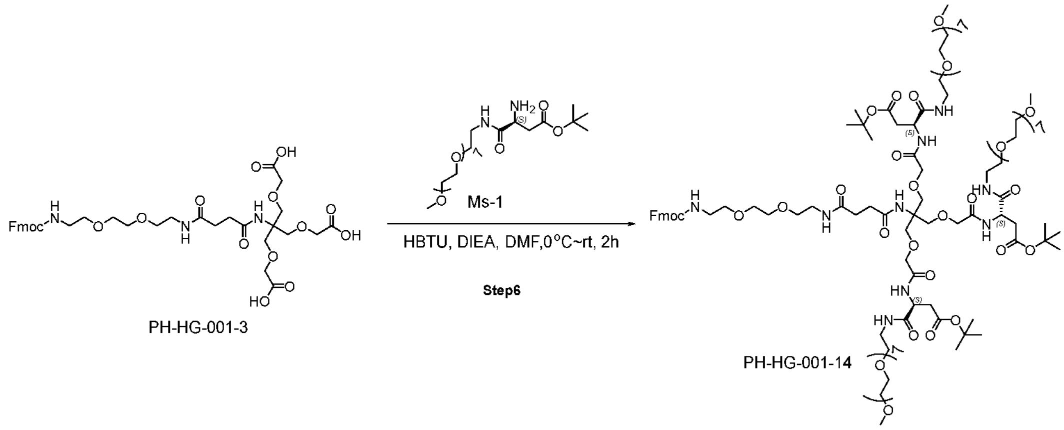


To a solution of PH-HG-001-3 (265.0 mg, 0.35 mmol, 1.0 eq) in DMF (10.0 mL) were added successively Ms-1 (648.7 mg, 1.2 mmol, 3.3 eq), N,N-diisopropylethylamine (206.1 mg, 1.6 mmol, 4.5 equivalents) and benzotriazole-N,N,N',N'-tetramethyluronium hexafluorophosphate (HBTU) (604.8 mg, 1.6 mmol, 4.5 eq.) at 0°C under nitrogen. The reaction solution was stirred at 25°C for 2 hours, diluted with 100 mL of water, and extracted with dichloromethane (3×200 mL). The combined organic phase was dried over anhydrous sodium sulfate, and filtered. The filtrate was concentrated by rotary-evaporation. The crude product was purified by Flash-Prep-HPLC (conditions: C18 silica gel column; mobile phase water and acetonitrile (10% to 100% acetonitrile, 20 minutes), detector UV 210 nm) to give 550 mg (66%) of PH -HG-001-14 as a colorless oil.

MS m/z $[M/2+H]^+$ (ESI): 1179.45.

**Step 7: Synthesis of PH-HG-001-6**

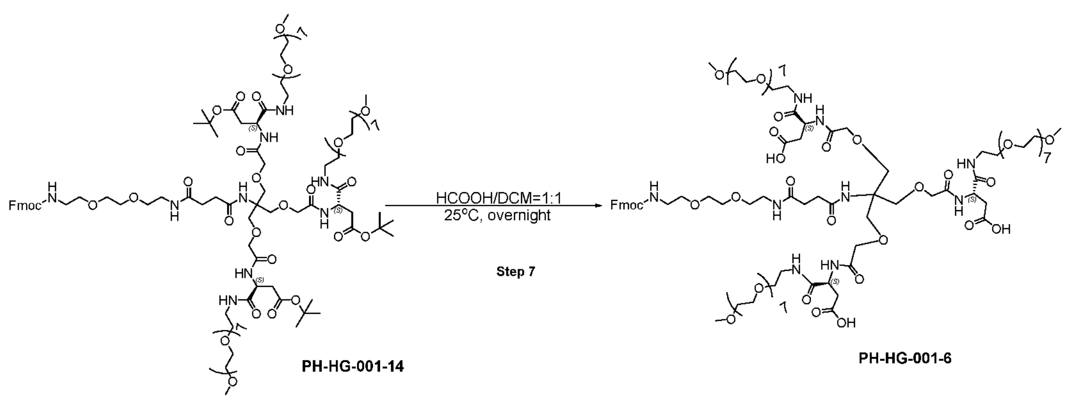


To a solution of PH-HG-001-14 (550.0 mg, 0.2 mmol, 1.0 eq.) in dichloromethane (2.5 mL) was added formic acid (2.5 mL) at 25 °C under nitrogen. The reaction solution was stirred overnight at 25°C and then concentrated by rotary-evaporation. The crude product was purified by Flash-Prep-HPLC (conditions: C18 silica gel column; mobile phase

water and acetonitrile (10% to 80% acetonitrile, 20 min), detector UV 210 nm) to give 360 mg (70%) of PH -HG-001-6 as a colorless oil.

MS m/z $[M/2+H]^+$ (ESI): 1095.40. $^1H$ NMR (300 MHz, Methanol-$d_4$) $\delta$: 2.48-2.55 (m, 4H), 2.85-2.87 (m, 6H), 3.35-3.40 (m, 19H), 3.53-3.60 (m, 17H), 3.62-3.64 (m, 81H), 3.89 (s, 6H), 4.09 (m, 6H), 4.38 (m, 3H), 4.77-4.81 (m, 3H), 7.33-7.42 (m, 4H), 7.68 (d, J= 7.2 Hz, 2H), 7.83 (d, *J* = 7.5 Hz, 2H).

**Step 8: Synthesis of PH-HG-001-9**

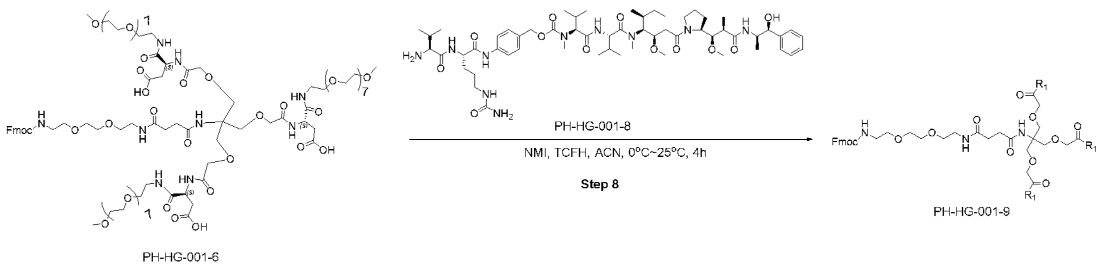


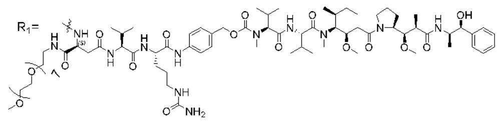

To a solution of PH-HG-001-6 (150.0 mg, 0.07 mmol, 1.0 eq.) in acetonitrile (3.0 mL) were added PH-HG-001-8 (254.0 mg, 0.23 mmol, 3.3 eq.), N-methylimidazole (50.6 mg, 0.63 mmol, 9.0 eq.) and N,N,N',N'-tetramethylchloroformamidine hexafluorophosphate (TCFH) (69.2 mg, 0.25 mmol, 3.6 eq.) at 0°C under nitrogen. The reaction solution was stirred at 25°C for 4 hours and concentrated by rotary-evaporation. The crude product was purified by preparative HPLC (conditions: X Select CSHPrep C18 OBDTM column; size 19mm×250mm, 5 μm; mobile phase, water (0.05% formic acid) and acetonitrile (54.0% to 68.0% acetonitrile, 10 min); detector, UV 254 nm) to give 160 mg (42%) of product PH-HG-001-9 as a white solid.

MS m/z $[M/4+H]^+$ (ESI): 1377.00.

**Step 9: Synthesis of PH-HG-001-10**

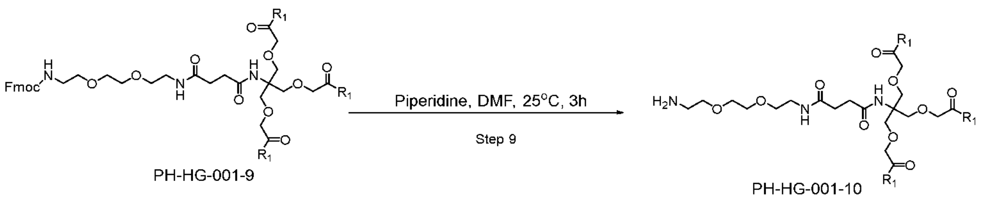


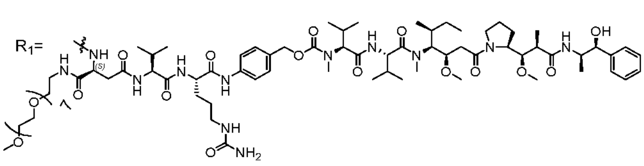

To a solution of PH-HG-001-9 (150.0 mg, 0.027 mmol, 1.0 eq.) in DMF (1.5 mL) was added piperidine (0.5 mL) under nitrogen. The reaction solution was stirred at 25°C for 3 h and then concentrated by rotary-evaporation. The crude product was purified by preparative HPLC (conditions: X Select CSH Prep C18 OBDTM column; size 19 mm × 250 mm, 5 μm; mobile phase, water (0.05% formic acid) and acetonitrile (41.0% to 50.0% acetonitrile, 10 min); detector, UV 254 nm) to give 90 mg (43%) of product PH-HG-001-10 as a white solid.

MS m/z $[M/4+H]^+$ (ESI): 1321.45.

**Step 10: Synthesis of HG-PL1**

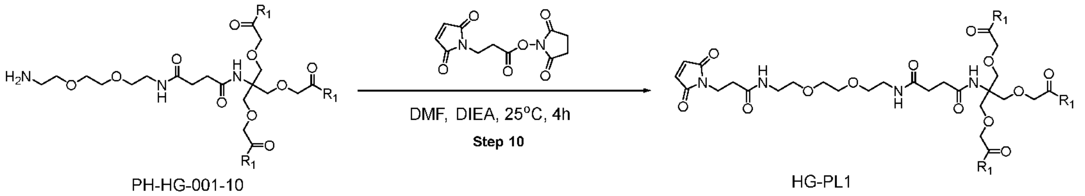


PH-HG-001-10 HG-PL1

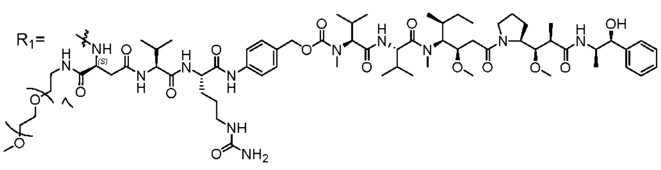

To a solution of PH-HG-001-10 (215.0 mg, 0.04 mmol, 1.0 eq.) in DMF (4.0 mL) were added succinimidyl 3-maleimidopropionate (21.7 mg, 0.08 mmol, 2.0 eq.) and N,N-diisopropylethylamine (15.8 mg, 0.12 mmol, 3.0 eq.). The reaction solution was stirred at 25 °C for 4 h and then concentrated by rotary-evaporation. The crude product was purified by preparative HPLC (conditions: X Select CSH Prep C18 OBDTM column; size 19 mm × 250 mm, 5 μm; mobile phase, water (0.05% formic acid) and acetonitrile (41.0% to 71.0% acetonitrile, 10 min); detector, UV 254 nm) to give 126.4 mg (57%) of product HG-PL1 as a white solid.

MS m/z $[M/3+H]^+$ (ESI): 1811.90. $^1H$ NMR (400 MHz, DMSO-$d_6$) δ: 0.75-0.77 (m, 30H), 0.83-0.84 (m, 49H), 0.88-0.97 (m, 15H), 0.98-0.99 (m, 6H), 1.01-1.03 (m, 7H), 1.04-1.05 (m, 9H), 1.10-1.20 (m, 2H), 1.30-1.48 (m, 14H), 1.50-1.73 (m, 7H), 1.99-2.01 (m, 5H), 2.32-2.34 (m, 8H), 2.30-2.39 (m, 5H), 2.40-2.50 (m, 3H), 2.83-2.85 (m, 11H), 2.87-2.87 (m, 11H), 2.88-2.97 (m, 9H), 3.12-3.15 (m, 7H), 3.17-3.19 (m, 8H), 3.23-3.24 (m, 24H), 3.33-3.35 (m, 2H), 3.40-3.41 (m, 9H), 3.42-3.43 (m, 4H), 3.45-3.49 (m, 64H), 3.57-3.59 (m, 5H), 3.60-3.61 (m, 7H), 3.70-3.76 (m, 13H), 3.92-3.96 (m, 7H), 4.00-4.27 (m, 10H), 4.40-4.49 (m, 6H), 5.00-5.10 (m, 5H), 5.40-5.43 (m, 9H), 5.99 (s, 3H), 6.99 (s, 2H), 7.26-7.28 (m, 4H), 7.29-7.32 (m, 22H), 7.60-7.62 (m, 9H), 8.02-8.10 (m, 18H), 8.11-8.20 (m, 4H), 9.80 (s, 3H).

**Example 6 Synthesis of HG-PL2**

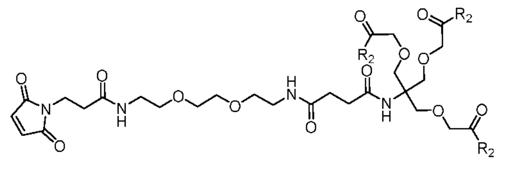

HG-PL2

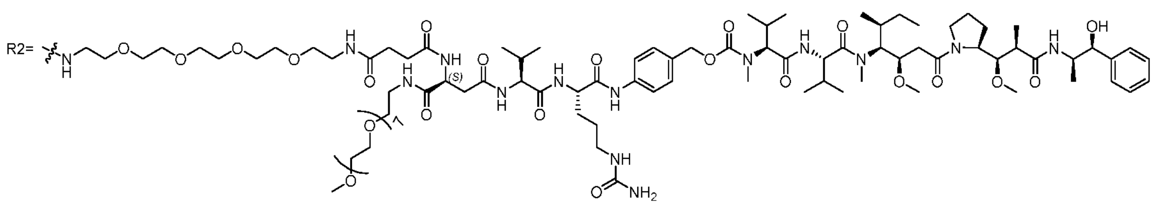

**Step 1: Synthesis of PH-HG-002-1**

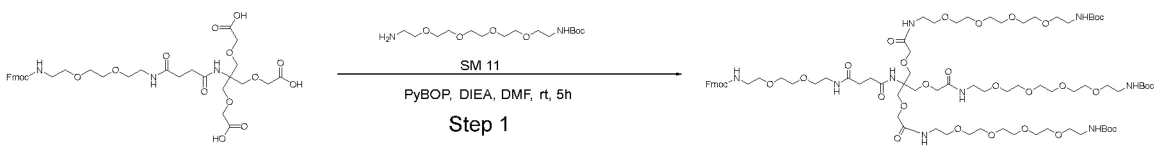


PH-HG-001-3 PH-HG-002-1

To a solution of PH-HG-001-3 (3.8g, 5.1mmol, 1.0 eq.) in DMF (60mL) were added successively N,N-diisopropylethylamine (3.0g, 23.2mmol, 4.5 eq.), SM11 (5.7g, 17.0mmol, 3.3 eq.) and 1H-benzotriazole-1-yl-oxytripyrrolidino hexafluorophosphate (PyBOP) (12.1g, 23.3mmol, 4.5 eq.) under nitrogen. The reaction solution was stirred at 25°C for 5 hours, then diluted with 300 mL of water, and extracted with ethyl acetate (3×300 mL). The combined organic phase was washed with saturated brine (3×300mL), dried over anhydrous sodium sulfate, and filtered. The filtrate was concentrated by rotary-evaporation. The crude product was purified by Flash-Prep-HPLC (conditions: C18 silica gel column; mobile phase water and acetonitrile (10% to 100% acetonitrile, 20 minutes), detector UV 210 nm) to give 6.1 g (70%) of PH-HG-002-1 as a colorless oil.

MS m/z $[M+H]^+$ (ESI): 1702.93. $^1H$ NMR (400 MHz, DMSO-$d_6$) $\delta$: 1.30-1.42 (s, 27H), 2.25-2.40 (m, 4H), 3.03-3.08 (m, 6H), 3.12-3.20 (m, 4H), 3.25-3.29 (m, 6H), 3.35-3.44 (m, 18H), 3.49-3.50 (m, 38H), 3.71 (s, 6H), 3.88 (s, 6H), 4.21-4.30 (m, 3H), 6.71-6.73 (m, 3H), 7.33 (t, $J$ = 7.6 Hz, 3H), 7.42 (t, $J$ = 7.6 Hz, 2H), 7.68-7.75 (m, 6H), 7.88-7.90 (m, 3H).

**Step 2: Synthesis of PH-HG-002-2**

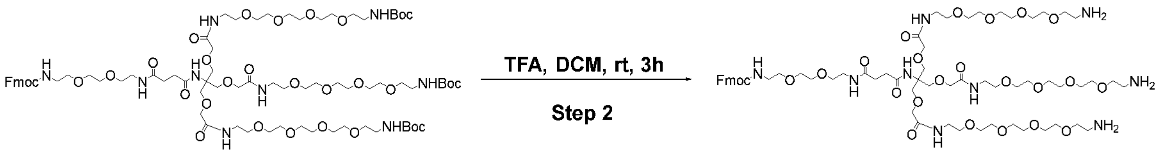


**PH-HG-002-1** **PH-HG-002-2**

To a solution of PH-HG-002-1 (3.1g, 1.8mmol, 1.0 eq.) in dichloromethane (15mL) was added trifluoroacetic acid (15mL) under nitrogen. The reaction solution was stirred at 25°C for 3 hours, and then concentrated by rotary-evaporation. The crude product was purified by Flash-Prep-HPLC (conditions: C18 silica gel column; mobile phase water and acetonitrile (10% to 80% acetonitrile, 20 minutes), detector UV 210 nm) to give 1.6g (64%) of PH-HG-002-2 as a colorless oil.

MS m/z [M/2+H]+ (ESI): 1402.77.

**Step 3: Synthesis of PH-HG-002-3**

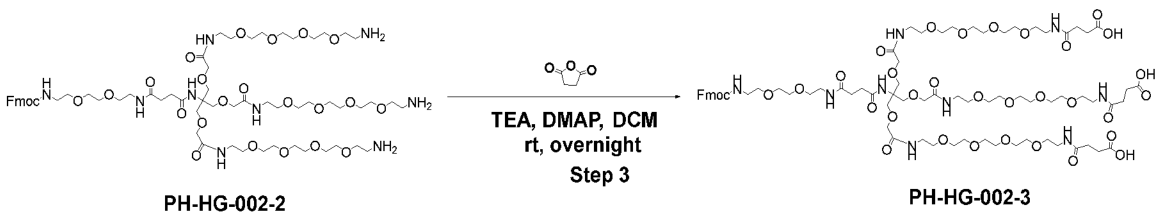


**PH-HG-002-2** **PH-HG-002-3**

To a solution of PH-HG-002-2 (3.0g, 2.1mmol, 1.0 eq.) in dichloromethane (45mL) were added succinic anhydride (1.9g, 19.3mmol, 9.0 eq.), triethylamine (2.6g, 25.7mmol, 12.0 eq.), and 4-dimethylaminopyridine (0.8g, 6.4mmol, 3.0 eq.) under nitrogen at 0°C. The reaction solution was stirred at 25°C for 12 hours, and then concentrated by rotary-evaporation. The crude product was purified by Flash-Prep-HPLC (conditions: C18 silica gel column; mobile phase water and acetonitrile (10% to 80% acetonitrile, 30 minutes), detector UV 210 nm) to give 1.6 g (44%) of PH-HG-002-3 as a colorless oil.

MS m/z $[M/2+H]^+$ (ESI): 1402.77. $^1H$ NMR (300 MHz, DMSO-$d_6$) $\delta$: 2.34-2.39 (m, 10H), 2.41-2.44 (m, 6H),

3.13-3.28 (m, 16H), 3.37-3.51 (m, 56H), 3.72 (s, 6H), 3.89 (s, 6H), 4.19-4.31 (m, 3H), 7.32 (t, *J* = 7.5 Hz, 3H), 7.43 (t, *J* = 7.5 Hz, 2H), 7.68-7.77 (m, 6H), 7.88-7.91 (m, 6H), 12.00-12.06 (m, 2H).

**Step 4: Synthesis of PH-HG-002-4**

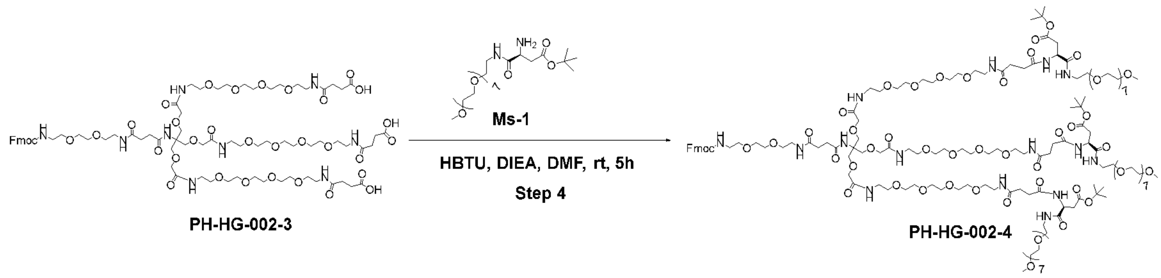


To a solution of PH-HG-002-3 (1.6g, 0.9mmol, 1.0 eq.) in DMF (30mL) were added Ms-1 (1.7g, 3.1mmol, 3.3 eq.), N,N-diisopropylethylamine (546.5mg, 4.2mmol, 4.5 eq.) and benzotriazole-N,N,N',N'-tetramethyluronium hexafluorophosphate (HBTU) (1.6g,4.2mmol, 4.5 eq.) under nitrogen at 0°C. The reaction solution was stirred at 25°C for 5 hours, then diluted with 300 mL of water, and extracted with ethyl acetate (3×300 mL). The combined organic phase was washed with saturated brine (3×300mL), dried over anhydrous sodium sulfate, and filtered. The filtrate was concentrated by rotary-evaporation. The crude product was purified by Flash-Prep-HPLC (conditions: C18 silica gel column; mobile phase water (with 0.1% formic acid) and acetonitrile (10% to 100% acetonitrile, 20 minutes), detector UV 210 nm) to give 1.7 g (55%) of PH-HG-002-4 as a yellow oil.

MS m/z $[M/2+H]^+$ (ESI): 1657.15.

**Step 5: Synthesis of PH-HG-002-5**

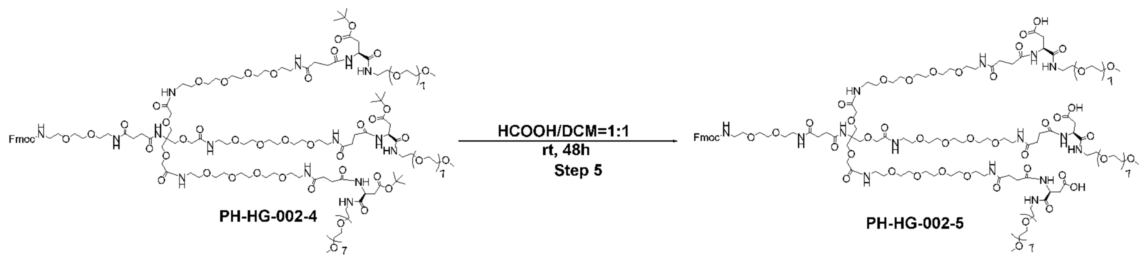


To a solution of PH-HG-002-4 (1.0g, 0.3mmol, 1.0 eq.) in dichloromethane (5mL) was added formic acid (5.0mL) under nitrogen. The reaction solution was stirred at 25°C for 48 hours, and then concentrated by rotary-evaporation. The crude product was purified by Flash-Prep-HPLC (conditions: C18 silica gel column; mobile phase water (with 0.1% formic acid) and acetonitrile (10% to 80% acetonitrile, 30 minutes), detector UV 210 nm) to give 700 mg (74%) of PH-HG-002-5 as a colorless oil.

MS m/z $[M/2+H]^+$ (ESI): 1573.20. $^1$H NMR (300 MHz, Methanol-$d_4$) $\delta$: 2.48-2.58 (m, 16H), 2.65-2.84 (m, 6H), 3.20-3.30 (m, 3H), 3.33-3.47 (m, 30H), 3.53-3.65 (m, 144H), 3.88 (s, 6H), 4.03 (s, 6H), 4.20-4.40 (m, 3H), 4.73-4.85 (m, 3H), 7.34-7.42 (m, 4H), 7.67 (m, 2H), 7.83 (d, *J* = 7.5 Hz, 2H).

**Step 6: Synthesis of PH-HG-002-6**

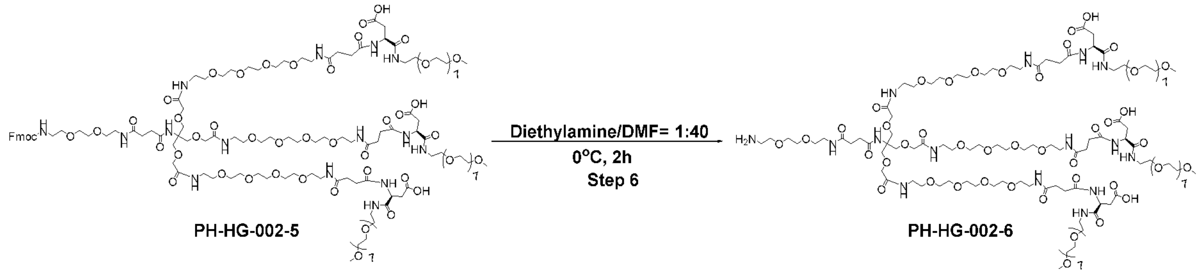


PH-HG-002-5

PH-HG-002-6

To a solution of PH-HG-002-5 (300mg, O.lmmol, 1.0 eq.) in DMF (4mL) was added diethylamine (0.1mL) under nitrogen at 0°C. The reaction solution was stirred at 0°C for 2 hours and then purified by preparative HPLC (conditions: X Select CSH Prep C18 OBD column; size 19mm×250mm, 5 μm; mobile phase, water (0.05% formic acid) and acetonitrile (8.0% to 23.0% acetonitrile, 11 min); detector, UV 200 nm) to give 140 mg (50%) of product PH-HG-002-6 as a colorless oil.

MS m/z $[M/2+H]^+$ (ESI): 1461.95.

**Step 7: Synthesis of PH-HG-002-7**

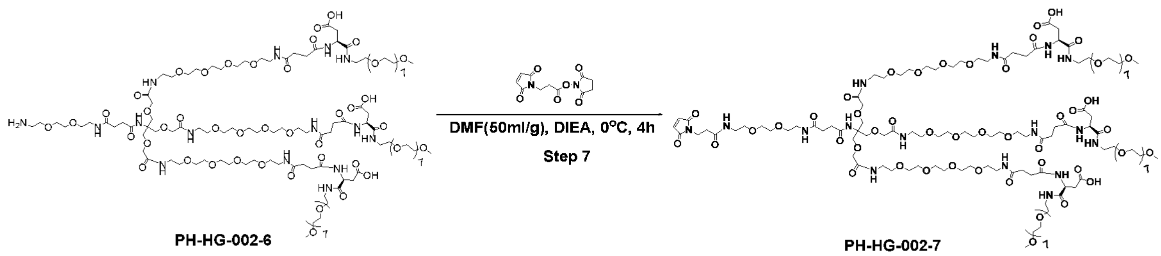


PH-HG-002-6

PH-HG-002-7

To a solution of PH-HG-002-6 (140mg, O.lmmol, 1.0 eq.) in DMF (5mL) were added succinimidyl 3-maleimidopropionate (19.1mg, 0.07mmol, 1.5 eq.) and N,N-diisopropylethylamine (10.5mg, 0.14mmol, 3.0 eq.) under nitrogen. The reaction solution was stirred at 0°C for 4 hours and then purified by preparative HPLC (conditions: XBridge Prep C18 OBD column; size 30mm×150mm, 5 μm; mobile phase, water (0.05% formic acid ) and acetonitrile (8.0% to 38.0% acetonitrile, 7 min); detector, UV 200 nm) to give 75 mg (51%) of product PH-HG-002-7 as a colorless oil.

MS m/z $[M/2+H]^+$ (ESI): 1537.55.

**Step 8: Synthesis of HG-PL2**

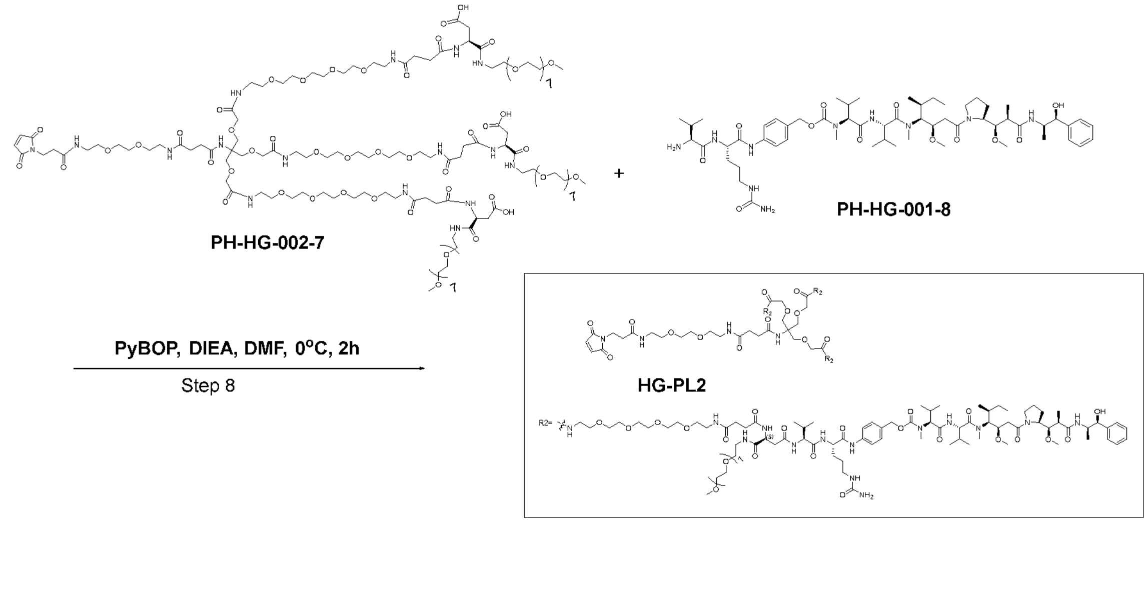


PH-HG-002-7

PH-HG-001-8

HG-PL2

To a solution of PH-HG-002-7 (120mg, 0.039mmol, 1.0 eq.) in DMF (4mL) were added PH-HG-001-8 (144.8 mg, 0.13 mmol, 3.3 eq.), N,N-diisopropylethylamine (22.7 mg, 0.18mmol, 4.5 eq.), and 1H-benzotriazole-1-yl-oxytripyrrolidino hexafluorophosphate (PyBOP) (91.4mg, 0.18mmol, 4.5 eq.) under nitrogen at 0°C. The reaction solution was stirred at 0°C for 2 hours and then purified by preparative HPLC (conditions: XBridge Prep C18 OBD column; size 30mm×150mm, 5 μm; mobile phase, water (0.01% formic acid) and acetonitrile (35.0% to 65.0% acetonitrile, 7 min); detector, UV 254 nm) to give 23.4mg (9%) of product HG-PL2 as a white solid.

MS m/z $[M/4+H]^+$ (ESI): 1598.50. $^1H$ NMR (400 MHz, DMSO-$d_6$) δ: 0.75-1.05 (m, 101H), 1.24-1.39 (m, 7H), 1.42-1.54 (m, 11H), 1.64-1.82 (m, 16H), 1.90-2.15 (m, 13H), 2.21-2.47 (m, 31H), 2.67-2.78 (m, 5H), 2.85-3.09 (m, 23H), 3.12-3.30 (m, 76H), 3.38-3.60 (m, 96H), 3.62-3.76 (m, 11H), 3.88-4.05 (m, 14H), 4.14-4.37 (m, 11H), 4.39-4.55 (m, 11H), 4.62-4.77 (m, 4H), 4.93-5.12 (m, 7H), 5.37-5.45 (m, 10H), 5.97-6.02 (m, 3H), 7.00 (s, 2H), 7.18-7.32 (m, 23H), 7.59-7.63 (m, 8H), 7.74-7.77 (m, 4H), 7.88-8.08 (m, 16H), 8.19-8.34 (m, 8H), 9.72 (s, 3H).

**Example 7 Synthesis of HG-PL3**

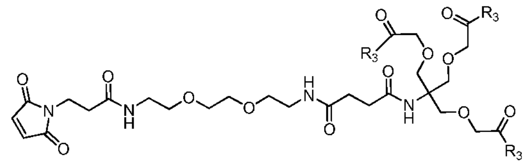

HG-PL3

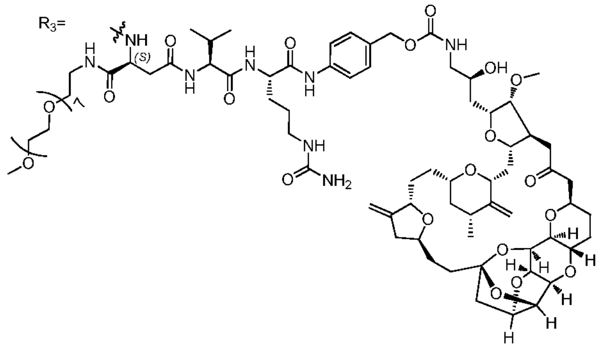

**Step 1: Synthesis of PH-HG-003-1**

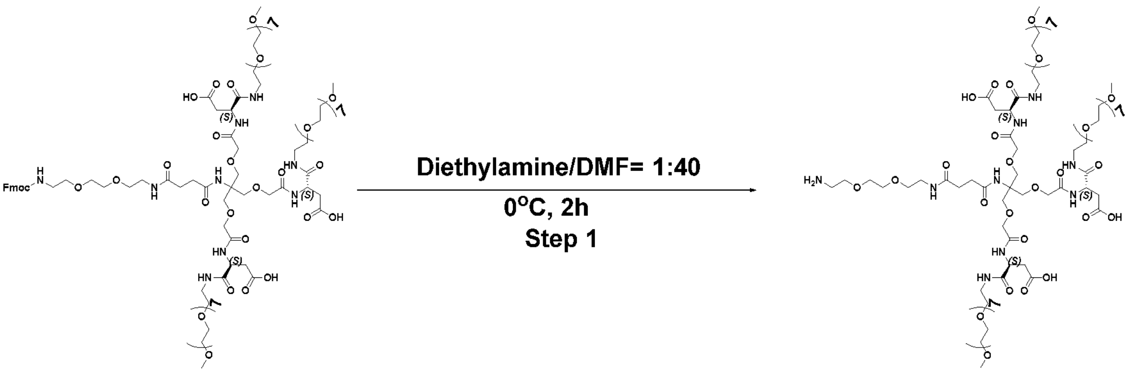


PH-HG-001-6

PH-HG-003-1

To a solution of PH-HG-001-6 (1.0g, O.lmmol, 1.0 eq.) in DMF (10mL) was added diethylamine (0.25mL) under nitrogen at 0°C. The reaction solution was stirred at 0°C for 2 hours and then purified by preparative HPLC (conditions:

XSelect CSH Prep C18 OBD column; size 19mm×250mm, 5 μm; mobile phase, water (0.1% formic acid ) and acetonitrile (8.0% to 23.0% acetonitrile, 11 min); detector, UV 200 nm) to give 400 mg (45%) of product PH-HG-003-1 as a colorless oil.

MS m/z $[M+H]^+$ (ESI):1966.55.

**Step 2: Synthesis of PH-HG-003-2**

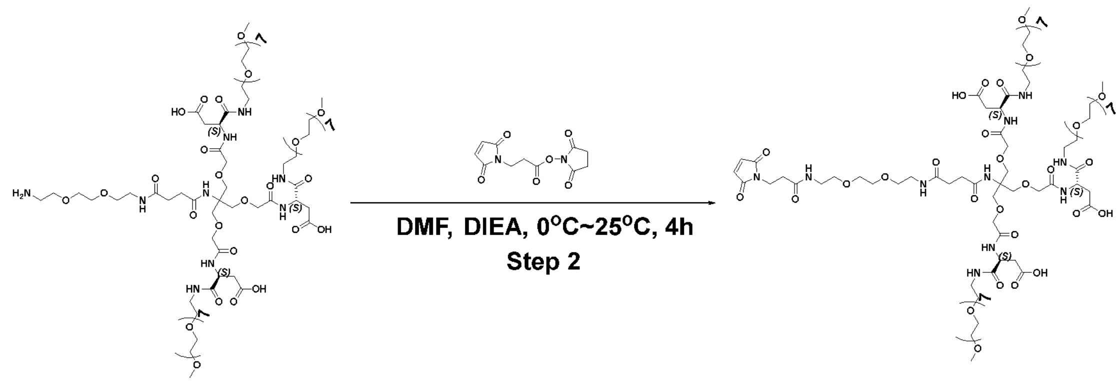


PH-HG-003-1

PH-HG-003-2

To a solution of PH-HG-003-1 (100mg, 0.05mmol, 1.0 eq.) in DMF (2.0mL) were added succinimidyl 3-maleimidopropionate (20.3mg, 0.08mmol, 1.5 eq.) and N,N-diisopropylethylamine (19.7mg, 0.15mmol, 3.0 eq.) under nitrogen. The reaction solution was stirred at 0°C for 4 hours and then purified by preparative HPLC (conditions: XBridge Prep C18 OBD column; size 30mm×150mm, 5 μm; mobile phase, water (0.1% formic acid) and acetonitrile (17.0% to 27.0% acetonitrile, 10 min); detector, UV 200 nm) to give 50 mg (46%) of product PH-HG-003-2 as a colorless oil.

MS m/z $[M/2+H]^+$ (ESI): 1059.95.

**Step 3: Synthesis of PH-HG-003-3**

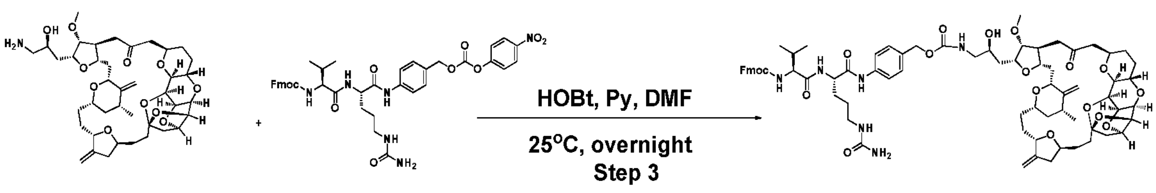


Eribulin CS-2 PH-HG-003-3

To a solution of CS-2 (88mg, 0.115mmol, 1.0 eq.) in DMF (2.0mL) were added eribulin (100.5mg, 0.138mmol, 1.2 eq.), 1-hydroxybenzotriazole (HOBT) (3.1mg, 0.023mmol, 0.2 eq.) and pyridine (2.7 mg, 0.035 mmol, 0.3 eq.) under nitrogen. The reaction solution was stirred at 25°C overnight, diluted with 20mL of water, and extracted with dichloromethane (3×20mL). The combined organic phase was washed with saturated brine (3×20mL), dried over anhydrous sodium sulfate, and filtered. The filtrate was concentrated by rotary-evaporation. The crude product was purified by Flash-Prep-HPLC (conditions: C18 silica gel column; mobile phase water and acetonitrile (10% to 100% acetonitrile, 30 minutes), detector UV 254 nm) to give 60mg (39%) of PH-HG-003-3 as a white solid.

MS m/z $[M+H]^+$ (ESI): 1357.68.

**Step 4: Synthesis of PH-HG-003-4**

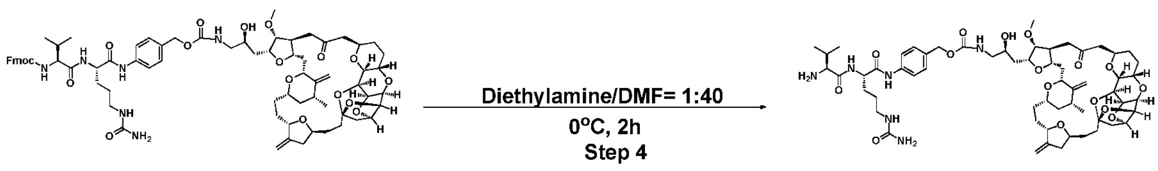


PH-HG-003-3

PH-HG-003-4

To a solution of PH-HG-003-3 (100 mg, 0.07mmol, 1.0 eq.) in DMF (2.0mL) was added diethylamine (0.05mL) under nitrogen at 0°C. The reaction solution was stirred at 0°C for 2 hours and then concentrated. The crude product was purified by Flash-Prep-HPLC (conditions: C18 silica gel column; mobile phase water and acetonitrile (10% to 100% acetonitrile, 30 minutes), detector UV 210 nm) to give 70mg (84%) of PH-HG-003-4 as a white solid.

MS m/z $[M+H]^+$(ESI): 1135.61; $^1H$ NMR (300 MHz, DMSO-$d_6$) δ: 0.81 (d, *J* = 6.6 Hz, 3H), 0.90 (d, *J* = 6.9 Hz, 3H), 1.02-1.05 (m, 3H), 1.1.12-1.20 (m, 2H), 1.27-1.40 (m, 4H), 1.47-1.58 (m, 4H), 1.62-1.75 (m, 7H), 1.88-2.05 (m, 7H), 2.11-2.13 (m, 1H), 2.18-2.28 (m, 4H), 2.32-2.35 (m, 1H), 2.58-2.61 (m, 1H), 2.65-2.75 (m, 2H), 2.81-2.87 (m, 2H), 2.93-3.05 (m, 4H), 3.07-3.11 (m, 1H), 3.21-3.24 (m, 4H), 3.45-3.57 (m, 4H), 3.68-3.83 (m, 3H), 4.01-4.13 (m, 4H), 4.18-4.31 (m, 2H), 4.48-4.65 (m, 4H), 4.75-4.85 (m, 2H), 4.91-5.09 (m, 4H), 5.42 (s, 2H), 5.98-6.02 (m, 1H), 7.09-7.11 (m, 1H), 7.28 (d, *J* = 8.1 Hz, 2H), 7.58 (d, *J* = 8.4 Hz, 2H), 8.21-8.25 (m, 1H), 10.14 (s, 1H).

**Step 5: Synthesis of HG-PL3**

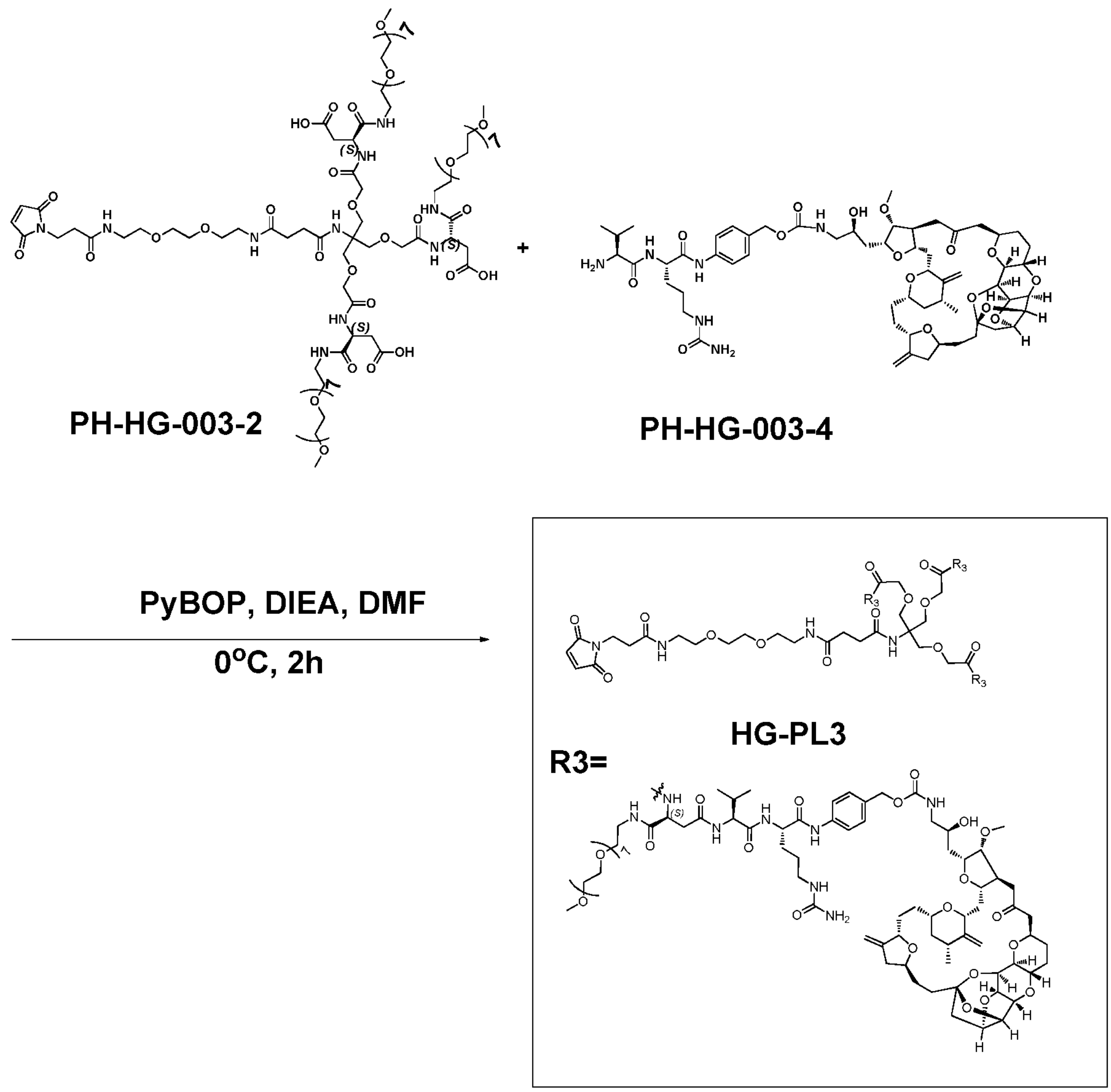


PH-HG-003-2

PH-HG-003-4

HG-PL3

To a solution of PH-HG-003-2 (40mg, 0.02mmol, 1.0 eq.) in DMF (1mL) were added PH-HG-003-4 (70.8 mg, 0.063 mmol, 3.3 eq.), N,N-diisopropylethylamine (11.0 mg, 0.085mmol, 4.5 eq.), and 1H-benzotriazole-1-yl-oxytripyrrolidino hexafluorophosphate (PyBOP) (44.2mg, 0.085mmol, 4.5 eq.) under nitrogen at 0°C. The reaction solution was stirred at 0°C for 2 hours and then purified by preparative HPLC (conditions: XBridge Shield RP18 OBD column; size 30mm×150mm, 5 μm; mobile phase, water and acetonitrile (36.0% to 66.0% acetonitrile, 10 min); detector, UV 254 nm) to give 14.2mg (14%) of product HG-PL3 as a white solid.

MS m/z $[M/3+H]^+$ (ESI): 1823.65.

**Example 8 Synthesis of HG-PL4**

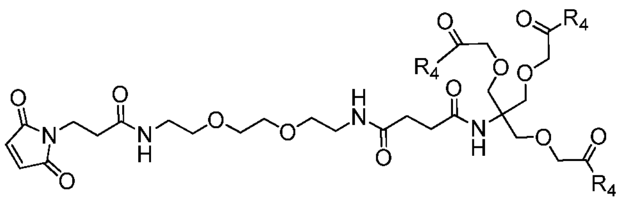

**HG-PL4**

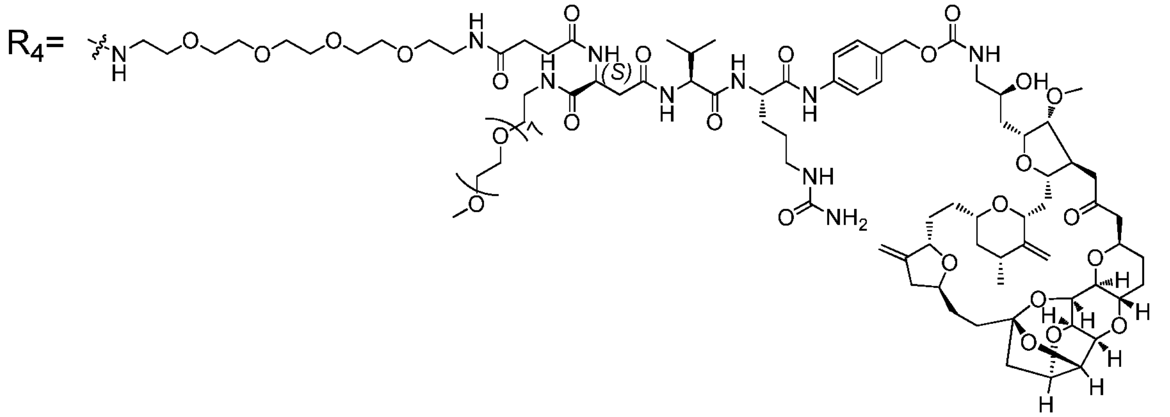

Step 1: Synthesis of HG-PL4

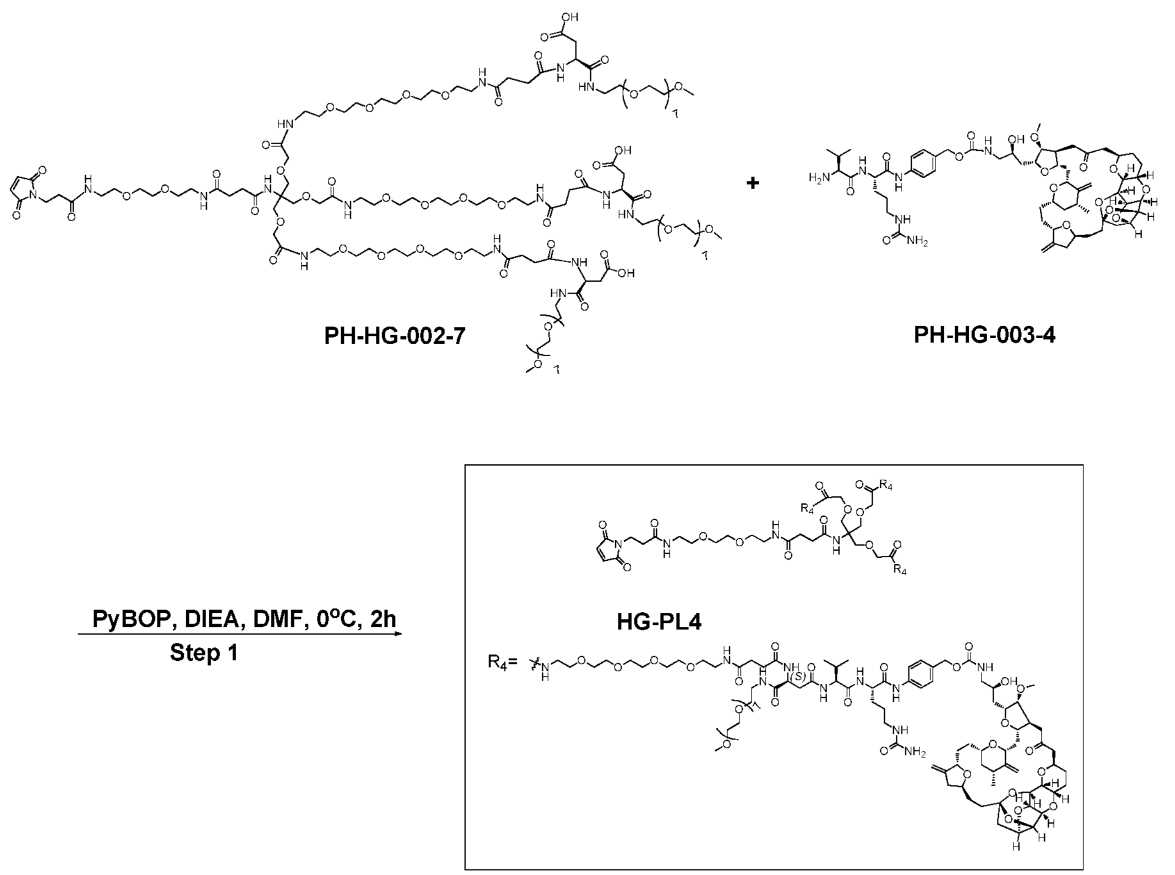


PH-HG-002-7

PH-HG-003-4

HG-PL4

To a solution of PH-HG-002-7 (65mg, 0.021mmol, 1.0 eq.) in DMF (2.0mL) were added PH-HG-003-4 (79.2 mg, 0.069 mmol, 3.3 eq.), N,N-diisopropylethylamine (12.3 mg, 0.095mmol, 4.5 eq.), and 1H-benzotriazole-1-yl-oxytripyrrolidino hexafluorophosphate (PyBOP) (49.5mg, 0.095mmol, 4.5 eq.) under nitrogen at 0°C. The reaction solution was stirred at 0°C for 2 hours and then purified by preparative HPLC (conditions: XBridge Shield RP18 OBD column; size 19mm×150mm, 5 μm; mobile phase, water and acetonitrile (35.0% to 65.0% acetonitrile, 10 min); detector, UV 254 nm) to give 20.9mg (15%) of product HG-PL4 as a white solid.

MS m/z $[M/4+H]^+$ (ESI): 1606.80. $^1$H NMR (400 MHz, DMSO-$d_6$) δ: 0.75-0.80 (m, 34H), 0.90-0.97 (m, 22H), 1.12-1.43 (m, 51H), 1.59-1.70 (m, 38H), 1.84-1.97 (m, 35H), 2.19-2.25 (m, 41H), 2.67-2.77 (m, 21H), 2.90-2.95 (m, 24H), 3.10-3.18 (m, 53H), 3.64-3.68 (m, 23H), 3.76-3.82 (m, 16H), 3.98-4.11 (m, 30H), 4.19-4.26 (m, 10H), 4.49-4.59 (m, 17H), 4.66-4.78 (m, 6H), 4.85-4.98 (m, 14H), 5.31-5.38 (m, 5H), 6.97-7.23 (m, 2H), 7.58-7.95 (m, 32H), 8.10-8.23 (m, 10H), 9.61 (m, 6H).

**Example 9 Synthesis** of **HG-PL5**

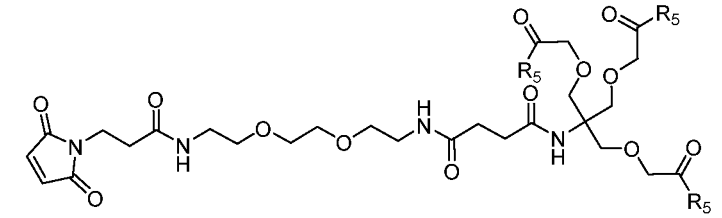

HG-PL5

R5=

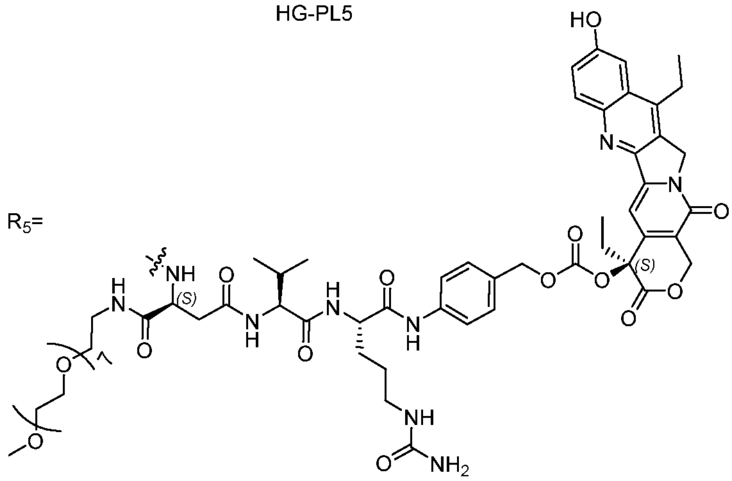

**Step 1: Synthesis of PH-HG-005-1**

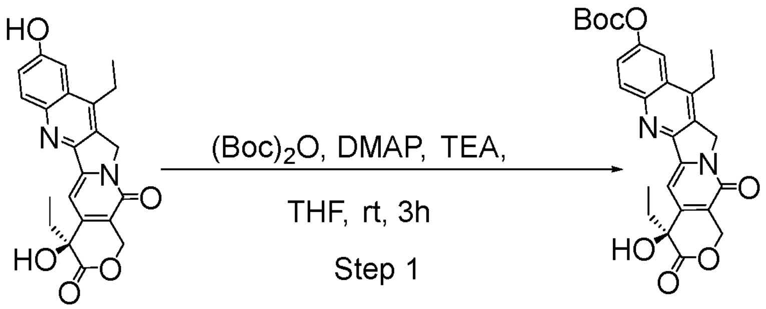


**SN-38** **PH-HG-005-1**

To a solution of SN-38 (2.5g, 6.4mmol, 1.0 eq.) in tetrahydrofuran (35.0mL) were added 4-(dimethylamino)pyridine (77.8mg, 0.64mmol, 0.1 eq.), triethylamine (1.9g, 19.1mmol, 3.0 eq.) and di-tert-butyl dicarbonate (1.67g, 7.7mmol, 1.2 eq.) under nitrogen at 0°C. The reaction solution was stirred at 25°C for 3 hours and then concentrated. The crude product was purified by Flash-Prep-HPLC (conditions: C18 silica gel column; mobile phase water and acetonitrile (10% to 100% acetonitrile, 30 minutes), detector UV 254 nm) to give 2.1g (64%) of PH-HG-005-1 as a yellow solid.

MS m/z $[M+H]^+$ (ESI): 493.19. $^1H$ NMR (300 MHz, DMSO-$d_6$) δ: 0.91 (t, *J* = 6.9 Hz, 3H), 1.31 (t, *J* = 7.5 Hz, 3H), 1.55 (s, 9H), 1.84-1.93 (m, 2H), 3.18-3.25 (m, 2H), 5.34 (s, 2H), 5.45 (s, 2H), 6.54 (s, 1H), 7.34 (s, 1H), 7.74 (d, *J* = 9.3 Hz, 1H), 8.10 (s, 1H), 8.21 (d, *J* = 9.3 Hz, 1H).

**Step 2: Synthesis of PH-HG-005-2**

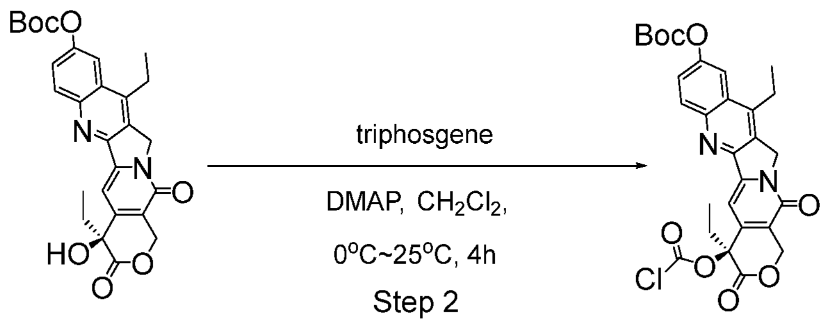


**PH-HG-005-1** **PH-HG-005-2**

To a solution of PH-HG-005-1 (500.0mg, 1.0mmol, 1.0 eq.) in dichloromethane (10.0mL) were added 4-(dimethylamino)pyridine (620.1mg, 5.1mmol, 5.0 eq.) and triphosgene (301.3mg, 1.0mmol, 1.0 eq.) under nitrogen at 0°C. The reaction solution was stirred at 25°C for 4 hours and then concentrated. This product was used directly in the next step without further purification.

**Step 3: Synthesis of PH-HG-005-3**

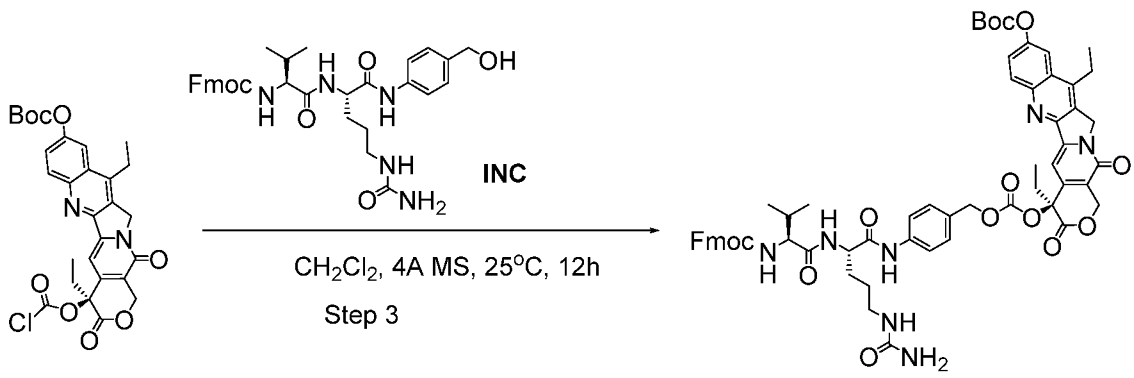


**PH-HG-005-2** **PH-HG-005-3**

To a solution of PH-HG-005-2 (crude product in Step 2) in dichloromethane (10.0mL) were added INC (650.5mg, 1.1mmol, 1.1 eq.) and 4A molecular sieve (500mg) under nitrogen at 0°C. The reaction solution was stirred at 25°C for 12 hours and then concentrated. The crude product was purified by Flash-Prep-HPLC (conditions: C18 silica gel column; mobile phase water and acetonitrile (10% to 100% acetonitrile, 30 minutes), detector UV 254 nm) to give 240mg (21% yield in two steps) of PH-HG-005-3 as a yellow solid.

MS m/z [M+H]+ (ESI): 1120.46; 1H NMR (300 MHz, DMSO-d6) δ: 0.86-0.91 (m, 9H), 1.28-1.30 (m, 3H), 1.39-1.43 (m, 2H), 1.55 (s, 9H), 1.62-1.75 (m, 2H), 1.98-2.07 (m, 1H), 2.11-2.23 (m, 2H), 2.90-3.10 (m, 2H), 3.18-3.23 (m, 2H), 3.91-3.97 (m, 1H), 4.21-4.32 (m, 3H), 4.41-4.47 (m, 1H), 5.06-5.16 (m, 2H), 5.37-5.42 (m, 4H), 5.54 (s, 2H),

5.98-6.02 (m, 1H), 7.06 (s, 1H), 7.33-7.37 (m, 4H), 7.40-7.42 (m, 3H), 7.54-7.63 (m, 2H), 7.75-7.78 (m, 3H), 7.87-7.91 (m, 2H), 8.12-8.15 (m, 2H), 8.24-8.26 (m, 1H), 10.11 (s, 1H).

**Step 4: Synthesis of PH-HG-005-4**

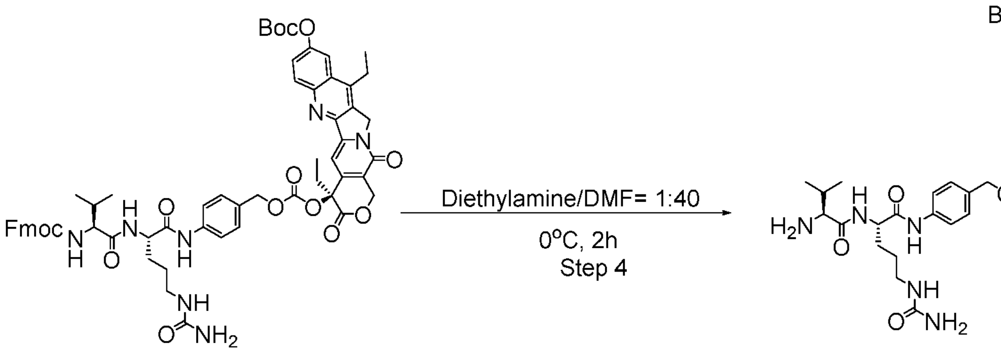


PH-HG-005-3 PH-HG-005-4

To a solution of PH-HG-005-3 (400.0mg, 0.36mmol, 1.0 eq.) in DMF (4.0mL) were added diethylamine (0.1mL) under nitrogen at 0°C. The reaction solution was stirred at 0°C for 2 hours and then concentrated. The crude product was purified by Flash-Prep-HPLC (conditions: C18 silica gel column; mobile phase water (10 mmol ammonium bicarbonate) and acetonitrile (10% to 80% acetonitrile, 20 minutes), detector UV 210 nm) to give 170mg (53%) of PH-HG-005-4 as a yellow solid.
MS m/z $[M+H]^+$ (ESI): 898.39.

**Step 5: Synthesis of PH-HG-005-5**

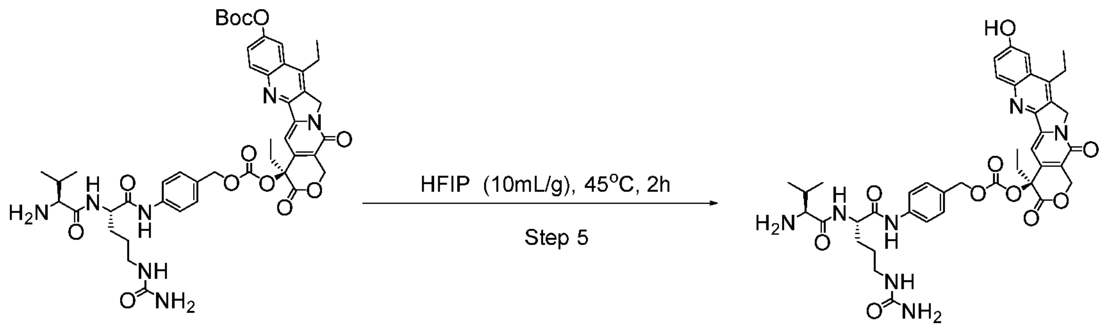


PH-HG-005-4 PH-HG-005-5

To a solution of PH-HG-005-4 (100.0mg, 0.11mmol, 1.0 eq.) in hexafluoroisopropanol (2.0mL) were added diethylamine (0.1mL) under nitrogen at 0°C. The reaction solution was stirred at 45°C for 2 hours and then concentrated. The crude product was purified by Flash-Prep-HPLC (conditions: C18 silica gel column; mobile phase water and acetonitrile (10% to 80% acetonitrile, 30 minutes), detector UV 210 nm) to give 45mg (51%) of PH-HG-005-5 as a yellow solid.
MS m/z $[M+H]^+$ (ESI): 798.34.

**Step 6: Synthesis of HG-PL5**

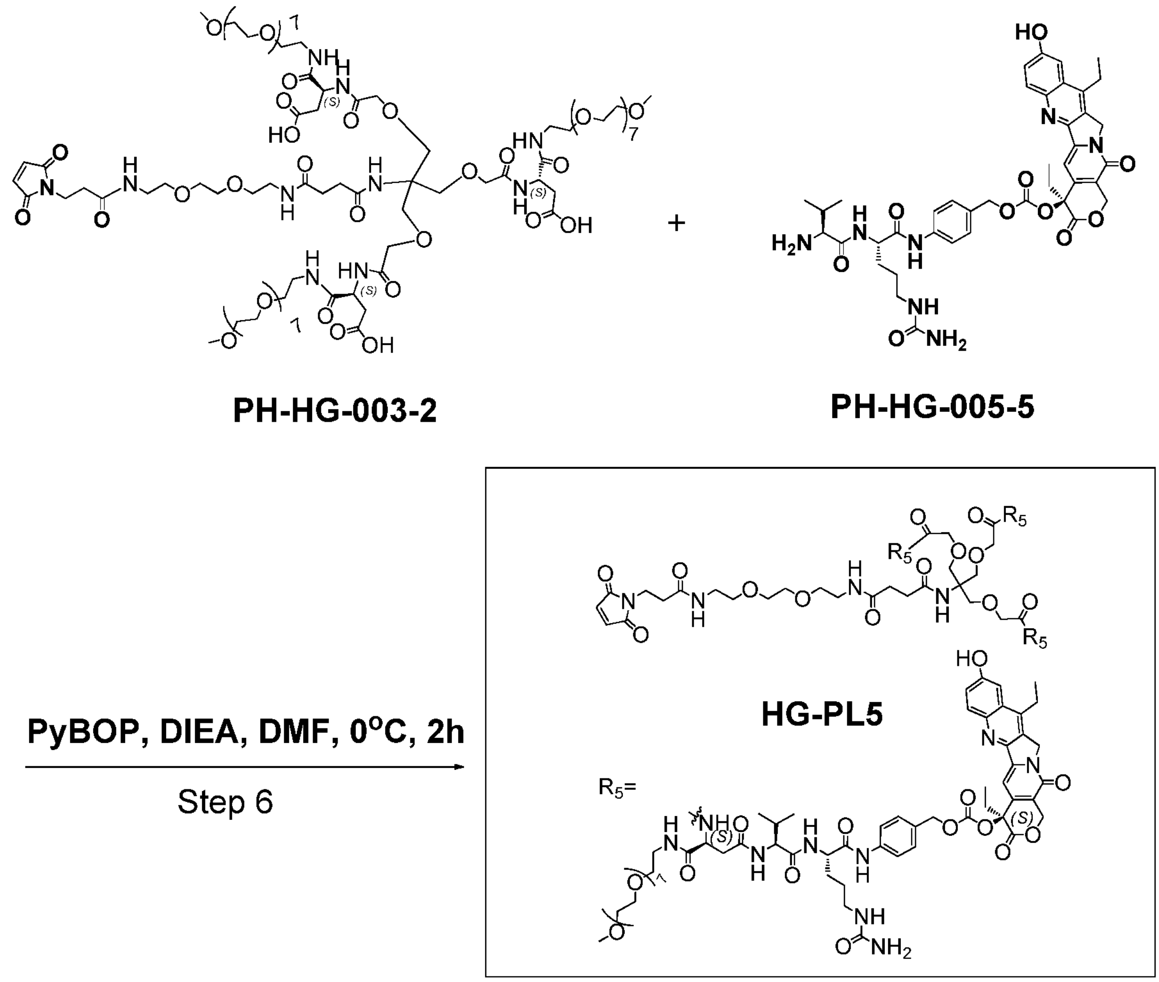


To a solution of PH-HG-003-2 (20mg, 0.01mmol, 1.0 eq.) in DMF (1.0mL) were added PH-HG-005-5 (24.9mg, 0.03 mmol, 3.3 eq.), N,N-diisopropylethylamine (5.5 mg, 0.04mmol, 4.5 eq.), and 1H-benzotriazole-1-yl-oxytripyrrolidino hexafluorophosphate (PyBOP) (22.1mg, 0.04mmol, 4.5 eq.) under nitrogen at 0°C. The reaction solution was stirred at 0°C for 2 hours and then purified by preparative HPLC (conditions: XBridge Prep OBD C18 column; size 19mm×250mm, 5 μm; mobile phase, water (with 0.01% formic acid) and acetonitrile (20.0% to 55.0% acetonitrile, 13 min); detector, UV 254 nm) to give 2.9mg (7%) of product HG-PL5 as a yellow solid.

MS m/z $[M/3+H]^+$ (ESI): 1486.35.

**Example 10 Synthesis of HG-PL6**

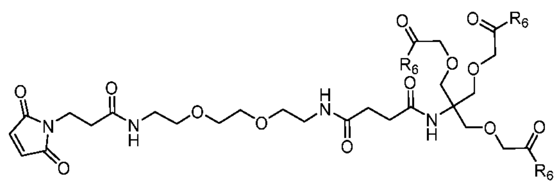

## HG-PL6

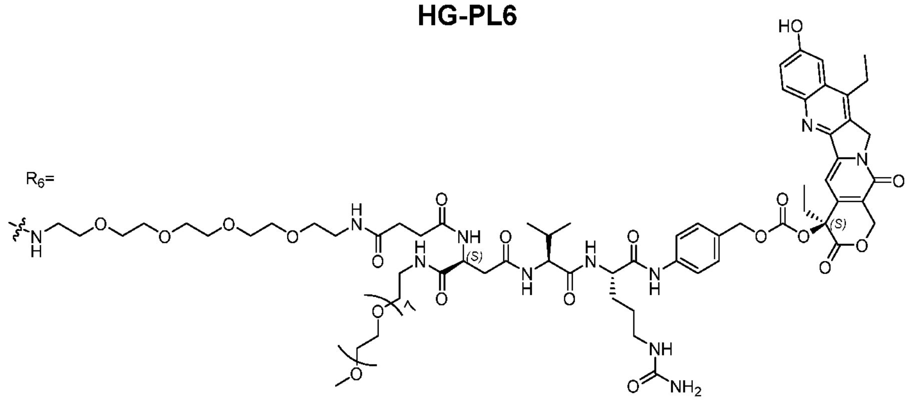

**Step 1: Synthesis of HG-PL6**

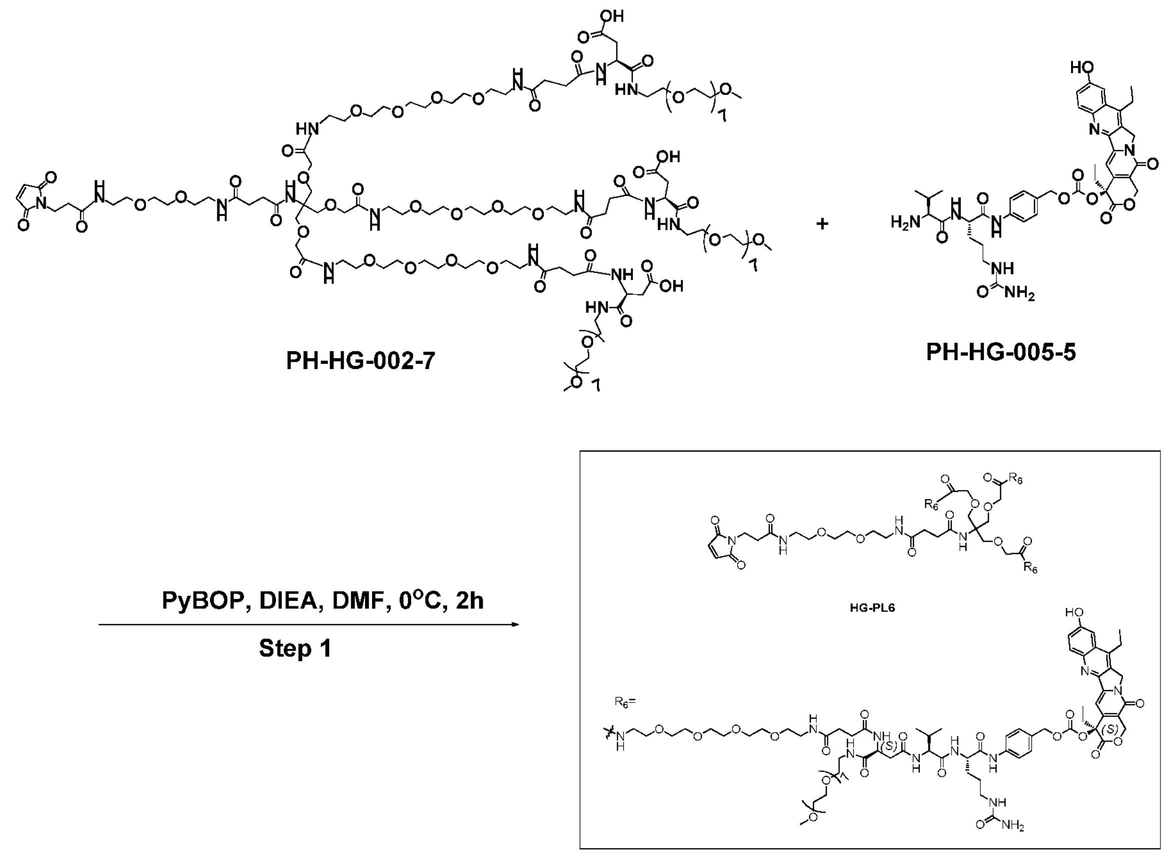


PH-HG-002-7

PH-HG-005-5

HG-PL6

To a solution of PH-HG-002-7 (25mg, 0.0081mmol, 1.0 eq.) in DMF (1.0mL) were added PH-HG-005-5 (21.4mg, 0.026 mmol, 3.3 eq.), N,N-diisopropylethylamine (4.7 mg, 0.036mmol, 4.5 eq.) and 1H-benzotriazole-1-yl-oxytripyrrolidino hexafluorophosphate (PyBOP) (19.1mg, 0.036mmol, 4.5 eq.) under nitrogen at 0°C. The reaction solution was stirred at 0°C for 2 hours and then purified by preparative HPLC (conditions: XSelect CSH Prep C18 column; size 19mm×250mm, 5 μm; mobile phase, water (with 0.01% formic acid) and acetonitrile (30.0% to 48.0% acetonitrile, 12 min); detector, UV 254 nm) to give 2.6mg (5%) of product HG-PL6 as a yellow solid.

MS m/z $[M/4+H]^+$ (ESI): 1354.40.

**Example 11 Synthesis of HG-PL7**

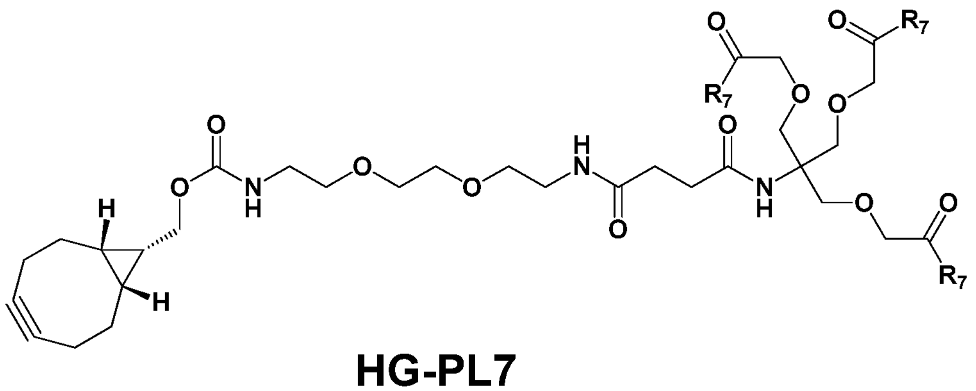

HG-PL7

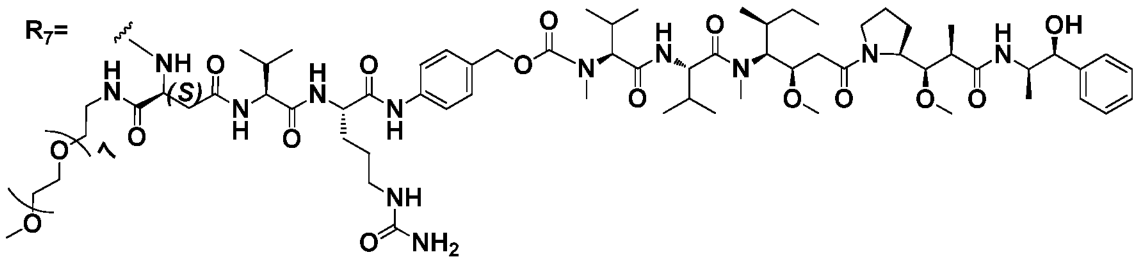

Step 1: Synthesis of PH-HG-007-1

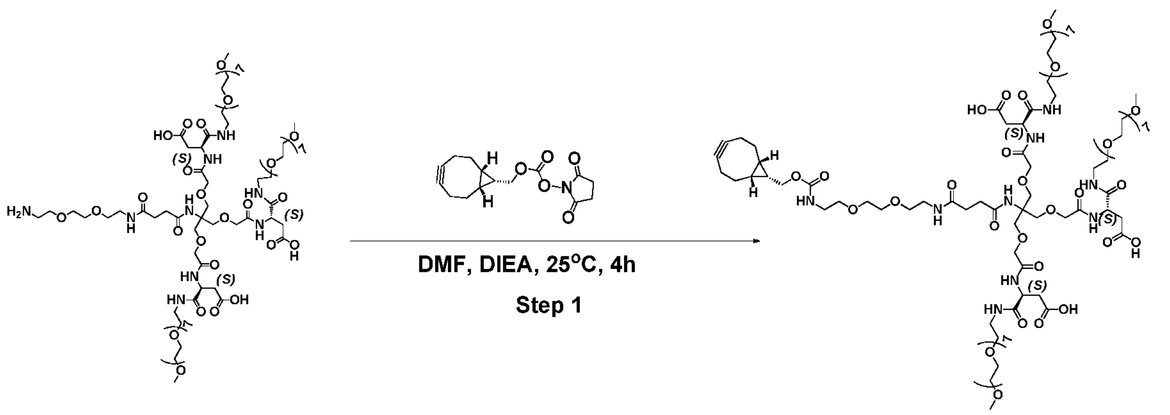


PH-HG-003-1

PH-HG-007-1

To a solution of PH-HG-003-1 (260mg, 0.13mmol, 1.0 eq.) in DMF (4.0mL) were added (1R,8S,9S)-bicyclo[6.1.0] non-4-yn-9-ylmethyl succinimidyl carbonate (BCN-Osu) (57.8mg, 0.2mmol, 1.5 eq.) and N,N-diisopropylethylamine (51.3mg, 0.4mmol, 3.0 eq.) under nitrogen. The reaction solution was stirred at 25°C for 4 hours and then purified by preparative HPLC (conditions: XBridge Prep OBD C18 column; size 30mm×250mm, 5 μm; mobile phase, water (with 0.1% formic acid) and acetonitrile (19.0% to 49.0% acetonitrile, 7 min); detector, UV 200nm) to give 100 mg (35%) of PH-HG-007-1 as a colorless oil.

MS m/z $[M/2+H]^+$ (ESI): 1072.35.

**Step 2: Synthesis of HG-PL7**

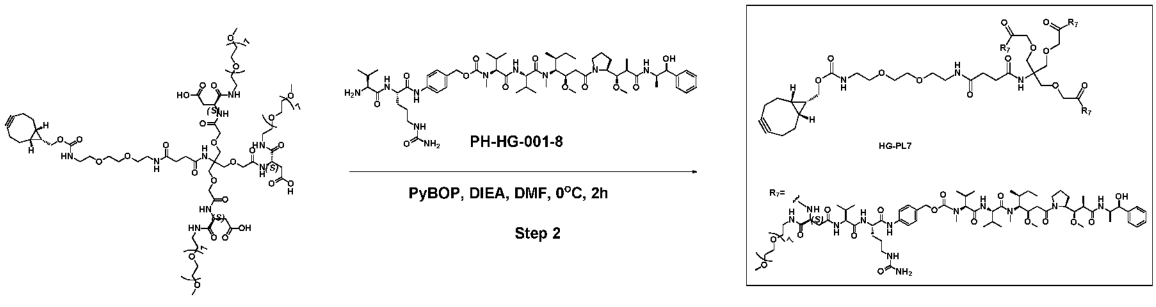


PH-HG-007-1

To a solution of PH-HG-007-1 (50mg, 0.023mmol, 1.0 eq.) in DMF (2.0mL) were added PH-HG-001-8 (86.5mg, 0.08mmol, 3.3 eq.), N,N-diisopropylethylamine (13.6mg, 0.103mmol, 4.5 eq.) and 1H-benzotriazole-1-yl-oxytripyrrolidino hexafluorophosphate (PyBOP) (54.6mg, 0.103mmol, 4.5 eq.) under nitrogen at 0°C. The reaction solution was stirred at 0°C for 2 hours and then purified by preparative HPLC (conditions: XBridge Shield Prep OBD column; size 30mm×150mm, 5 μm; mobile phase, water (with 0.01% formic acid) and acetonitrile (29.0% to 59.0% acetonitrile, 7 min); detector, UV 254nm) to give 10.4mg (8%) of HG-PL7 as a white solid.

MS m/z [M/3+H]$^+$ (ESI): 1820.90. $^1$H NMR (300 MHz, DMSO-$d_6$) δ: 0.75-0.90 (m, 84H), 0.95-1.05 (m, 23H), 1.15-1.24 (m, 1H), 1.25-1.52 (m, 17H), 1.60-1.88 (m, 14H), 1.90-2.24 (m, 18H), 2.38-2.42 (m, 6H), 2.80-2.90 (m, 7H), 3.07-3.13 (m, 13H), 3.17-3.28 (m, 36H), 3.48-3.52 (m, 89H), 3.72-3.81 (m, 13H), 3.91-4.06 (m, 20H), 4.20-4.56 (m, 21H), 4.62-4.78 (m, 7H), 4.94-5.17 (m, 7H), 5.35-5.43 (m, 10H), 5.95-6.03 (m, 3H), 7.14-7.35 (m, 22H), 7.60-7.75 (m, 8H), 7.89-8.35 (m, 21H), 9.81 (s, 3H).

## Example 12 Synthesis of HG-PL8

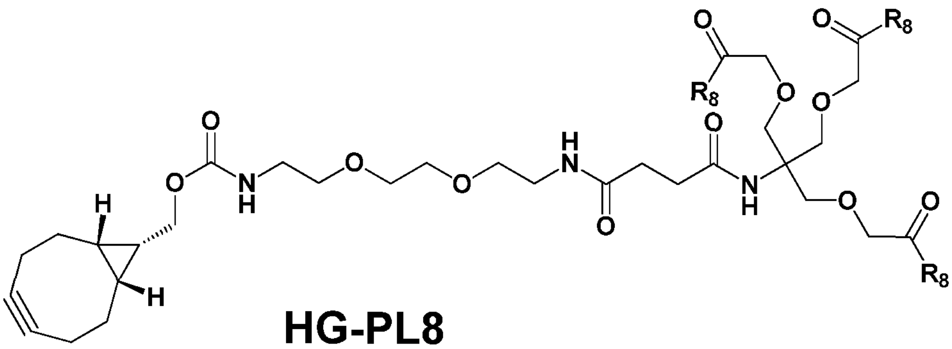

HG-PL8

$R_8$=

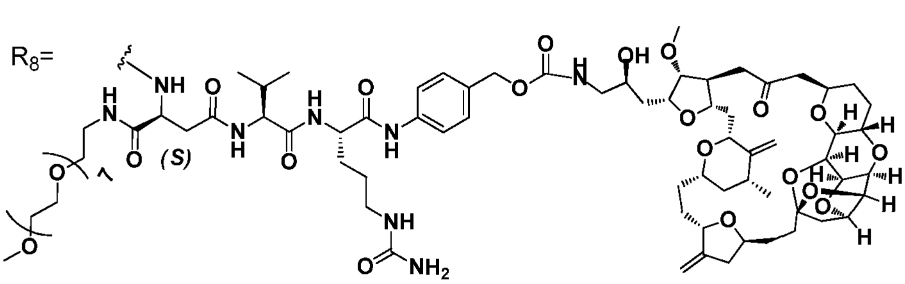

### Step 1: Synthesis of HG-PL8

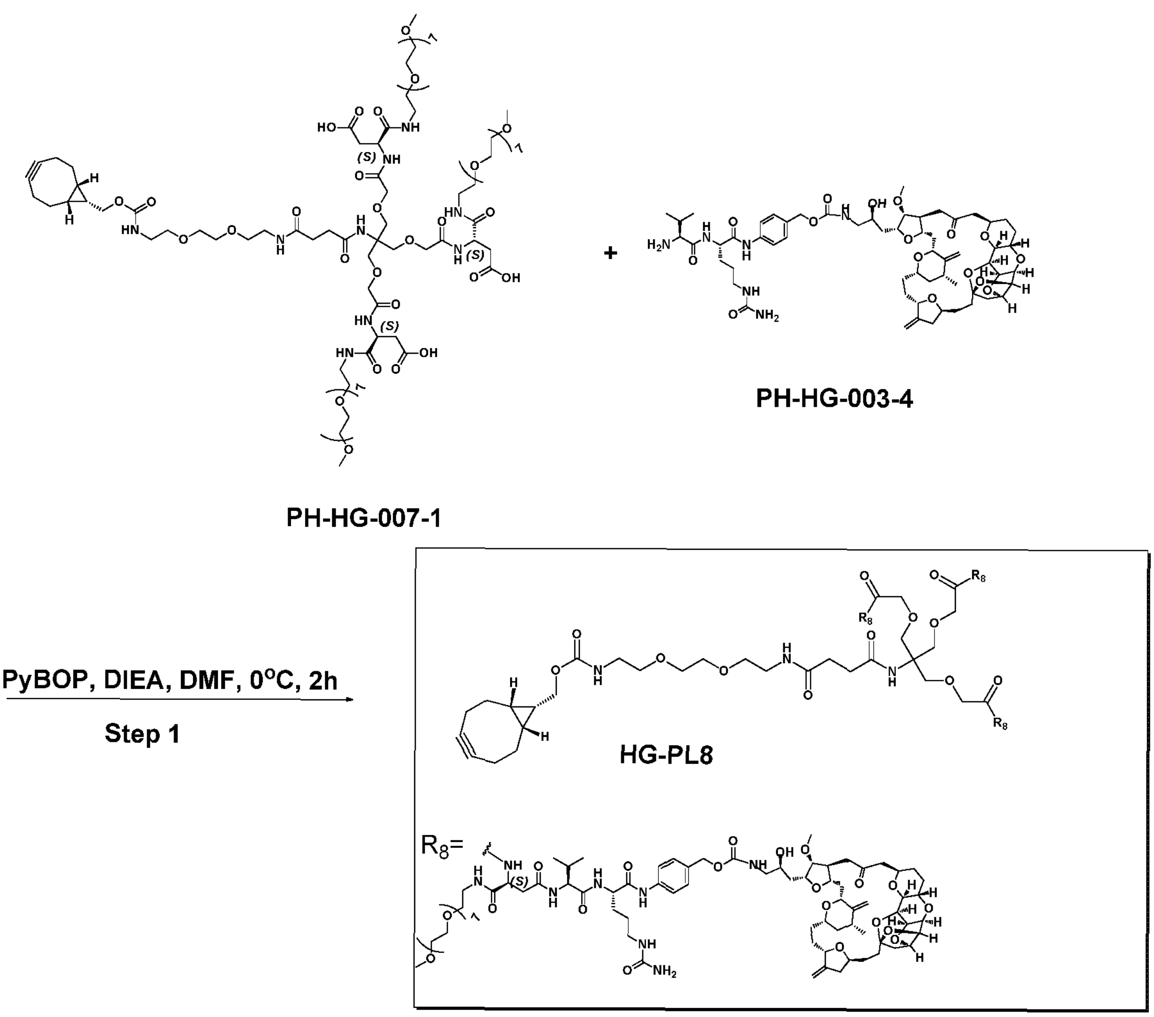


PH-HG-007-1

PH-HG-003-4

HG-PL8

To a solution of PH-HG-007-1 (100mg, 0.047mmol, 1.0 eq.) in DMF (4.0mL) were added PH-HG-003-4 (174.8mg, 0.155mmol, 3.3 eq.), N,N-diisopropylethylamine (27.1mg, 0.211mmol, 4.5 eq.) and 1H-benzotriazole-1-yl-oxytripyrrolidino hexafluorophosphate (PyBOP) (109.3mg, 0.211mmol, 4.5 eq.) under nitrogen at 0°C. The reaction solution was stirred at 0°C for 2 hours and then purified by preparative HPLC (conditions: XBridge Shield RP18 OBD column; size 19mm×150mm, 10 μm; mobile phase, water and acetonitrile (53.0% to 65.0% acetonitrile, 11 min); detector, UV 254nm) to give 21.2mg (8%) of HG-PL8 as a white solid.

MS m/z [M/3+H]+ (ESI): 1832.75. 1H NMR (300 MHz, DMSO-d6) δ: 0.86-0.89 (m, 22H), 0.97-1.06 (m, 13H), 1.22-1.40 (m, 27H), 1.51-1.60 (m, 14H), 1.67-1.75 (m, 22H), 1.94-2.02 (m, 21H), 2.13-2.36 (m, 28H), 2.62-2.87 (m, 25H), 2.99-3.11 (m, 14H), 3.24-3.26 (m, 27H), 3.51-3.55 (m, 76H), 3.70-3.89 (m, 20H), 3.94-4.40 (m, 35H), 4.57-4.77 (m, 15H), 4.84-5.07 (m, 15H), 5.44 (s, 6H), 5.96-6.04 (m, 3H), 7.09-7.11 (m, 3H), 7.27-7.29 (m, 8H), 7.61-7.64 (m, 7H), 7.80-8.20 (m, 15H), 9.78 (s, 3H).

**Example 13 Synthesis of HG-PL9**

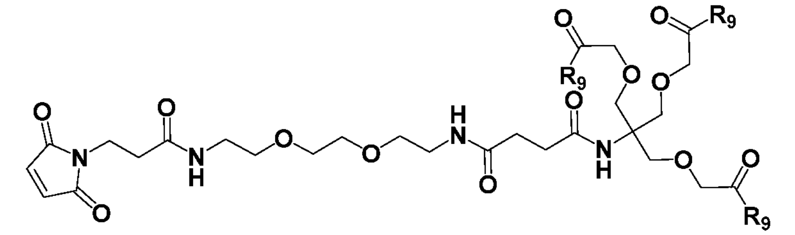

**HG-PL9**

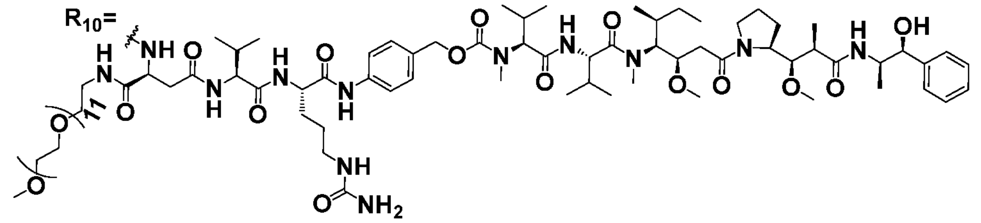

**Step 1: Synthesis of PH-HG-009-1**

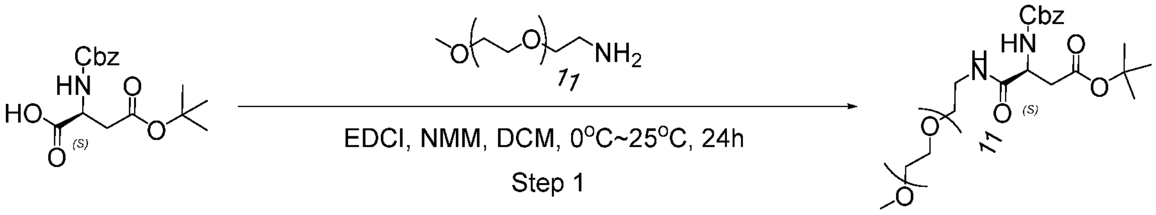


**SM2** PH-HG-09-1

To a solution of SM2 (0.81g, 2.8mmol, 1.1 eq.) in dichloromethane (15.0mL) were added successively aminododeca(ethylene glycol) monomethyl ether (1.4g, 2.5mmol,1.0 eq.), 1-(3-dimethylaminopropyl)-3-ethylcarbodiimide hydrochloride EDCI (0.7g, 3.8mmol, 1.5 eq.) and N-methylmorpholine (0.56g, 5.5mmol, 2.2 eq.) under nitrogen at 0°C. The reaction solution was stirred at 25°C for 24 hours, then diluted with 100 mL of water, and extracted with dichloromethane (3×30 mL). The combined organic phase was washed with saturated brine (3×30mL), dried over anhydrous sodium sulfate, and filtered. The filtrate was concentrated by rotary-evaporation. The crude product was purified by Flash-Prep-HPLC (conditions: C18 silica gel column; mobile phase water and acetonitrile (10% to 100% acetonitrile, 20 minutes), detector UV 210 nm) to give 1.2g (56%) of PH-HG-009-1 as a colorless oil.

MS m/z $[M+H]^+$ (ESI): 865.45; $^1H$ NMR (400 MHz, Methanol-$d_4$) δ: 1.41 (s, 9H), 2.52-2.58 (m, 1H), 2.74-2.79 (m, 1H), 3.34-3.37 (m, 9H), 3.51-3.54 (m, 4H), 3.60-3.62 (m, 38H), 4.48-4.51 (m, 1H), 5.05-5.15 (m, 2H), 7.29-7.38 (m, 5H).

**Step 2: Synthesis of PH-HG-009-2**

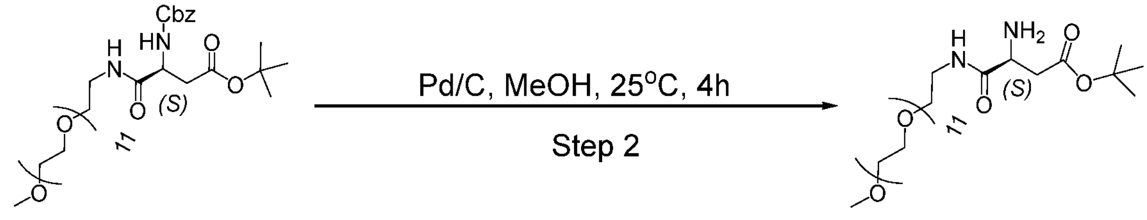


PH-HG-009-1 PH-HG-009-2

To a solution of PH-HG-009-1 (1.2 g, 1.3 mmol, 1.0 eq.) in methanol (15.0mL) was added palladium on carbon (0.2g), and the atmosphere in the reaction system was replaced 5 times with hydrogen. The mixture was hydrogenated with stirring at 25 °C for 4 hours. The reaction solution was filtered and concentrated to dryness by rotary-evaporation under reduced pressure to give 780.0 mg of PH-HG-009-2 (77%) as a colorless oil. This product was used directly in the next step without further purification.

MS m/z $[M+H]^+$ (ESI): 731.35

**Step 3: Synthesis of PH-HG-009-3**

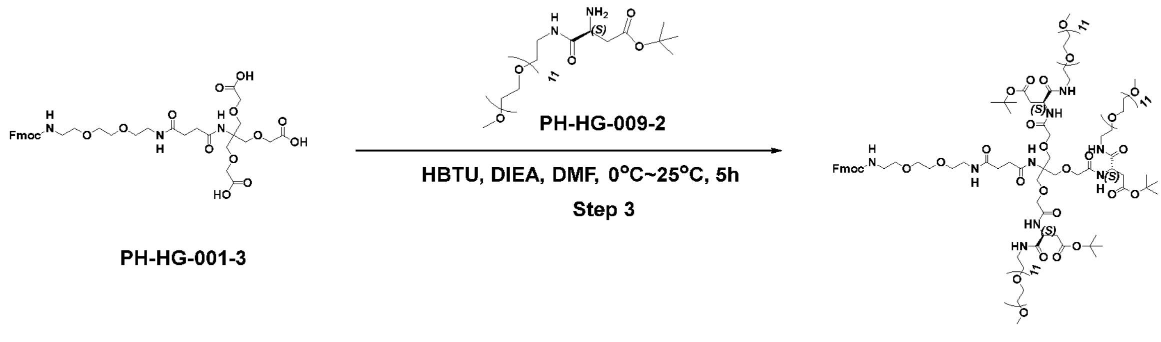

PH-HG-001-3

PH-HG-009-3

To a solution of PH-HG-009-2 (774.0mg, 1.1mmol, 3.3 eq.) in DMF (8.0mL) were added successively PH-HG-001-3 (240mg, 0.3mmol, 1.0 eq.), N,N-diisopropylethylamine (187mg, 1.4mmol, 4.5 eq.) and benzotriazole-N,N,N',N'-tetramethyluronium hexafluorophosphate (548.0mg, 1.4mmol, 4.5 eq.) under nitrogen at 0°C. The reaction solution was stirred at 25°C for 5 hours, then diluted with 100 mL of water, and extracted with dichloromethane (3×30 mL). The combined organic phase was washed with saturated brine (3×30mL), dried over anhydrous sodium sulfate, and filtered. The filtrate was concentrated by rotary-evaporation. The crude product was purified by Flash-Prep-HPLC (conditions: C18 silica gel column; mobile phase water and acetonitrile (10% to 100% acetonitrile, 20 minutes), detector UV 210 nm) to give 450mg (49%) of PH-HG-009-3 as a colorless oil.

MS m/z $[M/3+H]^+$ (ESI): 963.05.

**Step 4: Synthesis of PH-HG-009-4**

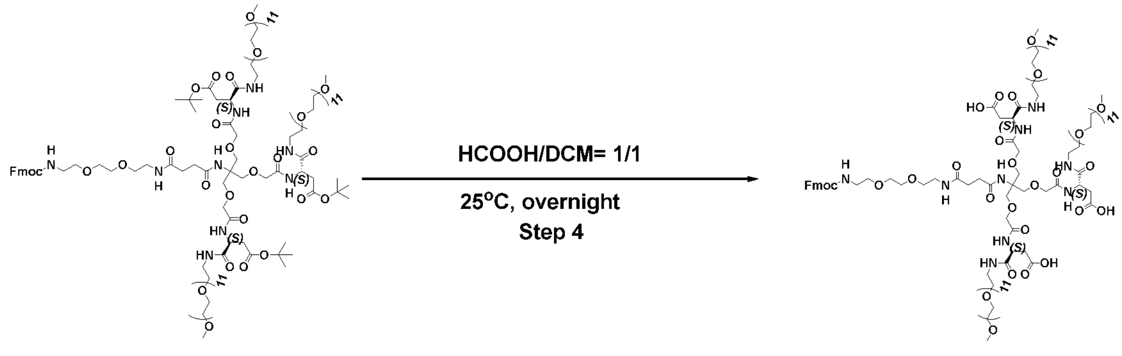

PH-HG-009-3

PH-HG-009-4

To a solution of PH-HG-009-3 (450.0mg, 0.2mmol, 1.0 eq.) in dichloromethane (2.5mL) was added formic acid (2.5mL) under nitrogen. The reaction solution was stirred at 25 °C for 12 hours and then concentrated. The crude product was purified by Flash-Prep-HPLC (conditions: C18 silica gel column; mobile phase water and acetonitrile (10% to 80% acetonitrile, 20 minutes), detector UV 210 nm) to give 200mg (47%) of PH-HG-009-4 as a colorless oil.

MS m/z $[M/2+H]^+$ (ESI): 1359.85 .

**Step 5: Synthesis of PH-HG-009-5**

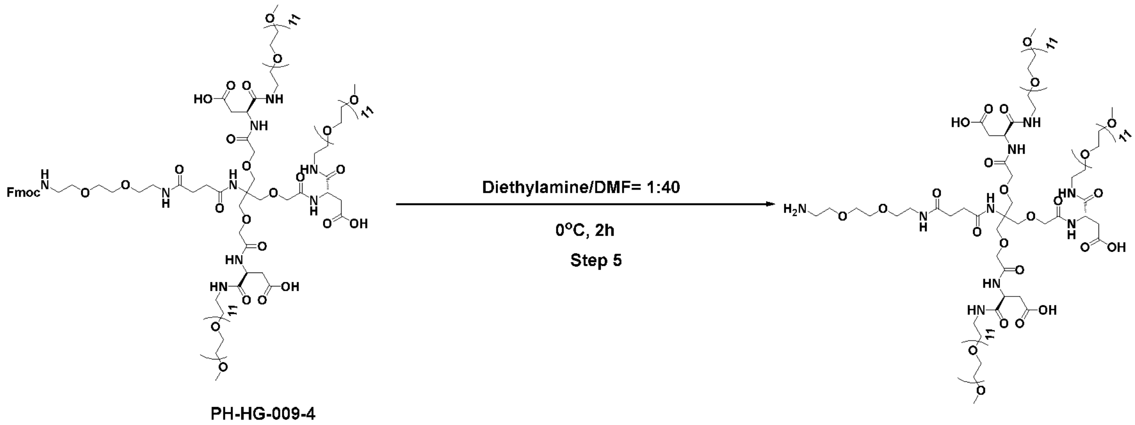


PH-HG-009-4

PH-HG-009-5

To a solution of PH-HG-009-4 (200mg, 0.07mmol, 1.0 eq.) in DMF (4.0mL) was added diethylamine (0.1mL) under nitrogen at 0°C. The reaction solution was stirred at 0°C for 2 hours and then purified by preparative HPLC (conditions: XSelect CSH Prep C18 OBD column; size 19mm×250mm, 5 μm; mobile phase, water (with 0.1% formic acid) and acetonitrile (8.0% to 23.0% acetonitrile, 11 min); detector, UV 200 nm) to give 96mg (52%) of PH-HG-009-5 as a colorless oil.

MS m/z $[M/3+H]^+$ (ESI): 832.80

**Step 6: Synthesis of PH-HG-009-6**

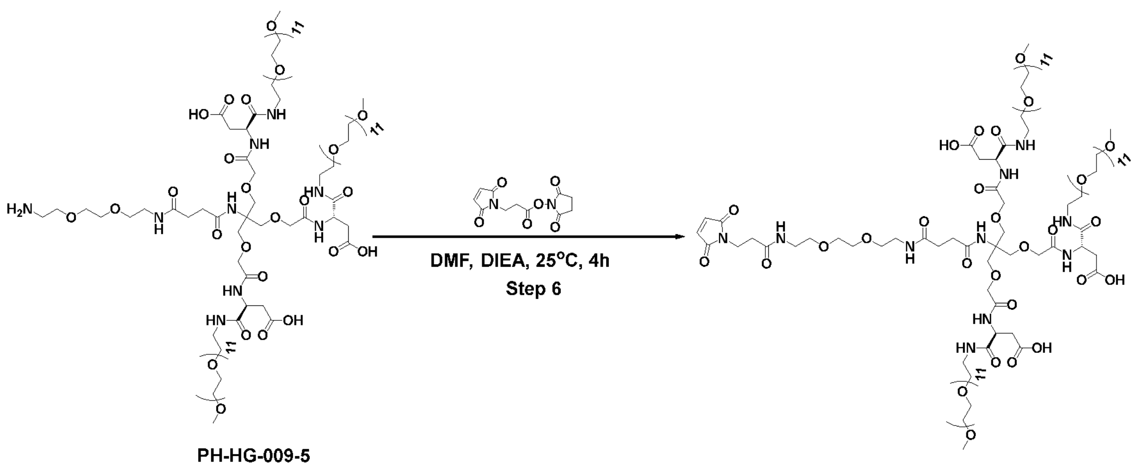


PH-HG-009-5

PH-HG-009-6

To a solution of PH-HG-009-5 (96mg, 0.04mmol, 1.0 eq.) in DMF (2.0mL) were added succinimidyl 3-maleimidopropionate (15.4mg, 0.06mmol, 1.5 eq.) and N,N-diisopropylethylamine (14.9mg, 0.11mmol, 3.0 eq.) under nitrogen. The reaction solution was stirred at 25°C for 4 hours and then purified by preparative HPLC (conditions: XBridge Prep C18 OBD column; size 30mm×150mm, 5 μm; mobile phase, water (0.01% formic acid) and acetonitrile (8.0% to 38.0% acetonitrile, 7 min); detector, UV 200 nm) to give 51mg (50%) of product PH-HG-009-6 as a colorless oil.

MS m/z $[M/2+H]^+$ (ESI): 1323.85

**Step 7: Synthesis of HG-PL9**

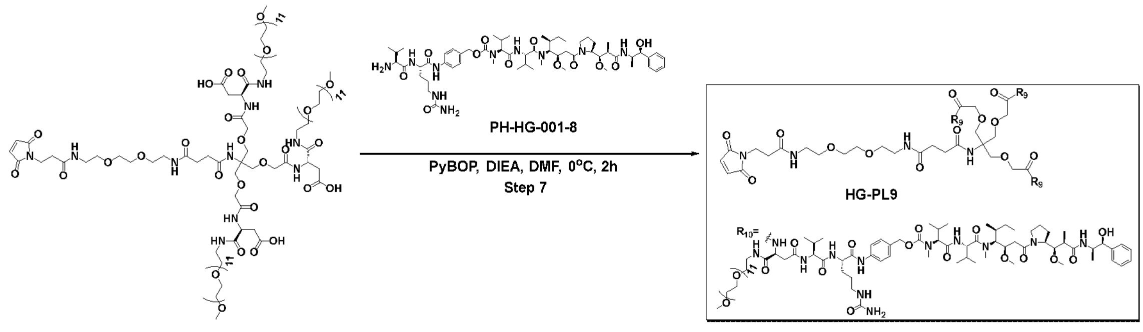


PH-HG-009-6

To a solution of PH-HG-009-6 (70mg, 0.026mmol, 1.0 eq.) in DMF (4.0mL) were added PH-HG-001-8 (98mg, 0.086mmol, 3.3 eq.), N,N-diisopropylethylamine (15.4mg, 0.12mmol, 4.5 eq.) and 1H-benzotriazole-1-yl-oxytripyrrolidino hexafluorophosphate (PyBOP) (61.9mg, 0.12mmol, 4.5 eq.) under nitrogen. The reaction solution was stirred at 0°C for 2 hours and then purified by preparative HPLC (conditions: XBridge Prep OBD C18 column; size 30mm×150mm, 5 μm; mobile phase, water (with 0.01% formic acid) and acetonitrile (35.0% to 65.0% acetonitrile, 7 min); detector, UV 254nm) to give 23.6mg (15%) of HG-PL9 as a white solid.

MS m/z $[M/4+H]^+$ (ESI): 1491.45; $^1$H NMR (300 MHz, DMSO-$d_6$) δ: 0.74-1.05 (m, 108H), 1.10-1.80 (m, 35H), 1.90-2.45 (m, 27H), 2.60-2.75 (m, 5H), 2.82-3.05 (m, 24H), 3.12-3.19 (m, 27H), 3.21-3.48 (m, 108H), 3.51-3.80 (m, 47H), 3.88-4.07 (m, 15H), 4.19-4.52 (m, 17H), 4.59-5.14 (m, 13H), 5.92-6.08 (m, 2H), 6.90-7.07 (m, 3H), 7.12-7.20 (m, 3H), 7.22-7.35 (m, 20H), 7.57-7.67 (m, 7H), 7.75-8.37 (m, 20H), 9.78 (s, 3H).

**Example 14 Synthesis of HG-PL10**

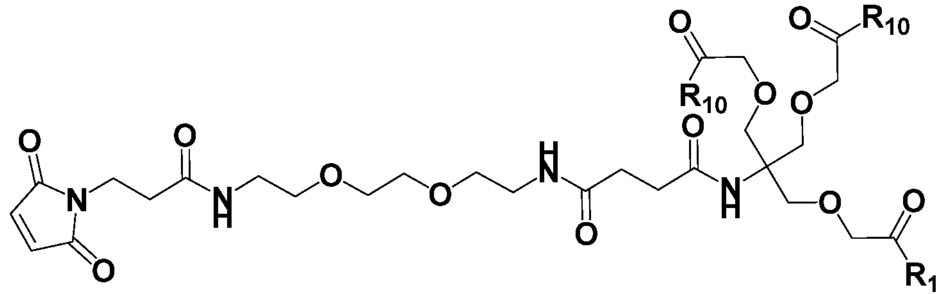

HG-PL10

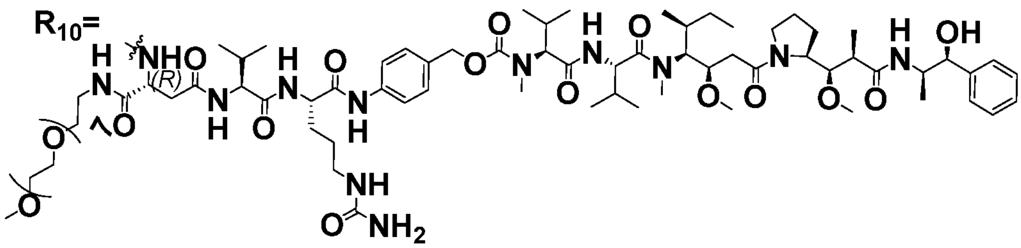

**Step 1: Synthesis of PH-HG-010-1**

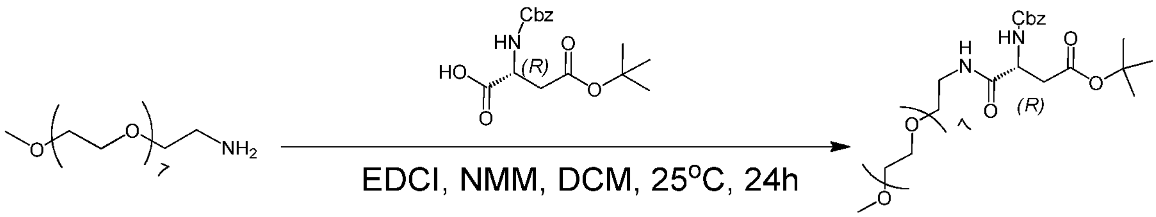


PH-HG-010-1

To a solution of (R)-2-(((benzyloxy)carbonyl)amino)-4-(tert-butoxy)-4-oxobutanoic acid (1.3g, 4.0mmol, 1.1 eq.) in dichloromethane (30.0mL) were added successively aminoocta(ethylene glycol) monomethyl ether (1.4g, 3.65mmol, 1.0 eq.), 1-(3-dimethylaminopropyl)-3-ethylcarbodiimide hydrochloride EDCI (1.1g, 5.84mmol, 1.5 eq.) and N-methylmorpholine (553.5mg, 5.48mmol, 1.5 eq.) under nitrogen at 0°C. The reaction solution was stirred at 25°C for 24 hours, then diluted with 100 mL of water, and extracted with dichloromethane (3×30 mL). The combined organic phase was washed with saturated brine (3×30mL), dried over anhydrous sodium sulfate, and filtered. The filtrate was concentrated by rotary-evaporation. The crude product was purified by Flash-Prep-HPLC (conditions: C18 silica gel column; mobile phase water and acetonitrile (10% to 100% acetonitrile, 20 minutes), detector UV 210 nm) to give 1.2g (48%) of PH-HG-010-1 as a colorless oil.

MS m/z $[M+H]^+$ (ESI): 689.25.

**Step 2: Synthesis of PH-HG-010-2**

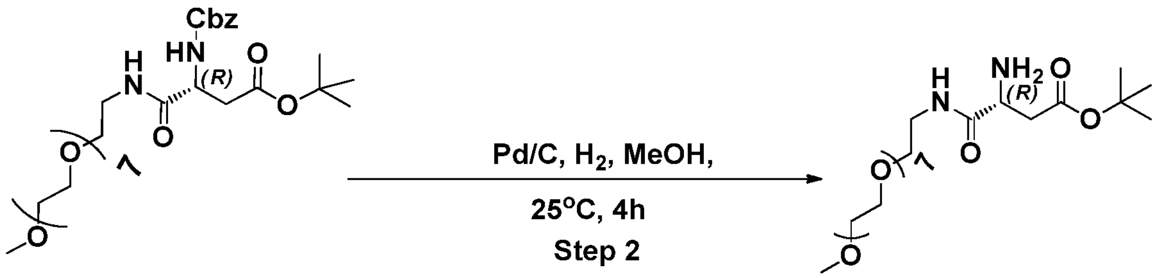


**PH-HG-010-1** **PH-HG-010-2**

To a solution of PH-HG-010-1 (1.2 g, 1.7 mmol, 1.0 eq.) in methanol (15.0mL) was added palladium on carbon (0.25g), and the atmosphere in the reaction system was replaced 5 times with hydrogen. The mixture was hydrogenated with stirring at 25 °C for 4 hours. The reaction solution was filtered and concentrated to dryness by rotary-evaporation under reduced pressure to give 800.0 mg of PH-HG-010-2 (83%) as a colorless oil. This product was used directly in the next step without further purification.

MS m/z $[M+H]^+$ (ESI): 555.45

**Step 3: Synthesis of PH-HG-010-3**

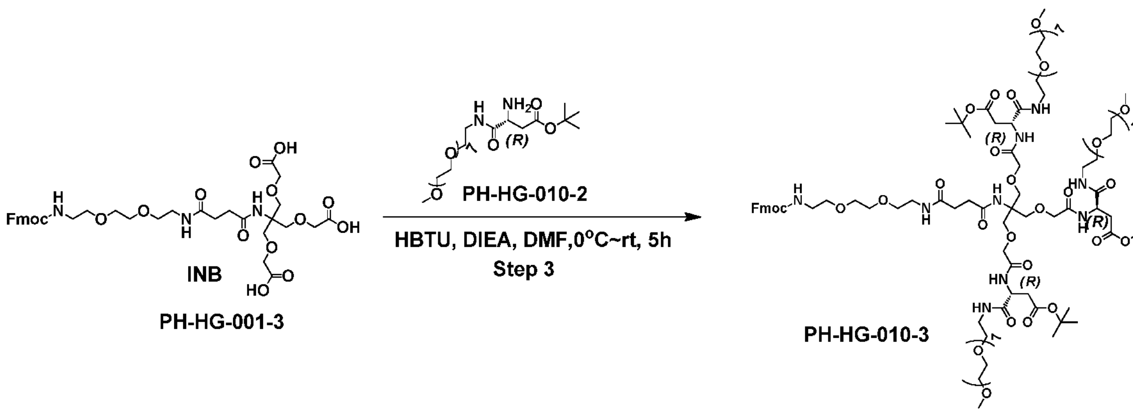

INB
PH-HG-001-3
PH-HG-010-2
PH-HG-010-3

To a solution of PH-HG-010-2 (735.4.0mg, 1.3mmol, 3.3 eq.) in DMF (10.0mL) were added successively PH-HG-001-3 (300mg, 0.3mmol, 1.0 eq.), N,N-diisopropylethylamine (230.4mg, 1.8mmol, 4.5 eq.) and benzotriazole-N,N,N',N'-tetramethyluronium hexafluorophosphate (686.7mg, 1.8mmol, 4.5 eq.) under nitrogen at 0°C. The reaction solution was stirred at 25°C for 5 hours, then diluted with 100 mL of water, and extracted with dichloromethane (3×30 mL). The combined organic phase was washed with saturated brine (3×30mL), dried over anhydrous sodium sulfate, and filtered. The filtrate was concentrated by rotary-evaporation. The crude product was purified by Flash-Prep-HPLC (conditions: C18 silica gel column; mobile phase water and acetonitrile (10% to 100% acetonitrile, 20 minutes), detector UV 210 nm) to give 470mg (50%) of PH-HG-010-3 as a colorless oil.

MS m/z $[M/2+H]^+$ (ESI): 1179.45

**Step 4: Synthesis of PH-HG-010-4**

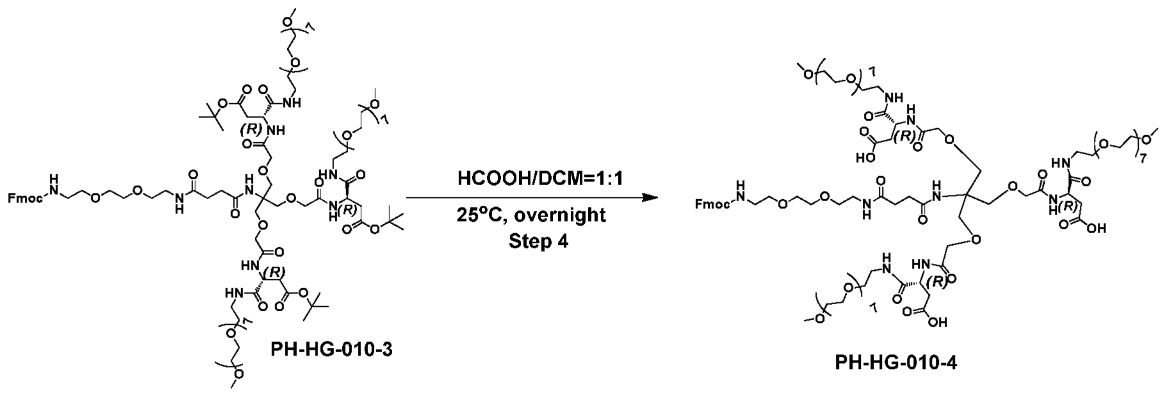

PH-HG-010-3
PH-HG-010-4

To a solution of PH-HG-010-3 (470.0mg, 0.2mmol, 1.0 eq.) in dichloromethane (2.5mL) was added formic acid (2.5mL) under nitrogen. The reaction solution was stirred at 25 °C for 12 h and then concentrated. The crude product was purified by Flash-Prep-HPLC (conditions: C18 silica gel column; mobile phase water and acetonitrile (10% to 80% acetonitrile, 20 minutes), detector UV 210 nm) to give 210mg (48%) of PH-HG-010-4 as a colorless oil.

MS m/z $[M/2+H]^+$ (ESI): 1095.40

**Step 5: Synthesis of PH-HG-010-5**

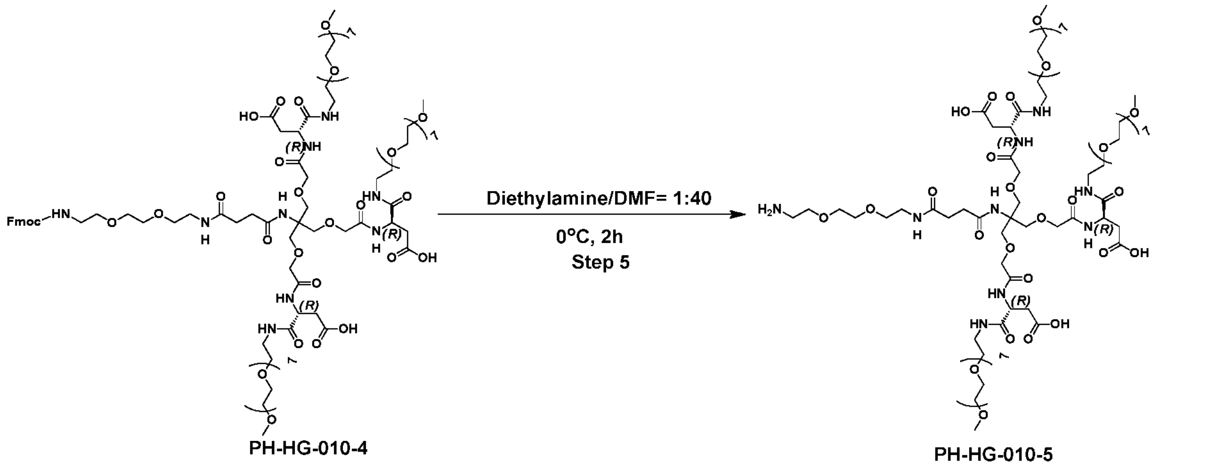


PH-HG-010-4 PH-HG-010-5

To a solution of PH-HG-010-4 (210mg, 0.1mmol, 1.0 eq.) in DMF (4.0mL) was added diethylamine (0.1mL) under nitrogen at 0°C. The reaction solution was stirred at 0°C for 2 hours and then purified by preparative HPLC (conditions: XSelect CSH Prep C18 OBD column; size 19mm×250mm, 5 μm; mobile phase, water (with 0.1% formic acid) and acetonitrile (8.0% to 23.0% acetonitrile, 11 min); detector, UV 200 nm) to give 100mg (53%) of PH-HG-010-5 as a colorless oil.

MS m/z $[M+H]^+$ (ESI): 1967.17

**Step 6: Synthesis of PH-HG-010-6**

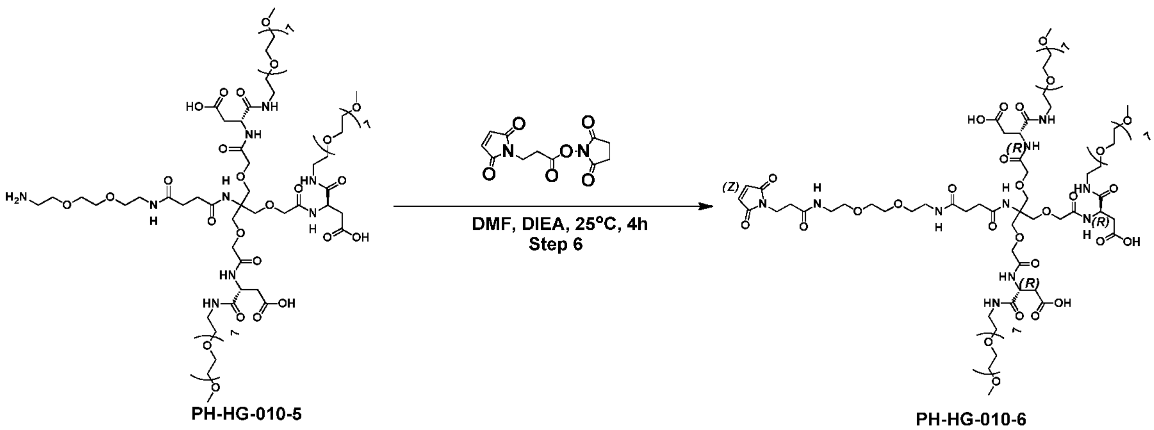


PH-HG-010-5 PH-HG-010-6

To a solution of PH-HG-010-5 (100mg, 0.05mmol, 1.0 eq.) in DMF (5.0mL) were added succinimidyl 3-maleimidopropionate (20.2mg, 0.07mmol, 1.5 eq.) and N,N-diisopropylethylamine (19.6mg, 0.14mmol, 3.0 eq.) under nitrogen. The reaction solution was stirred at 25°C for 4 hours and then purified by preparative HPLC (conditions: XBridge Prep C18 OBD column; size 30mm×150mm, 5 μm; mobile phase, water (0.01% formic acid) and acetonitrile (8.0% to 38.0% acetonitrile, 7 min); detector, UV 200 nm) to give 55mg (51%) of product PH-HG-010-6 as a colorless oil.

MS m/z $[M/2+H]^+$ (ESI): 1060.00

**Step 7: Synthesis of HG-PL10**

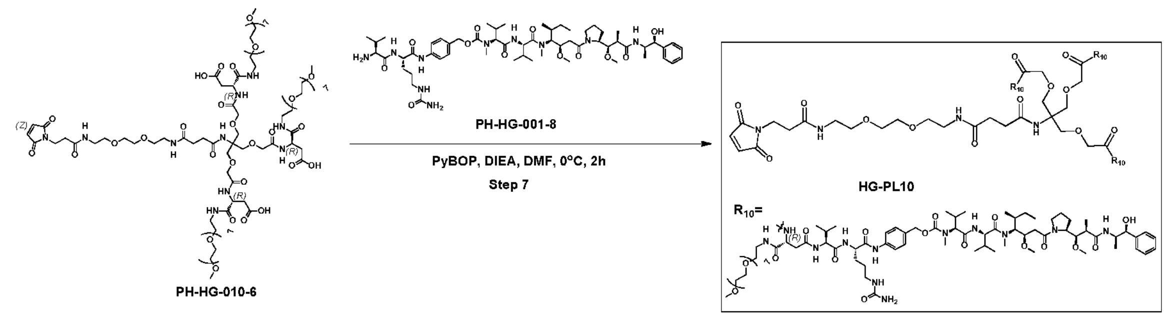


To a solution of PH-HG-010-6 (48mg, 0.023mmol, 1.0 eq.) in DMF (4.0mL) were added PH-HG-001-8 (85.4mg, 0.076mmol, 3.3 eq.), N,N-diisopropylethylamine (12.9mg, 0.10mmol, 4.5 eq.) and 1H-benzotriazole-1-yl-oxytripyrrolidino hexafluorophosphate (PyBOP) (52.0mg, 0.10mmol, 4.5 eq.) under nitrogen at 0°C. The reaction solution was stirred at 0°C for 2 hours and then purified by preparative HPLC (conditions: XBridge Prep OBD C18 column; size 30mm×150mm, 5 μm; mobile phase, water (with 0.01% formic acid) and acetonitrile (35.0% to 65.0% acetonitrile, 7 min); detector, UV 254nm) to give 18.2mg (15%) of HG-PL10 as a white solid.

MS m/z $[M/4+H]^+$ (ESI): 1359.35; $^1H$ NMR (300 MHz, DMSO-$d_6$) δ: 0.73-0.89 (m,70H), 0.96-1.05 (m, 21H), 1.05-2.31 (m, 34H), 2.31-2.32 (m, 26H), 2.24-2.51 (m, 9H), 2.84-2.88 (m, 9H), 3.13-3.31 (m, 46H), 3.38-3.45 (m, 14H), 3.64-3.49 (m, 95H), 3.76-3.86 (m, 8H), 3.96-4.07 (m, 12H), 4.30-4.21 (m, 6H), 4.36-4.52 (m, 9H), 4.71-4.78 (m, 6H), 4.91-5.15 (m, 6H), 6.01-6.35 (m, 1H), 7.48-5.60 (m, 6H), 6.98 (s, 3H), 7.26 (s, 2H), 7.12-7.35 (m, 21H), 7.50-7.80 (m, 9H), 7.80-8.19 (m, 15H), 8.25-8.53 (m, 5H), 9.95 (s, 3H).

## Biological Example

The following abbreviations are used throughout this disclosure:

| | |
|---|---|
| ADC | Antibody-drug conjugate |
| BSA | Bovine serum albumin |
| DAR | Drug-to-antibody ratio |
| $EC_{50}$ | Half maximal effective concentration |
| FACS | Fluorescence-activated cell sorting |
| FBS | Fetal bovine serum |
| $IC_{50}$ | Half maximal inhibitory concentration |
| MFI | Mean fluorescence |
| NA | Not Available |
| PBS | Phosphate Buffered Saline |

## Example 15 Synthesis of Trastuzumab Conjugate (HG-ADC-1) Using HG-PL1 Prepared in Examples as Scaffold

### 1. Preparation of HG-ADC-1-D2

Antibody Herceptin was transferred into PB buffer (40 mM PB, 2 mM EDTA, pH 7.0) by solution replacement, and then reduced by adding 1.0 eq. of tris(2-carboxyethyl)phosphine (TCEP). The reaction solution was reacted at 22 °C for 3 hours. The reaction solution was adjusted to a pH value of 5 with 400 mM acetic acid solution, and then directly used for the next coupling reaction. Water and an aqueous solution of linker-payload HG-PL1 (10 mg/mL, 3 eq.) were slowly added to the reduced antibody in an ice bath. After mixing well, the coupling reaction solution was reacted at 4 °C for 2 hours. After the coupling reaction was completed, the ADC solution was filtered with a 0.22 μm PVDF needle filter, and then purified using a Hydrophobic Interaction Chromatography column (Thermo SCIENTIFIC ProPac™ HIC-10 column, 5 μm, 300 A, 7.8*75 mm) desalting column on the AKTA instrument. Before the purification, the desalting column was equilibrated with 30 mL of buffer A (20 mM histidine, 2.0 M NaCl, pH 5.5). The NaCl concentration in the ADC solution was adjusted to 2.0 M with buffer (20 mM histidine, 5.0 M NaCl, pH 5.5), so that the ADC could bind to the desalting column packing material. After loading the sample, the desalting column was washed with buffer A until

the absorbance at 280 nm wavelength reached the baseline equilibrium. Subsequently, within 20 minutes (buffer flow rate was 1 mL/min), the buffer system was continuously converted from 100% buffer A (20 mM histidine, 2.0 M NaCl, pH 5.5) to 100% buffer B (20 mM histidine, pH 5.5) for elution. The eluted and purified sample (HG-ADC-1-D2) was then concentrated into 20 mM histidine buffer by solution replacement using an Amicon ultrafiltration tube (50 kDa), and filtered with a 0.2 μm PVDF needle filter to give the ADC product, which was sampled for evaluation.

**2. Preparation of HG-ADC-1-D4**

In PB buffer (40 mM PB, 2 mM EDTA, pH 7.0), the antibody Herceptin was reduced with 2.2 eq. of TCEP at 22 °C for 3 hours. Without removing excess TCEP, the reaction solution was directly used for the next coupling reaction. The reaction solution was adjusted to a pH of 5 with 400 mM acetic acid solution, and water and a HG-PL1 solution (10 mg/mL, dissolved in water, 6 eq.) were then slowly added to the reaction solution under ice bath conditions. After mixing well, the reaction solution was reacted at 4 °C for 2 hours. After the reaction was completed, the reaction solution was concentrated into 20 mM histidine buffer by solution replacement with an Amicon ultrafiltration tube (50 kDa), and filtered with a 0.2 μm PVDF needle filter to give the ADC product, which was sampled for evaluation.

**3. Preparation of DS8201**

In PB buffer (40 mM PB, 2 mM EDTA, pH 7.0), the antibody Herceptin was reduced with 7.5 eq. of TCEP, and the reaction mixture was shaken on a shaker at 32 °C for 3 hours (with a shaking speed of 60 rpm). Without removing excess TCEP, the reaction solution was directly used for the next coupling reaction. DMA (dimethylacetamide) and a Deruxtecan solution (10 mg/mL, dissolved in DMA, 20 eq.) were slowly added to the reaction solution. After mixing well, the reaction solution was reacted at 22 °C for 2 hours. After the reaction was completed, the reaction solution was transferred into 20 mM histidine buffer by solution replacement with a desalting column (40 K), and filtered with a 0.2 μm PVDF needle filter to give the ADC product, which was sampled for evaluation.

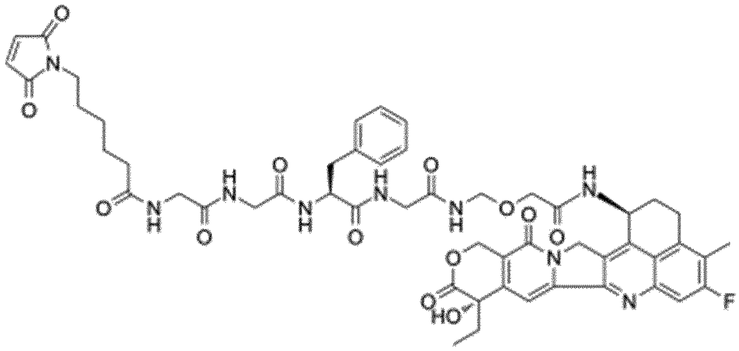

Deruxtecan

**4. Data Summary of Antibody Drug Conjugates**

RP-DAR assay

To 10.0 μl of ADC solution were added 37.5 μL of 8.0 mol/L guanidine hydrochloride solution, 2.5 μL of 1.0 mol/L Tris-HCl, and 1.0 μL of 1 mol/L dithiothreitol. The mixture was mixed well and sampled for HPLC detection.

The chromatographic conditions are shown in the table below:

| | |
|---|---|
| Chromatographic column | Agilent, PLRP-S, 2.1*50mm, 8μm |
| Column temperature | 80°C |
| Sample temperature | 2~8°C |
| Wavelength | 280 nm |
| Injection volume | 4 μg |
| Mobile phase system | Mobile phase A: 0.05% trifluoroacetic acid in water |
| | Mobile phase B: 0.05% trifluoroacetic acid in acetonitrile |

(continued)

| Gradient | Time (min) | %A | %B | Flow rate (mL/min) |
|---|---|---|---|---|
| | 0.0 | 75 | 25 | 0.5 |
| | 0.7 | 66 | 34 | |
| | 5.0 | 55 | 45 | |
| | 6.0 | 10 | 90 | |
| | 7.0 | 10 | 90 | |
| | 7.1 | 75 | 25 | |
| | 10.0 | 75 | 25 | |

Test results:

The DAR of antibody-drug conjugate HG-ADC-1-D2 was 6.54, the DAR of HG-ADC-1-D4 was 12.60, and the DAR of DS8201 was 8.00.

**Example 16 Synthesis of Trastuzumab Conjugate (HG-ADC-2) Using HG-PL2 Prepared in Examples as Scaffold**

**1. Preparation of HG-ADC-2-D1**

In PB buffer (40 mM PB, 2 mM EDTA, pH 7.0), the antibody Herceptin was reduced with 1 eq. of tris(2-carboxyethyl)phosphine (TCEP), and the reaction mixture was shaken on a shaker at 22 °C for 3 hours (with a shaking speed of 60 rpm). Without removing excess TCEP, the reaction solution was directly used for the next coupling reaction. PB buffer, dimethylacetamide (DMA) and a solution of linker-payload HG-PL2 in dimethylacetamide (DMA) (6.39 mg /mL, 1 eq.) were slowly added to the reduced antibody in an ice bath. After mixing well, the coupling reaction solution was reacted at 4 °C for 1.5 hours. L-cysteine (1.21 mg/ml aqueous solution, 4 eq.) was added to the reaction solution, and the mixture was mixed well and reacted at 4 °C for 0.5 hours. After the reaction was completed, the ADC solution was filtered with a 0.22 μm PVDF needle filter, and then purified using a hydrophobic interaction chromatography column (GE HiTrap Butyl HP column, 1 mL) on a protein purification apparatus (AKTA). Before the purification, the column was equilibrated with 10 mL of buffer A (20 mM Histidine, 1.5 M NaCl, pH 5.5). The NaCl concentration in the ADC solution was adjusted to 2.0 M with buffer (20 mM histidine, 5.0 M NaCl, pH 5.5), so that the ADC could be bound to the chromatographic column packing material. After loading the sample, the chromatographic column was washed with buffer A until the absorbance at 280 nm wavelength reached the baseline equilibrium. Subsequently, within 2 minutes (buffer flow rate was 1 mL/min), the buffer system was continuously converted from 100% buffer A (20 mM histidine, 1.5 M NaCl, pH 5.5) to 100% buffer B (20 mM histidine, pH 5.5) for elution. The eluted and purified HG-ADC-2-D1 was then subjected to solution replacement to be concentrated into 20 mM histidine buffer with an Amicon ultrafiltration tube (10 kDa), and filtered with a 0.2 μm PVDF needle filter to give the ADC product, which was sampled for evaluation.

**2. Preparation of HG-ADC-2-D2**

In PB buffer (40 mM PB, 2 mM EDTA, pH 7.0), the antibody Herceptin was reduced with 1 eq. of tris(2-carboxyethyl)phosphine (TCEP), and the reaction mixture was shaken on a shaker at 22 °C for 3 hours (with a shaking speed of 60 rpm). Without removing excess TCEP, the reaction solution was directly used for the next coupling reaction. PB buffer, dimethylacetamide (DMA) and a solution of linker-payload HG-PL2 in dimethylacetamide (DMA) (6.43 mg /mL, 3 eq.) were slowly added to the reduced antibody in an ice bath. After mixing well, the coupling reaction solution was reacted at 4 °C for 2 hours. After the reaction was completed, the ADC solution was filtered with a 0.22 μm PVDF needle filter, and then purified using a hydrophobic interaction chromatography column (Polar MC30 HIC Ether column, 1 mL) on a protein purification apparatus (AKTA). Before the purification, the column was equilibrated with 10 mL of buffer A (20 mM Histidine, 4M NaCl, pH 5.5). The NaCl concentration in the ADC solution was adjusted to 4 M with buffer (20 mM histidine, 5.0 M NaCl, pH 5.5), so that the ADC could be bound to the chromatographic column packing material. After loading the sample, the chromatographic column was washed with buffer A until the absorbance at 280 nm wavelength reached the baseline equilibrium. Subsequently, within 2 minutes (buffer flow rate was 1 mL/min), the buffer system was continuously converted from 100% buffer A (20 mM histidine, 4 M NaCl, pH 5.5) to 100% buffer B (20 mM histidine, pH 5.5) for elution. The eluted and purified HG-ADC-2-D2 was then subjected to solution replacement

to be concentrated into 20 mM histidine buffer with an Amicon ultrafiltration tube (10 kDa), and filtered with a 0.2 μm PVDF needle filter to give the ADC product, which was sampled for evaluation.

**Example 17 Synthesis of Trastuzumab Conjugate (HG-ADC-3) Using HG-PL3 Prepared in Examples as Scaffold**

**1. Preparation of HG-ADC-3-D1**

In PB buffer (40 mM PB, 2 mM EDTA, pH 7.0), the antibody Herceptin was reduced with 1 eq. of tris(2-carboxyethyl)phosphine (TCEP), and the reaction mixture was shaken on a shaker at 22 °C for 3 hours (with a shaking speed of 60 rpm). Without removing excess TCEP, the reaction solution was directly used for the next coupling reaction. PB buffer, dimethylacetamide (DMA) and an linker-payload HG-PL3 solution (a mixed solution of water:DMA = 3:1 (volume ratio), 5.47 mg/mL, 1 eq.) were slowly added to the reduced antibody in an ice bath. After mixing well, the coupling reaction solution was reacted at 4 °C for 1.5 hours. L-cysteine (1.21 mg/ml aqueous solution, 4 eq.) was added to the reaction solution, and the mixture was mixed well and reacted at 4 °C for 0.5 hours. After the reaction was completed, the ADC solution was filtered with a 0.22 μm PVDF needle filter, and then purified using a hydrophobic interaction chromatography column (GE HiTrap Butyl HP column, 1 mL) on a protein purification apparatus (AKTA). Before the purification, the column was equilibrated with 10 mL of buffer A (20 mM Histidine, 1.5 M NaCl, pH 5.5). The NaCl concentration in the ADC solution was adjusted to 2 M with buffer (20 mM histidine, 5.0 M NaCl, pH 5.5), so that the ADC could be bound to the chromatographic column packing material. After loading the sample, the chromatographic column was washed with buffer A until the absorbance at 280 nm wavelength reached the baseline equilibrium. Subsequently, within 2 minutes (buffer flow rate was 1 mL/min), the buffer system was continuously converted from 100% buffer A (20 mM histidine, 1.5 M NaCl, pH 5.5) to 100% buffer B (20 mM histidine, pH 5.5) for elution. The eluted and purified HG-ADC-3-D1 was then subjected to solution replacement to be concentrated into 20 mM histidine buffer with an Amicon ultrafiltration tube (10 kDa), and filtered with a 0.2 μm PVDF needle filter to give the ADC product, which was sampled for evaluation.

**2. Preparation of HG-ADC-3-D2**

In PB buffer (40 mM PB, 2 mM EDTA, pH 7.0), the antibody Herceptin was reduced with 1 eq. of tris(2-carboxyethyl)phosphine (TCEP), and the reaction mixture was shaken on a shaker at 22 °C for 3 hours (with a shaking speed of 60 rpm). Without removing excess TCEP, the reaction solution was directly used for the next coupling reaction. PB buffer, dimethylacetamide (DMA) and an linker-payload HG-PL3 solution (a mixed solution of water:DMA = 3:1 (volume ratio), 5.47 mg/mL, 3 eq.) were slowly added to the reduced antibody in an ice bath. After mixing well, the coupling reaction solution was reacted at 4 °C for 2 hours. After the reaction was completed, the ADC solution was filtered with a 0.22 μm PVDF needle filter, and then purified using a hydrophobic interaction chromatography column (GE HiTrap Butyl HP column, 1 mL) on a protein purification apparatus (AKTA). Before the purification, the column was equilibrated with 10 mL of buffer A (20 mM Histidine, 1.2 M NaCl, pH 5.5). The NaCl concentration in the ADC solution was adjusted to 1.5 M with buffer (20 mM histidine, 5.0 M NaCl, pH 5.5), so that the ADC could be bound to the chromatographic column packing material. After loading the sample, the chromatographic column was washed with buffer A until the absorbance at 280 nm wavelength reached the baseline equilibrium. Subsequently, within 2 minutes (buffer flow rate was 1 mL/min), the buffer system was continuously converted from 100% buffer A (20 mM histidine, 1.2 M NaCl, pH 5.5) to 100% buffer B (20 mM histidine, pH 5.5) for elution. The eluted and purified HG-ADC-3-D2 was then subjected to solution replacement to be concentrated into 20 mM histidine buffer with an Amicon ultrafiltration tube (10 kDa), and filtered with a 0.2 μm PVDF needle filter to give the ADC product, which was sampled for evaluation.

**Example 18 Synthesis of Trastuzumab Conjugate (HG-ADC-4) Using HG-PL4 Prepared in Examples as Scaffold**

**1. Preparation of HG-ADC-4-D1**

In PB buffer (40 mM PB, 2 mM EDTA, pH 7.0), the antibody Herceptin was reduced with 1 eq. of tris(2-carboxyethyl)phosphine (TCEP), and the reaction mixture was shaken on a shaker at 22 °C for 3 hours (with a shaking speed of 60 rpm). Without removing excess TCEP, the reaction solution was directly used for the next coupling reaction. PB buffer, dimethylacetamide (DMA) and a solution of linker-payload HG-PL4 in dimethylacetamide (DMA) (6.43 mg/mL, 1 eq.) were slowly added to the reduced antibody in an ice bath. After mixing well, the coupling reaction solution was reacted at 4 °C for 1.5 hours. L-cysteine (1.21 mg/ml aqueous solution, 4 eq.) was added to the reaction solution, and the mixture was mixed well and reacted at 4 °C for 0.5 hours. After the reaction was completed, the ADC solution was filtered with a 0.22 μm PVDF needle filter, and then purified using a hydrophobic interaction chromatography column (GE HiTrap Butyl HP column, 1 mL) on a protein purification apparatus (AKTA). Before the purification, the column was

equilibrated with 10 mL of buffer A (20 mM Histidine, 1.5 M NaCl, pH 5.5). The NaCl concentration in the ADC solution was adjusted to 2.0 M with buffer (20 mM histidine, 5.0 M NaCl, pH 5.5), so that the ADC could be bound to the chromatographic column packing material. After loading the sample, the chromatographic column was washed with buffer A until the absorbance at 280 nm wavelength reached the baseline equilibrium. Subsequently, within 2 minutes (buffer flow rate was 1 mL/min), the buffer system was continuously converted from 100% buffer A (20 mM histidine, 1.5 M NaCl, pH 5.5) to 100% buffer B (20 mM histidine, pH 5.5) for elution. The eluted and purified HG-ADC-4-D1 was then subjected to solution replacement to be concentrated into 20 mM histidine buffer with an Amicon ultrafiltration tube (10 kDa), and filtered with a 0.2 μm PVDF needle filter to give the ADC product, which was sampled for evaluation.

**2. Preparation of HG-ADC-4-D2**

In PB buffer (40 mM PB, 2 mM EDTA, pH 7.0), the antibody Herceptin was reduced with 1 eq. of tris(2-carboxyethyl)phosphine (TCEP), and the reaction mixture was shaken on a shaker at 22 °C for 3 hours (with a shaking speed of 60 rpm). Without removing excess TCEP, the reaction solution was directly used for the next coupling reaction. PB buffer, dimethylacetamide (DMA) and a solution of linker-payload HG-PL4 in dimethylacetamide (DMA) (6.43 mg/mL, 3 eq.) were slowly added to the reduced antibody in an ice bath. After mixing well, the coupling reaction solution was reacted at 4 °C for 2 hours. After the reaction was completed, the ADC solution was filtered with a 0.22 μm PVDF needle filter, and then purified using a hydrophobic interaction chromatography column (GE HiTrap Butyl HP column, 1 mL) on a protein purification apparatus (AKTA). Before the purification, the column was equilibrated with 10 mL of buffer A (20 mM Histidine, 1.2 M NaCl, pH 5.5). The NaCl concentration in the ADC solution was adjusted to 1.5 M with buffer (20 mM histidine, 5.0 M NaCl, pH 5.5), so that the ADC could be bound to the chromatographic column packing material. After loading the sample, the chromatographic column was washed with buffer A until the absorbance at 280 nm wavelength reached the baseline equilibrium. Subsequently, within 2 minutes (buffer flow rate was 1 mL/min), the buffer system was continuously converted from 100% buffer A (20 mM histidine, 1.2 M NaCl, pH 5.5) to 100% buffer B (20 mM histidine, pH 5.5) for elution. The eluted and purified HG-ADC-4-D2 was then subjected to solution replacement to be concentrated into 20 mM histidine buffer with an Amicon ultrafiltration tube (10 kDa), and filtered with a 0.2 μm PVDF needle filter to give the ADC product, which was sampled for evaluation.

**Data Summary of Antibody Drug Conjugates:**

**A determination method for free linker-payload (Free drug) level:**

A free linker-payload level in an ADC product was detected by reverse-phase high-performance liquid chromatography (RP-HPLC).

1) The linker-payload was diluted to 20 μg/mL with DMA, and then the solvent was diluted to 2 X mol/L and 0.8 X mol/L with 20 mM histidine buffer (pH 5.5) and DMA, with the final solution containing 10% DMA.

$$5/100 = \frac{\mathrm{X\ mol/L}}{\mathrm{X} + \mathrm{DAR} * \frac{\mathrm{1mg/mL}}{\mathrm{MW_{protein}} + \mathrm{DAR} * \mathrm{MW_{drug}}}}$$

2) Antibodies were diluted to 2 mg/mL with 20 mM histidine buffer (pH 5.5) and DMA, with the final solution containing 10% DMA.

3) 12.5 μL of the solution of 2) was added to 12.5 μL of the solution of 1) respectively, resulting in a standard solution with an antibody concentration of 1 mg/mL and a linker-payload concentration of 5% or 2% (molar percentage), respectively.

4) ADC was diluted to 1 mg/mL with 20 mM histidine buffer (pH 5.5) and DMA, with the final solution containing 10% DMA.

5) The prepared ADC solution and the 5% or 2% (molar percentage) linker-payload standard solution were detected by LC-MS.

| | |
|---|---|
| Chromatographic column | Agilent, PLRP-S, 2.1*50mm, 8μm |
| Wavelength | 252 nm |
| Column temperature | 80 °C |
| Flow rate | 0.4 mL/min |

(continued)

| | | | |
|---|---|---|---|
| Time | 13 min | | |
| Injection volume | 10 μL | | |
| Mobile phase system | Mobile phase A: 0.025% trifluoroacetic acid and 0.1% formic acid in water<br>Mobile phase B: 0.025% trifluoroacetic acid and 0.1% formic acid in acetonitrile | | |
| Gradient | Time (min)<br>0.0<br>0.5<br>2.0<br>8.0<br>8.5<br>10.0<br>10.01<br>13.0 | A (%)<br>95<br>95<br>70<br>20<br>10<br>10<br>95<br>95 | B (%)<br>5<br>5<br>30<br>80<br>90<br>90<br>5<br>5 |

The DAR of antibody drug conjugate HG-ADC-2-D1 was 2.85, the DAR of HG-ADC-2-D2 was 7.17, the DAR of HG-ADC-3-D1 was 4.50, the DAR of HG-ADC-3-D2 was 7.80, the DAR of HG-ADC-4-D1 was 3.81, and the DAR of HG-ADC-4-D2 was 6.51.

**Example 19 In Vitro Characterization Example**

Sample information:

| Sample | **Concentration (mg/mL)** | **Buffer** |
|---|---|---|
| DS8201 | 2.87 | 20 mM **histidine** pH 5.5 |
| HG-ADC-1-D2 | 1.22 | 20 mM **histidine** pH 5.5 |
| HG-ADC-1-D4 | 3.48 | 20 **mM histidine pH** 5.5 |
| HG-ADC-2-D1 | 0.71 | 20 mM **histidine** pH 5.5 |
| HG-ADC-2-D2 | 0.61 | 20 mM **histidine** pH 5.5 |
| HG-ADC-3-D1 | 4.16 | 20 mM **histidine** pH 5.5 |
| HG-ADC-3-D2 | 3.87 | 20 mM **histidine** pH 5.5 |
| HG-ADC-4-D1 | 0.48 | 20 mM **histidine** pH 5.5 |
| HG-ADC-4-D2 | 0.63 | 20 mM **histidine** pH 5.5 |
| MMAE | 0.5 | DMSO |
| Eribulin | 0.5 | DMSO |
| mAb- Hercetin. | 20.98 | 40 mM PB, 2 mM EDTA, pH7.0 |
| Human·IgG1·isotype ( hIgG1_Isotype ) control. | 13.60 | 20 mM**histidine**←5% (W/V)**sucrose, pH.** 5.5 |
| HG-PL1 | 10.00 | $H_2O$ |
| DS8201 scaffold (no antibody) | 10.00 | DMA |

Equipment

| Instrument | Supplier |
|---|---|
| Centrifuge 5810R | Eppendorf |
| Flow Cytometry FACS CantoII | BD Biosciences |
| Multifunctional microplate reader EnVision | PerkinElmer |

Reagents and materials

| Reagents/Materials/Cells | Supplier | Item No. |
|---|---|---|
| NCI-N87 | ATCC | CRL-5822 |
| RPMI 1640 medium | Gibco | 22400-089 |
| FBS | ExCell Bio | FND500 |
| Polystyrene round bottom 96-well microplate | Corning | 3799 |
| Goat Anti-Human-IgG Fc--Alexa 647 Antibody | Jackson | 109-605-098 |
| 96-well black-walled, clear-bottom microplate | Greinier | 655090 |
| Cell Titer Glo | Promega | G7573 |

**Assay method**

**Assay of FACS binding specificity**

When the confluence of cultured NCI-N87 cells reached 70-90%, the adherent cells were digested with trypsin. The digestion was terminated with RPMI 1640 medium (Gibco, 22400-089) containing 10% FBS (ExCell Bio, FND500). The cell suspension was centrifuged at 1500 rpm at 4°C for 5 minutes (Eppendorf, 5810R). Then the cells were resuspended by adding PBS containing 1% BSA, and the cell suspension was adjusted to a density of $1\times10^6$ cells/mL. 100 μL of cell suspension with $1\times10^6$ cells/mL was added to each well of a 96-well round-bottom microplate (Corning, 3799). The plate was centrifuged and the supernatant was discarded. ADC or monoclonal sample was serially diluted in a ratio of 1:5 (with a maximum concentration of 100 nM) in PBS containing 1% BSA, and 100 μL of the diluted sample was added to each well. The plate was incubated at 4°C for 1 hour. At the same time, human IgG1 antibody was used as an isotype control. After the incubation was completed, each well was washed twice by adding 160 μL of PBS containing 1% BSA, and then 100 μL of goat anti-human-IgG Fc--Alexa 647 antibody diluted in a ratio of 1:500 in PBS containing 1% BSA was added as a secondary antibody. The plate was incubated at 4°C for 30 minutes in the dark. After the incubation was completed, the plate was washed twice and the cells were resuspended in 80 μL of PBS containing 1% BSA. The fluorescence intensity was detected by flow cytometry (BD Biosciences, FACS CantoII), and the fluorescence value was expressed as mean fluorescence intensity (MFI).

The raw data obtained from the FACS assay were analyzed with FlowJo software, and the $EC_{50}$ value was calculated by using GraphPad Prism 6 non-linear four-parameter non-linear regression with the sample wells not containing antibodies or in which only a secondary antibody was incubated as the background fluorescence intensity of cells.

**Cytotoxicity assay**

When the confluence of cultured NCI-N87 cells reached 70-90%, the cells were digested with trypsin. The trypsin was removed by centrifugation. The cells were resuspended to $1\times10^5$ cells/mL in RPMI 1640 medium containing 10% FBS. 50 μL of cell suspension was added to each well of a 96-well black-walled, clear-bottom microplate (Greinier, 655090), and incubated in a 5% carbon dioxide incubator at 37°C overnight to make the cells adhere to the wall. The next day, ADC or linker-toxin sample was serially diluted in a ratio of 1:5 (with a maximum concentration of 50 nM) in RPMI 1640 medium containing 10% FBS, and 50 μL of diluted ADC or linker-toxin sample was added to each well. The plate was incubated in a 5% $CO_2$ incubator at 37°C for 6 days. After the incubation was completed, the cell viability was detected with CellTiter-Glo reagent (Promega, G7573), and the specific cell viability value was measured by a multifunctional microplate reader (PerkinElmer, EnVision).

The cytotoxicity of each sample well was calculated by comparing the cell viability value of the sample well with that of the control well having only cells, and the calculation formula was: cytotoxicity = 100* (cell viability value $_{\text{well having only cells}}$ - cell viability value $_{\text{sample well}}$) / cell viability value $_{\text{well having only cells}}$; $IC_{50}$ values were calculated using GraphPad Prism 6 nonlinear four-parameter nonlinear regression.

**Assay results**

**Assay of FACS binding specificity**

In this assay, the specific binding of DS8201, HG-ADC-1-D2, HG-ADC-1-D4, HG-ADC-2-D1, HG-ADC-2-D2, HG-ADC-3-D1, HG-ADC-3-D2, HG-ADC-4-D1, HG-ADC-4-D2, mAb-Herceptin and hIgG1_Isotype to HER2-positive NCI-N87 cells was analyzed by FACS. The assay results show that the $EC_{50}$ value and the binding curve of nine ADC samples DS8201, HG-ADC-1-D2, HG-ADC-1-D4, HG-ADC-2-D1, HG-ADC-2-D2, HG-ADC-3-D1, HG-ADC-3-D2, HG-ADC-4-D1, and HG-ADC-4-D2 with NCI-N87 cells were comparable to those of naked Herceptin antibody (i.e. mAb-Herceptin in the Table), while the binding curves of all these samples reached the upper plateau. However, the hIgG1_Isotype control (an antibody without antigen-binding properties) was not bound to NCI-N87 cells. See Table 1 to Table 3 for the corresponding sample information and $EC_{50}$.

Table 1: Summary of FACS detection of binding of ADC1 and corresponding monoclonal antibody to NCI-N87 cells

| Sample name | NCI-N87 | | |
|---|---|---|---|
| | $EC_{50}$ (nM) | Max MFI | Neg fluorescence value |
| DS8201 | 1.565 | 47400.0 | 12.6 |
| HG-ADC-1-D2 | 1.928 | 45100.0 | |
| HG-ADC-1-D4 | 1.876 | 44400.0 | |
| mAb-Herceptin | 1.543 | 51000.0 | |
| hIgG1_Isotype | NA | 32.5 | |
| Note: Neg, a parallel control well in which only a secondary antibody was incubated, background fluorescence value. | | | |

Table 2: Summary of FACS detection of binding of ADC2 and corresponding monoclonal antibody to NCI-N87 cells

| Sample name | NCI-N87 | | |
|---|---|---|---|
| | $EC_{50}$ (nM) | Max MFI | Neg fluorescence value |
| HG-ADC-2-D1 | 3.31 | 103000 | 80.8 |
| HG-ADC-2-D2 | 3.27 | 102000 | |
| mAb-Herceptin | 2.10 | 106000 | |
| hIgG1_Isotype | NA | 216 | |

Table 3: Summary of FACS detection of binding of ADC3, ADC4 and corresponding monoclonal antibodies to NCI-N87 cells

| Sample name | NCI-N87 | | |
|---|---|---|---|
| | $EC_{50}$ (nM) | Max MFI | Neg fluorescence value |
| HG-ADC-3-D1 | 2.63 | 107000 | 59.3 |
| HG-ADC-3-D2 | 2.80 | 106000 | |
| HG-ADC-4-D1 | 2.37 | 108000 | |
| HG-ADC-4-D2 | 2.30 | 108000 | |
| mAb-Herceptin | 1.38 | 123000 | |
| hIgG1_Isotype | NA | 118 | |

**Cytotoxicity assay**

In this assay, the cytotoxicity of DS8201, HG-ADC-1-D2, HG-ADC-1-D4, HG-ADC-2-D1, HG-ADC-2-D2, HG-ADC-3-D1, HG-ADC-3-D2, HG-ADC-4-D1, HG-ADC-4-D2, scaffold HG-PL1, and DS8201 scaffold to NCI-N87 cells were detected by the cytotoxic method described above. The assay results show that HG-ADC-1-D2, HG-ADC-1-D4, HG-ADC-2-D1, HG-ADC-2-D2, HG-ADC-3-D1, HG-ADC-3-D2, HG-ADC-4-D1, and HG-ADC-4-D2 showed more sensitive cytotoxicity to NCI-N87 cells compared to DS8201. The results are shown in Table 4.

Table 4: Summary of toxicity of ADC to NCI-N87 cells

| Sample name | NCI-N87 | |
|---|---|---|
| | $IC_{50}$ (nM) | Max Cvtotoxicitv% |
| DS8201 | 0.152600 | 92.17 |
| HG-ADC-1-D2 | 0.009839 | 92.88 |
| HG-ADC-1-D4 | 0.006813 | 94.41 |
| HG-ADC-2-D1 | 0.056 | 92.15 |
| HG-ADC-2-D2 | 0.020 | 93.31 |
| HG-ADC-3-D1 | 0.015 | 93.22 |
| HG-ADC-3-D2 | 0.0086 | 89.97 |
| HG-ADC-4-D1 | 0.026 | 93.56 |
| HG-ADC-4-D2 | 0.0083 | 90.31 |
| Eribulin | 0.22 | 93.99 |
| MMAE | 0.13 | 93.50 |
| HG-PL1 | NA | 49.53 |
| DS8201 scaffold | NA | 9.38 |

All features disclosed in this specification may be combined in any combination. Each feature disclosed in this specification may be replaced by alternative features serving the same, equivalent or similar purpose. Thus, unless expressly stated otherwise, each feature disclosed is only an example of a series of equivalent or similar features.

From the above description, one skilled in the art can easily ascertain the essential characteristics of this invention, and can make various changes and modifications to the invention without departing from the spirit and scope of the invention to adapt this invention to various usages and conditions. Accordingly, other examples are also within the scope of the appended claims.

## Claims

1. A compound of formula (I), or a tautomer, a stereoisomer or a pharmaceutically acceptable salt thereof, wherein

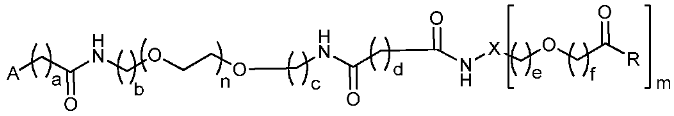

(I)

a, b, c, d, e, and f are each independently 0, 1, 2, 3, 4, 5, 6, 7, 8, 9 or 10; alternatively 0, 1, 2, 3, 4 or 5; alternatively 0, 1, 2 or 3; alternatively 1 or 2;
n is 1, 2, 3, 4, 5, 6, 7, 8, 9, 10, 11, 12, 13, 14, 15, 16, 17, 18, 19 or 20; alternatively 1, 2, 3, 4, 5, 6, 7, 8, 9 or 10; alternatively 1, 2, 3, 4 or 5; alternatively 1, 2 or 3; alternatively 1;
m is 2 or 3;
X is C, N or Si;
A is $-NH_2$, -NH-PG1,

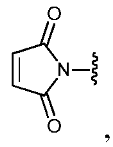, 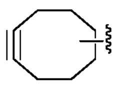 or 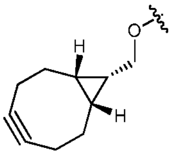;

R is -OH, -O-PG2,

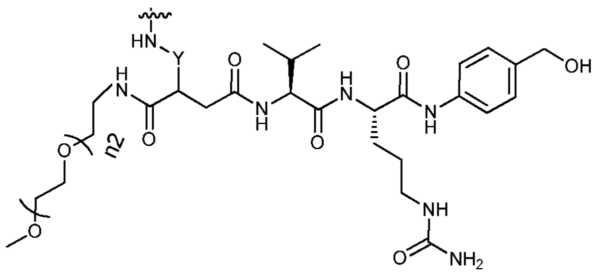,

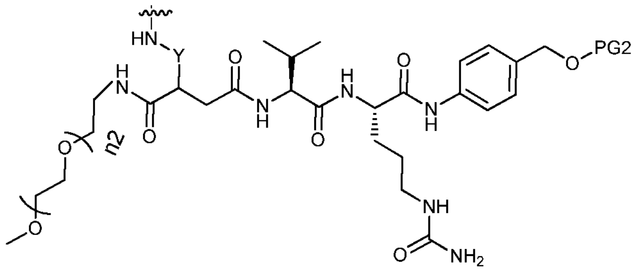,

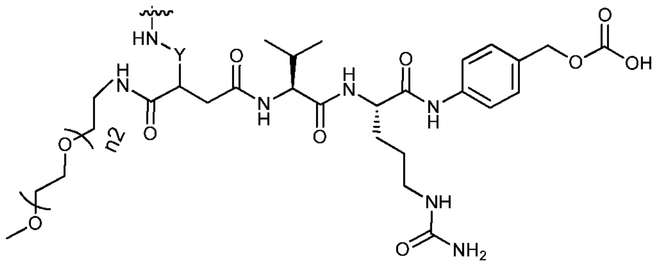

or

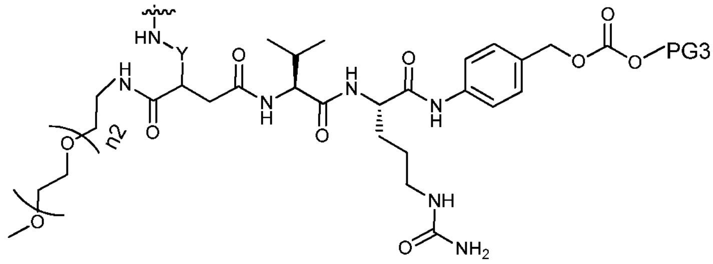

;

wherein PG1, PG2 and PG3 are protecting groups;
n2 is 5, 6, 7, 8, 9, 10, 11, 12, 13, 14, 15, 16, 17, 18, 19 or 20; alternatively 5, 6, 7, 8, 9, 10, 11, 12, 13, 14 or 15; alternatively 7, 8, 9, 10 or 11;
Y is a bond or

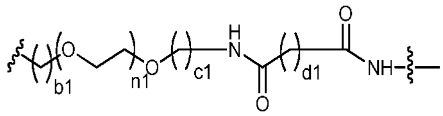

;

wherein n1 is 1, 2, 3, 4, 5, 6, 7, 8, 9, 10, 11, 12, 13, 14, 15, 16, 17, 18, 19 or 20; alternatively 1, 2, 3, 4, 5, 6, 7, 8, 9 or 10; alternatively 1, 2, 3, 4 or 5; alternatively 1, 2 or 3;
b1, c1, and d1 are each independently 0, 1, 2, 3, 4, 5, 6, 7, 8, 9 or 10; alternatively 0, 1, 2, 3, 4 or 5; alternatively 0, 1, 2 or 3; alternatively 1 or 2;
when X is C or Si, m is 3;
when X is N, m is 2.

2. The compound according to claim 1, which is a compound represented by formula (II), or a tautomer, a stereoisomer or a pharmaceutically acceptable salt thereof,

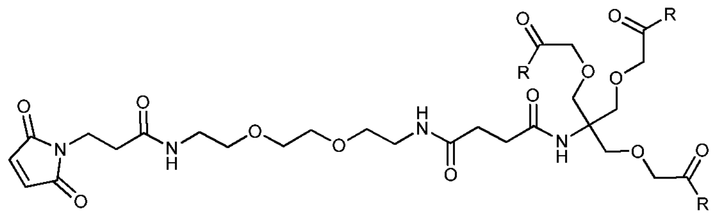

(II).

3. The compound according to claim 1, **characterized in that** the compound is a compound represented by formula (III), or a tautomer, a stereoisomer or a pharmaceutically acceptable salt thereof,

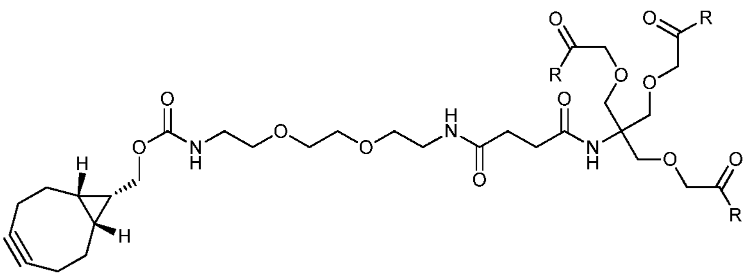

(III).

4. The compound according to any one of claims 1 to 3, **characterized in that** R is -OH, -O-PG2,

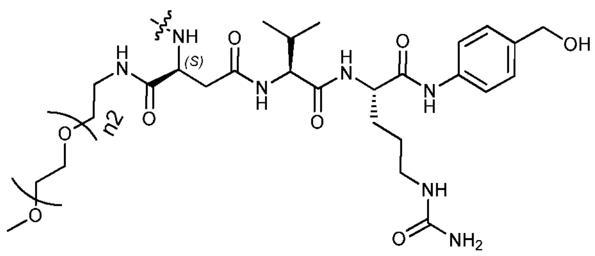

,

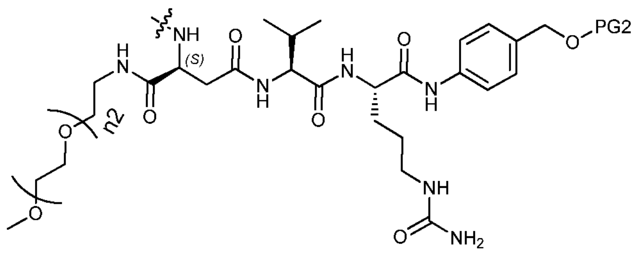

,

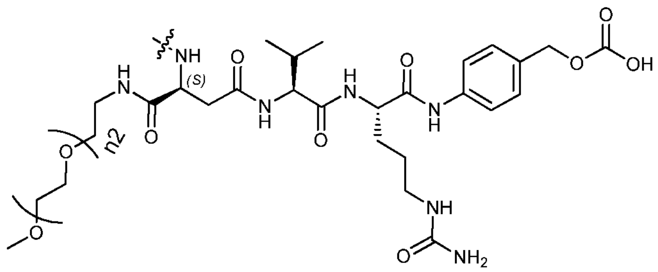

,

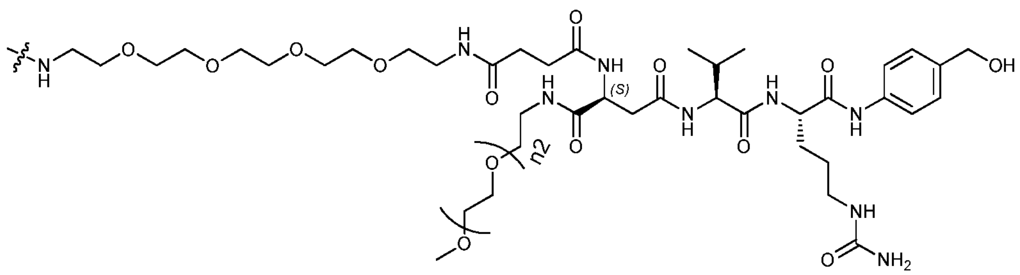

,

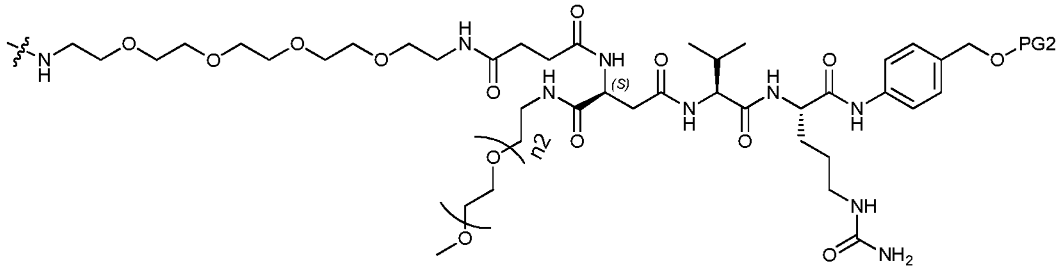

,

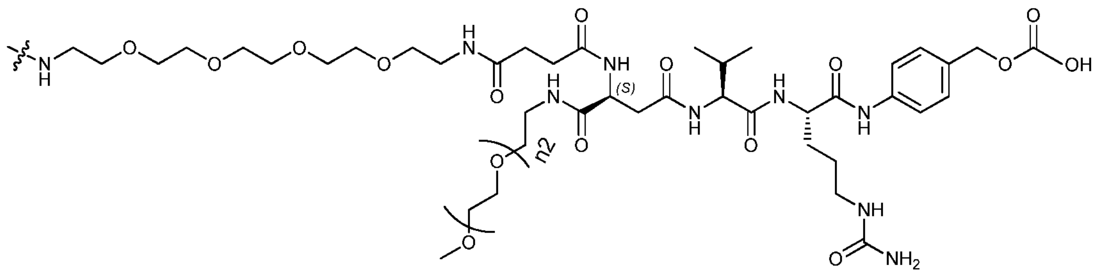

,

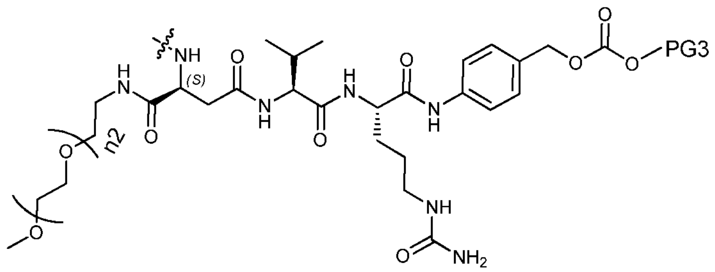

,

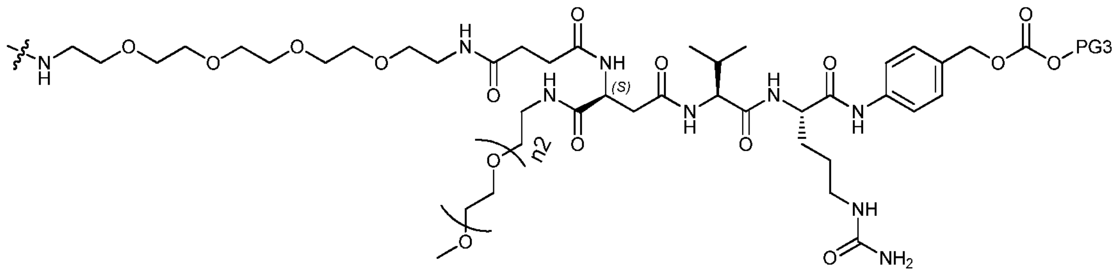

,

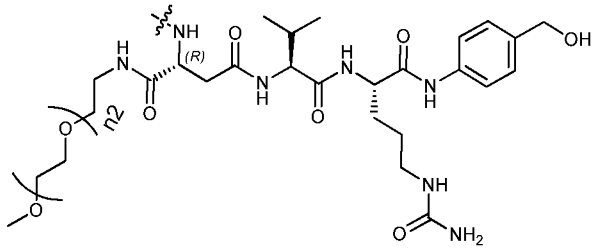

,

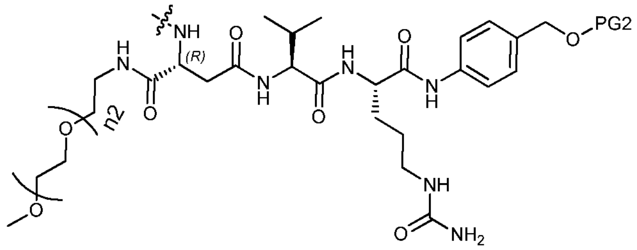

,

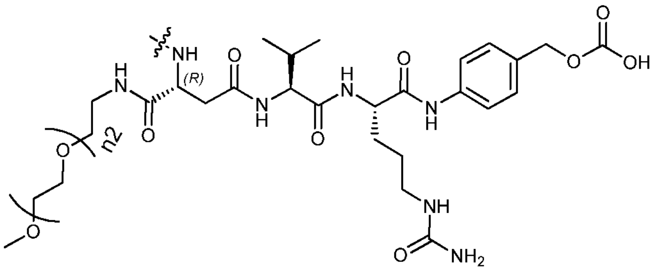

,

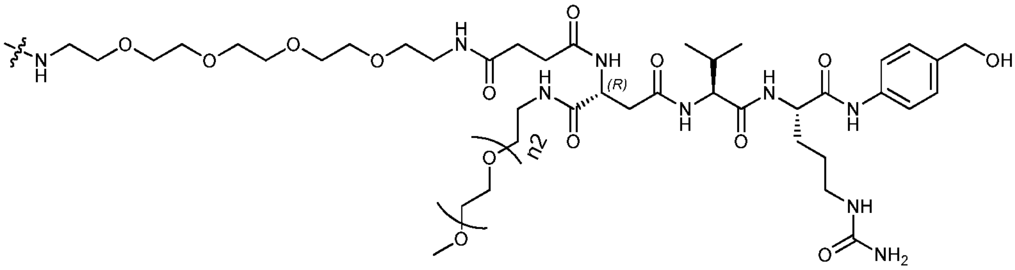

,

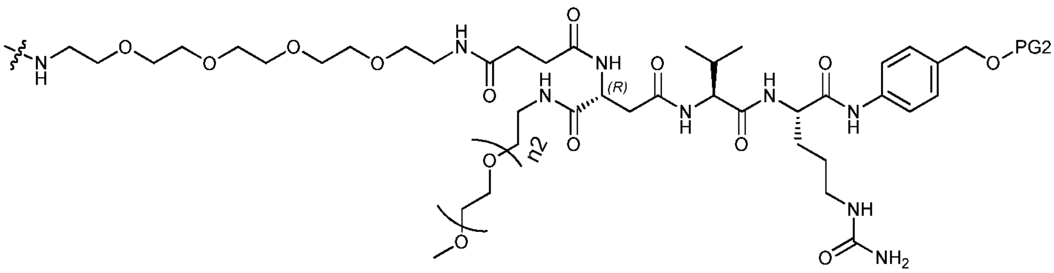

,

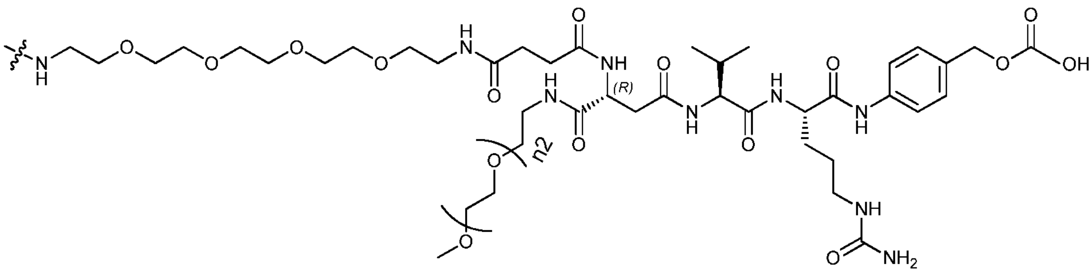

,

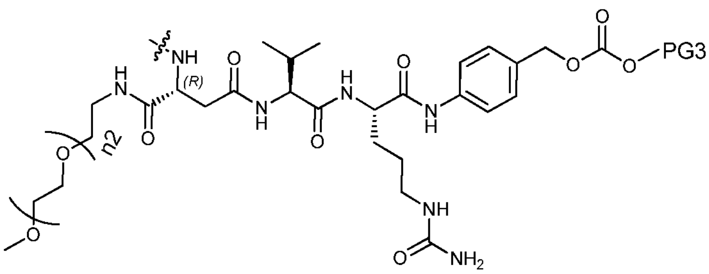

,

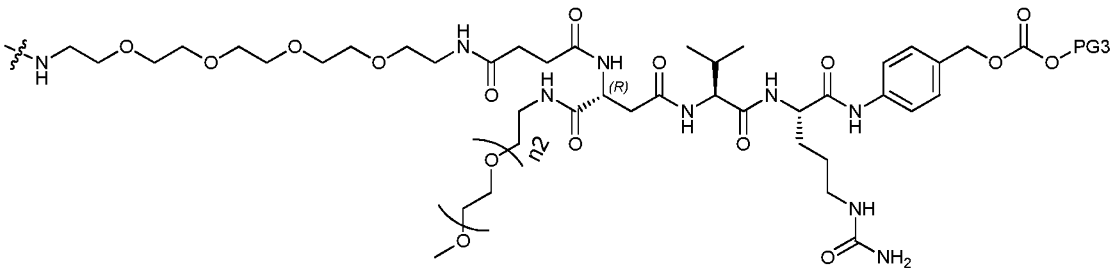

.

5. The compound according to any one of claims 1 to 4, wherein n2 is 7 or 11.

6. The compound according to any one of claims 1 to 5, wherein the PG1 is an amino-protecting group;

optionally, the amino-protecting group is selected from acetyl, trifluoroacetyl, tert-butoxycarbonyl (BOC, Boc), benzyloxycarbonyl (CBZ, Cbz) and 9-fluorenylmethyleneoxycarbonyl (Fmoc);
the PG2 is a hydroxyl-protecting group;
optionally, the hydroxyl-protecting group is selected from acetyl and silyl;
the PG3 is a carboxyl-protecting group;
optionally, the carboxyl-protecting group is selected from $-CH_2CH_2SO_2Ph$, cyanoethyl, 2-(trimethylsilyl)ethyl, 2-(trimethylsilyl)ethoxymethyl, 2-(p-toluenesulfonyl)ethyl, 2-(p-nitrobenzenesulfonyl)ethyl, 2-(diphenylphosphino)ethyl and nitroethyl.

7. A conjugate comprising the compound of any one of claims 1 to 6 and a drug moiety, wherein the compound is covalently bound to the drug moiety via an R group.

8. The conjugate according to claim 7, wherein the drug is selected from one or more of eribulin, methyl auristatin E and SN-38.

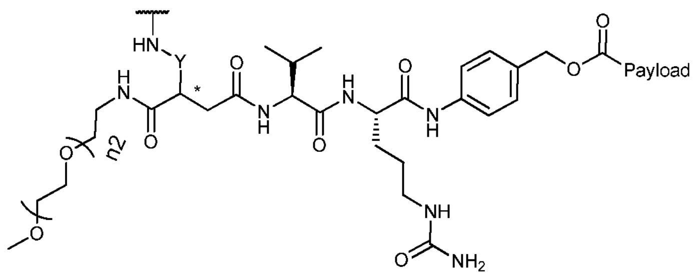

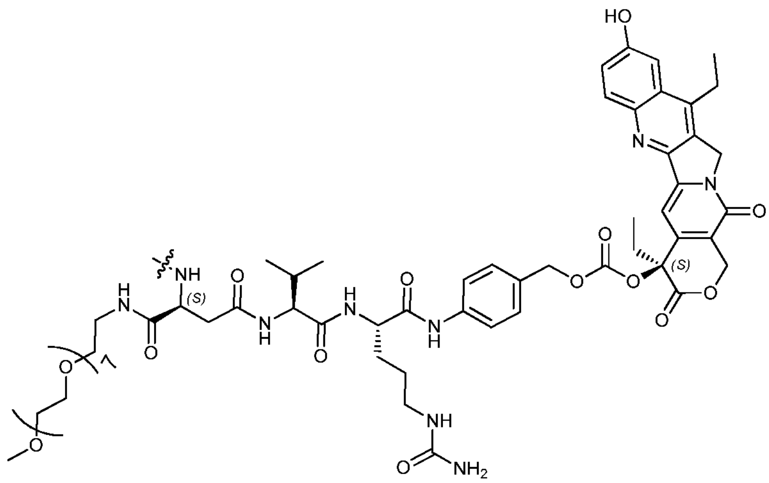

9. The conjugate according to any one of claims 7 to 8, further comprising a targeting moiety, wherein one or more of the conjugates are covalently bound to the targeting moiety via an A group.

**10.** A conjugate comprising a targeting moiety and one or more compounds according to any one of claims 1 to 6, wherein the compound is covalently bound to the targeting moiety via an A group.

**11.** The conjugate according to claim 9 or 10, wherein the targeting moiety is a protein-based recognition molecule.

**12.** The conjugate according to claim 11, wherein the recognition molecule is an internalizing antibody or an internalizing antigen-binding fragment thereof targeting tumor cells.

**13.** The conjugate of claim 12, wherein the antibody or antigen-binding fragment binds to an antibody selected from the group consisting of: anti-human epidermal growth factor receptor (HER2) antibody, EGFR, GPNMB, CD56, TACSTD2 (TROP2), CEACAM5, folate receptor-a, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin outer domain B, endothelin receptor ETB, tenascin c, collagen IV, VEGFR2, periostin, CD30, CD79b, CD19, CD22, CD138, CD37, CD33, CD74, etc.

**14.** A pharmaceutical composition comprising the conjugate according to any one of claims 7 to 13 and a pharmaceutically acceptable carrier.

**15.** Use of the conjugate according to any one of claims 7 to 13 or the pharmaceutical composition according to claim 14 in the manufacture of a medicament for treating a patient having or at risk of having a cancer expressing a target antigen.

**16.** The conjugate according to any one of claims 7 to 13 or the pharmaceutical composition according to claim 14, for use in treating a patient having or at risk of having a cancer expressing a target antigen.

**17.** A method of treating a patient having or at risk of having a cancer expressing a target antigen, comprising administering to the patient the conjugate according to any one of claims 7 to 13 or the pharmaceutical composition according to claim 14.

**18.** The use according to claim 15 or the conjugate or pharmaceutical composition for use according to claim 16 or the method according to claim 17, wherein the target antigen is human epidermal growth factor receptor 2.

**19.** The use according to claim 15 or the conjugate or pharmaceutical composition for use according to claim 16 or the method according to claim 17, wherein the cancer expresses a high level of human epidermal growth factor receptor 2.

**20.** The use according to claim 15 or the conjugate or pharmaceutical composition for use according to claim 16 or the method according to claim 17, wherein the cancer is selected from breast cancer, gastric cancer, bladder cancer and urothelial cell carcinoma.

## INTERNATIONAL SEARCH REPORT

International application No.
**PCT/CN2022/074523**

**A. CLASSIFICATION OF SUBJECT MATTER**

A61K 47/68(2017.01)i； A61K 47/66(2017.01)i； A61K 47/60(2017.01)i； A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, DWPI, SIPOABS, CNTXT, EPTXT, USTXT, WOTXT, CNKI, PUBMED-CHEM, BING, STNext, 百度学术, BAIDU SCHOLAR, 读秀, DUXIU: 南京桦冠生物, NANJING HUAGUAN BIOTECHNOLOGY, 陈剑, 赵海波, 顾榕, 抗体, 偶联, 抗体药物偶联, 缀合, antibody, drug, conjugate?, ADC, ADCs, 毒素, payload, 连接子, linker, 连接臂, 结构检索, structural search

**C. DOCUMENTS CONSIDERED TO BE RELEVANT**

| Category* | Citation of document, with indication, where appropriate, of the relevant passages | Relevant to claim No. |
|---|---|---|
| X | CN 110234357 A (MERSANA THERAPEUTICS INC.) 13 September 2019 (2019-09-13) description, paragraphs 0004-0008, 0022-0038, 0045, 0048, 0049, 0072, 0084, 0090, 0091, 0652, and 0706, and tables B and C | 1-20 |
| Y | CN 110234357 A (MERSANA THERAPEUTICS INC.) 13 September 2019 (2019-09-13) description, paragraphs 0004-0008, 0022-0038, 0045, 0048, 0049, 0072, 0084, 0090, 0091, 0652, and 0706, and tables B and C | 1-20 |
| X | EP 2913064 A1 (CELARES GMBH) 02 September 2015 (2015-09-02) description, paragraphs 0008-0020, and figure 6 | 1-20 |
| Y | CN 101541332 A (ENZON PHARMACEUTICALS, INC.) 23 September 2009 (2009-09-23) abstract, and claims 1 and 25 | 1-20 |
| A | WO 2015105083 A1 (SHIONOGI & CO., LTD.) 16 July 2015 (2015-07-16) claims 1-9, and description, paragraph 0211, compound 165 | 1-20 |
| A | CN 106061981 A (SOLSTICE BIOLOG LTD.) 26 October 2016 (2016-10-26) claims 1-278 | 1-20 |
| A | CN 107109405 A (SOLSTICE BIOLOG LTD.) 29 August 2017 (2017-08-29) claims 1-111 | 1-20 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 April 2022** | **28 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No. PCT/CN2022/074523

**C. DOCUMENTS CONSIDERED TO BE RELEVANT**

| Category* | Citation of document, with indication, where appropriate, of the relevant passages | Relevant to claim No. |
|---|---|---|
| A | CN 102006890 A (ALNYLAM PHARMACEUTICALS, INC.) 06 April 2011 (2011-04-06) claims 1-23 | 1-20 |
| A | CN 110520161 A (UNIVERSITY OF ILLINOIS) 29 November 2019 (2019-11-29) claims 1-122 | 1-20 |
| A | CN 103491982 A (ARROWHEAD RESEARCH CORP.) 01 January 2014 (2014-01-01) claims 1-20 | 1-20 |
| A | US 2013317080 A1 (ALNYLAM PHARMACEUTICALS, INC.) 28 November 2013 (2013-11-28) claims 1-33, and description, paragraphs 0269-0307 | 1-20 |
| X | LIANG, Chihui et al.,. "Effects of Neighboring Glycans on Antibody-Carbohydrate Interaction，" *Angewandte Chemie International Edition,* Vol. 50, No. 7, 05 January 2011 (2011-01-05), pp. 1608-1612 | 1, 4-6 |
| A | GEEL, R. V. et al.,. "Chemoenzymatic conjugation of toxic payloads to the globally conserved N-glycan of native mAbs provides homogeneous and highly efficacious antibody-drug conjugates," *Bioconjugate Chemistry,* Vol. 26, 10 June 2015 (2015-06-10), pp. 2233-2242 | 1-20 |

Form PCT/ISA/210 (second sheet) (January 2015)

**INTERNATIONAL SEARCH REPORT**

International application No.
**PCT/CN2022/074523**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17、18-20**（部分）
because they relate to subject matter not required to be searched by this Authority, namely:

[1] The subject matter of claim 17 relates to a method for treating a human body. Dependent claims 18-20 (in part) correspondingly referring to claim 17 also relate to a method for treating a human body, and therefore do not comply with PCT Rule 39.1(iv). The search report is formed on the basis that the subject matter of claim 17 is "a use of the conjugate according to any one of claims 7-13 or the pharmaceutical composition according to claim 14 in the preparation of drugs for treating a patient who suffers from a cancer expressing a target antigen or is at the risk of developing a cancer expressing a target antigen", and that dependent claims 18-20 (in part) refers to the use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/074523**

| Patent document cited in search report | | | Publication date (day/month/year) | Patent family member(s) | | | Publication date (day/month/year) |
|---|---|---|---|---|---|---|---|
| CN | 110234357 | A | 13 September 2019 | BR | 112019008854 | A2 | 09 July 2019 |
| | | | | WO | 2018098269 | A2 | 31 May 2018 |
| | | | | CA | 3041559 | A1 | 31 May 2018 |
| | | | | EP | 3544635 | A2 | 02 October 2019 |
| | | | | JP | 2020510669 | A | 09 April 2020 |
| | | | | TW | 201827082 | A | 01 August 2018 |
| | | | | US | 2018154018 | A1 | 07 June 2018 |
| | | | | MX | 2019005879 | A | 09 December 2019 |
| | | | | KR | 20190087535 | A | 24 July 2019 |
| | | | | IL | 266310 | D0 | 30 June 2019 |
| | | | | AU | 2017363246 | A1 | 16 May 2019 |
| EP | 2913064 | A1 | 02 September 2015 | None | | | |
| CN | 101541332 | A | 23 September 2009 | RU | 2009114154 | A | 20 October 2010 |
| | | | | WO | 2008034124 | A2 | 20 March 2008 |
| | | | | KR | 20090057383 | A | 05 June 2009 |
| | | | | BR | PI0716812 | A2 | 05 November 2013 |
| | | | | EP | 2073820 | A2 | 01 July 2009 |
| | | | | CA | 2662981 | A1 | 20 March 2008 |
| | | | | MX | 2009002855 | A | 30 March 2009 |
| | | | | AU | 2007296056 | A1 | 20 March 2008 |
| | | | | IL | 197517 | D0 | 24 December 2009 |
| | | | | JP | 2010503708 | A | 04 February 2010 |
| WO | 2015105083 | A1 | 16 July 2015 | JP | WO2015105083 | A1 | 23 March 2017 |
| CN | 106061981 | A | 26 October 2016 | WO | 2015069932 | A1 | 14 May 2015 |
| | | | | JP | 2016537027 | A | 01 December 2016 |
| | | | | EP | 3066105 | A1 | 14 September 2016 |
| | | | | AU | 2014346658 | A1 | 02 June 2016 |
| | | | | CA | 2929651 | A1 | 14 May 2015 |
| | | | | US | 2016257961 | A1 | 08 September 2016 |
| CN | 107109405 | A | 29 August 2017 | AU | 2001269053 | A1 | 13 June 2002 |
| | | | | AU | 2015269053 | A1 | 22 December 2016 |
| | | | | WO | 2015188197 | A2 | 10 December 2015 |
| | | | | US | 2020392498 | A1 | 17 December 2020 |
| | | | | JP | 2017522046 | A | 10 August 2017 |
| | | | | CA | 2950960 | A1 | 10 December 2015 |
| | | | | US | 2017114341 | A1 | 27 April 2017 |
| | | | | EP | 3152308 | A2 | 12 April 2017 |
| CN | 102006890 | A | 06 April 2011 | CA | 2708153 | A1 | 11 June 2009 |
| | | | | AU | 2008333811 | A1 | 11 June 2009 |
| | | | | EP | 3705125 | A1 | 09 September 2020 |
| | | | | EP | 3141265 | A1 | 15 March 2017 |
| | | | | JP | 2021080299 | A | 27 May 2021 |
| | | | | US | 2012136042 | A1 | 31 May 2012 |
| | | | | US | 2019184018 | A1 | 20 June 2019 |
| | | | | US | 2013178512 | A1 | 11 July 2013 |
| | | | | JP | 2011505426 | A | 24 February 2011 |
| | | | | CA | 3043911 | A1 | 02 July 2009 |
| | | | | AU | 2008340355 | A1 | 02 July 2009 |
| | | | | US | 2018326070 | A1 | 15 November 2018 |

**INTERNATIONAL SEARCH REPORT**
**Information on patent family members**

International application No.
**PCT/CN2022/074523**

| Patent document cited in search report | Publication date (day/month/year) | Patent family member(s) | Publication date (day/month/year) |
|---|---|---|---|
| | | US 2014179761 A1 | 26 June 2014 |
| | | EP 3156077 A1 | 19 April 2017 |
| | | US 2015119445 A1 | 30 April 2015 |
| | | CA 3146103 A1 | 11 June 2009 |
| | | WO 2009082607 A2 | 02 July 2009 |
| | | JP 2016034974 A | 17 March 2016 |
| | | AU 2014340355 A1 | 28 April 2016 |
| | | EP 2229186 A2 | 22 September 2010 |
| | | CA 2930393 A1 | 11 June 2009 |
| | | JP 2019023241 A | 14 February 2019 |
| | | CA 2708173 A1 | 02 July 2009 |
| | | US 2015119444 A1 | 30 April 2015 |
| | | CA 2910760 A1 | 02 July 2009 |
| | | EP 2231195 A2 | 29 September 2010 |
| | | US 2015011615 A1 | 08 January 2015 |
| | | JP 2019147846 A | 05 September 2019 |
| | | US 2009239814 A1 | 24 September 2009 |
| | | JP 2014139232 A | 31 July 2014 |
| | | JP 2011505425 A | 24 February 2011 |
| | | JP 2021020928 A | 18 February 2021 |
| | | WO 2009073809 A2 | 11 June 2009 |
| | | JP 2015025007 A | 05 February 2015 |
| | | US 2009247608 A1 | 01 October 2009 |
| | | US 2022008541 A1 | 13 January 2022 |
| | | US 2020297853 A1 | 24 September 2020 |
| | | US 2019099493 A1 | 04 April 2019 |
| | | JP 2017002082 A | 05 January 2017 |
| | | JP 2018029599 A | 01 March 2018 |
| | | US 2016051691 A1 | 25 February 2016 |
| CN 110520161 A | 29 November 2019 | WO 2018148650 A1 | 16 August 2018 |
| | | US 2019367550 A1 | 05 December 2019 |
| | | JP 2020507584 A | 12 March 2020 |
| | | EP 3595729 A1 | 22 January 2020 |
| | | KR 20190126314 A | 11 November 2019 |
| CN 103491982 A | 01 January 2014 | KR 20130136494 A | 12 December 2013 |
| | | NZ 611656 A | 31 October 2014 |
| | | WO 2012092373 A2 | 05 July 2012 |
| | | IN 4209DELNP2013 A | 24 October 2014 |
| | | CL 2013001876 A1 | 11 April 2014 |
| | | BR 112013014115 A2 | 24 September 2019 |
| | | CL 2015003580 A1 | 15 July 2016 |
| | | MX 334578 B | 03 November 2015 |
| | | MX 347298 B | 21 April 2017 |
| | | SG 189942 A1 | 28 June 2013 |
| | | ZA 201302896 B | 25 July 2014 |
| | | MX 2013007316 A | 29 July 2013 |
| | | EP 2658579 A2 | 06 November 2013 |
| | | AU 2011352204 A1 | 02 May 2013 |
| | | CA 2816041 A1 | 05 July 2012 |
| | | JP 2014505685 A | 06 March 2014 |

Form PCT/ISA/210 (patent family annex) (January 2015)

**INTERNATIONAL SEARCH REPORT**
**Information on patent family members**

International application No.
**PCT/CN2022/074523**

| Patent document cited in search report | | | Publication date (day/month/year) | Patent family member(s) | | | Publication date (day/month/year) |
|---|---|---|---|---|---|---|---|
| | | | | RU | 2013117286 | A | 10 March 2015 |
| | | | | PE | 20140198 | A1 | 21 February 2014 |
| US | 2013317080 | A1 | 28 November 2013 | CA | 2812046 | A1 | 22 March 2012 |
| | | | | AU | 2011302152 | A1 | 02 May 2013 |
| | | | | EP | 2616543 | A1 | 24 July 2013 |
| | | | | WO | 2012037254 | A1 | 22 March 2012 |
| | | | | JP | 2013541334 | A | 14 November 2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9005144 A, Winter
- US 5821337 A


### Non-patent literature cited in the description


- McGraw-Hill Dictionary of Chemical Terms. McGraw-Hill Book Company, 1984
- **ELIEL, E. ; WILEN, S.** Stereochemistry of Organic Compounds. John Wiley & Sons, Inc, 1994
- **JACQUES et al.** Enantiomers, Racemates and Resolutions. Wiley Interscience, 1981
- **ROBERT E. GAWLEY ; JEFFREY AUBÉ.** Principles of Asymmetric Synthesis. Elsevier, 2012
- **ELIEL, E.L.** Stereochemistry of Carbon Compounds. McGraw-Hill, 1962
- **WILEN, S.H.** Tables of Resolving Agents and Optical Resolutions. Univ. of Notre Dame Press, 1972, 268
- Chiral Separation Techniques: A Practical Approach. Wiley-VCH Verlag GmbH & Co. KGaA, 2007
- **T W. GREENE.** Protective Groups in Organic Synthesis. John Wiley&Sons, 1991
- **P. J. KOCIENSKI.** Protecting Groups. Thieme, 2005
- *J. Pharmaceutical Sciences,* 1977, vol. 66, 1-19
- **WARD et al.** *Nature,* 1989, vol. 341, 544-6
- **BIRD et al.** *Science,* 1988, vol. 242, 423-6
- **HUSTON et al.** *Proc. Natl. Acad. Sci. USA,* 1988, vol. 85, 5879-83
- **HE et al.** *J. Nucl. Med.,* 2010, vol. 51, 427-32
- **FITTING et al.** *MAbs,* 2015, vol. 7, 390-402
- **HOLLIGER et al.** *Proc. Natl. Acad. Sci. USA,* 1993, vol. 90, 6444-8
- **POLJAK et al.** *Structure,* 1994, vol. 2, 1121-3
- Periodic Table of the Elements, CAS Edition. Handbook of Chemistry and Physics. 1994
- **THOMAS SORRELL.** Organic Chemistry. University Science Books, 1999
- **MICHAEL B. SMITH ; JERRY MARCH.** March's Advanced Organic Chemistry. John Wiley & Sons, 2007